# EUROPÄISCHE PATENTSCHRIFT

(11) **EP 2 921 485 B1**
(45) Veröffentlichungstag und Bekanntmachung des Hinweises auf die Patenterteilung: **18.07.2018**
(21) Anmeldenummer: 15154130.7
(22) Anmeldetag: 21.12.2012
(51) Int. Cl.: C07D 261/08

(54) **ISOXAZOLDERIVATE**
ISOXAZOLE DERIVATIVES
DÉRIVÉS D'ISOXAZOLE

(30) Priorität: 27.12.2011 EP 11195764
(43) Veröffentlichungstag der Anmeldung: 23.09.2015
(62) Teilanmeldung aus: 12806489.6
(73) Patentinhaber: Bayer CropScience AG, 40789 Monheim (DE)
(72) Erfinder: Tsuchiya, Tomoki, 69009 Lyon (FR); Wasnaire, Pierre, 49225 Düsseldorf (DE); Hoffmann, Sebastian, 41470 Neuss (DE); Seitz, Thomas, 40764 Langenfeld (DE); Hillebrand, Stefan, 41462 Neuss (DE); Benting, Jürgen, 42799 Leichlingen (DE); Schmidt, Jan Peter, 95630 Folsom (US); Cristau Pierre, 69009 Lyon (FR)
(74) Vertreter: BIP Patents

(56) Entgegenhaltungen:
- US-A- 5 814 627
- JONATHAN D. TOKER ET AL: "Exploring the Scope of the 29G12 Antibody Catalyzed 1,3-Dipolar Cycloaddition Reaction", THE JOURNAL OF ORGANIC CHEMISTRY, Bd. 70, Nr. 20, 1. September 2005 (2005-09-01), Seiten 7810-7815, XP055195563, ISSN: 0022-3263, DOI: 10.1021/jo050410b
- ALAM ATHAR ET AL: "Synthesis and bio-evaluation of human macrophage migration inhibitory factor inhibitor to develop anti-inflammatory agent", BIOORGANIC & MEDICINAL CHEMISTRY, PERGAMON, GB, Bd. 19, Nr. 24, 24. Oktober 2011 (2011-10-24), Seiten 7365-7373, XP028597161, ISSN: 0968-0896, DOI: 10.1016/J.BMC.2011.10.056
- V. SINGH ET AL: "Interesting Results of Catalytic Hydrogenation of 3-(2-Nitrophenyl)isoxazoles and 3-(Nitrophenyl)-4,5-dihydroisoxazoles", SYNTHESIS, Bd. 2006, Nr. 12, 1. Juni 2006 (2006-06-01), Seiten 1995-2004, XP055195562, ISSN: 0039-7881, DOI: 10.1055/s-2006-942388
- ZAGOZDA ET AL: "Optically active nitrile oxides: synthesis and 1,3-dipolar cycloaddition reactions", TETRAHEDRON ASYMMETRY, PERGAMON PRESS LTD, OXFORD, GB, Bd. 18, Nr. 12, 4. Juli 2007 (2007-07-04), Seiten 1457-1464, XP022157444, ISSN: 0957-4166, DOI: 10.1016/J.TETASY.2007.05.021
- MOLINA C T ET AL: "SYNTHESIS OF NEW 4,5-DIHYDROISOXAZOLES WITH POTENTIAL ANTI-INFLAMMATORY ACTIVITY", HETEROCYCLIC COMMUNICATIONS, FREUND PUBLISHING HOUSE, TEL AVIV, IL, Bd. 9, Nr. 5, 1. Januar 2003 (2003-01-01), Seiten 535-538, XP009044208, ISSN: 0793-0283

## Beschreibung

Die Erfindung betrifft Verbindungen, die sich zur Herstellung von Heteroarylpiperidinderivatenund Mitteln zur Bekämpfung von pflanzenpathogenen Schadpilzen in und/oder auf Pflanzen oder in und/oder auf Saatgut von Pflanzen enthaltend diese Heteroarylpiperidinderivate eignen.

Die Synthese von Aryl-substituierten Isoxazolinen durch 1,3-dipolare Cycloaddition ist bereits bekannt (J. D. Toker et al., J. Org. Chem. 70 (2005), 7810-7815; A. Alam et al., Bioorg. Med. Chem. 19 (2011), 7365-7373).

Es ist außerdem bereits bekannt, dass bestimmte heterocyclisch substituierte Thiazole als fungizide Pflanzenschutzmittel benutzt werden können (siehe WO 07/014290, WO 08/013925, WO 08/013622, WO 08/091594, WO 08/091580, WO 09/055514, WO 09/094407, WO 09/094445, WO 09/132785, WO 10/037479, WO 10/065579, WO 11/076510, WO 11/018415, WO 11/018401, WO 11/076699, WO 11/146182, WO 12/055837, WO 12/025557, WO 12/082580). Die fungizide Wirksamkeit dieser Verbindungen ist jedoch gerade bei niedrigeren Aufwandmengen nicht immer ausreichend.

Da sich die ökologischen und ökonomischen Anforderungen an moderne Pflanzenschutzmittel laufend erhöhen, beispielsweise was Wirkspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Pflanzenschutzmittel, insbesondere Fungizide zu entwickeln, die zumindest in Teilbereichen Vorteile gegenüber den bekannten aufweisen.

Überraschenderweise wurde nun gefunden, dass die vorliegenden Heteroarylpiperidin und -piperazinderivate die genannten Aufgaben zumindest in Teilaspekten lösen und sich zur Herstelllung von Pflanzenschutzmitteln, insbesondere Fungiziden eignen.

Gegenstand der Erfindung sind Verbindungen der Formeln **(VIIIa)** und **(VIIIb),** 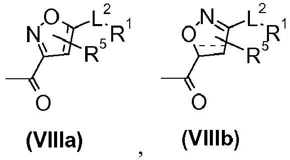 in welchen die Restedefinitionen folgende Bedeutungen haben:
- R⁵: steht für Wasserstoff,
- L²: steht für eine direkte Bindung,
- R¹: steht für Phenyl, das 1, 2 oder 3 Substituenten enthält, wobei die Substituenten unabhängig voneinander mindestens einmal aus Z⁴ und gegebenenfalls aus folgender Liste ausgewählt sind: Fluor, Chlor, Brom, Iod, Cyano, Nitro, Hydroxy, Amino, Methyl, Ethyl, *n*-Propyl, 1-Methylethyl, *n*-Butyl, 1,1-Dimethylethyl, 1,2-Dimethylethyl, Ethenyl, Ethinyl, Trifluormethyl, Difluormethyl, Trichlormethyl, Dichlormethyl, Cyclopropyl, Methoxy, Ethoxy, *n*-Propoxy, 1-Methylethoxy, 1,1-Dimethylethoxy, Methylcarbonyl, Ethylcarbonyl, Methoxycarbonyl, Ethoxycarbonyl, *n*-Propoxycarbonyl, 1-Methylethoxycarbonyl, 1,1-Dimethylethoxycarbonyl, 1-Methylcarbonyloxy, Methylthio, Ethylthio, Methylsulfonyl oder -L³R³,
- L³: steht für eine direkte Bindung,
- R³: steht für Wasserstoff, C₁-C₆-Alkyl, C₁-C₆-Halogenalkyl, C₃-C₈-Cycloalkyl, C₁-C₄-Alkylcarbonyl, C₁-C₄-Halogenalkylcarbonyl, C₁-C₄-Alkoxycarbonyl oder C₁-C₄-Halogenalkoxycarbonyl,
- Z³: steht für einen Phenylrest, der bis zu zwei Substituenten enthalten kann, wobei die Substituenten unabhängig voneinander ausgewählt sind aus folgender Liste:
Chlor, Brom, Iod, Fluor, Cyano, Nitro, Hydroxy, Amino, -SH, Methyl, Ethyl, *n*-Propyl, 1-Methylethyl, 1,1-Dimethylethyl, Ethenyl, Propen-2-yl, Ethinyl, Propin-2-yl, Trifluormethyl, Difluormethyl, Methoxymethyl, Methylcarbonyl, Ethylcarbonyl, Trifluormethylcarbonyl, Methoxycarbonyl, Ethoxycarbonyl, *n*-Propoxycarbonyl, 1-Methylethoxycarbonyl, 1,1-Dimethylethoxycarbonyl, Methoxy, Ethoxy, *n*-Propoxy, 1-Methylethoxy, 1,1-Dimethylethoxy, Trifluormethoxy, Ethenyloxy, 2-Propenyloxy, Ethinyloxy, 2-Propinyloxy, Methylthio, Ethylthio, Trifluormethylthio, Methylsulfonyl, Ethylsulfonyl, Propylthionyl, 1-Methylethylthio, Trifluormethylsulfonyl, Methylamino, Ethylamino, *n*-Propylamino, 1-Methylethylamino, 1,1-Dimethylethylamino oder Dimethylamino, oder
- Z³: steht für Naphthalenyl,
- R¹³ und R¹⁴: stehen gleich oder verschieden unabhängig voneinander für Wasserstoff, Methyl, Ethyl, *n-*Propyl, 1-Methylethyl, *n*-Butyl oder 1,1-Dimethylethyl,
- Z⁴: steht für -Formyl, Methoxymethoxy, 2-Methoxyethoxy, Allyloxy, 2-Fluorprop-2-en-1-yloxy, 2-Chlorprop-2-en-1-yloxy, 3-Chlorprop-2-en-1-yloxy, 2-Bromprop-2-en-1-yloxy, 2-Methylprop-2-en-1-yloxy, 3,3-Dichlorprop-2-en-1-yloxy, 3,3-Dichlor-2-fluorprop-2-en-1-yloxy, But-2-en-1-yloxy, But-3-en-2-yloxy, But-3-en-1-yloxy, 3-Chlorbut-2-en-1-yloxy 3-Methylbut-2-en-1-yloxy, 4,4,4-Trifluorbut-2-en-1-yloxy, Prop-2-in-1-yloxy, 3-Chlorprop-2-in-1-yloxy, 3-Bromprop-2-in-1-yloxy, But-2-in-1-yloxy, Pent-2-in-1-yloxy, 2-Fluor-2-methylpropanoyloxy, 3,3,3-Trifluorpropanoyloxy, Cyclopropylcarbonyloxy, Cyclohexylcarbonyloxy, (1-Chlorcyclopropyl)carbonyloxy, But-2-enoyloxy, Acryloyloxy, Cyanomethoxy, Methylsulfonyloxy, Ethylsulfonyloxy, Trifluormethylsulfonyloxy, Cyclopropylsulfonyloxy, 2-Methoxyethoxymethyl, Allyloxymethyl, Prop-2-in-1-yloxymethyl, Methylsulfonylmethyl, Methylcarbonylaminomethyl, Methylsulfonylaminomethyl, -C(=NOR⁷)R⁸, Dimethylaminosulfonyl, Ethylaminosulfonyl, Trimethylsilylethinyl, Diethylaminosulfonyl, Methylaminosulfonyl, Trimethylsilyloxy, Trimethylsilylprop-2-in-1-yloxy, Trifluormethylamino, Dimethylaminocarbonylamino, -C(=O)OH, -NHC(=O)H,-C(=O)NH₂, -C(=S)NR¹³R¹⁴ 1,1-Dimethylethylcarbonylamino, Chlormethylcarbonylamino, Trifluormethylcarbonylamino, 1,1-Dimethylethoxycarbonylamino, Ethylcarbonylamino, 1-Methylethoxycarbonylamino, Trifluormethylcarbonylamino, Methylcarbonylamino, Methoxycarbonylamino, Ethoxycarbonylamino, *iso*-Propoxycarbonylamino, 1-Methyl-ethylcarbonylamino, Methylsulfonylamino oder Phenylsulfonylamino, 3-Bromprop-2-en-1-yloxy, oder -L⁴Z³,
- L⁴: steht für -C(=O)O- oder -OCH₂C≡C-,
- R⁷: steht für Wasserstoff, Methyl, Ethyl, *n*-Propyl, 1-Methylethyl, *n*-Butyl, 1,1-Dimethylethyl oder 2-Methylpropyl,
- R⁸: steht für Wasserstoff, Methyl, Ethyl, *n*-Propyl, 1-Methylethyl, *n*-Butyl, 1,1-Dimethylethyl oder 2-Methylpropyl,
sowie Salze davon.

Die Verbindungen der Formeln **(VIIIa)** und **(VIIIb)** eignen sich zur Herstellung von Verbindungen der Formel **(I)**, 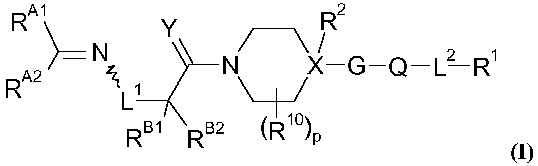 in welcher L² und R¹ wie in den Formeln **(VIIIa)** und **(VIIIb)** definiert sind, und wobei die verbleibenden Restedefinitionen folgende Bedeutungen haben:
- R^{A1}: steht für Wasserstoff, Halogen, Cyano, Amino, -CHO, -C(=O)OH, -C(=O)NH2, Alkyl, Alkenyl, Alkinyl, Halogenalkyl, Halogenalkenyl, Halogenalkinyl, Cycloalkyl, Halogencycloalkyl, Alkylcycloalkyl, Cycloalkylalkyl, Halogencycloalkylalkyl, Cycloalkenyl, Halogencycloalkenyl, Alkoxyalkyl, Alkylthioalkyl, Alkylsulfinylalkyl, Alkylsulfonylalkyl, Alkylaminoalkyl, Dialkylaminoalkyl, Halogenalkylaminoalkyl, Alkylcarbonyl, Halogenalkylcarbonyl, Cycloalkylcarbonyl, Alkoxycarbonyl, Cycloalkoxycarbonyl, Cycloalkylalkoxycarbonyl, Alkylaminocarbonyl, Dialkylaminocarbonyl, Alkoxy, Halogenalkoxy, Cycloalkoxy, Halogencycloalkoxy, Alkenyloxy, Halogenalkenyloxy, Alkinyloxy, Halogenalkinyloxy, Alkoxyalkoxy, Alkylcarbonyloxy, Halogenalkylcarbonyloxy, Alkylthio, Halogenalkylthio, Cycloalkylthio, Alkylamino, Dialkylamino, Halogenalkylamino, Halogendialkylamino, Cycloalkylamino, Alkylcarbonylamino, Halogenalkylcarbonylamino, Alkylsulfonylamino oder Halogenalkylsulfonylamino,
- R^{A2}: steht für Wasserstoff, Halogen, Cyano, Hydroxy, Alkyl, Halogenalkyl, Alkoxy, Halogenalkoxy, Alkylthio, oder
- R^{A2}: steht für ein unsubstituiertes oder substituiertes Phenyl, ein gegebenenfalls benzokondensiertes, substituiertes 5- oder 6-gliedriges Heterocyclyl, wobei die Substituenten unabhängig voneinander aus folgender Liste ausgewählt sind: Hydroxy, Cyano, Halogen, Alkyl, Halogenalkyl, Alkoxy, Halogenalkoxy, oder
- R^{A1} und R^{A2}: bilden zusammen mit dem Kohlenstoffatom, an das sie gebunden sind, einen drei- bis siebengliedrigen gesättigten oder partiell ungesättigten Ring, enthaltend gegebenenfalls, ein, zwei, drei oder vier Heteroatome aus der Gruppe Sauerstoff, Stickstoff, Silicium oder Schwefel, wobei gegebenenfalls, ein, zwei oder drei Kohlenstoffringglieder ausgewählt sind aus C(=O) und C(=S) ist und die Schwefelringglieder ausgewählt sind aus S(=O)ₛ(=NR^{A3})_{f}, und die Siliciumringglieder ausgewählt sind aus SiR^{A4}R^{A5}, wobei der Ring unsubstituiert oder substituiert sein kann, wobei die Substituenten unabhängig voneinander ausgewählt aus R^{A6} sind,
- R^{A3}: steht für Wasserstoff, Cyano, Alkyl, Halogenalkyl, Cycloalkyl, Halogencycloalkyl, Alkoxy, Halogenalkoxy, Alkylamino, Dialkylamino, Halogenalkylamino oder Phenyl,
- R^{A4} und R^{A5}: stehen gleich oder verschieden und unabhängig voneinander für Alkyl, Alkenyl, Alkinyl, Cycloalkyl, Halogencycloalkyl, Cycloalkylalkyl, Alkylcycloalkyl, Alkylcycloalkylalkyl, Halogenalkyl, Alkoxy oder Halogenalkoxy,
- s: für 0, 1 oder 2 steht, und
- f: für 0, 1 oder 2 steht, und
- s + f: für 0, 1 oder 2 steht,
- R^{A6}: für Halogen, Cyano, Alkyl, Halogenalkyl, Alkoxy oder Halogenalkoxy an den Kohlenstoffringgliedern und für Cyano, Alkyl oder Alkoxy an den Stickstoffringgliedern steht,
- L¹: steht für Sauerstoff, Schwefel, -N(R^{L1})-, -C(R^{L2})₂-, -OC(R^{L2})₂-, -SC(R^{L2})₂-, - N(R^{L1})-C(R^{L2})₂-, wobei die nach links weisende Bindung mit dem Stickstoffatom von Formel I verbunden ist und die nach rechts weisende Bindung mit dem Stickstoffatom von Formel I verbunden ist
- R^{L1}: steht für Wasserstoff, Cyano, Alkyl, Halogenalkyl, Alkoxyalkyl, Alkylthioalkyl, Alkylcarbonyl, Halogenalkylcarbonyl, Alkoxycarbonyl, Alkylaminocarbonyl, Dialkylaminocarbonyl, Alkylsulfonyl oder Halogenalkylsulfonyl, oder die beiden Reste R^{L1} und R^{A2} bilden zusammen mit dem Kohlenstoffatom, an das sie gebunden sind, einen fünf- bis siebengliedrigen partiell ungesättigten Ring, enthaltend gegebenenfalls ein, zwei oder drei Heteroatome aus der Gruppe Sauerstoff, Stickstoff oder Schwefel, wobei der Ring unsubstituiert oder substituiert sein kann, wobei die Substituenten unabhängig voneinander ausgewählt aus R^{A6} sind,
- R^{L2}: steht für Wasserstoff, Alkyl oder Halogenalkyl,
- R^{B1}: steht für Wasserstoff, Halogen, Cyano, Hydroxy, Formyl, Alkyl, Alkenyl, Alkinyl, Halogenalkyl, Halogenalkenyl, Halogenalkinyl, Alkoxyalkyl, Alkylthioalkyl, Alkylsulfinylalkyl, Alkylsulfonylalkyl, Alkylcarbonyl, Halogenalkylcarbonyl, Alkoxycarbonyl, Alkylaminocarbonyl, Dialkylaminocarbonyl, Alkoxy, Halogenalkoxy, Alkylthio, Halogenalkylthio, Alkylsulfinyl, Halogenalkylsulfinyl, Alkylsulfonyl, Halogenalkylsulfonyl, Alkylcarbonyloxy, Halogenalkylcarbonyloxy, Alkoxycarbonyloxy, Alkylaminocarbonyloxy, Dialkylaminocarbonyloxy, oder
- R^{B1}: steht für einen Phenylrest, Naphthalenylrest oder einen 5- oder 6-gliedrigen Heteroarylrest, der jeweils 0, 1, 2 oder 3 Substituenten enthalten kann, wobei die Substituenten unabhängig voneinander ausgewählt sind aus folgender Liste:
Wasserstoff, Halogen, Alkyl, Halogenalkyl, Cycloalkyl, Alkoxy, Halogenalkoxy, Alkyl-carbonyloxy, Halogenalkylcarbonyloxy,
- R^{B2}: steht für Wasserstoff, Alkyl oder Halogenalkyl,
oder die beiden Reste R^{B1} und R^{B2} bilden zusammen mit dem Kohlenstoffatom, an das sie gebunden sind, einen drei- bis sechsgliedrigen gesättigten Ring,
- Y: steht für Schwefel oder Sauerstoff,
- X: steht für Kohlenstoff oder Stickstoff,
- R²: steht für Wasserstoff, Alkyl, Alkenyl, Halogenalkyl, Alkoxy, Halogen, Cyano oder Hydroxy,
- R¹⁰: steht gleich oder verschieden und unabhängig voneinander für Wasserstoff, Alkyl, Alkenyl, Halogenalkyl, Alkoxy, Halogen, Cyano oder Hydroxy,
- p: steht für 0, 1 oder 2,
- G: steht für 5-gliedriges Heteroaryl, das mit Q substituiert ist und ansonsten unsubstituiert oder substituiert sein kann,
- Q: steht für Q¹¹ = 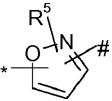 oder Q²⁴ = 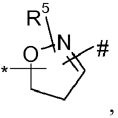 wobei die Bindung, die mit "*" identifiziert ist, direkt an G oder L² gebunden ist, und wobei die Bindung, die mit "#" identifiziert ist, direkt an L² oder G gebunden gebunden ist oder wobei die Bindung, die mit "*" identifiziert ist, direkt an L² gebunden ist, und gleichzeitig die Bindung, die mit "#" identifiziert ist, direkt an G gebunden ist,
- R⁵: steht für Wasserstoff,
sowie Salzen der Verbindungen der Formel **(I).**

Heteroarylpiperidin und -piperazinderivate der Formel **(I)** sowie deren Salze, Metallkomplexe und N-Oxide eignen sich sehr gut als zur Bekämpfung pflanzenpathogener Schadpilze. Die vorgenannten Verbindungen zeigen vor allem eine starke fungizide Wirksamkeit und lassen sich sowohl im Pflanzenschutz, im Bereich Haushalt und Hygiene als auch im Materialschutz verwenden.

Die Verbindungen der Formel **(I)** können sowohl in reiner Form als auch als Mischungen verschiedener möglicher isomerer Formen, insbesondere von Stereoisomeren, wie E- und Z-, threo- und erythro-, sowie optischen Isomeren, wie R- und S-Isomeren oder Atropisomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die Restedefinitionen der Verbindungen der Formel **(I)** haben bevorzugt, besonders bevorzugt und ganz besonders bevorzugt folgende Bedeutungen:
- R^{A1}: steht bevorzugt für Wasserstoff, Cyano, C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₂-C₄-Alkoxyalkyl, C₂-C₄-Alkylthioalkyl, C₁-C₃-Alkylcarbonyl, C₁-C₃-Halogenalkylcarbonyl, C₁-C₃-Alkoxycarbonyl, C₁-C₄-Alkoxy, C₁-C₄-Halogenalkoxy, C₂-C₄-Alkenyloxy, C₂-C₄-Halogenalkenyloxy, C₂-C₄-Alkinyloxy, C₂-C₄-Halogenalkinyloxy, C₂-C₄-Alkoxyalkoxy, C₁-C₄-Alkylthio, C₁-C₄-Halogenalkylthio, C₁-C₄-Alkylamino, C₂-C₄-Dialkylamino, C₁-C₄-Halogenalkylamino, C₂-C₄-Halogendialkylamino, C₃-C₆-Cycloalkyl und besonders bevorzugt für Wasserstoff, Methyl, Ethyl, Propan-2-yl, *t*-Butyl, Difluormethyl, Trifluormethyl, Methoxymethyl, Ethoxymethyl, Methoxycarbonyl, Ethoxycarbonyl oder für Cyclopropyl,
- R^{A2}: steht bevorzugt für Wasserstoff, C₁-C₃-Alkyl, C₁-C₃-Halogenalkyl, C₁-C₃-Alkoxy, oder
- R^{A2}: steht bevorzugt für ein unsubstituiertes oder substituiertes Phenyl, ein gegebenenfalls benzokondensiertes, substituiertes 5- oder 6-gliedriges Heterocyclyl, wobei die Substituenten unabhängig voneinander aus folgender Liste ausgewählt sind: Hydroxy, Cyano, Halogen, C₁-C₄-Alkyl, C₁-C₄-Halogenalkyl, C₁-C₄-Alkoxy, C₁-C₄-Halogenalkoxy,
- R^{A2}: steht besonders bevorzugt für Methyl, Ethyl, Propyl, iso-Propyl, Butyl, *tert-Butyl, iso*-Butyl, 1,3-Benzodioxolyl oder ein unsubstituiertes oder substituiertes Phenyl wobei die Substituenten unabhängig voneinander aus folgender Liste ausgewählt sind: Hydroxy, Cyano, Fluor, Chlor, Brom, Methyl, Difluormethyl, Trifluormethyl, Methoxy, Ethoxy, Difluormethoxy, Trifluormethoxy,
- R^{A1} und R^{A2}: bilden bevorzugt zusammen mit dem Kohlenstoffatom, an das sie gebunden sind, einen dreibis sechsgliedrigen gesättigten oder partiell ungesättigten Ring, enthaltend gegebenenfalls ein, zwei, drei oder vier Heteroatome aus der Gruppe Sauerstoff, Stickstoff oder Schwefel, wobei gegebenenfalls oder ein Kohlenstoffringglied ausgewählt aus C(=O) und C(=S) ist, wobei der Ring kein, ein, zwei oder drei Substituenten enthalten kann, wobei die Substituenten unabhängig voneinander ausgewählt aus R^{A6} sind,
- R^{A6}: steht bevorzugt für Halogen, Cyano, C₁-C₂-Alkyl, C₁-C₂-Halogenalkyl, C₁-C₂-Alkoxy oder C₁-C₂-Halogenalkoxy an den Kohlenstoffringgliedern und für Cyano, C₁-C₂-Alkyl oder C₁-C₂-Alkoxy an den Stickstoffringgliedern,
- L¹: steht bevorzugt für Sauerstoff, Schwefel, -N(R^{L1})-,
- R^{L1}: steht bevorzugt für Wasserstoff, C₁-C₂-Alkyl, C₁-C₂-Halogenalkyl, -C(=O)CH₃, -C(=O)CF₃, C(=O)OCH₃, oder die beiden Reste R^{L1} und R^{A2} bilden zusammen mit dem Kohlenstoffatom, an das sie gebunden sind, einen fünf- bis siebengliedrigen partiell ungesättigten Ring, enthaltend gegebenenfalls ein, zwei oder drei Heteroatome aus der Gruppe Sauerstoff, Stickstoff oder Schwefel, wobei der Ring unsubstituiert oder substituiert sein kann, wobei die Substituenten unabhängig voneinander ausgewählt aus R^{A6} sind,
- R^{B1}: steht bevorzugt für Wasserstoff, Cyano, Hydroxy, C₁-C₃-Alkyl, C₂-C₃-Alkenyl, C₂-C₃-Alkinyl, C₁-C₃-Halogenalkyl, C₂-C₃-Halogenalkenyl, C₂-C₃-Halogenalkinyl, C₂-C₃-Alkylcarbonyl, C₂-C₃-Halogenalkylcarbonyl, C₁-C₃-Alkoxy, C₁-C₃-Halogenalkoxy, C₁-C₃-Alkylthio, C₁-C₃-Halogenalkylthio, C₁-C₂-Alkylcarbonyloxy, C₁-C₂-Halogenalkylcarbonyloxy, oder
- R^{B1}: steht bevorzugt für einen Phenylrest, Naphthalenylrest oder einen 5- oder 6-gliedrigen Heteroarylrest, der jeweils 0, 1, 2 oder 3 Substituenten enthalten kann, wobei die Substituenten unabhängig voneinander ausgewählt sind aus folgender Liste: Wasserstoff, Fluor, Chlor, Brom, C₁-C₃-Alkyl, C₁-C₃-Halogenalkyl, C₁-C₃-Alkoxy, C₁-C₃-Halogenalkoxy,
- R^{B2}: steht bevorzugt für Wasserstoff oder C₁-C₂-Alkyl, und besonders bevorzugt für Wasserstoff,
- Y: steht bevorzugt für Schwefel oder Sauerstoff, und besonders bevorzugt für Sauerstoff,
- X: steht bevorzugt für Kohlenstoff oder Stickstoff, und besonders bevorzugt für Kohlenstoff,
- R²: steht bevorzugt für Wasserstoff, C₁-C₄-Alkyl, C₁-C₂-Halogenalkyl, C₁-C₂-Alkoxy, Halogen, Cyano oder Hydroxy, und besonders bevorzugt für Wasserstoff, Fluor, Methoxy oder Hydroxy, und ganz besonders bevorzugt für Wasserstoff,
- R¹⁰: steht bevorzugt gleich oder verschieden und unabhängig voneinander für Wasserstoff, C₁-C₄-Alkyl, C₁-C₂-Halogenalkyl, C₁-C₂-Alkoxy, Halogen, Cyano oder Hydroxy, und besonders bevorzugt für Wasserstoff, Fluor, Methoxy oder Hydroxy,
- p: steht bevorzugt für 0 bis 1, und besonders bevorzugt für 0,
- G: steht bevorzugt für 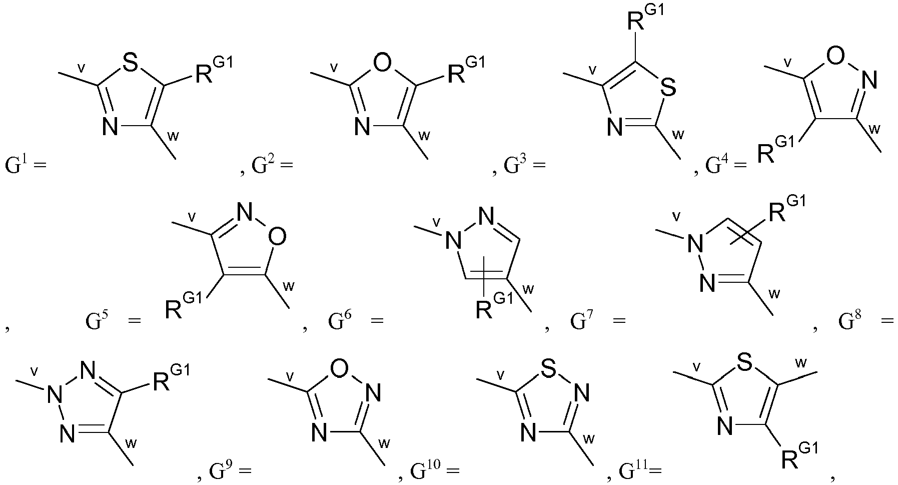 wobei die Bindung, die mit "v" identifiziert ist, direkt an X gebunden ist, und wobei die Bindung, die mit "w" identifiziert ist, direkt an Q gebunden ist,
- G: steht besonders bevorzugt für G¹, G² oder G³, und ganz besonders bevorzugt für G¹,
- R^{G1}: steht bevorzugt für Wasserstoff, C₁-C₃-Alkyl oder Halogen und besonders bevorzugt für Wasserstoff,
- Q: steht besonders bevorzugt für 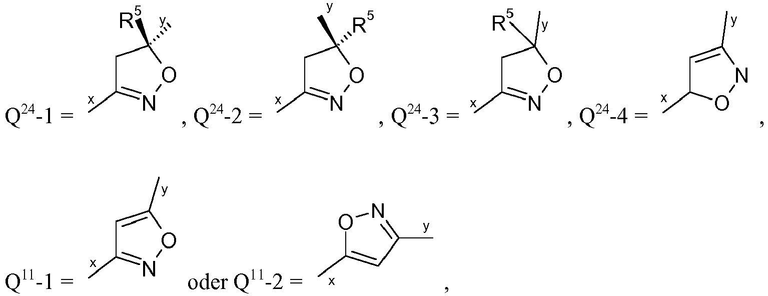 wobei die Bindung, die mit "x" identifiziert ist, direkt an G gebunden ist, und wobei die Bindung, die mit "y" identifiziert ist, direkt an L² gebunden ist,
- R⁵: steht für Wasserstoff,
- R³: steht für Wasserstoff, C₁-C₆-Alkyl, C₁-C₆-Halogenalkyl, C₃-C₈-Cycloalkyl, C₁-C₄-Alkylcarbonyl, C₁-C₄-Halogenalkylcarbonyl, C₁-C₄-Alkoxycarbonyl oder C₁-C₄-Halogenalkoxycarbonyl,
- L²: steht für eine direkte Bindung,
- R¹: steht für Phenyl, das 1, 2 oder 3 Substituenten enthält, wobei die Substituenten unabhängig voneinander mindestens einmal aus Z⁴ und gegebenenfalls aus folgender Liste ausgewählt sind: Fluor, Chlor, Brom, Iod, Cyano, Nitro, Hydroxy, Amino, Methyl, Ethyl, *n*-Propyl, 1-Methylethyl, *n*-Butyl, 1,1-Dimethylethyl, 1,2-Dimethylethyl, Ethenyl, Ethinyl, Trifluormethyl, Difluormethyl, Trichlormethyl, Dichlormethyl, Cyclopropyl, Methoxy, Ethoxy, *n*-Propoxy, 1-Methylethoxy, 1,1-Dimethylethoxy, Methylcarbonyl, Ethylcarbonyl, Methoxycarbonyl, Ethoxycarbonyl, *n*-Propoxycarbonyl, 1-Methylethoxycarbonyl, 1,1-Dimethylethoxycarbonyl, 1-Methylcarbonyloxy, Methylthio, Ethylthio, Methylsulfonyl oder -L³R³, sowie ganz besonders bevorzugt für Phenyl steht, das 1, 2 oder 3 Substituenten enthält, wobei die Substituenten unabhängig voneinander aus folgender Liste ausgewählt sind:
Formyl, Methoxymethoxy, 2-Methoxyethoxy, Allyloxy, 2-Fluorprop-2-en-1-yloxy, 2-Chlorprop-2-en-1-yloxy, 3-Chlorprop-2-en-1-yloxy, 2-Bromprop-2-en-1-yloxy, 2-Methylprop-2-en-1-yloxy, 3,3-Dichlorprop-2-en-1-yloxy, 3,3-Dichlor-2-fluorprop-2-en-1-yloxy, But-2-en-1-yloxy, But-3-en-2-yloxy, But-3-en-1-yloxy, 3-Chlorbut-2-en-1-yloxy 3-Methylbut-2-en-1-yloxy, 4,4,4-Trifluorbut-2-en-1-yloxy, Prop-2-in-1-yloxy, 3-Chlorprop-2-in-1-yloxy, 3-Bromprop-2-in-1-yloxy, But-2-in-1-yloxy, Pent-2-in-1-yloxy, 2-Fluor-2-methylpropanoyloxy, 3,3,3-Trifluorpropanoyloxy, Cyclopropylcarbonyloxy, Cyclohexylcarbonyloxy, (1-Chlorcyclopropyl)carbonyloxy, But-2-enoyloxy, Acryloyloxy, Benzoyloxy, 2-Fluorbenzoyloxy, 3-Fluorbenzoyloxy, 4-Fluorbenzoyloxy, Cyanomethoxy, Methylsulfonyloxy, Ethylsulfonyloxy, Trifluormethylsulfonyloxy, Cyclopropylsulfonyloxy, 2-Methoxyethoxymethyl, Allyloxymethyl, Prop-2-in-1-yloxymethyl, Methylsulfonylmethyl, Methylcarbonylaminomethyl, Methylsulfonylaminomethyl, -C(=NOH)H, -C(=NOCH₃)H, -C(=NOCH₂CH₃)H, - C(=NOCH(CH₃)CH₃)H, -C(=NOH)CH₃, -C(=NOCH₃)CH₃, -C(=NOCH₂CH₃)CH₃, - C(=NOCH(CH₃)CH₃)CH₃, Dimethylaminosulfonyl, C(=O)NH₂, Ethylaminosulfonyl, Trimethylsilylethinyl, Diethylaminosulfonyl, Methylaminosulfonyl, Trimethylsilyloxy, Trimethylsilylprop-2-in-1-yloxy, Trifluormethylamino, Dimethylaminocarbonylamino, - C(=O)OH, 1,1-Dimethylethylcarbonylamino, Chlormethylcarbonylamino, Trifluormethylcarbonylamino, 1,1-Dimethylethoxycarbonylamino, Ethylcarbonylamino, 1-Methylethoxycarbonylamino, Trifluormethylcarbonylamino, Methylcarbonylamino, Methoxycarbonylamino, Ethoxycarbonylamino, *iso*-Propoxycarbonylamino, 1-Methylethylcarbonylamino, Methylsulfonylamino oder Phenylsulfonylamino, 3-Bromprop-2-en-1-yloxy,
und zusätzliche Substituenten gegebenenfalls aus folgender Liste ausgewählt sind: Fluor, Chlor, Methyl, Trifluormethyl, Methoxy,
- L³: steht für eine direke Bindung,
- Z³: steht für einen Phenylrest, der bis zu zwei Substituenten enthalten kann, wobei die Substituenten unabhängig voneinander ausgewählt sind aus folgender Liste:
Chlor, Brom, Iod, Fluor, Cyano, Nitro, Hydroxy, Amino, -SH, Methyl, Ethyl, *n*-Propyl, 1-Methylethyl, 1,1-Dimethylethyl, Ethenyl, Propen-2-yl, Ethinyl, Propin-2-yl, Trifluormethyl, Difluormethyl, Methoxymethyl, Methylcarbonyl, Ethylcarbonyl, Trifluormethylcarbonyl, Methoxycarbonyl, Ethoxycarbonyl, *n*-Propoxycarbonyl, 1-Methylethoxycarbonyl, 1,1-Dimethylethoxycarbonyl, Methoxy, Ethoxy, *n*-Propoxy, 1-Methylethoxy, 1,1-Dimethylethoxy, Trifluormethoxy, Ethenyloxy, 2-Propenyloxy, Ethinyloxy, 2-Propinyloxy, Methylthio, Ethylthio, Trifluormethylthio, Methylsulfonyl, Ethylsulfonyl, Propylthionyl, 1-Methylethylthio, Trifluormethylsulfonyl, Methylamino, Ethylamino, *n*-Propylamino, 1-Methylethylamino, 1,1-Dimethylethylamino oder Dimethylamino, oder
- Z³: steht für Naphthalenyl,
- R¹³ und R¹⁴: stehen gleich oder verschieden unabhängig voneinander für Wasserstoff, Methyl, Ethyl, *n*-Propyl, 1-Methylethyl, *n*-Butyl oder 1,1-Dimethylethyl,
- Z⁴: steht für - Formyl, Methoxymethoxy, 2-Methoxyethoxy, Allyloxy, 2-Fluorprop-2-en-1-yloxy, 2-Chlorprop-2-en-1-yloxy, 3-Chlorprop-2-en-1-yloxy, 2-Bromprop-2-en-1-yloxy, 2-Methylprop-2-en-1-yloxy, 3,3-Dichlorprop-2-en-1-yloxy, 3,3-Dichlor-2-fluorprop-2-en-1-yloxy, But-2-en-1-yloxy, But-3-en-2-yloxy, But-3-en-1-yloxy, 3-Chlorbut-2-en-1-yloxy 3-Methylbut-2-en-1-yloxy, 4,4,4-Trifluorbut-2-en-1-yloxy, Prop-2-in-1-yloxy, 3-Chlorprop-2-in-1-yloxy, 3-Bromprop-2-in-1-yloxy, But-2-in-1-yloxy, Pent-2-in-1-yloxy, 2-Fluor-2-methylpropanoyloxy, 3,3,3-Trifluorpropanoyloxy, Cyclopropylcarbonyloxy, Cyclohexylcarbonyloxy, (1-Chlorcyclopropyl)carbonyloxy, But-2-enoyloxy, Acryloyloxy, Cyanomethoxy, Methylsulfonyloxy, Ethylsulfonyloxy, Trifluormethylsulfonyloxy, Cyclopropylsulfonyloxy, 2-Methoxyethoxymethyl, Allyloxymethyl, Prop-2-in-1-yloxymethyl, Methylsulfonylmethyl, Methylcarbonylaminomethyl, Methylsulfonylaminomethyl, -C(=NOR⁷)R⁸, Dimethylaminosulfonyl, Ethylaminosulfonyl, Trimethylsilylethinyl, Diethylaminosulfonyl, Methylaminosulfonyl, Trimethylsilyloxy, Trimethylsilylprop-2-in-1-yloxy, Trifluormethylamino, Dimethylaminocarbonylamino, -C(=O)OH, - NHC(=O)H, -C(=O)NH₂, -C(=S)NR¹³R¹⁴ 1,1-Dimethylethylcarbonylamino, Chlormethylcarbonylamino, Trifluormethylcarbonylamino, 1,1-Dimethylethoxycarbonylamino, Ethylcarbonylamino, 1-Methylethoxycarbonylamino, Trifluormethylcarbonylamino, Methylcarbonylamino, Methoxycarbonylamino, Ethoxycarbonylamino, *iso*-Propoxycarbonylamino, 1-Methylethylcarbonylamino, Methylsulfonylamino oder Phenylsulfonylamino, 3-Bromprop-2-en-1-yloxy, oder -L⁴Z³,
- L⁴: steht für -OCH₂C≡C- oder -C(=O)O-,
- R⁷: steht für Wasserstoff, Methyl, Ethyl, *n*-Propyl, 1-Methylethyl, *n*-Butyl, 1,1-Dimethylethyl oder 2-Methylpropyl,
R⁸ steht für Wasserstoff, Methyl, Ethyl, *n*-Propyl, 1-Methylethyl, *n*-Butyl, 1,1-Dimethylethyl oder 2-Methylpropyl.Die Restedefinitionen der vorstehenden und nachfolgend genannten Restedefinitionen der Formel **(I)** gelten für die Endprodukte der Formel **(I)** wie für alle Zwischenprodukte (siehe auch unten unter "Erläuterungen der Verfahren und Zwischenprodukte") gleichermaßen.

Die oben aufgeführten bzw. nachfolgenden allgemeinen oder in Vorzugsbereichen aufgeführten Reste-definitionen bzw. Erläuterungen können auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können einzelne Definitionen entfallen.

Bevorzugt sind solche Verbindungen der Formel **(I),** in welcher alle Reste jeweils die oben genannten bevorzugten Bedeutungen haben.

Besonders bevorzugt sind solche Verbindungen der Formel **(I),** in welcher alle Reste jeweils die oben genannten besonders bevorzugten Bedeutungen haben.

Ganz besonders bevorzugt sind solche Verbindungen der Formel **(I),** in welcher alle Reste jeweils die oben genannten ganz besonders bevorzugten Bedeutungen haben.

Weiterhin bevorzugt sind Verbindungen der Formel **(I)** sowie agrochemisch wirksame Salze, Metallkomplexe und N-Oxide davon in welcher:
R^{A1} für Methyl, Trifluormethyl oder für Cyclopropyl steht,
R^{A2} für Methyl oder Propan-2-yl steht,
R^{A2} für 1,3-Benzodioxol-5-yl, 4-Ethoxyphenyl, 3-Fluorphenyl, 3,4-Dimethylphenyl, 3-(Trifluormethoxy)-phenyl, 3,4-Dimethylphenyl oder 4-Ethoxyphenyl steht,
L¹ für Sauerstoff steht,
R^{B1} und R^{B2} für Wasserstoff stehen,
Y für Sauerstoff steht;
G für G¹ steht;
R^{G1} für Wasserstoff steht;
Q für Q²⁴-3 steht oder Q für Q¹¹-1 steht;
R⁵ für Wasserstoff steht oder R⁵ für Methyl steht;
L² für eine direkte Bindung steht;
R¹ für 2,3-Dichlor-4-(prop-2-in-1-yloxy)phenyl steht oder
R¹ für 2,3-Dichlor-4-[(methylsulfonyl)oxy]phenyl steht oder
R¹ für 2,3-Difluor-4-(prop-2-in-1-yloxy)phenyl steht oder
R¹ für 2,3-Difluor-4-[(methylsulfonyl)oxy]phenyl steht oder
R¹ für 2,3-Difluor-4-formylphenyl steht oder
R¹ für 2,4-Dichlor-3-(prop-2-in-1-yloxy)phenyl steht oder
R¹ für 2,4-Dichlor-3-[(methylsulfonyl)oxy]phenyl steht oder
R¹ für 2,4-Difluor-3-(prop-2-in-1-yloxy)phenyl steht oder
R¹ für 2,4-Difluor-3-[(methylsulfonyl)oxy]phenyl steht oder
R¹ für 2,4-Difluor-3-formylphenyl steht oder
R¹ für 2,5-Dichlor-3-(prop-2-in-1-yloxy)phenyl steht oder
R¹ für 2,5-Dichlor-3-[(methylsulfonyl)oxy]phenyl steht oder
R¹ für 2,5-Dichlor-4-(prop-2-in-1-yloxy)phenyl steht oder
R¹ für 2,5-Dichlor-4-[(methylsulfonyl)oxy]phenyl steht oder
R¹ für 2,5-Difluor-3-(prop-2-in-1-yloxy)phenyl steht oder
R¹ für 2,5-Difluor-3-[(methylsulfonyl)oxy]phenyl steht oder
R¹ für 2,5-Difluor-3-formylphenyl steht oder
R¹ für 2,5-Difluor-4-(prop-2-in-1-yloxy)phenyl steht oder
R¹ für 2,5-Difluor-4-[(methylsulfonyl)oxy]phenyl steht oder
R¹ für 2,5-Difluor-4-formylphenyl steht oder
R¹ für 2,6-Dichlor-3-(prop-2-in-1-yloxy)phenyl steht oder
R¹ für 2,6-Dichlor-3-[(methylsulfonyl)oxy]phenyl steht oder
R¹ für 2,6-Dichlor-4-(prop-2-in-1-yloxy)phenyl steht oder
R¹ für 2,6-Dichlor-4-[(methylsulfonyl)oxy]phenyl steht oder
R¹ für 2,6-Difluor-3-(prop-2-in-1-yloxy)phenyl steht oder
R¹ für 2,6-Difluor-3-[(methylsulfonyl)oxy]phenyl steht oder
R¹ für 2,6-Difluor-3-formylphenyl steht oder
R¹ für 2,6-Difluor-4-(prop-2-in-1-yloxy)phenyl steht oder
R¹ für 2,6-Difluor-4-[(methylsulfonyl)oxy]phenyl steht oder
R¹ für 2,6-Difluor-4-formylphenyl steht oder
R¹ für 2-(Allyloxy)-3,4-dichlorphenyl steht oder
R¹ für 2-(Allyloxy)-3,4-difluorphenyl steht oder
R¹ für 2-(Allyloxy)-3,5-dichlorphenyl steht oder
R¹ für 2-(Allyloxy)-3,5-difluorphenyl steht oder
R¹ für 2-(Allyloxy)-3,6-dichlorphenyl steht oder
R¹ für 2-(Allyloxy)-3,6-difluorphenyl steht oder
R¹ für 2-(Allyloxy)-3-chlorphenyl steht oder
R¹ für 2-(Allyloxy)-3-fluorphenyl steht oder
R¹ für 2-(Allyloxy)-3-methylphenyl steht oder
R¹ für 2-(Allyloxy)-4,5-dichlorphenyl steht oder
R¹ für 2-(Allyloxy)-4,5-difluorphenyl steht oder
R¹ für 2-(Allyloxy)-4,6-dichlorphenyl steht oder
R¹ für 2-(Allyloxy)-4,6-difluorphenyl steht oder
R¹ für 2-(Allyloxy)-4-chlorphenyl steht oder
R¹ für 2-(Allyloxy)-4-fluorphenyl steht oder
R¹ für 2-(Allyloxy)-4-methylphenyl steht oder
R¹ für 2-(Allyloxy)-5,6-dichlorphenyl steht oder
R¹ für 2-(Allyloxy)-5,6-difluorphenyl steht oder
R¹ für 2-(Allyloxy)-5-chlorphenyl steht oder
R¹ für 2-(Allyloxy)-5-fluorphenyl steht oder
R¹ für 2-(Allyloxy)-5-methylphenyl steht oder
R¹ für 2-(Allyloxy)-6-chlorphenyl steht oder
R¹ für 2-(Allyloxy)-6-fluorphenyl steht oder
R¹ für 2-(Allyloxy)-6-methylphenyl steht oder
R¹ für 2-(Allyloxy)phenyl steht oder
R¹ für 2-(Cyanomethoxy)-3,4-dichlorphenyl steht oder
R¹ für 2-(Cyanomethoxy)-3,4-difluorphenyl steht oder
R¹ für 2-(Cyanomethoxy)-3,5-dichlorphenyl steht oder
R¹ für 2-(Cyanomethoxy)-3,5-difluorphenyl steht oder
R¹ für 2-(Cyanomethoxy)-3,6-dichlorphenyl steht oder
R¹ für 2-(Cyanomethoxy)-3,6-difluorphenyl steht oder
R¹ für 2-(Cyanomethoxy)-3-chlorphenyl steht oder
R¹ für 2-(Cyanomethoxy)-3-fluorphenyl steht oder
R¹ für 2-(Cyanomethoxy)-3-methylphenyl steht oder
R¹ für 2-(Cyanomethoxy)-4,5-dichlorphenyl steht oder
R¹ für 2-(Cyanomethoxy)-4,5-difluorphenyl steht oder
R¹ für 2-(Cyanomethoxy)-4,6-dichlorphenyl steht oder
R¹ für 2-(Cyanomethoxy)-4,6-difluorphenyl steht oder
R¹ für 2-(Cyanomethoxy)-4-chlorphenyl steht oder
R¹ für 2-(Cyanomethoxy)-4-fluorphenyl steht oder
R¹ für 2-(Cyanomethoxy)-4-methylphenyl steht oder
R¹ für 2-(Cyanomethoxy)-5,6-dichlorphenyl steht oder
R¹ für 2-(Cyanomethoxy)-5,6-difluorphenyl steht oder
R¹ für 2-(Cyanomethoxy)-5-chlorphenyl steht oder
R¹ für 2-(Cyanomethoxy)-5-fluorphenyl steht oder
R¹ für 2-(Cyanomethoxy)-5-methylphenyl steht oder
R¹ für 2-(Cyanomethoxy)-6-chlorphenyl steht oder
R¹ für 2-(Cyanomethoxy)-6-fluorphenyl steht oder
R¹ für 2-(Cyanomethoxy)-6-methylphenyl steht oder
R¹ für 2-(Cyanomethoxy)phenyl steht oder
R¹ für 2-(Prop-2-in-1-yloxy)phenyl steht oder
R¹ für 2-(Prop-2-in-1-yloxy)-4-(trifluormethyl)phenyl steht oder
R¹ für 2-Chlor-3-(prop-2-in-1-yloxy)phenyl steht oder
R¹ für 2-Chlor-3-[(methylsulfonyl)oxy]phenyl steht oder
R¹ für 2-Chlor-3-formylphenyl steht oder
R¹ für 2-Chlor-4-(prop-2-in-1-yloxy)phenyl steht oder
R¹ für 2-Chlor-4-[(methylsulfonyl)oxy]phenyl steht oder
R¹ für 2-Chlor-4-formylphenyl steht oder
R¹ für 2-Fluor-3-(prop-2-in-1-yloxy)phenyl steht oder
R¹ für 2-Fluor-3-[(methylsulfonyl)oxy]phenyl steht oder
R¹ für 2-Fluor-3-formylphenyl steht oder
R¹ für 2-Fluor-4-(prop-2-in-1-yloxy)phenyl steht oder
R¹ für 2-Fluor-4-[(methylsulfonyl)oxy]phenyl steht oder
R¹ für 2-Fluor-4-formylphenyl steht oder
R¹ für 2-Formyl-3-methylphenyl steht oder
R¹ für 2-Formyl-4-methylphenyl steht oder
R¹ für 2-Formyl-5-methylphenyl steht oder
R¹ für 2-Formyl-6-methylphenyl steht oder
R¹ für 2-Formylphenyl steht oder
R¹ für 2-Methyl-3-(prop-2-in-1-yloxy)phenyl steht oder
R¹ für 2-Methyl-3-[(methylsulfonyl)oxy]phenyl steht oder
R¹ für 2-Methyl-4-(prop-2-in-1-yloxy)phenyl steht oder
R¹ für 2-Methyl-4-[(methylsulfonyl)oxy]phenyl steht oder
R¹ für 2-[(Hydroxyimino)methyl]-3,4-difluorphenyl steht oder
R¹ für 2-[(Hydroxyimino)methyl]-3,5-difluorphenyl steht oder
R¹ für 2-[(Hydroxyimino)methyl]-3,6-difluorphenyl steht oder
R¹ für 2-[(Hydroxyimino)methyl]-3-chlorphenyl steht oder
R¹ für 2-[(Hydroxyimino)methyl]-3-fluorphenyl steht oder
R¹ für 2-[(Hydroxyimino)methyl]-3-methylphenyl steht oder
R¹ für 2-[(Hydroxyimino)methyl]-4,5-difluorphenyl steht oder
R¹ für 2-[(Hydroxyimino)methyl]-4,6-difluorphenyl steht oder
R¹ für 2-[(Hydroxyimino)methyl]-4-chlorphenyl steht oder
R¹ für 2-[(Hydroxyimino)methyl]-4-fluorphenyl steht oder
R¹ für 2-[(Hydroxyimino)methyl]-4-methylphenyl steht oder
R¹ für 2-[(Hydroxyimino)methyl]-5,6-difluorphenyl steht oder
R¹ für 2-[(Hydroxyimino)methyl]-5-chlorphenyl steht oder
R¹ für 2-[(Hydroxyimino)methyl]-5-fluorphenyl steht oder
R¹ für 2-[(Hydroxyimino)methyl]-5-methylphenyl steht oder
R¹ für 2-[(Hydroxyimino)methyl]-6-chlorphenyl steht oder
R¹ für 2-[(Hydroxyimino)methyl]-6-fluorphenyl steht oder
R¹ für 2-[(Hydroxyimino)methyl]-6-methylphenyl steht oder
R¹ für 2-[(Hydroxyimino)methyl]phenyl steht oder
R¹ für 2-[(Methoxyimino)methyl]-3,4-difluorphenyl steht oder
R¹ für 2-[(Methoxyimino)methyl]-3,5-difluorphenyl steht oder
R¹ für 2-[(Methoxyimino)methyl]-3,6-difluorphenyl steht oder
R¹ für 2-[(Methoxyimino)methyl]-3-chlorphenyl steht oder
R¹ für 2-[(Methoxyimino)methyl]-3-fluorphenyl steht oder
R¹ für 2-[(Methoxyimino)methyl]-3-methylphenyl steht oder
R¹ für 2-[(Methoxyimino)methyl]-4,5-difluorphenyl steht oder
R¹ für 2-[(Methoxyimino)methyl]-4,6-difluorphenyl steht oder
R¹ für 2-[(Methoxyimino)methyl]-4-chlorphenyl steht oder
R¹ für 2-[(Methoxyimino)methyl]-4-fluorphenyl steht oder
R¹ für 2-[(Methoxyimino)methyl]-4-methylphenyl steht oder
R¹ für 2-[(Methoxyimino)methyl]-5,6-difluorphenyl steht oder
R¹ für 2-[(Methoxyimino)methyl]-5-chlorphenyl steht oder
R¹ für 2-[(Methoxyimino)methyl]-5-fluorphenyl steht oder
R¹ für 2-[(Methoxyimino)methyl]-5-methylphenyl steht oder
R¹ für 2-[(Methoxyimino)methyl]-6-chlorphenyl steht oder
R¹ für 2-[(Methoxyimino)methyl]-6-fluorphenyl steht oder
R¹ für 2-[(Methoxyimino)methyl]-6-methylphenyl steht oder
R¹ für 2-[(Methoxyimino)methyl]phenyl steht oder
R¹ für 2-[(Methylsulfonyl)oxy]phenyl steht oder
R¹ für 2-[(Methylsulfonyl)oxy]-4-(trifluormethyl)phenyl steht oder
R¹ für 3,4-Dichlor-2-(prop-2-in-1-yloxy)phenyl steht oder
R¹ für 3,4-Dichlor-2-[(methylsulfonyl)oxy]phenyl steht oder
R¹ für 3,4-Difluor-2-(prop-2-in-1-yloxy)phenyl steht oder
R¹ für 3,4-Difluor-2-[(methylsulfonyl)oxy]phenyl steht oder
R¹ für 3,4-Difluor-2-formylphenyl steht oder
R¹ für 3,5-Dichlor-2-(prop-2-in-1-yloxy)phenyl steht oder
R¹ für 3,5-Dichlor-2-[(methylsulfonyl)oxy]phenyl steht oder
R¹ für 3,5-Dichlor-4-(prop-2-in-1-yloxy)phenyl steht oder
R¹ für 3,5-Dichlor-4-[(methylsulfonyl)oxy]phenyl steht oder
R¹ für 3,5-Difluor-2-(prop-2-in-1-yloxy)phenyl steht oder
R¹ für 3,5-Difluor-2-[(methylsulfonyl)oxy]phenyl steht oder
R¹ für 3,5-Difluor-2-formylphenyl steht oder
R¹ für 3,5-Difluor-4-(prop-2-in-1-yloxy)phenyl steht oder
R¹ für 3,5-Difluor-4-[(methylsulfonyl)oxy]phenyl steht oder
R¹ für 3,5-Difluor-4-formylphenyl steht oder
R¹ für 3,6-Dichlor-2-(prop-2-in-1-yloxy)phenyl steht oder
R¹ für 3,6-Dichlor-2-[(methylsulfonyl)oxy]phenyl steht oder
R¹ für 3,6-Dichlor-4-(prop-2-in-1-yloxy)phenyl steht oder
R¹ für 3,6-Dichlor-4-[(methylsulfonyl)oxy]phenyl steht oder
R¹ für 3,6-Difluor-2-(prop-2-in-1-yloxy)phenyl steht oder
R¹ für 3,6-Difluor-2-[(methylsulfonyl)oxy]phenyl steht oder
R¹ für 3,6-Difluor-2-formylphenyl steht oder
R¹ für 3,6-Difluor-4-(prop-2-in-1-yloxy)phenyl steht oder
R¹ für 3,6-Difluor-4-[(methylsulfonyl)oxy]phenyl steht oder
R¹ für 3,6-Difluor-4-formylphenyl steht oder
R¹ für 3-(Allyloxy)-2,4-dichlorphenyl steht oder
R¹ für 3-(Allyloxy)-2,4-difluorphenyl steht oder
R¹ für 3-(Allyloxy)-2,5-dichlorphenyl steht oder
R¹ für 3-(Allyloxy)-2,5-difluorphenyl steht oder
R¹ für 3-(Allyloxy)-2,6-dichlorphenyl steht oder
R¹ für 3-(Allyloxy)-2,6-difluorphenyl steht oder
R¹ für 3-(Allyloxy)-2-chlorphenyl steht oder
R¹ für 3-(Allyloxy)-2-fluorphenyl steht oder
R¹ für 3-(Allyloxy)-2-methylphenyl steht oder
R¹ für 3-(Allyloxy)-4,5-dichlorphenyl steht oder
R¹ für 3-(Allyloxy)-4,5-difluorphenyl steht oder
R¹ für 3-(Allyloxy)-4,6-dichlorphenyl steht oder
R¹ für 3-(Allyloxy)-4,6-difluorphenyl steht oder
R¹ für 3-(Allyloxy)-4-chlorphenyl steht oder
R¹ für 3-(Allyloxy)-4-fluorphenyl steht oder
R¹ für 3-(Allyloxy)-4-methylphenyl steht oder
R¹ für 3-(Allyloxy)-5,6-dichlorphenyl steht oder
R¹ für 3-(Allyloxy)-5,6-difluorphenyl steht oder
R¹ für 3-(Allyloxy)-5-chlorphenyl steht oder
R¹ für 3-(Allyloxy)-5-fluorphenyl steht oder
R¹ für 3-(Allyloxy)-5-methylphenyl steht oder
R¹ für 3-(Allyloxy)-6-chlorphenyl steht oder
R¹ für 3-(Allyloxy)-6-fluorphenyl steht oder
R¹ für 3-(Allyloxy)-6-methylphenyl steht oder
R¹ für 3-(Allyloxy)phenyl steht oder
R¹ für 3-(Cyanomethoxy)-2,4-dichlorphenyl steht oder
R¹ für 3-(Cyanomethoxy)-2,4-difluorphenyl steht oder
R¹ für 3-(Cyanomethoxy)-2,5-dichlorphenyl steht oder
R¹ für 3-(Cyanomethoxy)-2,5-difluorphenyl steht oder
R¹ für 3-(Cyanomethoxy)-2,6-dichlorphenyl steht oder
R¹ für 3-(Cyanomethoxy)-2,6-difluorphenyl steht oder
R¹ für 3-(Cyanomethoxy)-2-chlorphenyl steht oder
R¹ für 3-(Cyanomethoxy)-2-fluorphenyl steht oder
R¹ für 3-(Cyanomethoxy)-2-methylphenyl steht oder
R¹ für 3-(Cyanomethoxy)-4,5-dichlorphenyl steht oder
R¹ für 3-(Cyanomethoxy)-4,5-difluorphenyl steht oder
R¹ für 3-(Cyanomethoxy)-4,6-dichlorphenyl steht oder
R¹ für 3-(Cyanomethoxy)-4,6-difluorphenyl steht oder
R¹ für 3-(Cyanomethoxy)-4-chlorphenyl steht oder
R¹ für 3-(Cyanomethoxy)-4-fluorphenyl steht oder
R¹ für 3-(Cyanomethoxy)-4-methylphenyl steht oder
R¹ für 3-(Cyanomethoxy)-5,6-dichlorphenyl steht oder
R¹ für 3-(Cyanomethoxy)-5,6-difluorphenyl steht oder
R¹ für 3-(Cyanomethoxy)-5-chlorphenyl steht oder
R¹ für 3-(Cyanomethoxy)-5-fluorphenyl steht oder
R¹ für 3-(Cyanomethoxy)-5-methylphenyl steht oder
R¹ für 3-(Cyanomethoxy)-6-chlorphenyl steht oder
R¹ für 3-(Cyanomethoxy)-6-fluorphenyl steht oder
R¹ für 3-(Cyanomethoxy)-6-methylphenyl steht oder
R¹ für 3-(Cyanomethoxy)phenyl steht oder
R¹ für 3-(Prop-2-in-1-yloxy)phenyl steht oder
R¹ für 3-Chlor-2-(prop-2-in-1-yloxy)phenyl steht oder
R¹ für 3-Chlor-2-[(methylsulfonyl)oxy]phenyl steht oder
R¹ für 3-Chlor-2-formylphenyl steht oder
R¹ für 3-Chlor-4-(prop-2-in-1-yloxy)phenyl steht oder
R¹ für 3-Chlor-4-[(methylsulfonyl)oxy]phenyl steht oder
R¹ für 3-Chlor-4-formylphenyl steht oder
R¹ für 3-Fluor-2-(prop-2-in-1-yloxy)phenyl steht oder
R¹ für 3-Fluor-2-[(methylsulfonyl)oxy]phenyl steht oder
R¹ für 3-Fluor-2-formylphenyl steht oder
R¹ für 3-Fluor-4-(prop-2-in-1-yloxy)phenyl steht oder
R¹ für 3-Fluor-4-[(methylsulfonyl)oxy]phenyl steht oder
R¹ für 3-Fluor-4-formylphenyl steht oder
R¹ für 3-Formyl-2-methylphenyl steht oder
R¹ für 3-Formyl-4-methylphenyl steht oder
R¹ für 3-Formyl-5-methylphenyl steht oder
R¹ für 3-Formyl-6-methylphenyl steht oder
R¹ für 3-Formylphenyl steht oder
R¹ für 3-Methyl-2-(prop-2-in-1-yloxy)phenyl steht oder
R¹ für 3-Methyl-2-[(methylsulfonyl)oxy]phenyl steht oder
R¹ für 3-Methyl-4-(prop-2-in-1-yloxy)phenyl steht oder
R¹ für 3-Methyl-4-[(methylsulfonyl)oxy]phenyl steht oder
R¹ für 3-[(Hydroxyimino)methyl]-2,4-difluorphenyl steht oder
R¹ für 3-[(Hydroxyimino)methyl]-2,5-difluorphenyl steht oder
R¹ für 3-[(Hydroxyimino)methyl]-2,6-difluorphenyl steht oder
R¹ für 3-[(Hydroxyimino)methyl]-2-chlorphenyl steht oder
R¹ für 3-[(Hydroxyimino)methyl]-2-fluorphenyl steht oder
R¹ für 3-[(Hydroxyimino)methyl]-2-methylphenyl steht oder
R¹ für 3-[(Hydroxyimino)methyl]-4,5-difluorphenyl steht oder
R¹ für 3-[(Hydroxyimino)methyl]-4,6-difluorphenyl steht oder
R¹ für 3-[(Hydroxyimino)methyl]-4-chlorphenyl steht oder
R¹ für 3-[(Hydroxyimino)methyl]-4-fluorphenyl steht oder
R¹ für 3-[(Hydroxyimino)methyl]-4-methylphenyl steht oder
R¹ für 3-[(Hydroxyimino)methyl]-5,6-difluorphenyl steht oder
R¹ für 3-[(Hydroxyimino)methyl]-5-chlorphenyl steht oder
R¹ für 3-[(Hydroxyimino)methyl]-5-fluorphenyl steht oder
R¹ für 3-[(Hydroxyimino)methyl]-5-methylphenyl steht oder
R¹ für 3-[(Hydroxyimino)methyl]-6-chlorphenyl steht oder
R¹ für 3-[(Hydroxyimino)methyl]-6-fluorphenyl steht oder
R¹ für 3-[(Hydroxyimino)methyl]-6-methylphenyl steht oder
R¹ für 3-[(Hydroxyimino)methyl]phenyl steht oder
R¹ für 3-[(Methoxyimino)methyl]-2,4-difluorphenyl steht oder
R¹ für 3-[(Methoxyimino)methyl]-2,5-difluorphenyl steht oder
R¹ für 3-[(Methoxyimino)methyl]-2,6-difluorphenyl steht oder
R¹ für 3-[(Methoxyimino)methyl]-2-chlorphenyl steht oder
R¹ für 3-[(Methoxyimino)methyl]-2-fluorphenyl steht oder
R¹ für 3-[(Methoxyimino)methyl]-2-methylphenyl steht oder
R¹ für 3-[(Methoxyimino)methyl]-4,5-difluorphenyl steht oder
R¹ für 3-[(Methoxyimino)methyl]-4,6-difluorphenyl steht oder
R¹ für 3-[(Methoxyimino)methyl]-4-chlorphenyl steht oder
R¹ für 3-[(Methoxyimino)methyl]-4-fluorphenyl steht oder
R¹ für 3-[(Methoxyimino)methyl]-4-methylphenyl steht oder
R¹ für 3-[(Methoxyimino)methyl]-5,6-difluorphenyl steht oder
R¹ für 3-[(Methoxyimino)methyl]-5-chlorphenyl steht oder
R¹ für 3-[(Methoxyimino)methyl]-5-fluorphenyl steht oder
R¹ für 3-[(Methoxyimino)methyl]-5-methylphenyl steht oder
R¹ für 3-[(Methoxyimino)methyl]-6-chlorphenyl steht oder
R¹ für 3-[(Methoxyimino)methyl]-6-fluorphenyl steht oder
R¹ für 3-[(Methoxyimino)methyl]-6-methylphenyl steht oder
R¹ für 3-[(Methoxyimino)methyl]phenyl steht oder
R¹ für 3-[(Methylsulfonyl)oxy]phenyl steht oder
R¹ für 4,5-Dichlor-2-(prop-2-in-1-yloxy)phenyl steht oder
R¹ für 4,5-Dichlor-2-[(methylsulfonyl)oxy]phenyl steht oder
R¹ für 4,5-Dichlor-3-(prop-2-in-1-yloxy)phenyl steht oder
R¹ für 4,5-Dichlor-3-[(methylsulfonyl)oxy]phenyl steht oder
R¹ für 4,5-Difluor-2-(prop-2-in-1-yloxy)phenyl steht oder
R¹ für 4,5-Difluor-2-[(methylsulfonyl)oxy]phenyl steht oder
R¹ für 4,5-Difluor-2-formylphenyl steht oder
R¹ für 4,5-Difluor-3-(prop-2-in-1-yloxy)phenyl steht oder
R¹ für 4,5-Difluor-3-[(methylsulfonyl)oxy]phenyl steht oder
R¹ für 4,5-Difluor-3-formylphenyl steht oder
R¹ für 4,6-Dichlor-2-(prop-2-in-1-yloxy)phenyl steht oder
R¹ für 4,6-Dichlor-2-[(methylsulfonyl)oxy]phenyl steht oder
R¹ für 4,6-Dichlor-3-(prop-2-in-1-yloxy)phenyl steht oder
R¹ für 4,6-Dichlor-3-[(methylsulfonyl)oxy]phenyl steht oder
R¹ für 4,6-Difluor-2-(prop-2-in-1-yloxy)phenyl steht oder
R¹ für 4,6-Difluor-2-[(methylsulfonyl)oxy]phenyl steht oder
R¹ für 4,6-Difluor-2-formylphenyl steht oder
R¹ für 4,6-Difluor-3-(prop-2-in-1-yloxy)phenyl steht oder
R¹ für 4,6-Difluor-3-[(methylsulfonyl)oxy]phenyl steht oder
R¹ für 4,6-Difluor-3-formylphenyl steht oder
R¹ für 4-(Allyloxy)-2,3-dichlorphenyl steht oder
R¹ für 4-(Allyloxy)-2,3-difluorphenyl steht oder
R¹ für 4-(Allyloxy)-2,5-dichlorphenyl steht oder
R¹ für 4-(Allyloxy)-2,5-difluorphenyl steht oder
R¹ für 4-(Allyloxy)-2,6-dichlorphenyl steht oder
R¹ für 4-(Allyloxy)-2,6-difluorphenyl steht oder
R¹ für 4-(Allyloxy)-2-chlorphenyl steht oder
R¹ für 4-(Allyloxy)-2-fluorphenyl steht oder
R¹ für 4-(Allyloxy)-2-methylphenyl steht oder
R¹ für 4-(Allyloxy)-3,5-dichlorphenyl steht oder
R¹ für 4-(Allyloxy)-3,5-difluorphenyl steht oder
R¹ für 4-(Allyloxy)-3,6-dichlorphenyl steht oder
R¹ für 4-(Allyloxy)-3,6-difluorphenyl steht oder
R¹ für 4-(Allyloxy)-3-chlorphenyl steht oder
R¹ für 4-(Allyloxy)-3-fluorphenyl steht oder
R¹ für 4-(Allyloxy)-3-methylphenyl steht oder
R¹ für 4-(Allyloxy)phenyl steht oder
R¹ für 4-(Cyanomethoxy)-2,3-dichlorphenyl steht oder
R¹ für 4-(Cyanomethoxy)-2,3-difluorphenyl steht oder
R¹ für 4-(Cyanomethoxy)-2,5-dichlorphenyl steht oder
R¹ für 4-(Cyanomethoxy)-2,5-difluorphenyl steht oder
R¹ für 4-(Cyanomethoxy)-2,6-dichlorphenyl steht oder
R¹ für 4-(Cyanomethoxy)-2,6-difluorphenyl steht oder
R¹ für 4-(Cyanomethoxy)-2-chlorphenyl steht oder
R¹ für 4-(Cyanomethoxy)-2-fluorphenyl steht oder
R¹ für 4-(Cyanomethoxy)-2-methylphenyl steht oder
R¹ für 4-(Cyanomethoxy)-3,5-dichlorphenyl steht oder
R¹ für 4-(Cyanomethoxy)-3,5-difluorphenyl steht oder
R¹ für 4-(Cyanomethoxy)-3,6-dichlorphenyl steht oder
R¹ für 4-(Cyanomethoxy)-3,6-difluorphenyl steht oder
R¹ für 4-(Cyanomethoxy)-3-chlorphenyl steht oder
R¹ für 4-(Cyanomethoxy)-3-fluorphenyl steht oder
R¹ für 4-(Cyanomethoxy)-3-methylphenyl steht oder
R¹ für 4-(Cyanomethoxy)phenyl steht oder
R¹ für 4-(Prop-2-in-1-yloxy)phenyl steht oder
R¹ für 4-Chlor-2-(prop-2-in-1-yloxy)phenyl steht oder
R¹ für 4-Chlor-2-[(methylsulfonyl)oxy]phenyl steht oder
R¹ für 4-Chlor-2-formylphenyl steht oder
R¹ für 4-Chlor-3-(prop-2-in-1-yloxy)phenyl steht oder
R¹ für 4-Chlor-3-[(methylsulfonyl)oxy]phenyl steht oder
R¹ für 4-Chlor-3-formylphenyl steht oder
R¹ für 4-Fluor-2-(prop-2-in-1-yloxy)phenyl steht oder
R¹ für 4-Fluor-2-[(methylsulfonyl)oxy]phenyl steht oder
R¹ für 4-Fluor-2-formylphenyl steht oder
R¹ für 4-Fluor-3-(prop-2-in-1-yloxy)phenyl steht oder
R¹ für 4-Fluor-3-[(methylsulfonyl)oxy]phenyl steht oder
R¹ für 4-Fluor-3-formylphenyl steht oder
R¹ für 4-Formyl-2-methylphenyl steht oder
R¹ für 4-Formyl-3-methylphenyl steht oder
R¹ für 4-Formylphenyl steht oder
R¹ für 4-Methyl-2-(prop-2-in-1-yloxy)phenyl steht oder
R¹ für 4-Methyl-2-[(methylsulfonyl)oxy]phenyl steht oder
R¹ für 4-Methyl-3-(prop-2-in-1-yloxy)phenyl steht oder
R¹ für 4-Methyl-3-[(methylsulfonyl)oxy]phenyl steht oder
R¹ für 4-[(Hydroxyimino)methyl]-2,3-difluorphenyl steht oder
R¹ für 4-[(Hydroxyimino)methyl]-2,5-difluorphenyl steht oder
R¹ für 4-[(Hydroxyimino)methyl]-2,6-difluorphenyl steht oder
R¹ für 4-[(Hydroxyimino)methyl]-2-chlorphenyl steht oder
R¹ für 4-[(Hydroxyimino)methyl]-2-fluorphenyl steht oder
R¹ für 4-[(Hydroxyimino)methyl]-2-methylphenyl steht oder
R¹ für 4-[(Hydroxyimino)methyl]-3,5-difluorphenyl steht oder
R¹ für 4-[(Hydroxyimino)methyl]-3,6-difluorphenyl steht oder
R¹ für 4-[(Hydroxyimino)methyl]-3-chlorphenyl steht oder
R¹ für 4-[(Hydroxyimino)methyl]-3-fluorphenyl steht oder
R¹ für 4-[(Hydroxyimino)methyl]-3-methylphenyl steht oder
R¹ für 4-[(Hydroxyimino)methyl]phenyl steht oder
R¹ für 4-[(Methoxyimino)methyl]-2,3-difluorphenyl steht oder
R¹ für 4-[(Methoxyimino)methyl]-2,5-difluorphenyl steht oder
R¹ für 4-[(Methoxyimino)methyl]-2,6-difluorphenyl steht oder
R¹ für 4-[(Methoxyimino)methyl]-2-chlorphenyl steht oder
R¹ für 4-[(Methoxyimino)methyl]-2-fluorphenyl steht oder
R¹ für 4-[(Methoxyimino)methyl]-2-methylphenyl steht oder
R¹ für 4-[(Methoxyimino)methyl]-3,5-difluorphenyl steht oder
R¹ für 4-[(Methoxyimino)methyl]-3,6-difluorphenyl steht oder
R¹ für 4-[(Methoxyimino)methyl]-3-chlorphenyl steht oder
R¹ für 4-[(Methoxyimino)methyl]-3-fluorphenyl steht oder
R¹ für 4-[(Methoxyimino)methyl]-3-methylphenyl steht oder
R¹ für 4-[(Methoxyimino)methyl]phenyl steht oder
R¹ für 4-[(Methylsulfonyl)oxy]phenyl steht oder
R¹ für 5,6-Dichlor-2-(prop-2-in-1-yloxy)phenyl steht oder
R¹ für 5,6-Dichlor-2-[(methylsulfonyl)oxy]phenyl steht oder
R¹ für 5,6-Dichlor-3-(prop-2-in-1-yloxy)phenyl steht oder
R¹ für 5,6-Dichlor-3-[(methylsulfonyl)oxy]phenyl steht oder
R¹ für 5,6-Difluor-2-(prop-2-in-1-yloxy)phenyl steht oder
R¹ für 5,6-Difluor-2-[(methylsulfonyl)oxy]phenyl steht oder
R¹ für 5,6-Difluor-2-formylphenyl steht oder
R¹ für 5,6-Difluor-3-(prop-2-in-1-yloxy)phenyl steht oder
R¹ für 5,6-Difluor-3-[(methylsulfonyl)oxy]phenyl steht oder
R¹ für 5,6-Difluor-3-formylphenyl steht oder
R¹ für 5-Chlor-2-(prop-2-in-1-yloxy)phenyl steht oder
R¹ für 5-Chlor-2-[(methylsulfonyl)oxy]phenyl steht oder
R¹ für 5-Chlor-2-formylphenyl steht oder
R¹ für 5-Chlor-3-(prop-2-in-1-yloxy)phenyl steht oder
R¹ für 5-Chlor-3-[(methylsulfonyl)oxy]phenyl steht oder
R¹ für 5-Chlor-3-formylphenyl steht oder
R¹ für 5-Fluor-2-(prop-2-in-1-yloxy)phenyl steht oder
R¹ für 5-Fluor-2-[(methylsulfonyl)oxy]phenyl steht oder
R¹ für 5-Fluor-2-formylphenyl steht oder
R¹ für 5-Fluor-3-(prop-2-in-1-yloxy)phenyl steht oder
R¹ für 5-Fluor-3-[(methylsulfonyl)oxy]phenyl steht oder
R¹ für 5-Fluor-3-formylphenyl steht oder
R¹ für 5-Methyl-2-(prop-2-in-1-yloxy)phenyl steht oder
R¹ für 5-Methyl-2-[(methylsulfonyl)oxy]phenyl steht oder
R¹ für 5-Methyl-3-(prop-2-in-1-yloxy)phenyl steht oder
R¹ für 5-Methyl-3-[(methylsulfonyl)oxy]phenyl steht oder
R¹ für 6-Chlor-2-(prop-2-in-1-yloxy)phenyl steht oder
R¹ für 6-Chlor-2-[(methylsulfonyl)oxy]phenyl steht oder
R¹ für 6-Chlor-2-formylphenyl steht oder
R¹ für 6-Chlor-3-(prop-2-in-1-yloxy)phenyl steht oder
R¹ für 6-Chlor-3-[(methylsulfonyl)oxy]phenyl steht oder
R¹ für 6-Chlor-3-formylphenyl steht oder
R¹ für 6-Fluor-2-(prop-2-in-1-yloxy)phenyl steht oder
R¹ für 6-Fluor-2-[(methylsulfonyl)oxy]phenyl steht oder
R¹ für 6-Fluor-2-formylphenyl steht oder
R¹ für 6-Fluor-3-(prop-2-in-1-yloxy)phenyl steht oder
R¹ für 6-Fluor-3-[(methylsulfonyl)oxy]phenyl steht oder
R¹ für 6-Fluor-3-formylphenyl steht oder
R¹ für 6-Methyl-2-(prop-2-in-1-yloxy)phenyl steht oder
R¹ für 6-Methyl-2-[(methylsulfonyl)oxy]phenyl steht oder
R¹ für 6-Methyl-3-(prop-2-in-1-yloxy)phenyl steht oder
R¹ für 6-Methyl-3-[(methylsulfonyl)oxy]phenyl steht.

Die zuvor genannten Reste-Definitionen können untereinander in beliebiger Weise kombiniert werden. Außerdem können einzelne Definitionen entfallen.

Je nach Art der oben definierten Substituenten weisen die Verbindungen der Formel **(I)** saure oder basische Eigenschaften auf und können mit anorganischen oder organischen Säuren oder mit Basen oder mit Metallionen Salze, gegebenenfalls auch innere Salze oder Addukte bilden. Tragen die Verbindungen der Formel **(I)** Amino, Alkylamino oder andere, basische Eigenschaften induzierende Gruppen, so können diese Verbindungen mit Säuren zu Salzen umgesetzt werden oder fallen durch die Synthese direkt als Salze an. Tragen die Verbindungen der Formel **(I)** Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit C₁-C₄-Alkyl-Gruppen, Mono-, Di- und Trialkanolamine von C₁-C₄-Alkanolen, Cholin sowie Chlorcholin.

Die so erhältlichen Salze weisen ebenfalls fungizide Eigenschaften auf.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄. Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, gesättigte oder einfach oder doppelt ungesättigte C₆-C₂₀-Fettsäuren, Alkylschwefelsäuremonoester, Alkylsulfonsäuren (Sulfonsäuren mit geradkettigen oder verzweigten Alkylresten mit 1 bis 20 Kohlenstoffatomen), Arylsulfonsäuren oder Aryldisulfonsäuren (aromatische Reste wie Phenyl und Naphthyl welche ein oder zwei Sulfonsäuregruppen tragen), Alkylphosphonsäuren (Phosphonsäuren mit geradkettigen oder verzweigten Alkylresten mit 1 bis 20 Kohlenstoffatomen), Arylphosphonsäuren oder Aryldiphosphonsäuren (aromatische Reste wie Phenyl und Naphthyl welche ein oder zwei Phosphonsäurereste tragen) in Betracht, wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Gegebenenfalls substituierte Gruppen können einfach oder mehrfach substituiert sein, wobei bei Mehrfachsubstitutionen die Substituenten gleich oder verschieden sein können.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Iod und vorzugsweise Fluor, Chlor, Brom und noch bevorzugter Fluor, Chlor.
**Alkyl:** gesättigte, geradkettige oder verzweigte Kohlenwasserstoffreste mit 1 bis 8 vorzugsweise 1 bis 6 und noch bevorzugter 1 bis 3 Kohlenstoffatomen, z.B. (aber nicht beschränkt auf) C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Diese Definition gilt auch für Alkyl as Bestandteil eines zusammengesetzen Substitutenten wie z.B. Cycloalkylalkyl, Hydroxyalkyl etc. sofern an anderer Stelle nicht definiert wie z.B. Alkylthio, Alkylsufinyl, Alkylsulfonyl, Halogenalkyl bzw. Halogenalkylthio. Steht das Alkyl am Ende eines zusammengesetzten Substituenten. wie z.B bei Alkylcylcloalkyl, so kann der am Anfang stehende Bestandteil des zusammengesetzten Substituenten, z.B. das Cycloalkyl, ein- bzw. mehrfach, gleich oder verschieden und unabhängig voneinander mit Alkyl substituiert sein. Das gleiche gilt auch für zusammengesetzte Substituenten bei denen andere Reste wie z.B. Alkenyl, Alkinyl, Hydroxy, Halogen, Formyl etc. am Ende stehen.
Alkenyl: ungesättigte, geradkettige oder verzweigte Kohlenwasserstoffreste mit 2 bis 8 vorzugsweise 2 bis 6 Kohlenstoffatomen und einer Doppelbindung in einer beliebigen Position, z.B. (aber nicht beschränkt auf) C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-l-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1,-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl. Diese Definition gilt auch für Alkenyl als Bestandteil eines zusammengesetzen Substitutenten wie z.B. Halogenalkenyl etc. sofern an anderer Stelle nicht definiert;
**Alkinyl**: geradkettige oder verzweigte Kohlenwasserstoffgruppen mit 2 bis 8 vorzugsweise 2 bis 6 Kohlenstoffatomen und einer Dreifachbindung in einer beliebigen Position, z.B. (aber nicht beschränkt auf) C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-l-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl. Diese Definition gilt auch für Alkinyl als Bestandteil eines zusammengesetzen Substitutenten wie z.B. Halogenalkinyl etc. sofern an anderer Stelle nicht definiert;
Alkoxy: gesättigte, geradkettige oder verzweigte Alkoxyreste mit 1 bis 8 vorzugsweise 1 bis 6 und noch bevorzugter 1 bis 3 Kohlenstoffatomen, z.B. (aber nicht beschränkt auf) C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Diese Definition gilt auch für Alkoxy als Bestandteil eines zusammengesetzen Substitutenten wie z.B. Halogenalkoxy, Alkinylalkoxy etc. sofern an anderer Stelle nicht definiert;
**Alkylthio:** gesättigte, geradkettige oder verzweigte Alkylthioreste mit 1 bis 8 vorzugsweise 1 bis 6 und noch bevorzugter 1 bis 3 Kohlenstoffatomen, z.B. (aber nicht beschränkt auf) C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio, 1 -Methylpentylthio, 2-Methylpentylthio, 3 -Methyl-pentylthio, 4-Methylpentylthio, 1,1-Dimethylbutylthio, 1,2-Dimethylbutylthio, 1,3-Dimethyl-butylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio. Diese Definition gilt auch für Alkylthio als Bestandteil eines zusammengesetzen Substitutenten wie z.B. Halogenalkylthio etc. sofern an anderer Stelle nicht definiert;
**Alkoxycarbonyl:** eine Alkoxygruppe mit 1 bis 6 bevorzugt 1 bis 3 Kohlenstoffatomen (wie vorstehend genannt), welche über eine Carbonylgruppe (-CO-) an das Gerüst gebunden ist. Diese Definition gilt auch für Alkoxycarbonyl als Bestandteil eines zusammengesetzen Substitutenten wie z.B. Cycloalkylalkoxycarbonyl etc. sofern an anderer Stelle nicht definiert;
**Alkylsulfinyl:** gesättigte, geradkettige oder verzweigte Alkylsulfinylreste mit 1 bis 8 vorzugsweise 1 bis 6 und noch bevorzugter 1 bis 3 Kohlenstoffatomen, z.B. (aber nicht beschränkt auf) C₁-C₆-Alkylsulfinyl wie Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Di-methylpropylsulfinyl, 1-Ethylpropylsulfinyl, Hexylsulfinyl, 1,1-Dimethylpropylsulfinyl, 1,2-Dimethylpropylsulfinyl, 1 -Methylpentylsulfinyl, 2-Methylpentyl-sulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl. Diese Definition gilt auch für Alkylsulfinyl als Bestandteil eines zusammengesetzen Substitutenten wie z.B. Halogenalkylsulfinyl etc. sofern an anderer Stelle nicht definiert;
**Alkylsulfonyl:** gesättigte, geradkettige oder verzweigte Alkylsulfonylreste mit 1 bis 8 vorzugsweise 1 bis 6 und noch bevorzugter 1 bis 3 Kohlenstoffatomen, z.B. (aber nicht beschränkt auf) C₁-C₆-Alkylsulfonyl wie Methylsulfonyl, Ethylsulfonyl, Propylsulfonyl, 1-Methylethylsulfonyl, Butylsulfonyl, 1-Methyl-propylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 2,2-Dimethylpropylsulfonyl, 1-Ethylpropylsulfonyl, Hexylsulfonyl, 1,1-Dimethylpropylsulfonyl, 1,2-Dimethylpropylsulfonyl,1-Methylpentylsulfonyl, 2-Methylpentyl-sulfonyl, 3 -Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl. Diese Definition gilt auch für Alkylsulfonyl als Bestandteil eines zusammengesetzen Substitutenten wie z.B. Alkylsulfonylalkyl etc. sofern an anderer Stelle nicht definiert;
**Cycloalkyl:** monocyclische, gesättigte Kohlenwasserstoffgruppen mit 3 bis 10 vorzugsweise 3 bis 8 und noch bevorzugter 3 bis 6 Kohlenstoffringgliedern, z.B. (aber nicht beschränkt auf) Cyclopropyl, Cyclopentyl und Cyclohexyl. Diese Definition gilt auch für Cycloalkyl als Bestandteil eines zusammengesetzen Substitutenten wie z.B. Cycloalkylalkyl etc. sofern an anderer Stelle nicht definiert;
**Cycloalkenyl:** monocyclische, partiell ungesättigter Kohlenwasserstoffgruppen mit 3 bis 10 vorzugsweise 3 bis 8 und noch bevorzugter 3 bis 6 Kohlenstoffringgliedern, z.B. (aber nicht beschränkt auf) Cyclopropenyl, Cyclopentyl und Cyclohexenyl. Diese Definition gilt auch für Cycloalkenyl als Bestandteil eines zusammengesetzen Substitutenten wie z.B. Cycloalkyenlalkyl etc. sofern an anderer Stelle nicht definiert;
**Cycloalkoxy:** monocyclische, gesättigte Cycloalkyloxyreste mit 3 bis 10 vorzugsweise 3 bis 8 und noch bevorzugter 3 bis 6 Kohlenstoffringgliedern, z.B. (aber nicht beschränkt auf) Cyclopropyloxy, Cyclopentyloxy und Cyclohexyloxy. Diese Definition gilt auch für Cycloalkoxy als Bestandteil eines zusammengesetzen Substitutenten wie z.B. Cycloalkoxyalkyl etc. sofern an anderer Stelle nicht definiert;
**Halogenalkyl:** geradkettige oder verzweigte Alkylgruppen mit 1 bis 8 vorzugsweise 1 bis 6 und noch bevorzugter 1 bis 3 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2-difluorethyl, 2,2-Dichlor-2-fluorethyl, 2,2,2-Trichlorethyl, Pentafluorethyl und 1,1,1-Trifluorprop-2-yl. Diese Definition gilt auch für Halogenalkyl als Bestandteil eines zusammengesetzen Substitutenten wie z.B. Halogenalkylaminoalkyl etc. sofern an anderer Stelle nicht definiert;
Halogenalkenyl und Halogenalkinyl sind analog wie Halogenalkyl definiert, wobei anstatt Alkylgruppen, Alkenyl und Alkinylgruppen als Bestandteil des Substituenten vorliegen.
**Halogenalkoxy:** geradkettige oder verzweigte Alkoxygruppen mit 1 bis 8 vorzugsweise 1 bis 6 und noch bevorzugter 1 bis 3 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor-2-difluorethoxy, 2,2-Dichlor-2-fluorethoxy, 2,2,2-Trichlorethoxy, Pentafluor-ethoxy und 1,1,1-Trifluorprop-2-oxy. Diese Definition gilt auch für Halogenalkoxy als Bestandteil eines zusammengesetzen Substitutenten wie z.B. Halogenalkoxyalkyl etc. sofern an anderer Stelle nicht definiert;
**Halogenalkylthio:** geradkettige oder verzweigte Alkylthiogruppen mit 1 bis 8 vorzugsweise 1 bis 6 und noch bevorzugter 1 bis 3 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluor-methylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor-2-difluorethylthio, 2,2-Dichlor-2-fluorethylthio, 2,2,2-Trichlorethylthio, Pentafluorethylthio und 1,1,1-Trifluorprop-2-ylthio. Diese Definition gilt auch für Halogenalkylthio als Bestandteil eines zusammengesetzen Substitutenten wie z.B. Halogenalkylthioalkyl etc. sofern an anderer Stelle nicht definiert;
**Heteroaryl:** 5 oder 6-gliedriges, vollständig ungesättigtes monocyclisches Ringsystem, enthaltend ein bis vier Heteroatome aus der Gruppe Sauerstoff, Stickstoff oder Schwefel, enthält der Ring mehrere Sauerstoffatome, so stehen diese nicht direkt benachbart;
**5-gliedriges Heteroaryl: enthaltend ein bis vier Stickstoffatome oder ein bis drei Stickstoffatome und ein Schwefel- oder Sauerstoffatom:** 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. (aber nicht beschränkt auf) 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
**über Stickstoff gebundenes 5-gliedriges Heteroaryl, enthaltend ein bis vier Stickstoffatome, oder über Stickstoff gebundenes benzokondensiertes 5-gliederiges Heteroaryl, enthaltend ein bis drei Stickstoffatome:** 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, in der ein oder zwei C-Atome durch N-Atome ersetzt sein können, wobei diese Ringe über eines der Stickstoffringglieder an das Gerüst gebunden sind, z.B. (aber nicht beschränkt auf) 1-Pyrrolyl, 1-Pyrazolyl, 1,2,4-Triazol-1-yl, 1-Imidazolyl, 1,2,3-Triazol-1-yl und 1,3,4-Triazol-1-yl;
**6-gliedriges Heteroaryl, enthaltend ein bis vier Stickstoffatome:** 6-Ring Heteroarylgruppen, welche neben Kohlenstoffatomen ein bis drei bzw. ein bis vier Stickstoffatome als Ringglieder enthalten können, beispielsweise (aber nicht beschränkt auf) 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
**Benzokondensiertes 5-gliedriges Heteroaryl, enthaltend ein bis drei Stickstoffatome oder ein Stickstoffatom und ein Sauerstoff- oder Schwefelatom:** z.B. (aber nicht beschränkt auf) Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl und 1,3-Benzoxazol-7-yl,
**Benzokondensiertes 6-gliedriges Heteroaryl, enthaltend ein bis drei Stickstoffatome:** : z.B. (aber nicht beschränkt auf) Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl, und Isochinolin-8-yl;
Diese Definition gilt auch für Heteroaryl als Bestandteil eines zusammengesetzen Substitutenten wie z.B. Heteroarylalkyl etc. sofern an anderer Stelle nicht definiert;
**Heterocyclyl:** drei- bis fünfzehngliedriger vorzugsweise ein drei- bis neungliedriger gesättigter oder partiell ungesättigter Heterocyclus, enthaltend ein bis vier Heteroatome aus der Gruppe Sauerstoff, Stickstoff oder Schwefel: mono-, bi- oder tricyclische Heterocyclen enthaltend neben Kohlenstoffringgliedern ein bis drei Stickstoffatome und/oder ein Sauerstoff- oder Schwefelatom oder ein oder zwei Sauerstoff- und/oder Schwefelatome; enthält der Ring mehrere Sauerstoffatome, so stehen diese nicht direkt benachbart; wie z.B. (aber nicht beschränkt auf) Oxiranyl, Aziridinyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydroopyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydro-pyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl. Diese Definition gilt auch für Heterocyclyl als Bestandteil eines zusammengesetzen Substitutenten wie z.B. Heterocyclylalkyl etc. sofern an anderer Stelle nicht definiert;
**Abgangsgruppe:** S_{N}1 oder S_{N}2-Abgangsgruppe, beispielsweise Chlor, Brom, Iod, Alkylsulfonate (-OSO₂-Alkyl, z.B. -OSO₂CH₃, -OSO₂CF₃) oder Arylsulfonate (-OSO₂-Aryl, z.B. -OSO₂Ph,-OSO₂PhMe);
Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

### Erläuterung der Herstellverfahren und Zwischenprodukte

Die Heteroarylpiperidin und -piperazinderivate der Formel (**I**) lassen sich auf unterschiedliche Weise herstellen. Im Folgenden sind die möglichen Verfahren zunächst schematisch dargestellt. Wenn nicht anders angegeben haben die angegebenen Reste die oben angegebenen Bedeutungen.

Die Verfahren zur Herstellung von Verbindungen der Formel **(I)** werden gegebenenfalls unter Verwendung eines oder mehrerer Reaktionshilfsmittel durchgeführt.

Als Reaktionshilfsmittel kommen gegebenenfalls anorganische oder organische Basen oder Säureakzeptoren in Betracht. Hierzu gehören vorzugsweise Alkalimetall- oder Erdalkalimetall- -acetate,-amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium-methanolat, -ethanolat, -n- oder -i-propanolat, -n-, -i-, -s- oder -tButanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl-und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Als Reaktionshilfsmittel kommen gegebenenfalls anorganische oder organische Säuren in Betracht. Hierzu gehören vorzugsweise anorganische Säuren wie beispielsweise Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄, oder organische Säuren wie beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, gesättigte oder einfach oder doppelt ungesättigte C₆-C₂₀-Fettsäuren, Alkylschwefelsäuremonoester, Alkylsulfonsäuren (Sulfonsäuren mit geradkettigen oder verzweigten Alkylresten mit 1 bis 20 Kohlenstoffatomen), Arylsulfonsäuren oder Aryldisulfonsäuren (aromatische Reste wie Phenyl und Naphthyl welche ein oder zwei Sulfonsäuregruppen tragen), Alkylphosphonsäuren (Phosphonsäuren mit geradkettigen oder verzweigten Alkylresten mit 1 bis 20 Kohlenstoffatomen), Arylphosphonsäuren oder Aryldiphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc.

Die Verfahren werden gegebenenfalls unter Verwendung eines oder mehrerer Verdünnungsmittel durchgeführt. Als Verdünnungsmittel kommen praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methyl-ethyl-, Methyl-isopropyl-oder Methyl-isobutyl-keton, Ester wie Essigsäuremethylester oder -ethylester, Nitrile wie z.B. Acetonitril oder Propionitril, Amide wie z.B. Dimethylformamid, Dimethylacetamid und N-Methyl-pyrrolidon, sowie Dimethylsulfoxid, Tetramethylensulfon und Hexamethylphosphorsäuretriamid und DMPU.

Die Reaktionstemperaturen können bei den Verfahren in einem größeren Bereich variiert werden. Im Allgemeinen arbeitet man bei Temperaturen zwischen 0 °C und 250 °C, vorzugsweise bei Temperaturen zwischen 10 °C und 185 °C.

Die Reaktionszeit variiert in Abhängigkeit vom Maßstab der Reaktion und der Reaktionstemperatur, liegt aber im Allgemeinen zwischen einigen Minuten und 48 Stunden.

Die Verfahren werden im Allgemeinen unter Normaldruck durchgeführt. Es ist jedoch auch möglich, unter erhöhtem oder vermindertem Druck zu arbeiten.

Zur Durchführung der Verfahren werden die jeweils benötigten Ausgangsstoffe im Allgemeinen in angenähert äquimolaren Mengen eingesetzt. Es ist jedoch auch möglich, eine der jeweils eingesetzten Komponenten in einem größeren Überschuss zu verwenden.

### Verfahren A

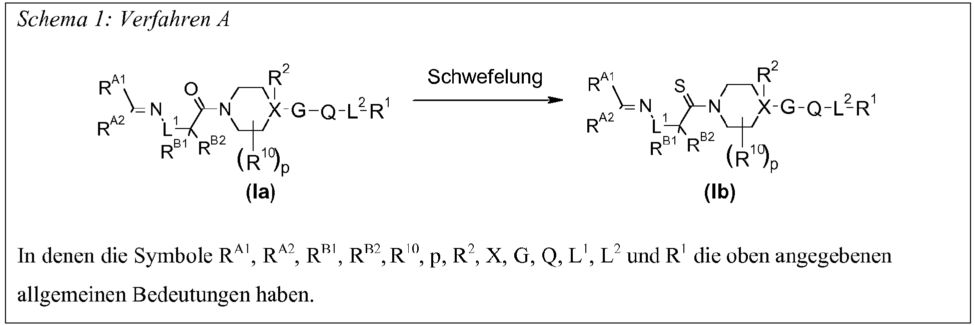

Die bei der Durchführung des *Verfahrens A* (Schema 1) erhaltenen Amide (**Ia**) lassen sich mittels literaturbeschriebener Methoden in die korrespondierenden Thioamide **(Ib)** überführen (z.B. Bioorganic & Medicinal Chemistry Letters, 2009, 19(2), 462-468). Hierbei werden die Verbindungen der Formel (**Ia**) typischerweise mit Phosphorpentasulfid oder 2,4-Bis(4-methoxyphenyl)-1,3-dithia-2,4-diphosphetan-2,4-disulfid (Lawesson Reagenz) umgesetzt (siehe Schema 7, *Verfahren F*).

Das *Verfahren A* wird vorzugsweise unter Verwendung eines oder mehrerer Verdünnungsmittel durchgeführt. Die bevorzugten Lösungsmittel sind Toluol, Tetrahydrofuran, 1,4-Dioxan und 1,2-Dimethoxyethan.

Nach Beendigung der Reaktion werden die Verbindungen **(Ib)** von der Reaktionsmischung durch eine der üblichen Trenntechniken getrennt. Falls notwendig werden die Verbindungen durch Umkristallisation oder Chromatographie gereinigt.

### Verfahren B

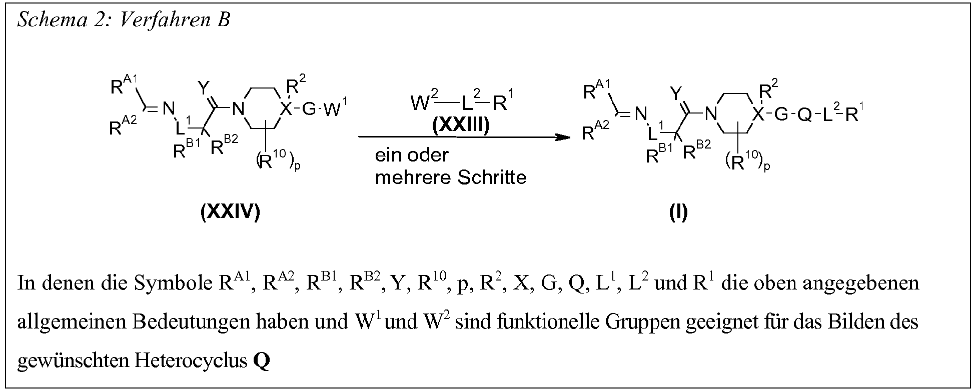

Im Allgemeinen ist es möglich Verbindungen der Formel (**I**) aus entsprechenden Verbindungen **(XXIII)** und **(XXIV)** mit geeigneten funktionellen Gruppen W¹ und W² **(I)** herzustellen (s. Schema 2, *Verfahren B*). Die möglichen funktionellen Gruppen für W¹ und W² sind z.B. Aldehyde, Ketone, Ester, Carbonsäuren, Amide, Thioamide, Nitrile, Alkohole, Thiole, Hydrazine, Oxime, Amidine, Amideoxidme, Olefine, Acetylene, Halide, Alkylhalide, Methansulfonate, Trifluormethansulfonate, Boronsäuren, Boronate, Dialkylacetal, Ketoxime, u.s.w., die unter geeigneten Reaktionsbedingungen den gewünschten Heterocyclus **Q** bilden können. In der Literatur finden sich zahlreiche Methoden für die Darstellung von Heterocyclen (s. WO 2008/013622; Comprehensive Heterocyclic Chemistry Vol. 4-6, A. R. Katritzky and C. W. Rees editors, Pergamon Press, New York, 1984; Comprehensive Heterocyclic Chemistry II, Vol 2-4, A. R. Katritzky, C. W. Rees and E. F: Scriven editors, Pergamon Press, New York, 1996; The Chemistry of Heterocyclic Compounds, E. C. Taylor, editor, Wiley, New York; Rodd's Chemistry of Carbon Compounds, Vol. 2-4, Elsevier, New York; Synthesis, 1982, 6, 508-509; Tetrahedron, 2000, 56, 1057-1094).

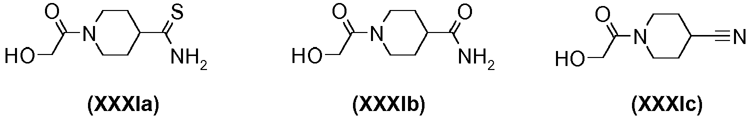

Die mit Hilfe der erfindungsgemäßen Verbindungen (**VIIIa**) und (**VIIIb**) hergestellten Stoffe der Formel **(I)** weisen eine starke mikrobizide Wirkung auf und können zur Bekämpfung von unerwünschten Mikroorganismen, wie Fungi und Bakterien, im Pflanzenschutz und im Materialschutz eingesetzt werden.

Die Heteroarylpiperidin und -piperazinderivate der Formel **(I)** besitzen sehr gute fungizide Eigenschaften und lassen sich im Pflanzenschutz beispielsweise zur Bekämpfung von Plasmodiophoromyceten, Oomyceten, Chytridiomyceten, Zygomyceten, Ascomyceten, Basidiomyceten und Deuteromyceten einsetzen.

Bakterizide lassen sich im Pflanzenschutz beispielsweise zur Bekämpfung von Pseudomonadaceae, Rhizobiaceae, Enterobacteriaceae, Corynebacteriaceae und Streptomycetaceae einsetzen.

Die mit Hilfe der erfindungsgemäßen Verbindungen hergestellten fungiziden Mittel können zur Bekämpfung von phytopathogenen Pilzen kurativ oder protektiv eingesetzt werden. Die Mittel zum Bekämpfen von phytopathogenen Pilzen im Pflanzenschutz umfassen eine wirksame, aber nicht-phytotoxische Menge der Wirkstoffe. "Wirksame, aber nicht-phytotoxische Menge" bedeutet eine Menge des Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der fungiziden Mittel.

Es können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Als Pflanzen, welche behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (beispielsweise Bananenbäume und -plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen..

Beispielhaft, aber nicht begrenzend, seien einige Erreger von pilzlichen Erkrankungen, die behandelt werden können, genannt:
and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die mit Hilfe der erfindungsgemäßen Verbindungen hergestellten Wirkstoffe weisen auch eine sehr gute stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze und Bakterien zu verstehen. Die mit Hilfe der erfindungsgemäßen Verbindungen hergestellten Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Dabei lassen sich die mit Hilfe der erfindungsgemäßen Verbindungen hergestellten Wirkstoffe mit besonders gutem Erfolg zur Bekämpfung von Krankheiten im Wein-, Obst-, Kartoffel- und Gemüseanbau, wie beispielsweise insbesondere gegen falsche Mehltaupilze, Oomyceten, wie beispielsweise Phytophthora-, Plasmopara-, Pseudoperonospora- und Pythium-Arten, einsetzen.

Die mit Hilfe der erfindungsgemäßen Verbindungen hergestellten Wirkstoffe eignen sich auch zur Steigerung des Ernteertrages. Sie sind außerdem mindertoxisch und weisen eine gute Pflanzenverträglichkeit auf.

Die mit Hilfe der erfindungsgemäßen Verbindungen hergestellten Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Insektizide verwenden. Sie lassen sich gegebenenfalls auch als Zwischen- oder Vorprodukte für die Synthese weiterer Wirkstoffe einsetzen.

Die mit Hilfe der erfindungsgemäßen Verbindungen hergestellten Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes in der Landwirtschaft, im Gartenbau, bei der Tierzucht, in Forsten, in Gärten und Freizeiteinrichtungen, im Vorrats- und Materialschutz sowie auf dem Hygienesektor. Sie können vorzugsweise als Pflanzenschutzmittel eingesetzt werden. Sie sind gegen normal sensible und resistente Arten sowie gegen alle oder einzelne Entwicklungsstadien wirksam.

Die Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die mit Hilfe der erfindungsgemäßen Verbindungen hergestellten Wirkstoffe bzw. Mittel können außerdem im Materialschutz zum Schutz von technischen Materialien gegen Befall und Zerstörung durch unerwünschte Mikroorganismen, wie z.B. Pilzen, eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch mit Hilfe der erfindungsgemäßen Verbindungen hergestellte Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die Wirkstoffe gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Die mit Hilfe der erfindungsgemäßen Verbindungen hergestellten Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Als Mischpartner kommen zum Beispiel bekannte Fungizide, Insektizide, Akarizide, Nematizide oder auch Bakterizide (siehe auch Pesticide Manual, 14th ed.) infrage.

Auch eine Mischung mit anderen bekannten Wirkstoffen, wie Herbiziden, oder mit Düngemitteln und Wachstumsregulatoren, Safenern bzw. Semiochemicals ist möglich.

Die Anwendung geschieht in einer den Anwendungsformen angepaßten üblichen Weise.

Die mit Hilfe der erfindungsgemäßen Verbindungen hergestellten Wirkstoffe bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von pflanzenpathogenen Schadpilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt wird, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die Bekämpfung von tierischen Schädlingen und/oder pflanzenpathogenen Schadpilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der mit Hilfe der erfindungsgemäßen Verbindungen hergestellten Mittel die Behandlung des Saatguts mit diesen Mitteln nicht nur das Saatgut selbst, sondern auch die daraus hervorgehenden Pflanzen nach dem Auflaufen vor tierischen Schädlingen und/oder pflanzenpathogenen Schadpilzen schützt. Auf diese Weise kann die unmittelbare Behandlung der Kultur zum Zeitpunkt der Aussaat oder kurz danach entfallen.

Ebenso ist es als vorteilhaft anzusehen, dass die mit Hilfe der erfindungsgemäßen Verbindungen hergestellten Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den mit Hilfe der erfindungsgemäßen Verbindungen hergestellten Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die mit Hilfe der erfindungsgemäßen Verbindungen hergestellten Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (wie Weizen, Gerste, Roggen, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Gemüse (wie Tomate, Gurke, Zwiebeln und Salat), Rasen und Zierpflanzen. Besondere Bedeutung kommt der Behandlung des Saatguts von Getreide (wie Weizen, Gerste, Roggen und Hafer), Mais und Reis zu.

Wie auch weiter unten beschrieben, ist die Behandlung von transgenem Saatgut mit den mit Hilfe der erfindungsgemäßen Verbindungen hergestellten Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus or Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis.

Das mit Hilfe der erfindungsgemäßen Verbindungen hergestellte Mittel kann alleine oder in einer geeigneten Formulierung auf das Saatgut aufgebracht werden. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten mit Hilfe der erfindungsgemäßen Verbindungen hergestellten Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die mit Hilfe der erfindungsgemäßen Verbindungen hergestellten Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417, US 4,245,432, US 4,808,430, US 5,876,739, US 2003/0176428, WO 2002/080675, WO 2002/028186.

Die mit Hilfe der erfindungsgemäßen Verbindungen hergestellten Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe oder Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den Beizmittel-Formulierungen kann innerhalb eines größeren Bereiches variiert werden. Sie richtet sich nach dem jeweiligen Gehalt der Wirkstoffe in den Formulierungen und nach dem Saatgut. Die Aufwandmengen an Wirkstoffkombination liegen im Allgemeinen zwischen 0,001 und 50 g pro Kilogramm Saatgut, vorzugsweise zwischen 0,01 und 15 g pro Kilogramm Saatgut.

Darüber hinaus weisen die mit Hilfe der erfindungsgemäßen Verbindungen hergestelltenVerbindungen der Formel **(I)** auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe der Formel (**I**) können daher sowohl in medizinischen als auch in nicht-medizinischen Anwendungen eingesetzt werden.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der Wirkstoffe als Fungizide können die Aufwandmengen je nach Applikationsart innerhalb eines größeren Bereiches variiert werden. Die Aufwandmenge der Wirkstoffe beträgt
- bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
- bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
- bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Die Anwendung der Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die mit Hilfe der erfindungsgemäßen Verbindungen hergestelltenVerbindungen der Formel **(I)** zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiterhin können die Verbindungen der Formel **(I)** allein oder in Kombinationen mit anderen Wirkstoffen als Antifouling-Mittel eingesetzt werden.

In gewissen Aufwandmengen können die Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die mit Hilfe der erfindungsgemäßen Verbindungen hergestellten Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im Allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls behandelt werden können, sind solche Pflanzen, die gegen einen oder mehrere abiotische Streßfaktoren resistent sind. Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosate-acetyltransferase-Enzym kodiert. Glyphosatetolerante Pflanzen können auch dadurch erhalten werden, daß man Pflanzen mit natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil_Crickmore/Bt/ beschrieben sind, zusammengestellt wurden, oder insektizide Teile davon, z.B. Proteine der Cry-Proteinklassen CrylAb, CrylAc, CrylF, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein CrylA.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bbl in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemischphysikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder - pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alteman produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls behandelt werden können, sind Pflanzen wie Raps oder verwandte Brassica-Pflanzen mit veränderten Eigenschaften der Ölzusammensetzung. Solche Pflanzen können durch

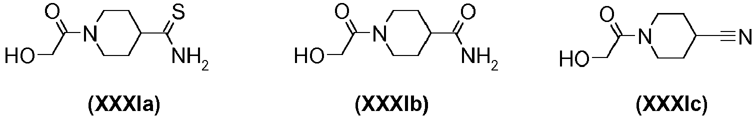

Die mit Hilfe der erfindungsgemäßen Verbindungen (**VIIIa**) und (**VIIIb**) hergestellten Stoffe der Formel **(I)** weisen eine starke mikrobizide Wirkung auf und können zur Bekämpfung von unerwünschten Mikroorganismen, wie Fungi und Bakterien, im Pflanzenschutz und im Materialschutz eingesetzt werden.

Die Heteroarylpiperidin und -piperazinderivate der Formel **(I)** besitzen sehr gute fungizide Eigenschaften und lassen sich im Pflanzenschutz beispielsweise zur Bekämpfung von Plasmodiophoromyceten, Oomyceten, Chytridiomyceten, Zygomyceten, Ascomyceten, Basidiomyceten und Deuteromyceten einsetzen.

Bakterizide lassen sich im Pflanzenschutz beispielsweise zur Bekämpfung von Pseudomonadaceae, Rhizobiaceae, Enterobacteriaceae, Corynebacteriaceae und Streptomycetaceae einsetzen.

Die mit Hilfe der erfindungsgemäßen Verbindungen hergestellten fungiziden Mittel können zur Bekämpfung von phytopathogenen Pilzen kurativ oder protektiv eingesetzt werden. Die Mittel zum Bekämpfen von phytopathogenen Pilzen im Pflanzenschutz umfassen eine wirksame, aber nicht-phytotoxische Menge der Wirkstoffe. "Wirksame, aber nicht-phytotoxische Menge" bedeutet eine Menge des Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der fungiziden Mittel.

Es können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Als Pflanzen, welche behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (beispielsweise Bananenbäume und -plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen..

Beispielhaft, aber nicht begrenzend, seien einige Erreger von pilzlichen Erkrankungen, die behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi oder Phakopsora meibomiae; Puccinia-Arten, wie beispielsweise Puccinia recondita, Puccinia graminis oder Puccinia striiformis; Uromyces-Arten, wie beispielsweise Uromyces appendiculatus;
Erkrankungen, hervorgerufen durch Erreger der Gruppe der Oomyceten wie z.B. Albugo-Arten, wie beispielsweise Albugo candida; Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladosporium-Arten, wie beispielsweise Cladosporium cucumerinum; Cochliobolus-Arten, wie beispielsweise Cochliobolus sativus (Konidienform: Drechslera, Syn: Helminthosporium) oder Cochliobolus miyabeanus; Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola, Mycosphaerella arachidicola oder Mycosphaerella fijiensis; Phaeosphaeria-Arten, wie beispielsweise Phaeosphaeria nodorum; Pyrenophora-Arten, wie beispielsweise Pyrenophora teres oder Pyrenophora tritici repentis; Ramularia-Arten, wie beispielsweise Ramularia collo-cygni oder Ramularia areola; Rhynchosporium-Arten, wie beispielsweise Rhynchosporium secalis; Septoria-Arten, wie beispielsweise Septoria apii oder Septoria lycopersici; Stagonospora-Arten, wie beispielsweise Stagonospora nodorum; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Plasmodiophora-Arten, wie beispielsweise Plasmodiophora brassicae; Rhizoctonia-Arten, wie beispielsweise Rhizoctonia solani; Sarocladium-Arten, wie beispielsweise Sarocladium oryzae; Sclerotium-Arten, wie beispielsweise Sclerotium oryzae; Tapesia-Arten, wie beispielsweise Tapesia acuformis; Thielaviopsis-Arten, wie beispielsweise Thielaviopsis basicola;
Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Stagonospora-Arten, wie beispielsweise Stagonospora nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries oder Tilletia controversa; Urocystis-Arten, wie beispielsweise Urocystis occulta; Ustilago-Arten, wie beispielsweise Ustilago nuda;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum oder Penicillium purpurogenum; Rhizopus -Arten, wie beispielsweise Rhizopus stolonifer; Sclerotinia-Arten, wie beispielsweise Sclerotinia sclerotiorum; Verticilium-Arten, wie beispielsweise Verticilium alboatrum;
Samen- und bodenbürtige Fäulen und Welken, sowie Sämlingserkrankungen, hervorgerufen durch z.B. Alternaria-Arten, wie beispielsweise Alternaria brassicicola; Aphanomyces-Arten, wie beispielsweise Aphanomyces euteiches; Ascochyta-Arten, wie beispielsweise Ascochyta lentis; Aspergillus-Arten, wie beispielsweise Aspergillus flavus; Cladosporium-Arten, wie beispielsweise Cladosporium herbarum; Cochliobolus-Arten, wie beispielsweise Cochliobolus sativus (Konidienform: Drechslera, Bipolaris Syn: Helminthosporium); Colletotrichum-Arten, wie beispielsweise Colletotrichum coccodes; Fusarium-Arten, wie beispielsweise Fusarium culmorum; Gibberella-Arten, wie beispielsweise Gibberella zeae; Macrophomina-Arten, wie beispielsweise Macrophomina phaseolina; Microdochium-Arten, wie beispielsweise Microdochium nivale; Monographella-Arten, wie beispielsweise Monographella nivalis; Penicillium-Arten, wie beispielsweise Penicillium expansum; Phoma-Arten, wie beispielsweise Phoma lingam; Phomopsis-Arten, wie beispielsweise Phomopsis sojae; Phytophthora Arten, wie beispielsweise Phytophthora cactorum; Pyrenophora-Arten, wie beispielsweise Pyrenophora graminea; Pyricularia-Arten, wie beispielsweise Pyricularia oryzae; Pythium-Arten, wie beispielsweise Pythium ultimum; Rhizoctonia-Arten, wie beispielsweise Rhizoctonia solani; Rhizopus-Arten, wie beispielsweise Rhizopus oryzae; Sclerotium-Arten, wie beispielsweise Sclerotium rolfsii; Septoria-Arten, wie beispielsweise Septoria nodorum; Typhula-Arten, wie beispielsweise Typhula incarnata; Verticillium-Arten, wie beispielsweise Verticillium dahliae;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Exobasidium-Arten, wie beispielsweise Exobasidium vexans; Taphrina-Arten, wie beispielsweise Taphrina deformans;
Degenerationserkrankungen holziger pflanzen, hervorgerufen durch z.B. Esca-Arten, wie beispielsweise Phaeomoniella chlamydospora, Phaeoacremonium aleophilum oder Fomitiporia mediterranea; Ganoderma-Arten, wie beispielsweise Ganoderma boninense;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora;
Bevorzugt können die folgenden Krankheiten von Soja-Bohnen bekämpft werden:
   Pilzkrankheiten an Blättern, Stängeln, Schoten und Samen verursacht durch z.B. Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).

Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die mit Hilfe der erfindungsgemäßen Verbindungen hergestellten Wirkstoffe weisen auch eine sehr gute stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze und Bakterien zu verstehen. Die mit Hilfe der erfindungsgemäßen Verbindungen hergestellten Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Dabei lassen sich die mit Hilfe der erfindungsgemäßen Verbindungen hergestellten Wirkstoffe mit besonders gutem Erfolg zur Bekämpfung von Krankheiten im Wein-, Obst-, Kartoffel- und Gemüseanbau, wie beispielsweise insbesondere gegen falsche Mehltaupilze, Oomyceten, wie beispielsweise Phytophthora-, Plasmopara-, Pseudoperonospora- und Pythium-Arten, einsetzen.

Die mit Hilfe der erfindungsgemäßen Verbindungen hergestellten Wirkstoffe eignen sich auch zur Steigerung des Ernteertrages. Sie sind außerdem mindertoxisch und weisen eine gute Pflanzenverträglichkeit auf.

Die mit Hilfe der erfindungsgemäßen Verbindungen hergestellten Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Insektizide verwenden. Sie lassen sich gegebenenfalls auch als Zwischen- oder Vorprodukte für die Synthese weiterer Wirkstoffe einsetzen.

Die mit Hilfe der erfindungsgemäßen Verbindungen hergestellten Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes in der Landwirtschaft, im Gartenbau, bei der Tierzucht, in Forsten, in Gärten und Freizeiteinrichtungen, im Vorrats- und Materialschutz sowie auf dem Hygienesektor. Sie können vorzugsweise als Pflanzenschutzmittel eingesetzt werden. Sie sind gegen normal sensible und resistente Arten sowie gegen alle oder einzelne Entwicklungsstadien wirksam.

Die Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die mit Hilfe der erfindungsgemäßen Verbindungen hergestellten Wirkstoffe bzw. Mittel können außerdem im Materialschutz zum Schutz von technischen Materialien gegen Befall und Zerstörung durch unerwünschte Mikroorganismen, wie z.B. Pilzen, eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch mit Hilfe der erfindungsgemäßen Verbindungen hergestellte Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die Wirkstoffe gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Die mit Hilfe der erfindungsgemäßen Verbindungen hergestellten Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Als Mischpartner kommen zum Beispiel bekannte Fungizide, Insektizide, Akarizide, Nematizide oder auch Bakterizide (siehe auch Pesticide Manual, 14th ed.) infrage.

Auch eine Mischung mit anderen bekannten Wirkstoffen, wie Herbiziden, oder mit Düngemitteln und Wachstumsregulatoren, Safenern bzw. Semiochemicals ist möglich.

Die Anwendung geschieht in einer den Anwendungsformen angepaßten üblichen Weise.

Die mit Hilfe der erfindungsgemäßen Verbindungen hergestellten Wirkstoffe bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von pflanzenpathogenen Schadpilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt wird, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die Bekämpfung von tierischen Schädlingen und/oder pflanzenpathogenen Schadpilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der mit Hilfe der erfindungsgemäßen Verbindungen hergestellten Mittel die Behandlung des Saatguts mit diesen Mitteln nicht nur das Saatgut selbst, sondern auch die daraus hervorgehenden Pflanzen nach dem Auflaufen vor tierischen Schädlingen und/oder pflanzenpathogenen Schadpilzen schützt. Auf diese Weise kann die unmittelbare Behandlung der Kultur zum Zeitpunkt der Aussaat oder kurz danach entfallen.

Ebenso ist es als vorteilhaft anzusehen, dass die mit Hilfe der erfindungsgemäßen Verbindungen hergestellten Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den mit Hilfe der erfindungsgemäßen Verbindungen hergestellten Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die mit Hilfe der erfindungsgemäßen Verbindungen hergestellten Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (wie Weizen, Gerste, Roggen, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Gemüse (wie Tomate, Gurke, Zwiebeln und Salat), Rasen und Zierpflanzen. Besondere Bedeutung kommt der Behandlung des Saatguts von Getreide (wie Weizen, Gerste, Roggen und Hafer), Mais und Reis zu.

Wie auch weiter unten beschrieben, ist die Behandlung von transgenem Saatgut mit den mit Hilfe der erfindungsgemäßen Verbindungen hergestellten Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus or Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis.

Das mit Hilfe der erfindungsgemäßen Verbindungen hergestellte Mittel kann alleine oder in einer geeigneten Formulierung auf das Saatgut aufgebracht werden. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten mit Hilfe der erfindungsgemäßen Verbindungen hergestellten Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die mit Hilfe der erfindungsgemäßen Verbindungen hergestellten Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417, US 4,245,432, US 4,808,430, US 5,876,739, US 2003/0176428, WO 2002/080675, WO 2002/028186.

Die mit Hilfe der erfindungsgemäßen Verbindungen hergestellten Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe oder Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den Beizmittel-Formulierungen kann innerhalb eines größeren Bereiches variiert werden. Sie richtet sich nach dem jeweiligen Gehalt der Wirkstoffe in den Formulierungen und nach dem Saatgut. Die Aufwandmengen an Wirkstoffkombination liegen im Allgemeinen zwischen 0,001 und 50 g pro Kilogramm Saatgut, vorzugsweise zwischen 0,01 und 15 g pro Kilogramm Saatgut.

Darüber hinaus weisen die mit Hilfe der erfindungsgemäßen Verbindungen hergestelltenVerbindungen der Formel **(I)** auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe der Formel (**I**) können daher sowohl in medizinischen als auch in nicht-medizinischen Anwendungen eingesetzt werden.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der Wirkstoffe als Fungizide können die Aufwandmengen je nach Applikationsart innerhalb eines größeren Bereiches variiert werden. Die Aufwandmenge der Wirkstoffe beträgt
- bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
- bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
- bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt.

Die Anwendung der Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die mit Hilfe der erfindungsgemäßen Verbindungen hergestelltenVerbindungen der Formel **(I)** zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiterhin können die Verbindungen der Formel **(I)** allein oder in Kombinationen mit anderen Wirkstoffen als Antifouling-Mittel eingesetzt werden.

In gewissen Aufwandmengen können die Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die mit Hilfe der erfindungsgemäßen Verbindungen hergestellten Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im Allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls behandelt werden können, sind solche Pflanzen, die gegen einen oder mehrere abiotische Streßfaktoren resistent sind. Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosate-acetyltransferase-Enzym kodiert. Glyphosatetolerante Pflanzen können auch dadurch erhalten werden, daß man Pflanzen mit natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil_Crickmore/Bt/ beschrieben sind, zusammengestellt wurden, oder insektizide Teile davon, z.B. Proteine der Cry-Proteinklassen CrylAb, CrylAc, CrylF, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein CrylA.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bbl in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemischphysikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder - pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alteman produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produzieren;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sulfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die aufgeführten Pflanzen können besonders vorteilhaft mit den Verbindungen der allgemeinen Formel (**I**) bzw. den Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die mit Hilfe der erfindungsgemäßen Verbindungen hergestellten Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Die Herstellung und die Verwendung der Wirkstoffe der Formeln (**I**) gehen aus den folgenden Beispielen hervor. Die Erfindung ist jedoch nicht auf diese Beispiele beschränkt.

### Herstellungsbeispiele

**Allgemeines:** Wenn nicht anders angegeben, werden alle chromatografischen Reinigungs- bzw. Trennungsschritte an Kieselgel und mit einem Lösungsmittelgradienten von 0:100 Essigsäure-ethylester/Cyclohexan zu 100:0 Essigsäure-ethylester/Cyclohexan durchgeführt.

### Herstellung von Verbindungen der Formel (I)

### 2-{3-[2-(1-{[(Propan-2-ylidenamino)oxy]acetyl}piperidin-4-yl)-1,3-thiazol-4-yl]-1,2-oxazol-5-yl}benzaldehyd (I-8)

Zu einer Lösung *tert*-Butyl-4-{4-[5-(2-formylphenyl)-1,2-oxazol-3-yl]-1,3-thiazol-2-yl}piperidin-1-carboxylat (2,20 g) in 1,4-Dioxan wurde bei 0 °C eine 4-molare Lösung von Chlorwasserstoff (12 mL) in 1,4-Dioxan getropft. Das Reaktionsgemisch wurde bei 0 °C gerührt und dann langsam auf Raumtemperatur erwärmt. Nach Rühren über Nacht wurden das Lösungsmittel und überschüssiger Chlorwasserstoff entfernt. Man erhielt 4-{4-[5-(2-Formylphenyl)-1,2-oxazol-3-yl]-1,3-thiazol-2-yl}piperidiniumchlorid (**XIIIa-99**, 2,0 g).

Zu einer Lösung von [(Propan-2-ylidenamino)oxy]essigsäure (185 mg) in Dichlormethan (10 mL) wurden bei 0 °C Oxalylchlorid (168 µL) und ein Tropfen N,N-Dimethylformamid gegeben. Das Reaktionsgemisch wurde bei Raumtemperatur für 120 Minuten gerührt. Danach wurden das Lösungsmittel und das überschüssige Reagenz unter vermindertem Druck entfernt. Der feste Rückstand wurde erneut in Dichlormethan gelöst und bei 0 °C tropfenweise zu einer Lösung von 4-{4-[5-(2-Formylphenyl)-1,2-oxazol-3-yl]-1,3-thiazol-2-yl}piperidiniumchlorid (484 mg) und Triethylamin (357 µL) in Dichlormethan (10 mL) gegeben. Die Reaktionsmischung wurde für 1 Stunde bei Raumtemperatur gerührt. Daraufhin wurde sie mit konzentrierter Natriumhydrogencarbonatlösung versetzt, die wässrige Phase abgetrennt und mit Essigsäureethylester extrahiert. Die vereinigten organischen Phasen wurden über Natriumsulfat getrocknet und eingeengt. Nach säulenchromatographischer Reinigung erhielt man 2-{3-[2-(1-{[(Propan-2-ylidenamino)oxy]acetyl}piperidin-4-yl)-1,3-thiazol-4-yl]-1,2-oxazol-5-yl}benzaldehyd (150 mg).

### 2-{3-[2-(1-{[(Propan-2-ylidenamino)oxy]acetyl}piperidin-4-yl)-1,3-thiazoL-4-yl]-4,5-dihydro-1,2-oxazol-5-yl}phenylmethansulfonat (I-12)

Zu einer Lösung von Acetonoxim (9,3 mg) in N,N-Dimethylformamid (0.28 mL) gab man bei Raumtemperatur 3Å Molekularsieb und rührte 2 Stunden lang bei dieser Temperatur. Dazu gab man danach 2-(3-{2-[1-(Chloracetyl)piperidin-4-yl]-1,3-thiazol-4-yl}-4,5-dihydro-1,2-oxazol-5-yl)phenylmethansulfonat (50 mg) und Caesiumcarbonat (64 mg) und rührte 18 Stunden lang bei Raumtemperatur. Anschließend filtrierte man das Gemisch und extrahierte die Mischung zweimal mit Essigsäureethylester. Die vereinigten organischen Phasen wurden über Magnesiumsulfat getrocknet und eingeengt. Nach säulenchromatographischer Reinigung erhielt man 2-{3-[2-(1-{[(Propan-2-ylidenamino)oxy]acetyl}piperidin-4-yl)-1,3-thiazol-4-yl]-4,5-dihydro-1,2-oxazol-5-yl}phenylmethansulfonat (5 mg, 10%).

### Herstellung von Verbindungen der Formel (IV)

### [(Propan-2-ylidenamino)oxy]essigsäure (IV-1)

Eine Mischung von (Aminooxy)essigsäure-hemihydrochlorid (2,51 g) und Aceton (6,0 g) wurde bei Raumtemperatur für 64 Stunden gerührt. Anschließend wurde die Mischung mit Dichlormethan (10 ml) versetzt. Danach wurden das Lösungsmittel und das überschüssige Reagenz unter vermindertem Druck entfernt. Man erhielt [(Propan-2-ylidenamino)oxy]essigsäure (3,1 g), das ohne weitere Reinigung weiter umgesetzt wurde.

### ({[1-(4-Fluorphenyl)ethyliden]amino}oxy)essigsäure (IV-2)

### Schritt 1:

Eine Mischung von 1-(4-Fluorphenyl)ethanonoxim (8,00 g) und Caesiumcarbonat (20,4 g) in Acetonitril wurde 30 Minuten lang bei 20 °C gerührt. Dazu gab man danach Ethylbromacetat (12,2 g) und Kaliumiodid (8,7 g) und rührte 3 Stunden lang bei 82 °C. Danach wurde das Reaktionsgemisch filtriert. Vom Filtrat wurde das Lösungsmittel unter vermindertem Druck entfernt. Nach säulenchromatographischer Reinigung erhielt man Ethyl-({[1-(4-fluorphenyl)ethyliden]amino}oxy)acetat (8,7 g).

### Schritt 2:

Zu einer Lösung von Ethyl-({[1-(4-fluorphenyl)ethyliden]amino}oxy)acetat (8,7 g) in einer Mischung aus 50 ml Tetrahydrofuran und 10 ml Wasser gab man bei 20 °C Lithiumhydroxid-monohydrat (2,3 g) und rührte 18 Stunden lang bei dieser Temperatur. Anschließend rührte man das Gemisch in eine eiskalte 10%ige Salzsäure ein und extrahierte die Mischung zweimal mit Essigsäureethylester (je 50 ml). Die vereinigten organischen Phasen wurden über Natriumsulfat getrocknet und eingeengt. Nach säulenchromatographischer Reinigung an Kieselgel mit einem Lösungsmittelgradienten von 0:100 Methanol/Dichlormethan zu 60:0 Methanol/Dichlormethan erhielt man ({[1-(4-Fluorphenyl)-ethyliden]amino}oxy)essigsäure (2,9 g).

### Herstellung von Verbindungen der Formel (X)

### 2-(3-{2-[1-(N,N-Dimethylglycyl)piperidin-4-yl]-1,3-thiazol-4-yl}-4,5-dihydro-1,2-oxazol-5-yl)phenylmethansulfonat (X-1)

Zu 4-[4-(5-{2-[(Methylsulfonyl)oxy]phenyl}-4,5-dihydro-1,2-oxazol-3-yl)-1,3-thiazol-2-yl]piperidiniumchlorid (500 mg) in Dimethylformamid (6 mL) wurden unter Argon N,N-Dimethylglycin (122 mg), Diisopropylethylamin (582 mg) und O-(Benzotriazol-1-yl)-N,N,N',N'-tetramethyluronium tetrafluoroborate (TBTU, 542 mg) gegeben. Die Reaktionsmischung wurde für 18 Stunden bei Raumtemperatur gerührt. Daraufhin wurde sie mit eiskalter Natriumhydrogencarbonat-Lösung versetzt, filtriert, die wässrige Phase abgetrennt und mit Essigsäureethylester extrahiert. Die vereinigten organischen Phasen wurden über Magnesiumsulfat getrocknet und eingeengt. Man erhielt 2-(3-{2-[1-(N,N-Dimethylglycyl)piperidin-4-yl]-1,3-thiazol-4-yl}-4,5-dihydro-1,2-oxazol-5-yl)phenylmethansulfonat (310 mg, 55%).
LogP (pH2.7): 1,48

### 2-(3-12-[1-({[tert-Butyl(dimethyl)silyl]oxy}acetyl)piperidin-4-yl]-1,3-thiazol-4-yl}-4,5-dihydro-1,2-oxazol-5-yl)phenylmethansulfonat (X-2)

Zu 4-[4-(5-{2-[(Methylsulfonyl)oxy]phenyl}-4,5-dihydro-1,2-oxazol-3-yl)-1,3-thiazol-2-yl]piperidiniumchlorid (500 mg) in Dimethylformamid (6 mL) wurden unter Argon {[tert-Butyl(dimethyl)silyl]oxy}essigsäure (225 mg), Diisopropylethylamin (582 mg) und O-(Benzotriazol-1-yl)-N,N,N',N'-tetramethyluronium tetrafluoroborate (TBTU, 542 mg) gegeben. Die Reaktionsmischung wurde für 18 Stunden bei Raumtemperatur gerührt. Daraufhin wurde sie mit eiskalter Natriumhydrogencarbonat-Lösung versetzt, filtriert, die wässrige Phase abgetrennt und mit Essigsäureethylester extrahiert. Die vereinigten organischen Phasen wurden über Magnesiumsulfat getrocknet und eingeengt. Nach säulenchromatographischer Reinigung erhielt man 2-(3-{2-[1-({[tert-Butyl(dimethyl)silyl]oxy}acetyl)piperidin-4-yl]-1,3-thiazol-4-yl}-4,5-dihydro-1,2-oxazol-5-yl)phenylmethansulfonat (150 mg, 22%).
LogP (pH2.7): 4,14

### 2-{4-[4-(5-{2-[(Methylsulfonyl)oxy]phenyl}-4,5-dihydro-1,2-oxazol-3-yl)-1,3-thiazol-2-yl]piperidin-1-yl}-2-oxoethylacetat (X-3)

Zu 4-[4-(5-{2-[(Methylsulfonyl)oxy]phenyl}-4,5-dihydro-1,2-oxazol-3-yl)-1,3-thiazol-2-yl]piperidiniumchlorid (500 mg) in Dichlormethan (6 mL) wurden unter Argon 2-Chlor-2-oxoethylacetat (154 mg) und Triethylamin (342 mg) gegeben. Die Reaktionsmischung wurde für 18 Stunden bei Raumtemperatur gerührt. Daraufhin wurde sie mit Wasser versetzt, filtriert, getrocknet und eingeengt. Nach säulenchromatographischer Reinigung erhielt man 2-{4-[4-(5-{2-[(Methylsulfonyl)oxy]phenyl}-4,5-dihydro-1,2-oxazol-3-yl)-1,3-thiazol-2-yl]piperidin-1-yl}-2-oxoethylacetat (170 mg, 30%).
LogP (pH2.7): 2,18

### 2-(3-{2-[1-(Chloracetyl)piperidin-4-yl]-1,3-thiazol-4-yl}-4,5-dihydro-1,2-oxazol-5-yl)phenylmethansulfonat (Xc-a-142)

Zu einer Lösung von Chloressigsäurechlorid (22 mg) wurde bei 0 °C eine Lösung von 4-[4-(5-{2-[(Methylsulfonyl)oxy]phenyl}-4,5-dihydro-1,2-oxazol-3-yl)-1,3-thiazol-2-yl]piperidiniumchlorid (87 mg) und Triethylamin (41 mg) in Dichlormethan (1 mL) gegeben. Die Reaktionsmischung wurde für 15 Minuten bei 0 °C gerührt, und für 18 Stunden bei Raumtemparatur nachgerührt. Daraufhin wurde sie mit Wasser versetzt, die wässrige Phase abgetrennt und mit Dichlormethan extrahiert. Die vereinigten organischen Phasen wurden über Magnesiumsulfat getrocknet und eingeengt. Nach säulenchromatographischer Reinigung erhielt man 2-(3-{2-[1-(Chloracetyl)piperidin-4-yl]-1,3-thiazol-4-yl}-4,5-dihydro-1,2-oxazol-5-yl)phenylmethansulfonat (60 mg, 60%).
LogP (pH2.7): 2,42
¹H NMR (250 MHz, CDCl₃): δₚₚₘ : 1.7-2.0 (m, 2H), 2.15-2.35 (m, 2H), 2.80-2.95 (m, 1H), 3.05-3.20 (m, 1H), 3.30 (s, 3H), 3.27-3.38 (m, 1H), 3.39-3.50 (dd, 1H), 3.85-3.97 (dd, 1H), 3.90-4.10 (m, 1H), 4.20 (s, 2H), 4.55-4.66 (m, 1H), 5.98-6.06 (dd, 1H), 7.30-7.42 (m, 3H), 7.55-7.62 (m, 1H), 7.62 (s, 1H)

### Herstellung von Verbindungen der Formel (XXVIIa)

### 2-{3-[2-(1-Glycoloylpiperidin-4-yl)-1,3-thiazol-4-yl]-4,5-dihydro-1,2-oxazol-5-yl}phenylmethansulfonat (XXVIIa-142)

Zu 4-[4-(5-{2-[(Methylsulfonyl)oxy]phenyl}-4,5-dihydro-1,2-oxazol-3-yl)-1,3-thiazol-2-yl]piperidiniumchlorid (110 mg) in Dimethylformamid (2.6 mL) wurden unter Argon Glycolsäure (19 mg), Diisopropylethylamin (32 mg) und O-(Benzotriazol-1-yl)-N,N,N',N'-tetramethyluronium tetrafluoroborate (TBTU, 159 mg) gegeben. Danach wurde zu der Reaktionmischung nochmals Diisopropylethylamin (64 mg) gegeben. Die Reaktionsmischung wurde für 1 Stunde bei Raumtemperatur gerührt. Daraufhin wurde sie mit eiskalter Natriumhydrogencarbonat-Lösung versetzt, filtriert, die wässrige Phase abgetrennt und mit Dichlormethan extrahiert. Die vereinigten organischen Phasen wurden über Magnesiumsulfat getrocknet und eingeengt. Nach säulenchromatographischer Reinigung erhielt man 2-{3-[2-(1-Glycoloylpiperidin-4-yl)-1,3-thiazol-4-yl]-4,5-dihydro-1,2-oxazol-5-yl}phenylmethansulfonat (15 mg, 12%).
LogP (pH2.7): 1,88
¹H NMR (500 MHz, CDCl₃): δₚₚₘ : 1.7-1.9 (m, 2H), 2.15-2.25 (m, 2H), 2.88-3.00 (m, 1H), 3.10-3.20 (m, 1H), 3.27 (s, 3H), 3.27-3.38 (m, 1H), 3.39-3.47 (dd, 1H), 3.62 (m, 1H), 3.89-3.97 (dd, 1H), 4.20 (s, 2H), 4.60-4.66 (m, 1H), 5.98-6.06 (dd, 1H), 7.30-7.40 (m, 3H), 7.55-7.60 (m, 1H), 7.61 (s, 1H)

### Herstellung von Verbindungen der Formel (XVI)

### tert-Butyl-4-[4-(5-{2-[(methylsulfonyl)oxy]-4-(trifluormethyl)phenyl}-4,5-dihydro-1,2-oxazol-3-yl)-1,3-thiazol-2-yl]piperidin-1-carboxylat (XVI-143)

Zu einer Lösung von 2-[3-(Chloracetyl)-4,5-dihydro-1,2-oxazol-5-yl]-5-(trifluormethyl)phenylmethan-sulfonat (200 mg) und *tert*-Butyl-4-carbamothioylpiperidin-1-carboxylat (108 mg) in Tetrahydrofuran (2 mL) wurde bei Raumtemperatur Tetrabutylammoniumbromid gegeben. Die Reaktionsmischung wurde für 12 Stunden bei Raumtemperatur gerührt. Daraufhin wurde sie mit Wasser versetzt, die wässrige Phase abgetrennt und mit Essigsäureethylester extrahiert. Die vereinigten organischen Phasen wurden über Magnesiumsulfat getrocknet und eingeengt. Nach säulenchromatographischer Reinigung erhielt man *tert-*Butyl-4-[4-(5-{2-[(methylsulfonyl)oxy]-4-(trifluormethyl)phenyl}-4,5-dihydro-1,2-oxazol-3-yl)-1,3-thiazol-2-yl]piperidin-1-carboxylat (168 mg, 56%).
LogP (pH2.7): 4,36

### Herstellung von Verbindungen der Formel (VIIa)

### 2-[3-(Chloracetyl)-4,5-dihydro-1,2-oxazol-5-yl]-5-(trifluormethyl)phenylmethansulfonat (VIIa-a-143)

Zu einer Lösung von 5-(Trifluormethyl)-2-vinylphenylmethansulfonat (1,05 g) in Acetonitril (10 mL) wurden Natriumhydrogencarbonat (2,55 g) und 3-Chlor-N-hydroxy-2-oxopropanimidoylchlorid (0,60 g) bein Raumtemperatur unter Argon gegeben. Die Reaktionsmishung wurde für ein Stunde bei Raumtemperatur gerührt. Der Feststoff wurde abgesaugt und das Filtrat im Vakuum eingeengt. Der Rückstand wurde mit Heptan verrührt und man erhielt 2-[3-(Chloracetyl)-4,5-dihydro-1,2-oxazol-5-yl]-5-(trifluormethyl)phenylmethansulfonat (1,33 g, 86% pur, 75%).
LogP (pH2.7): 3,25

### Herstellung von Verbindungen der Formel (VIII)

### 1-[5-(2-{[Prop-2-in-1-yl]oxy}pheny|)-4,5-dihydro-1,2-oxazol-3-yl]ethanon (VIIIa-a-81) Schritt 1:

Zu einer Lösung von 3,3-Dimethoxybutan-2-on (1,00 g) in Ethanol (10 mL) wurde Hydroxylamin (50% in Wasser, 0,23 mL) bei Raumtemperatur gegeben. Das Reaktiongemisch wurde bei 50 °C für 4 Stunden gerührt. Daraufhin wurde sie mit Wasser versetzt, die wässrige Phase abgetrennt und mit Essigsäureethylester extrahiert. Die vereinigten organischen Phasen wurden über Magnesiumsulfat getrocknet und eingeengt. Man erhielt 3,3-Dimethoxybutan-2-onoxim (800 mg, 72%).

### Schritt 2:

Zu einer Lösung von 3,3-Dimethoxybutan-2-onoxim (270 mg) in Tetrahydrofuran (2,7 mL) wurde bei 0°C unter Argon n-Butyllithium (2M in Tetrahydrofuran, 1,83 mL) getropft. Nach 5 Minuten nachrühren wurde zu der Reaktionsmischung eine Lösung von 2-{[3-(Trimethylsilyl)prop-2-in-1-yl]oxy}benzaldehyd (232 mg) in Tetrahydrofuran (1 mL) getropft, und für 1 Stunden nachgerührt. Anschließend wurde das Reaktiongemisch mit konzentrierter Ammoniumchloridlösung versetzt, die wässrige Phase abgetrennt und mit Essigsäureethylester extrahiert. Die vereinigten organischen Phasen wurden über Magnesiumsulfat getrocknet und eingeengt. Nach säulenchromatographischer Reinigung erhielt man 1-Hydroxy-4,4-dimethoxy-1-(2-{[3-(trimethylsilyl)prop-2-in-1-yl]oxy}phenyl)pentan-3-onoxim (482 mg, 69%).
LogP (pH2.7): 3,19

### Schritt 3:

Eine Lösung von Salzsäure (4M in Dioxan, 3,80 mL) wurde zu 1-Hydroxy-4,4-dimethoxy-1-(2-{[3-(trimethylsilyl)prop-2-in-1-yl]oxy}phenyl)pentan-3-onoxim gegeben. Nach 15 Minuten nachrühren wurde die Reaktionsmischung mit konzentrierter Natriumhydrogencarbonatlösung versetzt, die wässrige Phase abgetrennt und mit Dichlormethan extrahiert. Die vereinigten organischen Phasen wurden über Magnesiumsulfat getrocknet und eingeengt. Man erhielt 1-[5-(2-{[Prop-2-in-1-yl]oxy}phenyl)-4,5-dihydro-1,2-oxazol-3-yl]ethanon (315 mg, 99%).
LogP (pH2.7): 4,41

### Verbindungsbeispiele

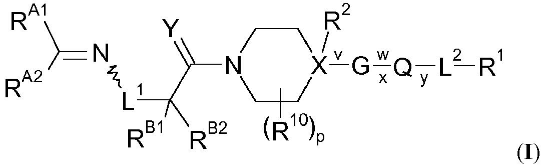

Die in Tabelle 1 aufgelisteteten Strukturelemente G und Q sind wie folgt definiert:
G= 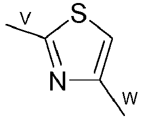 Q =

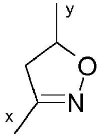

Für alle in Tabelle 1 aufgeführten Verbindungen, p=0 und L² = direkte Bindung

**Tabelle 1:**

| **Bsp**. | **R^{A1}** | **R^{A2}** | **L¹** | **R^{B1}** | **R^{B2}** | **Y** | **X** | **R²** | **R¹** | **Log P** |
|---|---|---|---|---|---|---|---|---|---|---|
| **I-1** | CH₃ | 1,3-Benzodioxol-5-yl | O | H | H | O | C | H | 2-Fluor-6-(prop-2-in-1-yloxy)phenyl | 3,29^{[a]}; 3,28^{[b]} |
| **I-2** | Propan-2-yl | 4-Ethoxyphenyl | O | H | H | O | C | H | 2-Fluor-6-(prop-2-in-1-yloxy)phenyl | 4,19^{[a]}; 4,21^{[b]} |
| **I-3** | CH₃ | 3-Fluorphenyl | O | H | H | O | C | H | 2-Fluor-6-(prop-2-in-1-yloxy)phenyl | 3,48^{[al}; 3,51^{[b]} |
| **I-4** | CH₃ | 3,4-Dimethylphenyl | O | H | H | O | C | H | 2-Fluor-6-(prop-2-in-1-yloxy)phenyl | 3,93^{[a]}; 3,94^{[b]} |
| **I-5** | CH₃ | 3-(Trifluormethoxy )-phenyl | O | H | H | O | C | H | 2-Fluor-6-(prop-2-in-1-yloxy)phenyl | 4,01^{[a]}; 4,01^{[b]} |
| **I-6** | CH₃ | 3,4-Dimethylphenyl | O | H | H | O | C | H | 5-Fluor-2-(prop-2-in-1-yloxy)phenyl | 4,18^{[a]}; 4,19^{[b]} |
| **I-7** | Propan-2-yl | 4-Ethoxyphenyl | O | H | H | O | C | H | 5-Fluor-2-(prop-2-in-1-yloxy)phenyl | 4,42^{[a]}; 4,42^{[b]} |
| **I-8** | CH₃ | CH₃ | O | H | H | O | C | H | 2-Formylphenyl | 2,45^{[a]} |
| **I-9** | CH₃ | Trifluormethyl | O | H | H | O | C | H | 2-Fluor-6-[(methylsulfonyl)oxy]phenyl | 2,9^{[a]} |
| **I-10** | CH₃ | Trifluormethyl | O | H | H | O | C | H | 2-[(methylsulfonyl)oxy]phenyl | 2,94^{[a]} |
| **I-11** | CH₃ | CH₃ | O | H | H | O | C | H | 2-(Prop-2-in-1-yloxy)phenyl | 2,75^{[a]} |
| **I-12** | CH₃ | CH₃ | O | H | H | O | C | H | 2-[(methylsulfonyl)oxy]phenyl | 2,36^{[a]} |
| **I-13** | CH₃ | CH₃ | O | H | H | O | C | H | 2-Fluor-6-[(methylsulfonyl)oxy]phenyl | 2,34^{[a]} |
| **I-14** | CH₃ | 1,1-Dimethylethyl | O | H | H | O | C | H | 2-[(methylsulfonyl)oxy]phenyl | 3,42^{[a]} |

**Tabelle 2:**

| | |
|---|---|
| 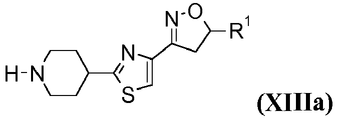 | |

| Bsp. | R¹ |
|---|---|
| XIIIa-1 | 2,3-Dichlor-4-(prop-2-in-1-yloxy)phenyl |
| XIIIa-2 | 2,3-Dichlor-4-[(methylsulfonyl)oxy]phenyl |
| XIIIa-3 | 2,3-Difluor-4-(prop-2-in-1-yloxy)phenyl |
| XIIIa-4 | 2,3-Difluor-4-[(methylsulfonyl)oxy]phenyl |
| XIIIa-5 | 2,3-Difluor-4-formylphenyl |
| XIIIa-6 | 2,4-Dichlor-3-(prop-2-in-1-yloxy)phenyl |
| XIIIa-7 | 2,4-Dichlor-3-[(methylsulfonyl)oxy]phenyl |
| XIIIa-8 | 2,4-Difluor-3-(prop-2-in-1-yloxy)phenyl |
| XIIIa-9 | 2,4-Difluor-3-[(methylsulfonyl)oxy]phenyl |
| XIIIa-10 | 2,4-Difluor-3-formylphenyl |
| XIIIa-11 | 2,5-Dichlor-3-(prop-2-in-1-yloxy)phenyl |
| XIIIa-12 | 2,5-Dichlor-3-[(methylsulfonyl)oxy]phenyl |
| XIIIa-13 | 2,5-Dichlor-4-(prop-2-in-1-yloxy)phenyl |
| XIIIa-14 | 2,5-Dichlor-4-[(methylsulfonyl)oxy]phenyl |
| XIIIa-15 | 2,5-Difluor-3-(prop-2-in-1-yloxy)phenyl |
| XIIIa-16 | 2,5-Difluor-3-[(methylsulfonyl)oxy]phenyl |
| XIIIa-17 | 2,5-Difluor-3-formylphenyl |
| XIIIa-18 | 2,5-Difluor-4-(prop-2-in-1-yloxy)phenyl |
| XIIIa-19 | 2,5-Difluor-4-[(methylsulfonyl)oxy]phenyl |
| XIIIa-20 | 2,5-Difluor-4-formylphenyl |
| XIIIa-21 | 2,6-Dichlor-3-(prop-2-in-1-yloxy)phenyl |
| XIIIa-22 | 2,6-Dichlor-3-[(methylsulfonyl)oxy]phenyl |
| XIIIa-23 | 2,6-Dichlor-4-(prop-2-in-1-yloxy)phenyl |
| XIIIa-24 | 2,6-Dichlor-4-[(methylsulfonyl)oxy]phenyl |
| XIIIa-25 | 2,6-Difluor-3-(prop-2-in-1-yloxy)phenyl |
| XIIIa-26 | 2,6-Difluor-3-[(methylsulfonyl)oxy]phenyl |
| XIIIa-27 | 2,6-Difluor-3-formylphenyl |
| XIIIa-28 | 2,6-Difluor-4-(prop-2-in-1-yloxy)phenyl |
| XIIIa-29 | 2,6-Difluor-4-[(methylsulfonyl)oxy]phenyl |
| XIIIa-30 | 2,6-Difluor-4-formylphenyl |
| XIIIa-31 | 2-(Allyloxy)-3,4-dichlorphenyl |
| XIIIa-32 | 2-(Allyloxy)-3,4-difluorphenyl |
| XIIIa-33 | 2-(Allyloxy)-3,5-dichlorphenyl |
| XIIIa-34 | 2-(Allyloxy)-3,5-difluorphenyl |
| XIIIa-35 | 2-(Allyloxy)-3,6-dichlorphenyl |
| XIIIa-36 | 2-(Allyloxy)-3,6-difluorphenyl |
| XIIIa-37 | 2-(Allyloxy)-3-chlorphenyl |
| XIIIa-38 | 2-(Allyloxy)-3-fluorphenyl |
| XIIIa-39 | 2-(Allyloxy)-3-methylphenyl |
| XIIIa-40 | 2-(Allyloxy)-4,5-dichlorphenyl |
| XIIIa-41 | 2-(Allyloxy)-4,5-difluorphenyl |
| XIIIa-42 | 2-(Allyloxy)-4,6-dichlorphenyl |
| XIIIa-43 | 2-(Allyloxy)-4,6-difluorphenyl |
| XIIIa-44 | 2-(Allyloxy)-4-chlorphenyl |
| XIIIa-45 | 2-(Allyloxy)-4-fluorphenyl |
| XIIIa-46 | 2-(Allyloxy)-4-methylphenyl |
| XIIIa-47 | 2-(Allyloxy)-5,6-dichlorphenyl |
| XIIIa-48 | 2-(Allyloxy)-5,6-difluorphenyl |
| XIIIa-49 | 2-(Allyloxy)-5-chlorphenyl |
| XIIIa-50 | 2-(Allyloxy)-5-fluorphenyl |
| XIIIa-51 | 2-(Allyloxy)-5-methylphenyl |
| XIIIa-52 | 2-(Allyloxy)-6-chlorphenyl |
| XIIIa-53 | 2-(Allyloxy)-6-fluorphenyl |
| XIIIa-54 | 2-(Allyloxy)-6-methylphenyl |
| XIIIa-55 | 2-(Allyloxy)phenyl |
| XIIIa-56 | 2-(Cyanomethoxy)-3,4-dichlorphenyl |
| XIIIa-57 | 2-(Cyanomethoxy)-3,4-difluorphenyl |
| XIIIa-58 | 2-(Cyanomethoxy)-3,5-dichlorphenyl |
| XIIIa-59 | 2-(Cyanomethoxy)-3,5-difluorphenyl |
| XIIIa-60 | 2-(Cyanomethoxy)-3,6-dichlorphenyl |
| XIIIa-61 | 2-(Cyanomethoxy)-3,6-difluorphenyl |
| XIIIa-62 | 2-(Cyanomethoxy)-3-chlorphenyl |
| XIIIa-63 | 2-(Cyanomethoxy)-3-fluorphenyl |
| XIIIa-64 | 2-(Cyanomethoxy)-3-methylphenyl |
| XIIIa-65 | 2-(Cyanomethoxy)-4,5-dichlorphenyl |
| XIIIa-66 | 2-(Cyanomethoxy)-4,5-difluorphenyl |
| XIIIa-67 | 2-(Cyanomethoxy)-4,6-dichlorphenyl |
| XIIIa-68 | 2-(Cyanomethoxy)-4,6-difluorphenyl |
| XIIIa-69 | 2-(Cyanomethoxy)-4-chlorphenyl |
| XIIIa-70 | 2-(Cyanomethoxy)-4-fluorphenyl |
| XIIIa-71 | 2-(Cyanomethoxy)-4-methylphenyl |
| XIIIa-72 | 2-(Cyanomethoxy)-5,6-dichlorphenyl |
| XIIIa-73 | 2-(Cyanomethoxy)-5,6-difluorphenyl |
| XIIIa-74 | 2-(Cyanomethoxy)-5-chlorphenyl |
| XIIIa-75 | 2-(Cyanomethoxy)-5-fluorphenyl |
| XIIIa-76 | 2-(Cyanomethoxy)-5-methylphenyl |
| XIIIa-77 | 2-(Cyanomethoxy)-6-chlorphenyl |
| XIIIa-78 | 2-(Cyanomethoxy)-6-fluorphenyl |
| XIIIa-79 | 2-(Cyanomethoxy)-6-methylphenyl |
| XIIIa-80 | 2-(Cyanomethoxy)phenyl |
| XIIIa-81 | 2-(Prop-2-in-1-yloxy)phenyl |
| XIIIa-82 | 2-(Prop-2-in-1-yloxy)-4-(trifluormethyl)phenyl |
| XIIIa-83 | 2-Chlor-3-(prop-2-in-1-yloxy)phenyl |
| XIIIa-84 | 2-Chlor-3-[(methylsulfonyl)oxy]phenyl |
| XIIIa-85 | 2-Chlor-3-formylphenyl |
| XIIIa-86 | 2-Chlor-4-(prop-2-in-1-yloxy)phenyl |
| XIIIa-87 | 2-Chlor-4-[(methylsulfonyl)oxy]phenyl |
| XIIIa-88 | 2-Chlor-4-formylphenyl |
| XIIIa-89 | 2-Fluor-3-(prop-2-in-1-yloxy)phenyl |
| XIIIa-90 | 2-Fluor-3-[(methylsulfonyl)oxy]phenyl |
| XIIIa-91 | 2-Fluor-3-formylphenyl |
| XIIIa-92 | 2-Fluor-4-(prop-2-in-1-yloxy)phenyl |
| XIIIa-93 | 2-Fluor-4-[(methylsulfonyl)oxy]phenyl |
| XIIIa-94 | 2-Fluor-4-formylphenyl |
| XIIIa-95 | 2-Formyl-3-methylphenyl |
| XIIIa-96 | 2-Formyl-4-methylphenyl |
| XIIIa-97 | 2-Formyl-5-methylphenyl |
| XIIIa-98 | 2-Formyl-6-methylphenyl |
| XIIIa-99 | 2-Formylphenyl |
| XIIIa-100 | 2-Methyl-3-(prop-2-in-1-yloxy)phenyl |
| XIIIa-101 | 2-Methyl-3-[(methylsulfonyl)oxy]phenyl |
| XIIIa-102 | 2-Methyl-4-(prop-2-in-1-yloxy)phenyl |
| XIIIa-103 | 2-Methyl-4-[(methylsulfonyl)oxy]phenyl |
| XIIIa-104 | 2-[(Hydroxyimino)methyl]-3,4-difluorphenyl |
| XIIIa-105 | 2-[(Hydroxyimino)methyl]-3,5-difluorphenyl |
| XIIIa-106 | 2-[(Hydroxyimino)methyl]-3,6-difluorphenyl |
| XIIIa-107 | 2-[(Hydroxyimino)methyl]-3-chlorphenyl |
| XIIIa-108 | 2-[(Hydroxyimino)methyl]-3-fluorphenyl |
| XIIIa-109 | 2- [(Hydroxyimino)methyl]-3 -methylphenyl |
| XIIIa-110 | 2-[(Hydroxyimino)methyl]-4,5-difluorphenyl |
| XIIIa-111 | 2-[(Hydroxyimino)methyl]-4,6-difluorphenyl |
| XIIIa-112 | 2-[(Hydroxyimino)methyl]-4-chlorphenyl |
| XIIIa-113 | 2-[(Hydroxyimino)methyl]-4-fluorphenyl |
| XIIIa-114 | 2-[(Hydroxyimino)methyl]-4-methylphenyl |
| XIIIa-115 | 2-[(Hydroxyimino)methyl]-5,6-difluorphenyl |
| XIIIa-116 | 2-[(Hydroxyimino)methyl]-5-chlorphenyl |
| XIIIa-117 | 2-[(Hydroxyimino)methyl]-5-fluorphenyl |
| XIIIa-118 | 2-[(Hydroxyimino)methyl]-5-methylphenyl |
| XIIIa-119 | 2-[(Hydroxyimino)methyl]-6-chlorphenyl |
| XIIIa-120 | 2-[(Hydroxyimino)methyl]-6-fluorphenyl |
| XIIIa-121 | 2-[(Hydroxyimino)methyl]-6-methylphenyl |
| XIIIa-122 | 2-[(Hydroxyimino)methyl]phenyl |
| XIIIa-123 | 2-[(Methoxyimino)methyl]-3,4-difluorphenyl |
| XIIIa-124 | 2-[(Methoxyimino)methyl]-3,5-difluorphenyl |
| XIIIa-125 | 2-[(Methoxyimino)methyl]-3,6-difluorphenyl |
| XIIIa-126 | 2-[(Methoxyimino)methyl]-3-chlorphenyl |
| XIIIa-127 | 2-[(Methoxyimino)methyl]-3-fluorphenyl |
| XIIIa-128 | 2-[(Methoxyimino)methyl]-3-methylphenyl |
| XIIIa-129 | 2-[(Methoxyimino)methyl]-4,5-difluorphenyl |
| XIIIa-130 | 2-[(Methoxyimino)methyl]-4,6-difluorphenyl |
| XIIIa-131 | 2-[(Methoxyimino)methyl]-4-chlorphenyl |
| XIIIa-132 | 2-[(Methoxyimino)methyl]-4-fluorphenyl |
| XIIIa-133 | 2-[(Methoxyimino)methyl]-4-methylphenyl |
| XIIIa-134 | 2-[(Methoxyimino)methyl]-5,6-difluorphenyl |
| XIIIa-135 | 2-[(Methoxyimino)methyl]-5-chlorphenyl |
| XIIIa-136 | 2-[(Methoxyimino)methyl]-5-fluorphenyl |
| XIIIa-137 | 2-[(Methoxyimino)methyl]-5-methylphenyl |
| XIIIa-138 | 2-[(Methoxyimino)methyl]-6-chlorphenyl |
| XIIIa-139 | 2-[(Methoxyimino)methyl]-6-fluorphenyl |
| XIIIa-140 | 2-[(Methoxyimino)methyl]-6-methylphenyl |
| XIIIa-141 | 2-[(Methoxyimino)methyl]phenyl |
| XIIIa-142 | 2-[(Methylsulfonyl)oxy]phenyl |
| XIIIa-143 | 2-[(Methylsulfonyl)oxy]-4-(trifluormethyl)phenyl |
| XIIIa-144 | 3,4-Dichlor-2-(prop-2-in-1-yloxy)phenyl |
| XIIIa-145 | 3,4-Dichlor-2-[(methylsulfonyl)oxy]phenyl |
| XIIIa-146 | 3,4-Difluor-2-(prop-2-in-1-yloxy)phenyl |
| XIIIa-147 | 3,4-Difluor-2-[(methylsulfonyl)oxy]phenyl |
| XIIIa-148 | 3,4-Difluor-2-formylphenyl |
| XIIIa-149 | 3,5-Dichlor-2-(prop-2-in-1-yloxy)phenyl |
| XIIIa-150 | 3,5-Dichlor-2-[(methylsulfonyl)oxy]phenyl |
| XIIIa-151 | 3,5-Dichlor-4-(prop-2-in-1-yloxy)phenyl |
| XIIIa-152 | 3,5-Dichlor-4-[(methylsulfonyl)oxy]phenyl |
| XIIIa-153 | 3,5-Difluor-2-(prop-2-in-1-yloxy)phenyl |
| XIIIa-154 | 3,5-Difluor-2-[(methylsulfonyl)oxy]phenyl |
| XIIIa-155 | 3,5-Difluor-2-formylphenyl |
| XIIIa-156 | 3,5-Difluor-4-(prop-2-in-1-yloxy)phenyl |
| XIIIa-157 | 3,5-Difluor-4-[(methylsulfonyl)oxy]phenyl |
| XIIIa-158 | 3,5-Difluor-4-formylphenyl |
| XIIIa-159 | 3,6-Dichlor-2-(prop-2-in-1-yloxy)phenyl |
| XIIIa-160 | 3,6-Dichlor-2-[(methylsulfonyl)oxy]phenyl |
| XIIIa-161 | 3,6-Dichlor-4-(prop-2-in-1-yloxy)phenyl |
| XIIIa-162 | 3,6-Dichlor-4-[(methylsulfonyl)oxy]phenyl |
| XIIIa-163 | 3,6-Difluor-2-(prop-2-in-1-yloxy)phenyl |
| XIIIa-164 | 3,6-Difluor-2-[(methylsulfonyl)oxy]phenyl |
| XIIIa-165 | 3,6-Difluor-2-formylphenyl |
| XIIIa-166 | 3,6-Difluor-4-(prop-2-in-1-yloxy)phenyl |
| XIIIa-167 | 3,6-Difluor-4-[(methylsulfonyl)oxy]phenyl |
| XIIIa-168 | 3,6-Difluor-4-formylphenyl |
| XIIIa-169 | 3-(Allyloxy)-2,4-dichlorphenyl |
| XIIIa-170 | 3-(Allyloxy)-2,4-difluorphenyl |
| XIIIa-171 | 3-(Allyloxy)-2,5-dichlorphenyl |
| XIIIa-172 | 3-(Allyloxy)-2,5-difluorphenyl |
| XIIIa-173 | 3-(Allyloxy)-2,6-dichlorphenyl |
| XIIIa-174 | 3-(Allyloxy)-2,6-difluorphenyl |
| XIIIa-175 | 3-(Allyloxy)-2-chlorphenyl |
| XIIIa-176 | 3-(Allyloxy)-2-fluorphenyl |
| XIIIa-177 | 3-(Allyloxy)-2-methylphenyl |
| XIIIa-178 | 3-(Allyloxy)-4,5-dichlorphenyl |
| XIIIa-179 | 3-(Allyloxy)-4,5-difluorphenyl |
| XIIIa-180 | 3-(Allyloxy)-4,6-dichlorphenyl |
| XIIIa-181 | 3-(Allyloxy)-4,6-difluorphenyl |
| XIIIa-182 | 3-(Allyloxy)-4-chlorphenyl |
| XIIIa-183 | 3-(Allyloxy)-4-fluorphenyl |
| XIIIa-184 | 3-(Allyloxy)-4-methylphenyl |
| XIIIa-185 | 3-(Allyloxy)-5,6-dichlorphenyl |
| XIIIa-186 | 3-(Allyloxy)-5,6-difluorphenyl |
| XIIIa-187 | 3-(Allyloxy)-5-chlorphenyl |
| XIIIa-188 | 3-(Allyloxy)-5-fluorphenyl |
| XIIIa-189 | 3-(Allyloxy)-5-methylphenyl |
| XIIIa-190 | 3-(Allyloxy)-6-chlorphenyl |
| XIIIa-191 | 3-(Allyloxy)-6-fluorphenyl |
| XIIIa-192 | 3-(Allyloxy)-6-methylphenyl |
| XIIIa-193 | 3-(Allyloxy)phenyl |
| XIIIa-194 | 3-(Cyanomethoxy)-2,4-dichlorphenyl |
| XIIIa-195 | 3-(Cyanomethoxy)-2,4-difluorphenyl |
| XIIIa-196 | 3-(Cyanomethoxy)-2,5-dichlorphenyl |
| XIIIa-197 | 3-(Cyanomethoxy)-2,5-difluorphenyl |
| XIIIa-198 | 3-(Cyanomethoxy)-2,6-dichlorphenyl |
| XIIIa-199 | 3-(Cyanomethoxy)-2,6-difluorphenyl |
| XIIIa-200 | 3-(Cyanomethoxy)-2-chlorphenyl |
| XIIIa-201 | 3-(Cyanomethoxy)-2-fluorphenyl |
| XIIIa-202 | 3-(Cyanomethoxy)-2-methylphenyl |
| XIIIa-203 | 3-(Cyanomethoxy)-4,5-dichlorphenyl |
| XIIIa-204 | 3-(Cyanomethoxy)-4,5-difluorphenyl |
| XIIIa-205 | 3-(Cyanomethoxy)-4,6-dichlorphenyl |
| XIIIa-206 | 3-(Cyanomethoxy)-4,6-difluorphenyl |
| XIIIa-207 | 3-(Cyanomethoxy)-4-chlorphenyl |
| XIIIa-208 | 3-(Cyanomethoxy)-4-fluorphenyl |
| XIIIa-209 | 3 -(Cyanomethoxy)-4-methylphenyl |
| XIIIa-210 | 3-(Cyanomethoxy)-5,6-dichlorphenyl |
| XIIIa-211 | 3-(Cyanomethoxy)-5,6-difluorphenyl |
| XIIIa-212 | 3-(Cyanomethoxy)-5-chlorphenyl |
| XIIIa-213 | 3-(Cyanomethoxy)-5-fluorphenyl |
| XIIIa-214 | 3-(Cyanomethoxy)-5-methylphenyl |
| XIIIa-215 | 3-(Cyanomethoxy)-6-chlorphenyl |
| XIIIa-216 | 3-(Cyanomethoxy)-6-fluorphenyl |
| XIIIa-217 | 3-(Cyanomethoxy)-6-methylphenyl |
| XIIIa-218 | 3-(Cyanomethoxy)phenyl |
| XIIIa-219 | 3-(Prop-2-in-1-yloxy)phenyl |
| XIIIa-220 | 3-Chlor-2-(prop-2-in-1-yloxy)phenyl |
| XIIIa-221 | 3-Chlor-2-[(methylsulfonyl)oxy]phenyl |
| XIIIa-222 | 3-Chlor-2-formylphenyl |
| XIIIa-223 | 3-Chlor-4-(prop-2-in-1-yloxy)phenyl |
| XIIIa-224 | 3-Chlor-4-[(methylsulfonyl)oxy]phenyl |
| XIIIa-225 | 3-Chlor-4-formylphenyl |
| XIIIa-226 | 3-Fluor-2-(prop-2-in-1-yloxy)phenyl |
| XIIIa-227 | 3-Fluor-2-[(methylsulfonyl)oxy]phenyl |
| XIIIa-228 | 3-Fluor-2-formylphenyl |
| XIIIa-229 | 3-Fluor-4-(prop-2-in-1-yloxy)phenyl |
| XIIIa-230 | 3-Fluor-4-[(methylsulfonyl)oxy]phenyl |
| XIIIa-231 | 3-Fluor-4-formylphenyl |
| XIIIa-232 | 3-Formyl-2-methylphenyl |
| XIIIa-233 | 3-Formyl-4-methylphenyl |
| XIIIa-234 | 3-Formyl-5-methylphenyl |
| XIIIa-235 | 3-Formyl-6-methylphenyl |
| XIIIa-236 | 3-Formylphenyl |
| XIIIa-237 | 3-Methyl-2-(prop-2-in-1-yloxy)phenyl |
| XIIIa-238 | 3-Methyl-2-[(methylsulfonyl)oxy]phenyl |
| XIIIa-239 | 3-Methyl-4-(prop-2-in-1-yloxy)phenyl |
| XIIIa-240 | 3-Methyl-4-[(methylsulfonyl)oxy]phenyl |
| XIIIa-241 | 3-[(Hydroxyimino)methyl]-2,4-difluorphenyl |
| XIIIa-242 | 3-[(Hydroxyimino)methyl]-2,5-difluorphenyl |
| XIIIa-243 | 3-[(Hydroxyimino)methyl]-2,6-difluorphenyl |
| XIIIa-244 | 3-[(Hydroxyimino)methyl]-2-chlorphenyl |
| XIIIa-245 | 3-[(Hydroxyimino)methyl]-2-fluorphenyl |
| XIIIa-246 | 3-[(Hydroxyimino)methyl]-2-methylphenyl |
| XIIIa-247 | 3-[(Hydroxyimino)methyl]-4,5-difluorphenyl |
| XIIIa-248 | 3-[(Hydroxyimino)methyl]-4,6-difluorphenyl |
| XIIIa-249 | 3-[(Hydroxyimino)methyl]-4-chlorphenyl |
| XIIIa-250 | 3-[(Hydroxyimino)methyl]-4-fluorphenyl |
| XIIIa-251 | 3-[(Hydroxyimino)methyl]-4-methylphenyl |
| XIIIa-252 | 3-[(Hydroxyimino)methyl]-5,6-difluorphenyl |
| XIIIa-253 | 3-[(Hydroxyimino)methyl]-5-chlorphenyl |
| XIIIa-254 | 3-[(Hydroxyimino)methyl]-5-fluorphenyl |
| XIIIa-255 | 3-[(Hydroxyimino)methyl]-5-methylphenyl |
| XIIIa-256 | 3-[(Hydroxyimino)methyl]-6-chlorphenyl |
| XIIIa-257 | 3-[(Hydroxyimino)methyl]-6-fluorphenyl |
| XIIIa-258 | 3-[(Hydroxyimino)methyl]-6-methylphenyl |
| XIIIa-259 | 3-[(Hydroxyimino)methyl]phenyl |
| XIIIa-260 | 3-[(Methoxyimino)methyl]-2,4-difluorphenyl |
| XIIIa-261 | 3-[(Methoxyimino)methyl]-2,5-difluorphenyl |
| XIIIa-262 | 3-[(Methoxyimino)methyl]-2,6-difluorphenyl |
| XIIIa-263 | 3-[(Methoxyimino)methyl]-2-chlorphenyl |
| XIIIa-264 | 3-[(Methoxyimino)methyl]-2-fluorphenyl |
| XIIIa-265 | 3-[(Methoxyimino)methyl]-2-methylphenyl |
| XIIIa-266 | 3-[(Methoxyimino)methyl]-4,5-difluorphenyl |
| XIIIa-267 | 3-[(Methoxyimino)methyl]-4,6-difluorphenyl |
| XIIIa-268 | 3-[(Methoxyimino)methyl]-4-chlorphenyl |
| XIIIa-269 | 3-[(Methoxyimino)methyl]-4-fluorphenyl |
| XIIIa-270 | 3-[(Methoxyimino)methyl]-4-methylphenyl |
| XIIIa-271 | 3-[(Methoxyimino)methyl]-5,6-difluorphenyl |
| XIIIa-272 | 3-[(Methoxyimino)methyl]-5-chlorphenyl |
| XIIIa-273 | 3-[(Methoxyimino)methyl]-5-fluorphenyl |
| XIIIa-274 | 3-[(Methoxyimino)methyl]-5-methylphenyl |
| XIIIa-275 | 3-[(Methoxyimino)methyl]-6-chlorphenyl |
| XIIIa-276 | 3-[(Methoxyimino)methyl]-6-fluorphenyl |
| XIIIa-277 | 3-[(Methoxyimino)methyl]-6-methylphenyl |
| XIIIa-278 | 3-[(Methoxyimino)methyl]phenyl |
| XIIIa-279 | 3-[(Methylsulfonyl)oxy]phenyl |
| XIIIa-280 | 4,5-Dichlor-2-(prop-2-in-1-yloxy)phenyl |
| XIIIa-281 | 4,5-Dichlor-2-[(methylsulfonyl)oxy]phenyl |
| XIIIa-282 | 4,5-Dichlor-3-(prop-2-in-1-yloxy)phenyl |
| XIIIa-283 | 4,5-Dichlor-3-[(methylsulfonyl)oxy]phenyl |
| XIIIa-284 | 4,5-Difluor-2-(prop-2-in-1-yloxy)phenyl |
| XIIIa-285 | 4,5-Difluor-2-[(methylsulfonyl)oxy]phenyl |
| XIIIa-286 | 4,5-Difluor-2-formylphenyl |
| XIIIa-287 | 4,5-Difluor-3-(prop-2-in-1-yloxy)phenyl |
| XIIIa-288 | 4,5-Difluor-3-[(methylsulfonyl)oxy]phenyl |
| XIIIa-289 | 4,5-Difluor-3-formylphenyl |
| XIIIa-290 | 4,6-Dichlor-2-(prop-2-in-1-yloxy)phenyl |
| XIIIa-291 | 4,6-Dichlor-2-[(methylsulfonyl)oxy]phenyl |
| XIIIa-292 | 4,6-Dichlor-3-(prop-2-in-1-yloxy)phenyl |
| XIIIa-293 | 4,6-Dichlor-3-[(methylsulfonyl)oxy]phenyl |
| XIIIa-294 | 4,6-Difluor-2-(prop-2-in-1-yloxy)phenyl |
| XIIIa-295 | 4,6-Difluor-2-[(methylsulfonyl)oxy]phenyl |
| XIIIa-296 | 4,6-Difluor-2-formylphenyl |
| XIIIa-297 | 4,6-Difluor-3-(prop-2-in-1-yloxy)phenyl |
| XIIIa-298 | 4,6-Difluor-3-[(methylsulfonyl)oxy]phenyl |
| XIIIa-299 | 4,6-Difluor-3-formylphenyl |
| XIIIa-300 | 4-(Allyloxy)-2,3-dichlorphenyl |
| XIIIa-301 | 4-(Allyloxy)-2,3-difluorphenyl |
| XIIIa-302 | 4-(Allyloxy)-2,5-dichlorphenyl |
| XIIIa-303 | 4-(Allyloxy)-2,5-difluorphenyl |
| XIIIa-304 | 4-(Allyloxy)-2,6-dichlorphenyl |
| XIIIa-305 | 4-(Allyloxy)-2,6-difluorphenyl |
| XIIIa-306 | 4-(Allyloxy)-2-chlorphenyl |
| XIIIa-307 | 4-(Allyloxy)-2-fluorphenyl |
| XIIIa-308 | 4-(Allyloxy)-2-methylphenyl |
| XIIIa-309 | 4-(Allyloxy)-3,5-dichlorphenyl |
| XIIIa-310 | 4-(Allyloxy)-3,5-difluorphenyl |
| XIIIa-311 | 4-(Allyloxy)-3,6-dichlorphenyl |
| XIIIa-312 | 4-(Allyloxy)-3,6-difluorphenyl |
| XIIIa-313 | 4-(Allyloxy)-3-chlorphenyl |
| XIIIa-314 | 4-(Allyloxy)-3-fluorphenyl |
| XIIIa-315 | 4-(Allyloxy)-3-methylphenyl |
| XIIIa-316 | 4-(Allyloxy)phenyl |
| XIIIa-317 | 4-(Cyanomethoxy)-2,3-dichlorphenyl |
| XIIIa-318 | 4-(Cyanomethoxy)-2,3 -difluorphenyl |
| XIIIa-319 | 4-(Cyanomethoxy)-2,5-dichlorphenyl |
| XIIIa-320 | 4-(Cyanomethoxy)-2,5-difluorphenyl |
| XIIIa-321 | 4-(Cyanomethoxy)-26-dichlorphenyl |
| XIIIa-322 | 4-(Cyanomethoxy)-2,6-difluorphenyl |
| XIIIa-323 | 4-(Cyanomethoxy)-2-chlorphenyl |
| XIIIa-324 | 4-(Cyanomethoxy)-2-fluorphenyl |
| XIIIa-325 | 4-(Cyanomethoxy)-2-methylphenyl |
| XIIIa-326 | 4-(Cyanomethoxy)-3,5-dichlorphenyl |
| XIIIa-327 | 4-(Cyanomethoxy)-3,5-difluorphenyl |
| XIIIa-328 | 4-(Cyanomethoxy)-3,6-dichlorphenyl |
| XIIIa-329 | 4-(Cyanomethoxy)-3,6-difluorphenyl |
| XIIIa-330 | 4-(Cyanomethoxy)-3-chlorphenyl |
| XIIIa-331 | 4-(Cyanomethoxy)-3-fluorphenyl |
| XIIIa-332 | 4-(Cyanomethoxy)-3-methylphenyl |
| XIIIa-333 | 4-(Cyanomethoxy)phenyl |
| XIIIa-334 | 4-(Prop-2-in-1-yloxy)phenyl |
| XIIIa-335 | 4-Chlor-2-(prop-2-in-1-yloxy)phenyl |
| XIIIa-33 6 | 4-Chlor-2-[(methylsulfonyl)oxy]phenyl |
| XIIIa-337 | 4-Chlor-2-formylphenyl |
| XIIIa-338 | 4-Chlor-3-(prop-2-in-1-yloxy)phenyl |
| XIIIa-339 | 4-Chlor-3-[(methylsulfonyl)oxy]phenyl |
| XIIIa-340 | 4-Chlor-3-formylphenyl |
| XIIIa-341 | 4-Fluor-2-(prop-2-in-1-yloxy)phenyl |
| XIIIa-342 | 4-Fluor-2-[(methylsulfonyl)oxy]phenyl |
| XIIIa-343 | 4-Fluor-2-formylphenyl |
| XIIIa-344 | 4-Fluor-3-(prop-2-in-1-yloxy)phenyl |
| XIIIa-345 | 4-Fluor-3-[(methylsulfonyl)oxy]phenyl |
| XIIIa-346 | 4-Fluor-3-formylphenyl |
| XIIIa-347 | 4-Formyl-2-methylphenyl |
| XIIIa-348 | 4-Formyl-3-methylphenyl |
| XIIIa-349 | 4-Formylphenyl |
| XIIIa-350 | 4-Methyl-2-(prop-2-in-1-yloxy)phenyl |
| XIIIa-351 | 4-Methyl-2-[(methylsulfonyl)oxy]phenyl |
| XIIIa-352 | 4-Methyl-3-(prop-2-in-1-yloxy)phenyl |
| XIIIa-353 | 4-Methyl-3-[(methylsulfonyl)oxy]phenyl |
| XIIIa-354 | 4-[(Hydroxyimino)methyl]-2,3-difluorphenyl |
| XIIIa-355 | 4-[(Hydroxyimino)methyl]-2,5-difluorphenyl |
| XIIIa-356 | 4-[(Hydroxyimino)methyl]-2, 6-difluorphenyl |
| XIIIa-357 | 4-[(Hydroxyimino)methyl]-2-chlorphenyl |
| XIIIa-358 | 4-[(Hydroxyimino)methyl]-2-fluorphenyl |
| XIIIa-359 | 4-[(Hydroxyimino)methyl]-2-methylphenyl |
| XIIIa-360 | 4-[(Hydroxyimino)methyl]-3,5-difluorphenyl |
| XIIIa-361 | 4-[(Hydroxyimino)methyl]-3,6-difluorphenyl |
| XIIIa-362 | 4-[(Hydroxyimino)methyl]-3-chlorphenyl |
| XIIIa-363 | 4-[(Hydroxyimino)methyl]-3-fluorphenyl |
| XIIIa-364 | 4-[(Hydroxyimino)methyl]-3-methylphenyl |
| XIIIa-365 | 4-[(Hydroxyimino)methyl]phenyl |
| XIIIa-366 | 4-[(Methoxyimino)methyl]-2,3-difluorphenyl |
| XIIIa-367 | 4-[(Methoxyimino)methyl]-2,5-difluorphenyl |
| XIIIa-368 | 4-[(Methoxyimino)methyl]-2,6-difluorphenyl |
| XIIIa-369 | 4-[(Methoxyimino)methyl]-2-chlorphenyl |
| XIIIa-370 | 4-[(Methoxyimino)methyl]-2-fluorphenyl |
| XIIIa-3 71 | 4-[(Methoxyimino)methyl]-2-methylphenyl |
| XIIIa-372 | 4-[(Methoxyimino)methyl]-3,5-difluorphenyl |
| XIIIa-373 | 4-[(Methoxyimino)methyl]-3,6-difluorphenyl |
| XIIIa-374 | 4-[(Methoxyimino)methyl]-3-chlorphenyl |
| XIIIa-375 | 4-[(Methoxyimino)methyl]-3-fluorphenyl |
| XIIIa-376 | 4-[(Methoxyimino)methyl]-3-methylphenyl |
| XIIIa-377 | 4-[(Methoxyimino)methyl]phenyl |
| XIIIa-378 | 4-[(Methylsulfonyl)oxy]phenyl |
| XIIIa-379 | 5,6-Dichlor-2-(prop-2-in-1-yloxy)phenyl |
| XIIIa-380 | 5,6-Dichlor-2-[(methylsulfonyl)oxy]phenyl |
| XIIIa-381 | 5,6-Dichlor-3-(prop-2-in-1-yloxy)phenyl |
| XIIIa- 382 | 5,6-Dichlor-3-[(methylsulfonyl)oxy]phenyl |
| XIIIa-383 | 5,6-Difluor-2-(prop-2-in-1-yloxy)phenyl |
| XIIIa-384 | 5,6-Difluor-2-[(methylsulfonyl)oxy]phenyl |
| XIIIa-385 | 5,6-Difluor-2-formylphenyl |
| XIIIa-386 | 5,6-Difluor-3-(prop-2-in-1-yloxy)phenyl |
| XIIIa-387 | 5,6-Difluor-3-[(methylsulfonyl)oxy]phenyl |
| XIIIa-388 | 5,6-Difluor-3-formylphenyl |
| XIIIa-389 | 5-Chlor-2-(prop-2-in-1-yloxy)phenyl |
| XIIIa-390 | 5-Chlor-2-[(methylsulfonyl)oxy]phenyl |
| XIIIa-391 | 5-Chlor-2-formylphenyl |
| XIIIa-392 | 5-Chlor-3-(prop-2-in-1-yloxy)phenyl |
| XIIIa-393 | 5-Chlor-3-[(methylsulfonyl)oxy]phenyl |
| XIIIa-394 | 5-Chlor-3-formylphenyl |
| XIIIa-395 | 5-Fluor-2-(prop-2-in-1-yloxy)phenyl |
| XIIIa-396 | 5-Fluor-2-[(methylsulfonyl)oxy]phenyl |
| XIIIa-397 | 5-Fluor-2-formylphenyl |
| XIIIa-398 | 5-Fluor-3-(prop-2-in-1-yloxy)phenyl |
| XIIIa-399 | 5-Fluor-3-[(methylsulfonyl)oxy]phenyl |
| XIIIa-400 | 5-Fluor-3-formylphenyl |
| XIIIa-401 | 5-Methyl-2-(prop-2-in-1-yloxy)phenyl |
| XIIIa-402 | 5-Methyl-2-[(methylsulfonyl)oxy]phenyl |
| XIIIa-403 | 5-Methyl-3-(prop-2-in-1-yloxy)phenyl |
| XIIIa-404 | 5-Methyl-3-[(methylsulfonyl)oxy]phenyl |
| XIIIa-405 | 6-Chlor-2-(prop-2-in-1-yloxy)phenyl |
| XIIIa-406 | 6-Chlor-2-[(methylsulfonyl)oxy]phenyl |
| XIIIa-407 | 6-Chlor-2-formylphenyl |
| XIIIa-408 | 6-Chlor-3-(prop-2-in-1-yloxy)phenyl |
| XIIIa-409 | 6-Chlor-3-[(methylsulfonyl)oxy]phenyl |
| XIIIa-410 | 6-Chlor-3-formylphenyl |
| XIIIa-411 | 6-Fluor-2-(prop-2-in-1-yloxy)phenyl |
| XIIIa-412 | 6-Fluor-2-[(methylsulfonyl)oxy]phenyl |
| XIIIa-413 | 6-Fluor-2-formylphenyl |
| XIIIa-414 | 6-Fluor-3-(prop-2-in-1-yloxy)phenyl |
| XIIIa-415 | 6-Fluor-3-[(methylsulfonyl)oxy]phenyl |
| XIIIa-416 | 6-Fluor-3-formylphenyl |
| XIIIa-417 | 6-Methyl-2-(prop-2-in-1-yloxy)phenyl |
| XIIIa-418 | 6-Methyl-2-[(methylsulfonyl)oxy]phenyl |
| XIIIa-419 | 6-Methyl-3-(prop-2-in-1-yloxy)phenyl |
| XIIIa-420 | 6-Methyl-3-[(methylsulfonyl)oxy]phenyl |

**Tabelle 3:**

| | |
|---|---|
| 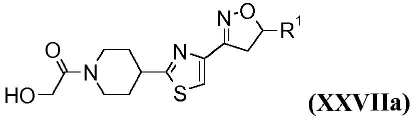 | |

| Bsp. | R¹ |
|---|---|
| XXVIIa-1 | 2,3-Dichlor-4-(prop-2-in-1-yloxy)phenyl |
| XXVIIa-2 | 2,3-Dichlor-4-[(methylsulfonyl)oxy]phenyl |
| XXVIIa-3 | 2,3-Difluor-4-(prop-2-in-1-yloxy)phenyl |
| XXVIIa-4 | 2,3-Difluor-4-[(methylsulfonyl)oxy]phenyl |
| XXVIIa-5 | 2,3-Difluor-4-fonnylphenyl |
| XXVIIa-6 | 2,4-Dichlor-3-(prop-2-in-1-yloxy)phenyl |
| XXVIIa-7 | 2,4-Dichlor-3-[(methylsulfonyl)oxy]phenyl |
| XXVIIa-8 | 2,4-Difluor-3-(prop-2-in-1-yloxy)phenyl |
| XXVIIa-9 | 2,4-Difluor-3-[(methylsulfonyl)oxy]phenyl |
| XXVIIa-10 | 2,4-Difluor-3-formylphenyl |
| XXVIIa-11 | 2,5-Dichlor-3-(prop-2-in-1-yloxy)phenyl |
| XXVIIa-12 | 2,5-Dichlor-3-[(methylsulfonyl)oxy]phenyl |
| XXVIIa-13 | 2,5-Dichlor-4-(prop-2-in-1-yloxy)phenyl |
| XXVIIa-14 | 2,5-Dichlor-4-[(methylsulfonyl)oxy]phenyl |
| XXVIIa-15 | 2,5-Difluor-3-(prop-2-in-1-yloxy)phenyl |
| XXVIIa-16 | 2,5-Difluor-3-[(methylsulfonyl)oxy]phenyl |
| XXVIIa-17 | 2,5-Difluor-3-formylphenyl |
| XXVIIa-18 | 2,5-Difluor-4-(prop-2-in-1-yloxy)phenyl |
| XXVIIa-19 | 2,5-Difluor-4-[(methylsulfonyl)oxy]phenyl |
| XXVIIa-20 | 2,5-Difluor-4-formylphenyl |
| XXVIIa-21 | 2,6-Dichlor-3-(prop-2-in-1-yloxy)phenyl |
| XXVIIa-22 | 2,6-Dichlor-3-[(methylsulfonyl)oxy]phenyl |
| XXVIIa-23 | 2,6-Dichlor-4-(prop-2-in-1-yloxy)phenyl |
| XXVIIa-24 | 2,6-Dichlor-4-[(methylsulfonyl)oxy]phenyl |
| XXVIIa-25 | 2,6-Difluor-3-(prop-2-in-1-yloxy)phenyl |
| XXVIIa-26 | 2,6-Difluor-3-[(methylsulfonyl)oxy]phenyl |
| XXVIIa-27 | 2,6-Difluor-3-formylphenyl |
| XXVIIa-28 | 2,6-Difluor-4-(prop-2-in-1-yloxy)phenyl |
| XXVIIa-29 | 2,6-Difluor-4-[(methylsulfonyl)oxy]phenyl |
| XXVIIa-30 | 2,6-Difluor-4-formylphenyl |
| XXVIIa-31 | 2-(Allyloxy)-3,4-dichlorphenyl |
| XXVIIa-32 | 2-(Allyloxy)-3,4-difluorphenyl |
| XXVIIa-33 | 2-(Allyloxy)-3,5-dichlorphenyl |
| XXVIIa-34 | 2-(Allyloxy)-3,5-difluorphenyl |
| XXVIIa-35 | 2-(Allyloxy)-3,6-dichlorphenyl |
| XXVIIa-36 | 2-(Allyloxy)-3,6-difluorphenyl |
| XXVIIa-37 | 2-(Allyloxy)-3-chlorphenyl |
| XXVIIa-38 | 2-(Allyloxy)-3-fluorphenyl |
| XXVIIa-39 | 2-(Allyloxy)-3-methylphenyl |
| XXVIIa-40 | 2-(Allyloxy)-4,5-dichlorphenyl |
| XXVIIa-41 | 2-(Allyloxy)-4,5-difluorphenyl |
| XXVIIa-42 | 2-(Allyloxy)-4,6-dichlorphenyl |
| XXVIIa-43 | 2-(Allyloxy)-4,6-difluorphenyl |
| XXVIIa-44 | 2-(Allyloxy)-4-chlorphenyl |
| XXVIIa-45 | 2-(Allyloxy)-4-fluorphenyl |
| XXVIIa-46 | 2-(Allyloxy)-4-methylphenyl |
| XXVIIa-47 | 2-(Allyloxy)-5,6-dichlorphenyl |
| XXVIIa-48 | 2-(Allyloxy)-5,6-difluorphenyl |
| XXVIIa-49 | 2-(Allyloxy)-5-chlorphenyl |
| XXVIIa-50 | 2-(Allyloxy)-5-fluorphenyl |
| XXVIIa-51 | 2-(Allyloxy)-5-methylphenyl |
| XXVIIa-52 | 2-(Allyloxy)-6-chlorphenyl |
| XXVIIa-53 | 2-(Allyloxy)-6-fluorphenyl |
| XXVIIa-54 | 2-(Allyloxy)-6-methylphenyl |
| XXVIIa-55 | 2-(Allyloxy)phenyl |
| XXVIIa-56 | 2-(Cyanomethoxy)-3,4-dichlorphenyl |
| XXVIIa-57 | 2-(Cyanomethoxy)-3,4-difluorphenyl |
| XXVIIa-58 | 2-(Cyanomethoxy)-3,5-dichlorphenyl |
| XXVIIa-59 | 2-(Cyanomethoxy)-3,5-difluorphenyl |
| XXVIIa-60 | 2-(Cyanomethoxy)-3,6-dichlorphenyl |
| XXVIIa-61 | 2-(Cyanomethoxy)-3,6-difluorphenyl |
| XXVIIa-62 | 2-(Cyanomethoxy)-3-chlorphenyl |
| XXVIIa-63 | 2-(Cyanomethoxy)-3-fluorphenyl |
| XXVIIa-64 | 2-(Cyanomethoxy)-3-methylphenyl |
| XXVIIa-65 | 2-(Cyanomethoxy)-4,5-dichlorphenyl |
| XXVIIa-66 | 2-(Cyanomethoxy)-4,5-difluorphenyl |
| XXVIIa-67 | 2-(Cyanomethoxy)-4,6-dichlorphenyl |
| XXVIIa-68 | 2-(Cyanomethoxy)-4,6-difluorphenyl |
| XXVIIa-69 | 2-(Cyanomethoxy)-4-chlorphenyl |
| XXVIIa-70 | 2-(Cyanomethoxy)-4-fluorphenyl |
| XXVIIa-71 | 2-(Cyanomethoxy)-4-methylphenyl |
| XXVIIa-72 | 2-(Cyanomethoxy)-5,6-dichlorphenyl |
| XXVIIa-73 | 2-(Cyanomethoxy)-5,6-difluorphenyl |
| XXVIIa-74 | 2-(Cyanomethoxy)-5-chlorphenyl |
| XXVIIa-75 | 2-(Cyanomethoxy)-5-fluorphenyl |
| XXVIIa-76 | 2-(Cyanomethoxy)-5-methylphenyl |
| XXVIIa-77 | 2-(Cyanomethoxy)-6-chlorphenyl |
| XXVIIa-78 | 2-(Cyanomethoxy)-6-fluorphenyl |
| XXVIIa-79 | 2-(Cyanomethoxy)-6-methylphenyl |
| XXVIIa-80 | 2-(Cyanomethoxy)phenyl |
| XXVIIa-81 | 2-(Prop-2-in-1-yloxy)phenyl |
| XXVIIa-82 | 2-(Prop-2-in-1-yloxy)-4-(trifluormethyl)phenyl |
| XXVIIa-83 | 2-Chlor-3-(prop-2-in-1-yloxy)phenyl |
| XXVIIa-84 | 2-Chlor-3-[(methylsulfonyl)oxy]phenyl |
| XXVIIa-85 | 2-Chlor-3-formylphenyl |
| XXVIIa-86 | 2-Chlor-4-(prop-2-in-1-yloxy)phenyl |
| XXVIIa-87 | 2-Chlor-4-[(methylsulfonyl)oxy]phenyl |
| XXVIIa-88 | 2-Chlor-4-formylphenyl |
| XXVIIa-89 | 2-Fluor-3-(prop-2-in-1-yloxy)phenyl |
| XXVIIa-90 | 2-Fluor-3-[(methylsulfonyl)oxy]phenyl |
| XXVIIa-91 | 2-Fluor-3-formylphenyl |
| XXVIIa-92 | 2-Fluor-4-(prop-2-in-1-yloxy)phenyl |
| XXVIIa-93 | 2-Fluor-4-[(methylsulfonyl)oxy]phenyl |
| XXVIIa-94 | 2-Fluor-4-formylphenyl |
| XXVIIa-95 | 2-Formyl-3-methylphenyl |
| XXVIIa-96 | 2-Formyl-4-methylphenyl |
| XXVIIa-97 | 2-Formyl-5-methylphenyl |
| XXVIIa-98 | 2-Formyl-6-methylphenyl |
| XXVIIa-99 | 2-Formylphenyl |
| XXVIIa-100 | 2-Methyl-3-(prop-2-in-1-yloxy)phenyl |
| XXVIIa-101 | 2-Methyl-3-[(methylsulfonyl)oxy]phenyl |
| XXVIIa-102 | 2-Methyl-4-(prop-2-in-1-yloxy)phenyl |
| XXVIIa-103 | 2-Methyl-4-[(methylsulfonyl)oxy]phenyl |
| XXVIIa-104 | 2-[(Hydroxyimino)methyl]-3,4-difluorphenyl |
| XXVIIa-105 | 2-[(Hydroxyimino)methyl]-3,5-difluorphenyl |
| XXVIIa-106 | 2-[(Hydroxyimino)methyl]-3,6-difluorphenyl |
| XXVIIa-107 | 2-[(Hydroxyimino)methyl]-3-chlorphenyl |
| XXVIIa-108 | 2-[(Hydroxyimino)methyl]-3-fluorphenyl |
| XXVIIa-109 | 2-[(Hydroxyimino)methyl]-3-methylphenyl |
| XXVIIa-110 | 2-[(Hydroxyimino)methyl]-4,5-difluorphenyl |
| XXVIIa-111 | 2-[(Hydroxyimino)methyl]-4,6-difluorphenyl |
| XXVIIa-112 | 2-[(Hydroxyimino)methyl]-4-chlorphenyl |
| XXVIIa-113 | 2-[(Hydroxyimino)methyl]-4-fluorphenyl |
| XXVIIa-114 | 2-[(Hydroxyimino)methyl]-4-methylphenyl |
| XXVIIa-115 | 2-[(Hydroxyimino)methyl]-5,6-difluorphenyl |
| XXVIIa-116 | 2-[(Hydroxyimino)methyl]-5-chlorphenyl |
| XXVIIa-117 | 2-[(Hydroxyimino)methyl]-5-fluorphenyl |
| XXVIIa-118 | 2-[(Hydroxyimino)methyl]-5-methylphenyl |
| XXVIIa-119 | 2-[(Hydroxyimino)methyl]-6-chlorphenyl |
| XXVIIa-120 | 2-[(Hydroxyimino)methyl]-6-fluorphenyl |
| XXVIIa-121 | 2-[(Hydroxyimino)methyl]-6-methylphenyl |
| XXVIIa-122 | 2-[(Hydroxyimino)methyl]phenyl |
| XXVIIa-123 | 2-[(Methoxyimino)methyl]-3,4-difluorphenyl |
| XXVIIa-124 | 2-[(Methoxyimino)methyl]-3,5-difluorphenyl |
| XXVIIa-125 | 2-[(Methoxyimino)methyl]-3,6-difluorphenyl |
| XXVIIa-126 | 2-[(Methoxyimino)methyl]-3-chlorphenyl |
| XXVIIa-127 | 2-[(Methoxyimino)methyl]-3-fluorphenyl |
| XXVIIa-128 | 2-[(Methoxyimino)methyl]-3-methylphenyl |
| XXVIIa-129 | 2-[(Methoxyimino)methyl]-4,5-difluorphenyl |
| XXVIIa-130 | 2-[(Methoxyimino)methyl]-4,6-difluorphenyl |
| XXVIIa-131 | 2-[(Methoxyimino)methyl]-4-chlorphenyl |
| XXVIIa-132 | 2-[(Methoxyimino)methyl]-4-fluorphenyl |
| XXVIIa-133 | 2-[(Methoxyimino)methyl]-4-methylphenyl |
| XXVIIa-134 | 2-[(Methoxyimino)methyl]-5,6-difluorphenyl |
| XXVIIa-135 | 2-[(Methoxyimino)methyl]-5-chlorphenyl |
| XXVIIa-136 | 2-[(Methoxyimino)methyl]-5-fluorphenyl |
| XXVIIa-137 | 2-[(Methoxyimino)methyl]-5-methylphenyl |
| XXVIIa-138 | 2-[(Methoxyimino)methyl]-6-chlorphenyl |
| XXVIIa-139 | 2-[(Methoxyimino)methyl]-6-fluorphenyl |
| XXVIIa-140 | 2-[(Methoxyimino)methyl]-6-methylphenyl |
| XXVIIa-141 | 2-[(Methoxyimino)methyl]phenyl |
| XXVIIa-142 | 2-[(Methylsulfonyl)oxy]phenyl |
| XXVIIa-143 | 2- [(Methylsulfonyl)oxy]-4-(trifluormethyl)phenyl |
| XXVIIa-144 | 3,4-Dichlor-2-(prop-2-in-1-yloxy)phenyl |
| XXVIIa-145 | 3,4-Dichlor-2-[(methylsulfonyl)oxy]phenyl |
| XXVIIa-146 | 3,4-Difluor-2-(prop-2-in-1-yloxy)phenyl |
| XXVIIa-147 | 3,4-Difluor-2-[(methylsulfonyl)oxy]phenyl |
| XXVIIa-148 | 3,4-Difluor-2-formylphenyl |
| XXVIIa-149 | 3,5-Dichlor-2-(prop-2-in-1-yloxy)phenyl |
| XXVIIa-150 | 3,5-Dichlor-2-[(methylsulfonyl)oxy]phenyl |
| XXVIIa-151 | 3,5-Dichlor-4-(prop-2-in-1-yloxy)phenyl |
| XXVIIa-152 | 3,5-Dichlor-4-[(methylsulfonyl)oxy]phenyl |
| XXVIIa-153 | 3,5-Difluor-2-(prop-2-in-1-yloxy)phenyl |
| XXVIIa-154 | 3,5-Difluor-2-[(methylsulfonyl)oxy]phenyl |
| XXVIIa-155 | 3,5-Difluor-2-formylphenyl |
| XXVIIa-156 | 3,5-Difluor-4-(prop-2-in-1-yloxy)phenyl |
| XXVIIa-157 | 3,5-Difluor-4-[(methylsulfonyl)oxy]phenyl |
| XXVIIa-158 | 3,5-Difluor-4-formylphenyl |
| XXVIIa-159 | 3,6-Dichlor-2-(prop-2-in-1-yloxy)phenyl |
| XXVIIa-160 | 3,6-Dichlor-2-[(methylsulfonyl)oxy]phenyl |
| XXVIIa-161 | 3,6-Dichlor-4-(prop-2-in-1-yloxy)phenyl |
| XXVIIa-162 | 3,6-Dichlor-4-[(methylsulfonyl)oxy]phenyl |
| XXVIIa-163 | 3,6-Difluor-2-(prop-2-in-1-yloxy)phenyl |
| XXVIIa-164 | 3,6-Difluor-2-[(methylsulfonyl)oxy]phenyl |
| XXVIIa-165 | 3,6-Difluor-2-formylphenyl |
| XXVIIa-166 | 3,6-Difluor-4-(prop-2-in-1-yloxy)phenyl |
| XXVIIa-167 | 3,6-Difluor-4-[(methylsulfonyl)oxy]phenyl |
| XXVIIa-168 | 3,6-Difluor-4-formylphenyl |
| XXVIIa-169 | 3-(Allyloxy)-2,4-dichlorphenyl |
| XXVIIa-170 | 3-(Allyloxy)-2,4-difluorphenyl |
| XXVIIa-171 | 3-(Allyloxy)-2,5-dichlorphenyl |
| XXVIIa-172 | 3-(Allyloxy)-2,5-difluorphenyl |
| XXVIIa-173 | 3-(Allyloxy)-2,6-dichlorphenyl |
| XXVIIa-174 | 3-(Allyloxy)-2,6-difluorphenyl |
| XXVIIa-175 | 3-(Allyloxy)-2-chlorphenyl |
| XXVIIa-176 | 3-(Allyloxy)-2-fluorphenyl |
| XXVIIa-177 | 3-(Allyloxy)-2-methylphenyl |
| XXVIIa-178 | 3-(Allyloxy)-4,5-dichlorphenyl |
| XXVIIa-179 | 3-(Allyloxy)-4,5-difluorphenyl |
| XXVIIa-180 | 3-(Allyloxy)-4,6-dichlorphenyl |
| XXVIIa-181 | 3-(Allyloxy)-4,6-difluorphenyl |
| XXVIIa-182 | 3-(Allyloxy)-4-chlorphenyl |
| XXVIIa-183 | 3-(Allyloxy)-4-fluorphenyl |
| XXVIIa-184 | 3-(Allyloxy)-4-methylphenyl |
| XXVIIa-185 | 3-(Allyloxy)-5,6-dichlorphenyl |
| XXVIIa-186 | 3-(Allyloxy)-5,6-difluorphenyl |
| XXVIIa-187 | 3-(Allyloxy)-5-chlorphenyl |
| XXVIIa-188 | 3-(Allyloxy)-5-fluorphenyl |
| XXVIIa-189 | 3 -(Allyloxy)- 5- methylphenyl |
| XXVIIa-190 | 3-(Allyloxy)-6-chlorphenyl |
| XXVIIa-191 | 3-(Allyloxy)-6-fluorphenyl |
| XXVIIa-192 | 3-(Allyloxy)-6-methylphenyl |
| XXVIIa-193 | 3-(Allyloxy)phenyl |
| XXVIIa-194 | 3-(Cyanomethoxy)-2,4-dichlorphenyl |
| XXVIIa-195 | 3-(Cyanomethoxy)-2,4-difluorphenyl |
| XXVIIa-196 | 3-(Cyanomethoxy)-2,5-dichlorphenyl |
| XXVIIa-197 | 3-(Cyanomethoxy)-2,5-difluorphenyl |
| XXVIIa-198 | 3-(Cyanomethoxy)-2,6-dichlorphenyl |
| XXVIIa-199 | 3-(Cyanomethoxy)-2,6-difluorphenyl |
| XXVIIa-200 | 3-(Cyanomethoxy)-2-chlorphenyl |
| XXVIIa-201 | 3-(Cyanomethoxy)-2-fluorphenyl |
| XXVIIa-202 | 3-(Cyanomethoxy)-2-methylphenyl |
| XXVIIa-203 | 3-(Cyanomethoxy)-4,5-dichlorphenyl |
| XXVIIa-204 | 3-(Cyanomethoxy)-4,5-difluorphenyl |
| XXVIIa-205 | 3-(Cyanomethoxy)-4,6-dichlorphenyl |
| XXVIIa-206 | 3-(Cyanomethoxy)-4,6-difluorphenyl |
| XXVIIa-207 | 3-(Cyanomethoxy)-4-chlorphenyl |
| XXVIIa-208 | 3-(Cyanomethoxy)-4-fluorphenyl |
| XXVIIa-209 | 3-(Cyanomethoxy)-4-methylphenyl |
| XXVIIa-210 | 3-(Cyanomethoxy)-5,6-dichlorphenyl |
| XXVIIa-211 | 3-(Cyanomethoxy)-5,6-difluorphenyl |
| XXVIIa-212 | 3-(Cyanomethoxy)-5-chlorphenyl |
| XXVIIa-213 | 3-(Cyanomethoxy)-5-fluorphenyl |
| XXVIIa-214 | 3-(Cyanomethoxy)-5-methylphenyl |
| XXVIIa-215 | 3-(Cyanomethoxy)-6-chlorphenyl |
| XXVIIa-216 | 3-(Cyanomethoxy)-6-fluorphenyl |
| XXVIIa-217 | 3-(Cyanomethoxy)-6-methylphenyl |
| XXVIIa-218 | 3-(Cyanomethoxy)phenyl |
| XXVIIa-219 | 3-(Prop-2-in-1-yloxy)phenyl |
| XXVIIa-220 | 3-Chlor-2-(prop-2-in-1-yloxy)phenyl |
| XXVIIa-221 | 3-Chlor-2-[(methylsulfonyl)oxy]phenyl |
| XXVIIa-222 | 3-Chlor-2-formylphenyl |
| XXVIIa-223 | 3-Chlor-4-(prop-2-in-1-yloxy)phenyl |
| XXVIIa-224 | 3-Chlor-4-[(methylsulfonyl)oxy]phenyl |
| XXVIIa-225 | 3-Chlor-4-formylphenyl |
| XXVIIa-226 | 3-Fluor-2-(prop-2-in-1-yloxy)phenyl |
| XXVIIa-227 | 3-Fluor-2-[(methylsulfonyl)oxy]phenyl |
| XXVIIa-228 | 3-Fluor-2-formylphenyl |
| XXVIIa-229 | 3-Fluor-4-(prop-2-in-1-yloxy)phenyl |
| XXVIIa-230 | 3-Fluor-4-[(methylsulfonyl)oxy]phenyl |
| XXVIIa-231 | 3-Fluor-4-formylphenyl |
| XXVIIa-232 | 3-Formyl-2-methylphenyl |
| XXVIIa-233 | 3-Formyl-4-methylphenyl |
| XXVIIa-234 | 3-Formyl-5-methylphenyl |
| XXVIIa-235 | 3-Formyl-6-methylphenyl |
| XXVIIa-236 | 3-Formylphenyl |
| XXVIIa-237 | 3-Methyl-2-(prop-2-in-1-yloxy)phenyl |
| XXVIIa-238 | 3-Methyl-2-[(methylsulfonyl)oxy]phenyl |
| XXVIIa-239 | 3-Methyl-4-(prop-2-in-1-yloxy)phenyl |
| XXVIIa-240 | 3-Methyl-4-[(methylsulfonyl)oxy]phenyl |
| XXVIIa-241 | 3-[(Hydroxyimino)methyl]-2,4-difluorphenyl |
| XXVIIa-242 | 3-[(Hydroxyimino)methyl]-2,5-difluorphenyl |
| XXVIIa-243 | 3-[(Hydroxyimino)methyl]-2,6-difluorphenyl |
| XXVIIa-244 | 3-[(Hydroxyimino)methyl]-2-chlorphenyl |
| XXVIIa-245 | 3-[(Hydroxyimino)methyl]-2-fluorphenyl |
| XXVIIa-246 | 3-[(Hydroxyimino)methyl]-2-methylphenyl |
| XXVIIa-247 | 3-[(Hydroxyimino)methyl]-4,5-difluorphenyl |
| XXVIIa-248 | 3-[(Hydroxyimino)methyl]-4,6-difluorphenyl |
| XXVIIa-249 | 3-[(Hydroxyimino)methyl]-4-chlorphenyl |
| XXVIIa-250 | 3-[(Hydroxyimino)methyl]-4-fluorphenyl |
| XXVIIa-251 | 3-[(Hydroxyimino)methyl]-4-methylphenyl |
| XXVIIa-252 | 3-[(Hydroxyimino)methyl]-5,6-difluorphenyl |
| XXVIIa-253 | 3-[(Hydroxyimino)methyl]-5-chlorphenyl |
| XXVIIa-254 | 3-[(Hydroxyimino)methyl]-5-fluorphenyl |
| XXVIIa-255 | 3-[(Hydroxyimmo)methyl]-5-methylphenyl |
| XXVIIa-256 | 3-[(Hydroxyimino)methyl]-6-chlorphenyl |
| XXVIIa-257 | 3-[(Hydroxyimino)methyl]-6-fluorphenyl |
| XXVIIa-258 | 3-[(Hydroxyimino)methyl]-6-methylphenyl |
| XXVIIa-259 | 3-[(Hydroxyimino)methyl]phenyl |
| XXVIIa-260 | 3-[(Methoxyimino)methyl]-2,4-difluorphenyl |
| XXVIIa-261 | 3-[(Methoxyimino)methyl]-2,5-difluorphenyl |
| XXVIIa-262 | 3-[(Methoxyimino)methyl]-2,6-difluorphenyl |
| XXVIIa-263 | 3-[(Methoxyimino)methyl]-2-chlorphenyl |
| XXVIIa-264 | 3-[(Methoxyimino)methyl]-2-fluorphenyl |
| XXVIIa-265 | 3-[(Methoxyimino)methyl]-2-methylphenyl |
| XXVIIa-266 | 3-[(Methoxyimino)methyl]-4,5-difluorphenyl |
| XXVIIa-267 | 3-[(Methoxyimino)methyl]-4,6-difluorphenyl |
| XXVIIa-268 | 3-[(Methoxyimino)methyl]-4-chlorphenyl |
| XXVIIa-269 | 3-[(Methoxyimino)methyl]-4-fluorphenyl |
| XXVIIa-270 | 3-[(Methoxyimino)methyl]-4-methylphenyl |
| XXVIIa-271 | 3-[(Methoxyimino)methyl]-5,6-difluorphenyl |
| XXVIIa-272 | 3-[(Methoxyimino)methyl]-5-chlorphenyl |
| XXVIIa-273 | 3-[(Methoxyimino)methyl]-5-fluorphenyl |
| XXVIIa-274 | 3-[(Methoxyimino)methyl]-5-methylphenyl |
| XXVIIa-275 | 3-[(Methoxyimino)methyl]-6-chlorphenyl |
| XXVIIa-276 | 3-[(Methoxyimino)methyl]-6-fluorphenyl |
| XXVIIa-277 | 3-[(Methoxyimino)methyl]-6-methylphenyl |
| XXVIIa-278 | 3-[(Methoxyimino)methyl]phenyl |
| XXVIIa-279 | 3-[(Methylsulfonyl)oxy]phenyl |
| XXVIIa-280 | 4,5-Dichlor-2-(prop-2-in-1-yloxy)phenyl |
| XXVIIa-281 | 4,5-Dichlor-2-[(methylsulfonyl)oxy]phenyl |
| XXVIIa-282 | 4,5-Dichlor-3-(prop-2-in-1-yloxy)phenyl |
| XXVIIa-283 | 4,5-Dichlor-3-[(methylsulfonyl)oxy]phenyl |
| XXVIIa-284 | 4,5-Difluor-2-(prop-2-in-1-yloxy)phenyl |
| XXVIIa-285 | 4,5-Difluor-2-[(methylsulfonyl)oxy]phenyl |
| XXVIIa-286 | 4,5-Difluor-2-formylphenyl |
| XXVIIa-287 | 4,5-Difluor-3-(prop-2-in-1-yloxy)phenyl |
| XXVIIa-288 | 4,5-Difluor-3-[(methylsulfonyl)oxy]phenyl |
| XXVIIa-289 | 4,5-Difluor-3-formylphenyl |
| XXVIIa-290 | 4,6-Dichlor-2-(prop-2-in-1-yloxy)phenyl |
| XXVIIa-291 | 4,6-Dichlor-2-[(methylsulfonyl)oxy]phenyl |
| XXVIIa-292 | 4,6-Dichlor-3-(prop-2-in-1-yloxy)phenyl |
| XXVIIa-293 | 4,6-Dichlor-3-[(methylsulfonyl)oxy]phenyl |
| XXVIIa-294 | 4,6-Difluor-2-(prop-2-in-1-yloxy)phenyl |
| XXVIIa-295 | 4,6-Difluor-2-[(methylsulfonyl)oxy]phenyl |
| XXVIIa-296 | 4,6-Difluor-2-formylphenyl |
| XXVIIa-297 | 4,6-Difluor-3-(prop-2-in-1-yloxy)phenyl |
| XXVIIa-298 | 4,6-Difluor-3-[(methylsulfonyl)oxy]phenyl |
| XXVIIa-299 | 4,6-Difluor-3-formylphenyl |
| XXVIIa-300 | 4-(Allyloxy)-2,3-dichlorphenyl |
| XXVIIa-301 | 4-(Allyloxy)-2,3-difluorphenyl |
| XXVIIa-302 | 4-(Allyloxy)-2,5-dichlorphenyl |
| XXVIIa-303 | 4-(Allyloxy)-2,5-difluorphenyl |
| XXVIIa-304 | 4-(Allyloxy)-2,6-dichlorphenyl |
| XXVIIa-305 | 4-(Allyloxy)-2,6-difluorphenyl |
| XXVIIa-306 | 4-(Allyloxy)-2-chlorphenyl |
| XXVIIa-307 | 4-(Allyloxy)-2-fluorphenyl |
| XXVIIa-308 | 4-(Allyloxy)-2-methylphenyl |
| XXVIIa-309 | 4-(Allyloxy)-3,5-dichlorphenyl |
| XXVIIa-310 | 4-(Allyloxy)-3,5-difluorphenyl |
| XXVIIa-311 | 4-(Allyloxy)-3,6-dichlorphenyl |
| XXVIIa-312 | 4-(Allyloxy)-3,6-difluorphenyl |
| XXVIIa-313 | 4-(Allyloxy)- 3 -chlorphenyl |
| XXVIIa-314 | 4-(Allyloxy)-3-fluorphenyl |
| XXVIIa-315 | 4-(Allyloxy)-3-methylphenyl |
| XXVIIa-316 | 4-(Allyloxy)phenyl |
| XXVIIa-317 | 4-(Cyanomethoxy)-2,3-dichlorphenyl |
| XXVIIa-318 | 4-(Cyanomethoxy)-2,3-difluorphenyl |
| XXVIIa-319 | 4-(Cyanomethoxy)-2,5-dichlorphenyl |
| XXVIIa-320 | 4-(Cyanomethoxy)-2,5-difluorphenyl |
| XXVIIa-321 | 4-(Cyanomethoxy)-2,6-dichlorphenyl |
| XXVIIa-322 | 4-(Cyanomethoxy)-2,6-difluorphenyl |
| XXVIIa-323 | 4-(Cyanomethoxy)-2-chlorphenyl |
| XXVIIa-324 | 4-(Cyanomethoxy)-2-fluorphenyl |
| XXVIIa-325 | 4-(Cyanomethoxy)-2-methylphenyl |
| XXVIIa-326 | 4-(Cyanomethoxy)-3,5-dichlorphenyl |
| XXVIIa-327 | 4-(Cyanomethoxy)-3,5-difluorphenyl |
| XXVIIa-328 | 4-(Cyanomethoxy)-3,6-dichlorphenyl |
| XXVIIa-329 | 4-(Cyanomethoxy)-3,6-difluorphenyl |
| XXVIIa-330 | 4-(Cyanomethoxy)-3-chlorphenyl |
| XXVIIa-331 | 4-(Cyanomethoxy)-3-fluorphenyl |
| XXVIIa-332 | 4-(Cyanomethoxy)-3-methylphenyl |
| XXVIIa-333 | 4-(Cyanomethoxy)phenyl |
| XXVIIa-334 | 4-(Prop-2-in-1-yloxy)phenyl |
| XXVIIa-335 | 4-Chlor-2-(prop-2-in-1-yloxy)phenyl |
| XXVIIa-336 | 4-Chlor-2-[(methylsulfonyl)oxy]phenyl |
| XXVIIa-337 | 4-Chlor-2-formylphenyl |
| XXVIIa-338 | 4-Chlor-3-(prop-2-in-1-yloxy)phenyl |
| XXVIIa-339 | 4-Chlor-3-[(methylsulfonyl)oxy]phenyl |
| XXVIIa-340 | 4-Chlor-3-formylphenyl |
| XXVIIa-341 | 4-Fluor-2-(prop-2-in-1-yloxy)phenyl |
| XXVIIa-342 | 4-Fluor-2-[(methylsulfonyl)oxy]phenyl |
| XXVIIa-343 | 4-Fluor-2-formylphenyl |
| XXVIIa-344 | 4-Fluor-3-(prop-2-in-1-yloxy)phenyl |
| XXVIIa-345 | 4-Fluor-3-[(methylsulfonyl)oxy]phenyl |
| XXVIIa-346 | 4-Fluor-3-formylphenyl |
| XXVIIa-347 | 4-Formyl-2-methylphenyl |
| XXVIIa-348 | 4-Formyl-3-methylphenyl |
| XXVIIa-349 | 4-Formylphenyl |
| XXVIIa-350 | 4-Methyl-2-(prop-2-in-1-yloxy)phenyl |
| XXVIIa-351 | 4-Methyl-2-[(methylsulfonyl)oxy]phenyl |
| XXVIIa-352 | 4-Methyl-3-(prop-2-in-1-yloxy)phenyl |
| XXVIIa-353 | 4-Methyl-3-[(methylsulfonyl)oxy]phenyl |
| XXVIIa-354 | 4-[(Hydroxyimino)methyl]-2,3-difluorphenyl |
| XXVIIa-355 | 4-[(Hydroxyimino)methyl]-2,5-difluorphenyl |
| XXVIIa-356 | 4-[(Hydroxyimino)methyl]-2,6-difluorphenyl |
| XXVIIa-357 | 4-[(Hydroxyimino)methyl]-2-chlorphenyl |
| XXVIIa-358 | 4-[(Hydroxyimino)methyl]-2-fluorphenyl |
| XXVIIa-359 | 4-[(Hydroxyimino)methyl]-2-methylphenyl |
| XXVIIa-360 | 4-[(Hydroxyimino)methyl]-3,5-difluorphenyl |
| XXVIIa-361 | 4-[(Hydroxyimino)methyl]-3,6-difluorphenyl |
| XXVIIa-362 | 4-[(Hydroxyimino)methyl]-3-chlorphenyl |
| XXVIIa-363 | 4-[(Hydroxyimino)methyl]-3-fluorphenyl |
| XXVIIa-364 | 4-[(Hydroxyimino)methyl]-3-methylphenyl |
| XXVIIa-365 | 4-[(Hydroxyimino)methyl]phenyl |
| XXVIIa-366 | 4-[(Methoxyimino)methyl]-2,3-difluorphenyl |
| XXVIIa-367 | 4-[(Methoxyimino)methyl]-2,5-difluorphenyl |
| XXVIIa-368 | 4-[(Methoxyimino)methyl]-2,6-difluorphenyl |
| XXVIIa-369 | 4-[(Methoxyimino)methyl]-2-chlorphenyl |
| XXVIIa-370 | 4-[(Methoxyimino)methyl]-2-fluorphenyl |
| XXVIIa-371 | 4-[(Methoxyimino)methyl]-2-methylphenyl |
| XXVIIa-372 | 4-[(Methoxyimino)methyl]-3,5-difluorphenyl |
| XXVIIa-373 | 4-[(Methoxyimino)methyl]-3,6-difluorphenyl |
| XXVIIa-374 | 4-[(Methoxyimino)methyl]-3-chlorphenyl |
| XXVIIa-375 | 4-[(Methoxyimino)methyl]-3-fluorphenyl |
| XXVIIa-376 | 4-[(Methoxyimino)methyl]-3-methylphenyl |
| XXVIIa-377 | 4-[(Methoxyimino)methyl]phenyl |
| XXVIIa-378 | 4-[(Methylsulfonyl)oxy]phenyl |
| XXVIIa-379 | 5,6-Dichlor-2-(prop-2-in-1-yloxy)phenyl |
| XXVIIa-380 | 5,6-Dichlor-2-[(methylsulfonyl)oxy]phenyl |
| XXVIIa-381 | 5,6-Dichlor-3-(prop-2-in-1-yloxy)phenyl |
| XXVIIa-382 | 5,6-Dichlor-3-[(methylsulfonyl)oxy]phenyl |
| XXVIIa-383 | 5,6-Difluor-2-(prop-2-in-1-yloxy)phenyl |
| XXVIIa-384 | 5,6-Difluor-2-[(methylsulfonyl)oxy]phenyl |
| XXVIIa-385 | 5,6-Difluor-2-formylphenyl |
| XXVIIa-386 | 5,6-Difluor-3-(prop-2-in-1-yloxy)phenyl |
| XXVIIa-387 | 5,6-Difluor-3-[(methylsulfonyl)oxy]phenyl |
| XXVIIa-388 | 5,6-Difluor-3-formylphenyl |
| XXVIIa-389 | 5-Chlor-2-(prop-2-in-1-yloxy)phenyl |
| XXVIIa-390 | 5-Chlor-2-[(methylsulfonyl)oxy]phenyl |
| XXVIIa-391 | 5-Chlor-2-formylphenyl |
| XXVIIa-392 | 5-Chlor-3-(prop-2-in-1-yloxy)phenyl |
| XXVIIa-393 | 5-Chlor-3-[(methylsulfonyl)oxy]phenyl |
| XXVIIa-394 | 5-Chlor-3-formylphenyl |
| XXVIIa-395 | 5-Fluor-2-(prop-2-in-1-yloxy)phenyl |
| XXVIIa-396 | 5-Fluor-2-[(methylsulfonyl)oxy]phenyl |
| XXVIIa-397 | 5-Fluor-2-formylphenyl |
| XXVIIa-398 | 5-Fluor-3-(prop-2-in-1-yloxy)phenyl |
| XXVIIa-399 | 5-Fluor-3-[(methylsulfonyl)oxy]phenyl |
| XXVIIa-400 | 5-Fluor-3-formylphenyl |
| XXVIIa-401 | 5-Methyl-2-(prop-2-in-1-yloxy)phenyl |
| XXVIIa-402 | 5-Methyl-2-[(methylsulfonyl)oxy]phenyl |
| XXVIIa-403 | 5-Methyl-3-(prop-2-in-1-yloxy)phenyl |
| XXVIIa-404 | 5-Methyl-3-[(methylsulfonyl)oxy]phenyl |
| XXVIIa-405 | 6-Chlor-2-(prop-2-in-1-yloxy)phenyl |
| XXVIIa-406 | 6-Chlor-2-[(methylsulfonyl)oxy]phenyl |
| XXVIIa-407 | 6-Chlor-2-formylphenyl |
| XXVIIa-408 | 6-Chlor-3-(prop-2-in-1-yloxy)phenyl |
| XXVIIa-409 | 6-Chlor-3-[(methylsulfonyl)oxy]phenyl |
| XXVIIa-410 | 6-Chlor-3-formylphenyl |
| XXVIIa-411 | 6-Fluor-2-(prop-2-in-1-yloxy)phenyl |
| XXVIIa-412 | 6-Fluor-2-[(methylsulfonyl)oxy]phenyl |
| XXVIIa-413 | 6-Fluor-2-formylphenyl |
| XXVIIa-414 | 6-Fluor-3-(prop-2-in-1-yloxy)phenyl |
| XXVIIa-415 | 6-Fluor-3-[(methylsulfonyl)oxy]phenyl |
| XXVIIa-416 | 6-Fluor-3-formylphenyl |
| XXVIIa-417 | 6-Methyl-2-(prop-2-in-1-yloxy)phenyl |
| XXVIIa-418 | 6-Methyl-2-[(methylsulfonyl)oxy]phenyl |
| XXVIIa-419 | 6-Methyl-3-(prop-2-in-1-yloxy)phenyl |
| XXVIIa-420 | 6-Methyl-3-[(methylsulfonyl)oxy]phenyl |

**Tabelle 4:**

| | |
|---|---|
| 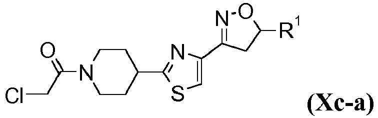 | |

| Bsp. | R¹ |
|---|---|
| Xc-a-1 | 2,3-Dichlor-4-(prop-2-in-1-yloxy)phenyl |
| Xc-a-2 | 2,3-Dichlor-4-[(methylsulfonyl)oxy]phenyl |
| Xc-a-3 | 2,3-Difluor-4-(prop-2-in-1-yloxy)phenyl |
| Xc-a-4 | 2,3-Difluor-4-[(methylsulfonyl)oxy]phenyl |
| Xc-a-5 | 2,3-Difluor-4-formylphenyl |
| Xc-a-6 | 2,4-Dichlor-3-(prop-2-in-1-yloxy)phenyl |
| Xc-a-7 | 2,4-Dichlor-3-[(methylsulfonyl)oxy]phenyl |
| Xc-a-8 | 2,4-Difluor-3-(prop-2-in-1-yloxy)phenyl |
| Xc-a-9 | 2,4-Difluor-3-[(methylsulfonyl)oxy]phenyl |
| Xc-a-10 | 2,4-Difluor-3-formylphenyl |
| Xc-a-11 | 2,5-Dichlor-3-(prop-2-in-1-yloxy)phenyl |
| Xc-a-12 | 2,5-Dichlor-3-[(methylsulfonyl)oxy]phenyl |
| Xc-a-13 | 2,5-Dichlor-4-(prop-2-in-1-yloxy)phenyl |
| Xc-a-14 | 2,5-Dichlor-4-[(methylsulfonyl)oxy]phenyl |
| Xc-a-15 | 2,5-Difluor-3-(prop-2-in-1-yloxy)phenyl |
| Xc-a-16 | 2,5-Difluor-3-[(methylsulfonyl)oxy]phenyl |
| Xc-a-17 | 2,5-Difluor-3-formylphenyl |
| Xc-a-18 | 2,5-Difluor-4-(prop-2-in-1-yloxy)phenyl |
| Xc-a-19 | 2,5-Difluor-4-[(methylsulfonyl)oxy]phenyl |
| Xc-a-20 | 2,5-Difluor-4-formylphenyl |
| Xc-a-21 | 2,6-Dichlor-3-(prop-2-in-1-yloxy)phenyl |
| Xc-a-22 | 2,6-Dichlor-3-[(methylsulfonyl)oxy]phenyl |
| Xc-23 | 2,6-Dichlor-4-(prop-2-in-1-yloxy)phenyl |
| Xc-a-24 | 2,6-Dichlor-4-[(methylsulfonyl)oxy]phenyl |
| Xc-a-25 | 2,6-Difluor-3-(prop-2-in-1-yloxy)phenyl |
| Xc-a-26 | 2,6-Difluor-3-[(methylsulfonyl)oxy]phenyl |
| Xc-a-27 | 2,6-Difluor-3-formylphenyl |
| Xc-a-28 | 2,6-Difluor-4-(prop-2-in-1-yloxy)phenyl |
| Xc-a-29 | 2,6-Difluor-4-[(methylsulfonyl)oxy]phenyl |
| Xc-a-30 | 2,6-Difluor-4-formylphenyl |
| Xc-a-31 | 2-(Allyloxy)-3,4-dichlorphenyl |
| Xc-a-32 | 2-(Allyloxy)-3,4-difluorphenyl |
| Xc-a-33 | 2-(Allyloxy)-3,5-dichlorphenyl |
| Xc-a-34 | 2-(Allyloxy)-3,5-difluorphenyl |
| Xc-a-35 | 2-(Allyloxy)-3,6-dichlorphenyl |
| Xc-a-36 | 2-(Allyloxy)-3,6-difluorphenyl |
| Xc-a-37 | 2-(Allyloxy)-3-chlorphenyl |
| Xc-a-38 8 | 2-(Allyloxy)-3-fluorphenyl |
| Xc-a-39 | 2-(Allyloxy)-3-methylphenyl |
| Xc-a-40 | 2-(Allyloxy)-4,5-dichlorphenyl |
| Xc-a-41 | 2-(Allyloxy)-4,5-difluorphenyl |
| Xc-a-42 | 2-(Allyloxy)-4,6-dichlorphenyl |
| Xc-a-43 | 2-(Allyloxy)-4,6-difluorphenyl |
| Xc-a-44 | 2-(Allyloxy)-4-chlorphenyl |
| Xc-a-45 | 2-(Allyloxy)-4-fluorphenyl |
| Xc-a-46 | 2-(Allyloxy)-4-methylphenyl |
| Xc-a-47 | 2-(Allyloxy)-5,6-dichlorphenyl |
| Xc-a-48 | 2-(Allyloxy)-5,6-difluorphenyl |
| Xc-a-49 | 2-(Allyloxy)-5-chlorphenyl |
| Xc-a-50 | 2-(Allyloxy)-5-fluorphenyl |
| Xc-a-51 | 2-(Allyloxy)-5-methylphenyl |
| Xc-a-52 | 2-(Allyloxy)-6-chlorphenyl |
| Xc-a-53 | 2-(Allyloxy)-6-fluorphenyl |
| Xc-a-54 | 2-(Allyloxy)-6-methylphenyl |
| Xc-a-55 | 2-(Allyloxy)phenyl |
| Xc-a-56 | 2-(Cyanomethoxy)-3,4-dichlorphenyl |
| Xc-a-57 | 2-(Cyanomethoxy)-3,4-difluorphenyl |
| Xc-a-58 | 2-(Cyanomethoxy)-3,5-dichlorphenyl |
| Xc-a-59 | 2-(Cyanomethoxy)-3,5-difluorphenyl |
| Xc-a-60 | 2-(Cyanomethoxy)-3,6-dichlorphenyl |
| Xc-a-61 | 2-(Cyanomethoxy)-3,6-difluorphenyl |
| Xc-a-62 | 2-(Cyanomethoxy)-3-chlorphenyl |
| Xc-a-63 | 2-(Cyanomethoxy)-3-fluorphenyl |
| Xc-a-64 | 2-(Cyanomethoxy)-3 -methylphenyl |
| Xc-a-65 | 2-(Cyanomethoxy)-4,5-dichlorphenyl |
| Xc-a-66 | 2-(Cyanomethoxy)-4,5-difluorphenyl |
| Xc-a-67 | 2-(Cyanomethoxy)-4,6-dichlorphenyl |
| Xc-a-68 | 2-(Cyanomethoxy)-4,6-difluorphenyl |
| Xc-a-69 | 2-(Cyanomethoxy)-4-chlorphenyl |
| Xc-a-70 | 2-(Cyanomethoxy)-4-fluorphenyl |
| Xc-a-71 | 2-(Cyanomethoxy)-4-methylphenyl |
| Xc-a-72 | 2-(Cyanomethoxy)-5,6-dichlorphenyl |
| Xc-a-73 | 2-(Cyanomethoxy)-5,6-difluorphenyl |
| Xc-a-74 | 2-(Cyanomethoxy)-5-chlorphenyl |
| Xc-a-75 | 2-(Cyanomethoxy)-5-fluorphenyl |
| Xc-a-76 | 2-(Cyanomethoxy)-5-methylphenyl |
| Xc-a-77 | 2-(Cyanomethoxy)-6-chlorphenyl |
| Xc-a-78 | 2-(Cyanomethoxy)-6-fluorphenyl |
| Xc-a-79 | 2-(Cyanomethoxy)-6-methylphenyl |
| Xc-a-80 | 2-(Cyanomethoxy)phenyl |
| Xc-a-81 | 2-(Prop-2-in-1-yloxy)phenyl |
| Xc-a-82 | 2-(Prop-2-in-1-yloxy)-4-(trifluormethyl)phenyl |
| Xc-a-83 | 2-Chlor-3-(prop-2-in-1-yloxy)phenyl |
| Xc-a-84 | 2-Chlor-3-[(methylsulfonyl)oxy]phenyl |
| Xc-a-85 | 2-Chlor-3-formylphenyl |
| Xc-a-86 | 2-Chlor-4-(prop-2-in-1-yloxy)phenyl |
| Xc-a-87 | 2-Chlor-4-[(methylsulfonyl)oxy]phenyl |
| Xc-a-88 | 2-Chlor-4-formylphenyl |
| Xc-a-89 | 2-Fluor-3-(prop-2-in-1-yloxy)phenyl |
| Xc-a-90 | 2-Fluor-3-[(methylsulfonyl)oxy]phenyl |
| Xc-a-91 | 2- Fluor- 3- formylphenyl |
| Xc-a-92 | 2-Fluor-4-(prop-2-in-1-yloxy)phenyl |
| Xc-a-93 | 2-Fluor-4-[(methylsulfonyl)oxy]phenyl |
| Xc-a-94 | 2-Fluor-4-formylphenyl |
| Xc-a-95 | 2-Formyl-3-methylphenyl |
| Xc-a-96 | 2-Formyl-4-methylphenyl |
| Xc-a-97 | 2-Formyl-5-methylphenyl |
| Xc-a-98 | 2-Formyl-6-methylphenyl |
| Xc-a-99 | 2-Formylphenyl |
| Xc-a-100 | 2-Methyl-3-(prop-2-in-1-yloxy)phenyl |
| Xc-a-101 | 2-Methyl-3-[(methylsulfonyl)oxy]phenyl |
| Xc-a-102 | 2-Methyl-4-(prop-2-in-1-yloxy)phenyl |
| Xc-a-103 | 2-Methyl-4-[(methylsulfonyl)oxy]phenyl |
| Xc-a-104 | 2-[(Hydroxyimino)methyl]-3,4-difluorphenyl |
| Xc-a-105 | 2-[(Hydroxyimino)methyl]-3,5-difluorphenyl |
| Xc-a-106 | 2-[(Hydroxyimino)methyl]-3,6-difluorphenyl |
| Xc-a-107 | 2-[(Hydroxyimino)methyl]-3-chlorphenyl |
| Xc-a-108 | 2-[(Hydroxyimino)methyl]-3-fluorphenyl |
| Xc-a-109 | 2-[(Hydroxyimino)methyl]-3-methylphenyl |
| Xc-a-110 | 2-[(Hydroxyimino)methyl]-4,5-difluorphenyl |
| Xc-a-111 | 2-[(Hydroxyimino)methyl]-4,6-difluorphenyl |
| Xc-a-112 | 2-[(Hydroxyimino)methyl]-4-chlorphenyl |
| Xc-a-113 | 2-[(Hydroxyimino)methyl]-4-fluorphenyl |
| Xc-a-114 | 2-[(Hydroxyimino)methyl]-4-methylphenyl |
| Xc-a-115 | 2-[(Hydroxyimino)methyl]-5,6-difluorphenyl |
| Xc-a-116 | 2-[(Hydroxyimino)methyl]-5-chlorphenyl |
| Xc-a-117 | 2-[(Hydroxyimino)methyl]-5-fluorphenyl |
| Xc-a-118 | 2-[(Hydroxyimino)methyl]-5-methylphenyl |
| Xc-a-119 | 2-[(Hydroxyimino)methyl]-6-chlorphenyl |
| Xc-a-120 | 2-[(Hydroxyimino)methyl]-6-fluorphenyl |
| Xc-a-121 | 2-[(Hydroxyimino)methyl]-6-methylphenyl |
| Xc-a-122 | 2-[(Hydroxyimino)methyl]phenyl |
| Xc-a-123 | 2-[(Methoxyimino)methyl]-3,4-difluorphenyl |
| Xc-a-124 | 2-[(Methoxyimino)methyl]-3,5-difluorphenyl |
| Xc-a-125 | 2-[(Methoxyimino)methyl]-3,6-difluorphenyl |
| Xc-a-126 | 2-[(Methoxyimino)methyl]-3-chlorphenyl |
| Xc-a-127 | 2-[(Methoxyimino)methyl]-3-fluorphenyl |
| Xc-a-128 | 2-[(Methoxyimino)methyl]-3-methylphenyl |
| Xc-a-129 | 2-[(Methoxyimino)methyl]-4,5-difluorphenyl |
| Xc-a-130 | 2-[(Methoxyimino)methyl]-4,6-difluorphenyl |
| Xc-a-131 | 2-[(Methoxyimino)methyl]-4-chlorphenyl |
| Xc-a-132 | 2-[(Methoxyimino)methyl]-4-fluorphenyl |
| Xc-a-133 | 2-[(Methoxyimino)methyl]-4-methylphenyl |
| Xc-a-134 | 2-[(Methoxyimino)methyl]-5,6-difluorphenyl |
| Xc-a-135 | 2-[(Methoxyimino)methyl]-5-chlorphenyl |
| Xc-a-136 | 2-[(Methoxyimino)methyl]-5-fluorphenyl |
| Xc-a-137 | 2-[(Methoxyimino)methyl]-5-methylphenyl |
| Xc-a-138 | 2-[(Methoxyimino)methyl]-6-chlorphenyl |
| Xc-a-139 | 2-[(Methoxyimino)methyl]-6-fluorphenyl |
| Xc-a-140 | 2-[(Methoxyimino)methyl]-6-methylphenyl |
| Xc-a-141 | 2-[(Methoxyimino)methyl]phenyl |
| Xc-a-142 | 2-[(Methylsulfonyl)oxy]phenyl |
| Xc-a-143 | 2-[(Methylsulfonyl)oxy]-4-(trifluormethyl)phenyl |
| Xc-a-144 | 3,4-Dichlor-2-(prop-2-in-1-yloxy)phenyl |
| Xc-a-145 | 3,4-Dichlor-2-[(methylsulfonyl)oxy]phenyl |
| Xc-a-146 | 3,4-Difluor-2-(prop-2-in-1-yloxy)phenyl |
| Xc-a-147 | 3,4-Difluor-2-[(methylsulfonyl)oxy]phenyl |
| Xc-a-148 | 3,4-Difluor-2-formylphenyl |
| Xc-a-149 | 3,5-Dichlor-2-(prop-2-in-1-yloxy)phenyl |
| Xc-a-150 | 3,5-Dichlor-2-[(methylsulfonyl)oxy]phenyl |
| Xc-a-151 | 3,5-Dichlor-4-(prop-2-in-1-yloxy)phenyl |
| Xc-a-152 | 3,5-Dichlor-4-[(methylsulfonyl)oxy]phenyl |
| Xc-a-153 | 3,5-Difluor-2-(prop-2-in-1-yloxy)phenyl |
| Xc-a-154 | 3,5-Difluor-2-[(methylsulfonyl)oxy]phenyl |
| Xc-a-155 | 3,5-Difluor-2-formylphenyl |
| Xc-a-156 | 3,5-Difluor-4-(prop-2-in-1-yloxy)phenyl |
| Xc-a-157 | 3,5-Difluor-4-[(methylsulfonyl)oxy]phenyl |
| Xc-a-158 | 3,5-Difluor-4-formylphenyl |
| Xc-a-159 | 3,6-Dichlor-2-(prop-2-in-1-yloxy)phenyl |
| Xc-a-160 | 3,6-Dichlor-2-[(methylsulfonyl)oxy]phenyl |
| Xc-a-161 | 3,6-Dichlor-4-(prop-2-in-1-yloxy)phenyl |
| Xc-a-162 | 3,6-Dichlor-4-[(methylsulfonyl)oxy]phenyl |
| Xc-a-163 | 3,6-Difluor-2-(prop-2-in-1-yloxy)phenyl |
| Xc-a-164 | 3,6-Difluor-2-[(methylsulfonyl)oxy]phenyl |
| Xc-a-165 | 3,6-Difluor-2-formylphenyl |
| Xc-a-166 | 3,6-Difluor-4-(prop-2-in-1-yloxy)phenyl |
| Xc-a-167 | 3,6-Difluor-4-[(methylsulfonyl)oxy]phenyl |
| Xc-a-168 | 3,6-Difluor-4-formylphenyl |
| Xc-a-169 | 3-(Allyloxy)-2,4-dichlorphenyl |
| Xc-a-170 | 3-(Allyloxy)-2,4-difluorphenyl |
| Xc-a-171 | 3-(Allyloxy)-2,5-dichlorphenyl |
| Xc-a-172 | 3-(Allyloxy)-2,5-difluorphenyl |
| Xc-a-173 | 3-(Allyloxy)-2,6-dichlorphenyl |
| Xc-a-174 | 3-(Allyloxy)-2,6-difluorphenyl |
| Xc-a-175 | 3-(Allyloxy)-2-chlorphenyl |
| Xc-a-176 | 3-(Allyloxy)-2-fluorphenyl |
| Xc-a-177 | 3-(Allyloxy)-2-methylphenyl |
| Xc-a-178 | 3-(Allyloxy)-4,5-dichlorphenyl |
| Xc-a-179 | 3-(Allyloxy)-4,5-difluorphenyl |
| Xc-a-180 | 3-(Allyloxy)-4,6-dichlorphenyl |
| Xc-a-181 | 3-(Allyloxy)-4,6-difluorphenyl |
| Xc-a-182 | 3-(Allyloxy)-4-chlorphenyl |
| Xc-a-183 | 3-(Allyloxy)-4-fluorphenyl |
| Xc-a-184 | 3-(Allyloxy)-4-methylphenyl |
| Xc-a-185 | 3-(Allyloxy)-5,6-dichlorphenyl |
| Xc-a-186 | 3-(Allyloxy)-5,6-difluorphenyl |
| Xc-a-187 | 3-(Allyloxy)-5-chlorphenyl |
| Xc-a-188 | 3-(Allyloxy)-5-fluorphenyl |
| Xc-a-189 | 3 -(Allyloxy)- 5- methylphenyl |
| Xc-a-190 | 3-(Allyloxy)-6-chlorphenyl |
| Xc-a-191 | 3-(Allyloxy)-6-fluorphenyl |
| Xc-a-192 | 3-(Allyloxy)-6-methylphenyl |
| Xc-a-193 | 3-(Allyloxy)phenyl |
| Xc-a-194 | 3-(Cyanomethoxy)-2,4-dichlorphenyl |
| Xc-a-195 | 3-(Cyanomethoxy)-2,4-difluorphenyl |
| Xc-a-196 | 3-(Cyanomethoxy)-2,5-dichlorphenyl |
| Xc-a-197 | 3-(Cyanomethoxy)-2,5-difluorphenyl |
| Xc-a-198 | 3-(Cyanomethoxy)-2,6-dichlorphenyl |
| Xc-a-199 | 3-(Cyanomethoxy)-2,6-difluorphenyl |
| Xc-a-200 | 3-(Cyanomethoxy)-2-chlorphenyl |
| Xc-a-201 | 3-(Cyanomethoxy)-2-fluorphenyl |
| Xc-a-202 | 3-(Cyanomethoxy)-2-methylphenyl |
| Xc-a-203 | 3-(Cyanomethoxy)-4,5-dichlorphenyl |
| Xc-a-204 | 3-(Cyanomethoxy)-4,5-difluorphenyl |
| Xc-a-205 | 3-(Cyanomethoxy)-4,6-dichlorphenyl |
| Xc-a-206 | 3-(Cyanomethoxy)-4,6-difluorphenyl |
| Xc-a-207 | 3-(Cyanomethoxy)-4-chlorphenyl |
| Xc-a-208 | 3-(Cyanomethoxy)-4-fluorphenyl |
| Xc-a-209 | 3-(Cyanomethoxy)-4-methylphenyl |
| Xc-a-210 | 3-(Cyanomethoxy)-5,6-dichlorphenyl |
| Xc-a-211 | 3-(Cyanomethoxy)-5,6-difluorphenyl |
| Xc-a-212 | 3-(Cyanomethoxy)-5-chlorphenyl |
| Xc-a-213 | 3-(Cyanomethoxy)-5-fluorphenyl |
| Xc-a- 214 | 3 -(Cyanomethoxy)-5-methylphenyl |
| Xc-a-215 | 3-(Cyanomethoxy)-6-chlorphenyl |
| Xc-a- 216 | 3 -(Cyanomethoxy)-6-fluorphenyl |
| Xc-a-217 | 3-(Cyanomethoxy)-6-methylphenyl |
| Xc-a-218 | 3-(Cyanomethoxy)phenyl |
| Xc-a-219 | 3-(Prop-2-in-1-yloxy)phenyl |
| Xc-a-220 | 3-Chlor-2-(prop-2-in-1-yloxy)phenyl |
| Xc-a-221 | 3-Chlor-2-[(methylsulfonyl)oxy]phenyl |
| Xc-a-222 | 3-Chlor-2-formylphenyl |
| Xc-a-223 | 3-Chlor-4-(prop-2-in-1-yloxy)phenyl |
| Xc-a-224 | 3-Chlor-4-[(methylsulfonyl)oxy]phenyl |
| Xc-a-225 | 3-Chlor-4-formylphenyl |
| Xc-a-226 | 3-Fluor-2-(prop-2-in-1-yloxy)phenyl |
| Xc-a-227 | 3-Fluor-2-[(methylsulfonyl)oxy]phenyl |
| Xc-a-228 | 3-Fluor-2-formylphenyl |
| Xc-a-229 | 3-Fluor-4-(prop-2-in-1-yloxy)phenyl |
| Xc-a-230 | 3-Fluor-4-[(methylsulfonyl)oxy]phenyl |
| Xc-a-231 | 3-Fluor-4-formylphenyl |
| Xc-a-232 | 3-Formyl-2-methylphenyl |
| Xc-a-233 | 3-Formyl-4-methylphenyl |
| Xc-a-234 | 3-Formyl-5-methylphenyl |
| Xc-a-235 | 3-Formyl-6-methylphenyl |
| Xc-a-236 | 3-Formylphenyl |
| Xc-a-237 | 3-Methyl-2-(prop-2-in-1-yloxy)phenyl |
| Xc-a-238 | 3-Methyl-2-[(methylsulfonyl)oxy]phenyl |
| Xc-a-239 | 3-Methyl-4-(prop-2-in-1-yloxy)phenyl |
| Xc-a-240 | 3-Methyl-4-[(methylsulfonyl)oxy]phenyl |
| Xc-a-241 | 3-[(Hydroxyimino)methyl]-2,4-difluorphenyl |
| Xc-a-242 | 3-[(Hydroxyimino)methyl]-2,5-difluorphenyl |
| Xc-a-243 | 3-[(Hydroxyimino)methyl]-2,6-difluorphenyl |
| Xc-a-244 | 3-[(Hydroxyimino)methyl]-2-chlorphenyl |
| Xc-a-245 | 3-[(Hydroxyimino)methyl]-2-fluorphenyl |
| Xc-a-246 | 3-[(Hydroxyimino)methyl]-2-methylphenyl |
| Xc-a-247 | 3-[(Hydroxyimino)methyl]-4,5-difluorphenyl |
| Xc-a-248 | 3-[(Hydroxyimino)methyl]-4,6-difluorphenyl |
| Xc-a-249 | 3-[(Hydroxyimino)methyl]-4-chlorphenyl |
| Xc-a-250 | 3-[(Hydroxyimino)methyl]-4-fluorphenyl |
| Xc-a-251 | 3-[(Hydroxyimino)methyl]-4-methylphenyl |
| Xc-a-252 | 3-[(Hydroxyimino)methyl]-5,6-difluorphenyl |
| Xc-a-253 | 3-[(Hydroxyimino)methyl]-5-chlorphenyl |
| Xc-a-254 | 3-[(Hydroxyimino)methyl]-5-fluorphenyl |
| Xc-a-255 | 3-[(Hydroxyimino)methyl]-5-methylphenyl |
| Xc-a-256 | 3-[(Hydroxyimino)methyl]-6-chlorphenyl |
| Xc-a-257 | 3-[(Hydroxyimino)methyl]-6-fluorphenyl |
| Xc-a-258 | 3-[(Hydroxyimino)methyl]-6-methylphenyl |
| Xc-a-259 | 3-[(Hydroxyimino)methyl]phenyl |
| Xc-a-260 | 3-[(Methoxyimino)methyl]-2,4-difluorphenyl |
| Xc-a-261 | 3-[(Methoxyimino)methyl]-2,5-difluorphenyl |
| Xc-a-262 | 3-[(Methoxyimino)methyl]-2,6-difluorphenyl |
| Xc-a-263 | 3-[(Methoxyimino)methyl]-2-chlorphenyl |
| Xc-a-264 | 3 - [(Methoxyimino)methyl]-2-fluorphenyl |
| Xc-a-265 | 3-[(Methoxyimino)methyl]-2-methylphenyl |
| Xc-a-266 | 3-[(Methoxyimino)methyl]-4,5-difluorphenyl |
| Xc-a-267 | 3-[(Methoxyimino)methyl]-4,6-difluorphenyl |
| Xc-a-268 | 3-[(Methoxyimino)methyl]-4-chlorphenyl |
| Xc-a-269 | 3-[(Methoxyimino)methyl]-4-fluorphenyl |
| Xc-a-270 | 3-[(Methoxyimino)methyl]-4-methylphenyl |
| Xc-a-271 | 3-[(Methoxyimino)methyl]-5,6-difluorphenyl |
| Xc-a-272 | 3-[(Methoxyimino)methyl]-5-chlorphenyl |
| Xc-a-273 | 3-[(Methoxyimino)methyl]-5-fluorphenyl |
| Xc-a-274 | 3-[(Methoxyimino)methyl]-5-methylphenyl |
| Xc-a-275 | 3-[(Methoxyimino)methyl]-6-chlorphenyl |
| Xc-a-276 | 3-[(Methoxyimino)methyl]-6-fluorphenyl |
| Xc-a-277 | 3-[(Methoxyimino)methyl]-6-methylphenyl |
| Xc-a-278 | 3-[(Methoxyimino)methyl]phenyl |
| Xc-a-279 | 3-[(Methylsulfonyl)oxy]phenyl |
| Xc-a-280 | 4,5-Dichlor-2-(prop-2-in-1-yloxy)phenyl |
| Xc-a-281 | 4,5-Dichlor-2-[(methylsulfonyl)oxy]phenyl |
| Xc-a-282 | 4,5-Dichlor-3-(prop-2-in-1-yloxy)phenyl |
| Xc-a-283 | 4,5-Dichlor-3-[(methylsulfonyl)oxy]phenyl |
| Xc-a-284 | 4,5-Difluor-2-(prop-2-in-1-yloxy)phenyl |
| Xc-a-285 | 4,5-Difluor-2-[(methylsulfonyl)oxy]phenyl |
| Xc-a-286 | 4,5-Difluor-2-formylphenyl |
| Xc-a-287 | 4,5-Difluor-3-(prop-2-in-1-yloxy)phenyl |
| Xc-a-288 | 4,5-Difluor-3-[(methylsulfonyl)oxy]phenyl |
| Xc-a-289 | 4,5-Difluor-3-formylphenyl |
| Xc-a-290 | 4,6-Dichlor-2-(prop-2-in-1-yloxy)phenyl |
| Xc-a-291 | 4,6-Dichlor-2-[(methylsulfonyl)oxy]phenyl |
| Xc-a-292 | 4,6-Dichlor-3-(prop-2-in-1-yloxy)phenyl |
| Xc-a-293 | 4,6-Dichlor-3-[(methylsulfonyl)oxy]phenyl |
| Xc-a-294 | 4,6-Difluor-2-(prop-2-in-1-yloxy)phenyl |
| Xc-a-295 | 4,6-Difluor-2-[(methylsulfonyl)oxy]phenyl |
| Xc-a-296 | 4,6-Difluor-2-formylphenyl |
| Xc-a-297 | 4,6-Difluor-3-(prop-2-in-1-yloxy)phenyl |
| Xc-a-298 | 4,6-Difluor-3-[(methylsulfonyl)oxy]phenyl |
| Xc-a-299 | 4,6-Difluor-3-formylphenyl |
| Xc-a-300 | 4-(Allyloxy)-2,3-dichlorphenyl |
| Xc-a-301 | 4-(Allyloxy)-2,3-difluorphenyl |
| Xc-a-302 | 4-(Allyloxy)-2,5-dichlorphenyl |
| Xc-a-303 | 4-(Allyloxy)-2,5-difluorphenyl |
| Xc-a-304 | 4-(Allyloxy)-2,6-dichlorphenyl |
| Xc-a-305 | 4-(Allyloxy)-2,6-difluorphenyl |
| Xc-a-306 | 4-(Allyloxy)-2-chlorphenyl |
| Xc-a-307 | 4-(Allyloxy)-2-fluorphenyl |
| Xc-a-308 | 4-(Allyloxy)-2-methylphenyl |
| Xc-a-309 | 4-(Allyloxy)-3,5-dichlorphenyl |
| Xc-a-310 | 4-(Allyloxy)-3,5-difluorphenyl |
| Xc-a-311 | 4-(Allyloxy)-3,6-dichlorphenyl |
| Xc-a-312 | 4-(Allyloxy)-3,6-difluorphenyl |
| Xc-a-313 | 4-(Allyloxy)-3-chlorphenyl |
| Xc-a-314 | 4-(Allyloxy)-3-fluorphenyl |
| Xc-a-315 | 4-(Allyloxy)-3-methylphenyl |
| Xc-a-316 | 4-(Allyloxy)phenyl |
| Xc-a-317 | 4-(Cyanomethoxy)-2,3-dichlorphenyl |
| Xc-a-318 | 4-(Cyanomethoxy)-2,3-difluorphenyl |
| Xc-a-319 | 4-(Cyanomethoxy)-2,5-dichlorphenyl |
| Xc-a-320 | 4-(Cyanomethoxy)-2,5-difluorphenyl |
| Xc-a-321 | 4-(Cyanomethoxy)-2,6-dichlorphenyl |
| Xc-a-322 | 4-(Cyanomethoxy)-2,6-difluorphenyl |
| Xc-a-323 | 4-(Cyanomethoxy)-2-chlorphenyl |
| Xc-a-324 | 4-(Cyanomethoxy)-2-fluorphenyl |
| Xc-a-325 | 4-(Cyanomethoxy)-2-methylphenyl |
| Xc-a-326 | 4-(Cyanomethoxy)-3,5-dichlorphenyl |
| Xc-a-327 | 4-(Cyanomethoxy)-3,5-difluorphenyl |
| Xc-a-328 | 4-(Cyanomethoxy)-3,6-dichlorphenyl |
| Xc-a-329 | 4-(Cyanomethoxy)-3,6-difluorphenyl |
| Xc-a-330 | 4-(Cyanomethoxy)-3-chlorphenyl |
| Xc-a-331 | 4-(Cyanomethoxy)-3-fluorphenyl |
| Xc-a-332 | 4-(Cyanomethoxy)-3-methylphenyl |
| Xc-a-333 | 4-(Cyanomethoxy)phenyl |
| Xc-a-334 | 4-(Prop-2-in-1-yloxy)phenyl |
| Xc-a-335 | 4-Chlor-2-(prop-2-in-1-yloxy)phenyl |
| Xc-a-336 | 4-Chlor-2-[(methylsulfonyl)oxy]phenyl |
| Xc-a-337 | 4-Chlor-2-formylphenyl |
| Xc-a-338 | 4-Chlor-3-(prop-2-in-1-yloxy)phenyl |
| Xc-a-33 9 | 4-Chlor-3-[(methylsulfonyl)oxy]phenyl |
| Xc-a-340 | 4-Chlor-3-formylphenyl |
| Xc-a-341 | 4-Fluor-2-(prop-2-in-1-yloxy)phenyl |
| Xc-a-342 | 4-Fluor-2-[(methylsulfonyl)oxy]phenyl |
| Xc-a-343 | 4-Fluor-2-formylphenyl |
| Xc-a-344 | 4-Fluor-3-(prop-2-in-1-yloxy)phenyl |
| Xc-a-345 | 4-Fluor-3-[(methylsulfonyl)oxy]phenyl |
| Xc-a-346 | 4-Fluor-3-formylphenyl |
| Xc-a-347 | 4-Formyl-2-methylphenyl |
| Xc-a-348 | 4-Formyl-3-methylphenyl |
| Xc-a-349 | 4-Formylphenyl |
| Xc-a-350 | 4-Methyl-2-(prop-2-in-1-yloxy)phenyl |
| Xc-a-351 | 4-Methyl-2-[(methylsulfonyl)oxy]phenyl |
| Xc-a-352 | 4-Methyl-3-(prop-2-in-1-yloxy)phenyl |
| Xc-a-353 | 4-Methyl-3-[(methylsulfonyl)oxy]phenyl |
| Xc-a-354 | 4-[(Hydroxyimino)methyl]-2,3-difluorphenyl |
| Xc-a-355 | 4-[(Hydroxyimino)methyl]-2,5-difluorphenyl |
| Xc-a-35 6 | 4-[(Hydroxyimino)methyl]-2,6-difluorphenyl |
| Xc-a-357 | 4-[(Hydroxyimino)methyl]-2-chlorphenyl |
| Xc-a-358 | 4-[(Hydroxyimino)methyl]-2-fluorphenyl |
| Xc-a-359 | 4-[(Hydroxyimino)methyl]-2-methylphenyl |
| Xc-a-360 | 4-[(Hydroxyimino)methyl]-3,5-difluorphenyl |
| Xc-a-361 | 4-[(Hydroxyimino)methyl]-3,6-difluorphenyl |
| Xc-a-362 | 4-[(Hydroxyimino)methyl]-3-chlorphenyl |
| Xc-a-363 | 4-[(Hydroxyimino)methyl]-3-fluorphenyl |
| Xc-a-364 | 4-[(Hydroxyimino)methyl]-3-methylphenyl |
| Xc-a-365 | 4-[(Hydroxyimino)methyl]phenyl |
| Xc-a-366 | 4-[(Methoxyimino)methyl]-2,3-difluorphenyl |
| Xc-a-367 | 4-[(Methoxyimino)methyl]-2,5-difluorphenyl |
| Xc-a-368 | 4-[(Methoxyimino)methyl]-2,6-difluorphenyl |
| Xc-a-369 | 4-[(Methoxyimino)methyl]-2-chlorphenyl |
| Xc-a-370 | 4-[(Methoxyimino)methyl]-2-fluorphenyl |
| Xc-a-371 | 4-[(Methoxyimino)methyl]-2-methylphenyl |
| Xc-a-372 | 4-[(Methoxyimino)methyl]-3,5-difluorphenyl |
| Xc-a-373 | 4-[(Methoxyimino)methyl]-3,6-difluorphenyl |
| Xc-a-374 | 4-[(Methoxyimino)methyl]-3-chlorphenyl |
| Xc-a-375 | 4-[(Methoxyimino)methyl]-3-fluorphenyl |
| Xc-a-376 | 4-[(Methoxyimino)methyl]-3-methylphenyl |
| Xc-a-377 | 4-[(Methoxyimino)methyl]phenyl |
| Xc-a-378 | 4-[(Methylsulfonyl)oxy]phenyl |
| Xc-a-379 | 5,6-Dichlor-2-(prop-2-in-1-yloxy)phenyl |
| Xc-a-380 | 5,6-Dichlor-2-[(methylsulfonyl)oxy]phenyl |
| Xc-a-381 | 5,6-Dichlor-3-(prop-2-in-1-yloxy)phenyl |
| Xc-a-382 | 5,6-Dichlor-3-[(methylsulfonyl)oxy]phenyl |
| Xc-a-383 | 5,6-Difluor-2-(prop-2-in-1-yloxy)phenyl |
| Xc-a-384 | 5,6-Difluor-2-[(methylsulfonyl)oxy]phenyl |
| Xc-a-385 | 5,6-Difluor-2-formylphenyl |
| Xc-a-386 | 5,6-Difluor-3-(prop-2-in-1-yloxy)phenyl |
| Xc-a-387 | 5,6-Difluor-3-[(methylsulfonyl)oxy]phenyl |
| Xc-a-388 | 5,6-Difluor-3-formylphenyl |
| Xc-a-389 | 5-Chlor-2-(prop-2-in-1-yloxy)phenyl |
| Xc-a-390 | 5-Chlor-2-[(methylsulfonyl)oxy]phenyl |
| Xc-a-391 | 5-Chlor-2-formylphenyl |
| Xc-a-392 | 5-Chlor-3-(prop-2-in-1-yloxy)phenyl |
| Xc-a-393 | 5-Chlor-3-[(methylsulfonyl)oxy]phenyl |
| Xc-a-394 | 5-Chlor-3-formylphenyl |
| Xc-a-395 | 5-Fluor-2-(prop-2-in-1-yloxy)phenyl |
| Xc-a-396 | 5-Fluor-2-[(methylsulfonyl)oxy]phenyl |
| Xc-a-397 | 5-Fluor-2-formylphenyl |
| Xc-a-398 | 5-Fluor-3-(prop-2-in-1-yloxy)phenyl |
| Xc-a-399 | 5-Fluor-3-[(methylsulfonyl)oxy]phenyl |
| Xc-a-400 | 5-Fluor-3-formylphenyl |
| Xc-a-401 | 5-Methyl-2-(prop-2-in-1-yloxy)phenyl |
| Xc-a-402 | 5-Methyl-2-[(methylsulfonyl)oxy]phenyl |
| Xc-a-403 | 5-Methyl-3-(prop-2-in-1-yloxy)phenyl |
| Xc-a-404 | 5-Methyl-3-[(methylsulfonyl)oxy]phenyl |
| Xc-a-405 | 6-Chlor-2-(prop-2-in-1-yloxy)phenyl |
| Xc-a-406 | 6-Chlor-2-[(methylsulfonyl)oxy]phenyl |
| Xc-a-407 | 6-Chlor-2-formylphenyl |
| Xc-a-408 | 6-Chlor-3-(prop-2-in-1-yloxy)phenyl |
| Xc-a-409 | 6-Chlor-3-[(methylsulfonyl)oxy]phenyl |
| Xc-a-410 | 6-Chlor-3-formylphenyl |
| Xc-a-411 | 6-Fluor-2-(prop-2-in-1-yloxy)phenyl |
| Xc-a-412 | 6-Fluor-2-[(methylsulfonyl)oxy]phenyl |
| Xc-a-413 | 6-Fluor-2-formylphenyl |
| Xc-a-414 | 6-Fluor-3-(prop-2-in-1-yloxy)phenyl |
| Xc-a-415 | 6-Fluor-3-[(methylsulfonyl)oxy]phenyl |
| Xc-a-416 | 6-Fluor-3-formylphenyl |
| Xc-a-417 | 6-Methyl-2-(prop-2-in-1-yloxy)phenyl |
| Xc-a-418 | 6-Methyl-2-[(methylsulfonyl)oxy]phenyl |
| Xc-a-419 | 6-Methyl-3-(prop-2-in-1-yloxy)phenyl |
| Xc-a-420 | 6-Methyl-3-[(methylsulfonyl)oxy]phenyl |

**Tabelle 5:**

| | |
|---|---|
| 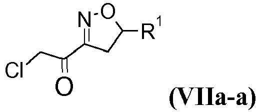 | |

| Bsp. | R¹ |
|---|---|
| VIIa-a-1 | 2,3-Dichlor-4-(prop-2-in-1-yloxy)phenyl |
| VIIa-a-2 | 2,3-Dichlor-4-[(methylsulfonyl)oxy]phenyl |
| VIIa-a-3 | 2,3-Difluor-4-(prop-2-in-1-yloxy)phenyl |
| VIIa-a-4 | 2,3-Difluor-4-[(methylsulfonyl)oxy]phenyl |
| VIIa-a-5 | 2,3-Difluor-4-formylphenyl |
| VIIa-a-6 | 2,4-Dichlor-3-(prop-2-in-1-yloxy)phenyl |
| VIIa-a-7 | 2,4-Dichlor-3-[(methylsulfonyl)oxy]phenyl |
| VIIa-a-8 | 2,4-Difluor-3-(prop-2-in-1-yloxy)phenyl |
| VIIa-a-9 | 2,4-Difluor-3-[(methylsulfonyl)oxy]phenyl |
| VIIa-a-10 | 2,4-Difluor-3-formylphenyl |
| VIIa-a-11 | 2,5-Dichlor-3-(prop-2-in-1-yloxy)phenyl |
| VIIa-a-12 | 2,5-Dichlor-3-[(methylsulfonyl)oxy]phenyl |
| VIIa-a-13 | 2,5-Dichlor-4-(prop-2-in-1 -yloxy)phenyl |
| VIIa-a-14 | 2,5-Dichlor-4-[(methylsulfonyl)oxy]phenyl |
| VIIa-a-15 | 2,5-Difluor-3-(prop-2-in-1-yloxy)phenyl |
| VIIa-a-16 | 2,5-Difluor-3-[(methylsulfonyl)oxy]phenyl |
| VIIa-a-17 | 2,5-Difluor-3-formylphenyl |
| VIIa-a-18 | 2,5-Difluor-4-(prop-2-in-1-yloxy)phenyl |
| VIIa-a-19 | 2,5-Difluor-4-[(methylsulfonyl)oxy]phenyl |
| VIIa-a-20 | 2,5-Difluor-4-formylphenyl |
| VIIa-a-21 | 2,6-Dichlor-3-(prop-2-in-1-yloxy)phenyl |
| VIIa-a-22 | 2,6-Dichlor-3-[(methylsulfonyl)oxy]phenyl |
| VIIa-a-23 | 2,6-Dichlor-4-(prop-2-in-1-yloxy)phenyl |
| VIIa-a-24 | 2,6-Dichlor-4-[(methylsulfonyl)oxy]phenyl |
| VIIa-a-25 | 2,6-Difluor-3-(prop-2-in-1-yloxy)phenyl |
| VIIa-a-26 | 2,6-Difluor-3-[(methylsulfonyl)oxy]phenyl |
| VIIa-a-27 | 2,6-Difluor-3-formylphenyl |
| VIIa-a-28 | 2,6-Difluor-4-(prop-2-in-1-yloxy)phenyl |
| VIIa-a-29 | 2,6-Difluor-4-[(methylsulfonyl)oxy]phenyl |
| VIIa-a-30 | 2,6-Difluor-4-formylphenyl |
| VIIa-a-31 | 2-(Allyloxy)-3,4-dichlorphenyl |
| VIIa-a-32 | 2-(Allyloxy)-3,4-difluorphenyl |
| VIIa-a-33 | 2-(Allyloxy)-3,5-dichlorphenyl |
| VIIa-a-34 | 2-(Allyloxy)-3,5-difluorphenyl |
| VIIa-a-35 | 2-(Allyloxy)-3,6-dichlorphenyl |
| VIIa-a-36 | 2-(Allyloxy)-3,6-difluorphenyl |
| VIIa-a-37 | 2-(Allyloxy)-3-chlorphenyl |
| VIIa-a-38 | 2-(Allyloxy)-3-fluorphenyl |
| VIIa-a-39 | 2-(Allyloxy)-3-methylphenyl |
| VIIa-a-40 | 2-(Allyloxy)-4,5-dichlorphenyl |
| VIIa-a-41 | 2-(Allyloxy)-4,5-difluorphenyl |
| VIIa-a-42 | 2-(Allyloxy)-4,6-dichlorphenyl |
| VIIa-a-43 | 2-(Allyloxy)-4,6-difluorphenyl |
| VIIa-a-44 | 2-(Allyloxy)-4-chlorphenyl |
| VIIa-a-45 | 2-(Allyloxy)-4-fluorphenyl |
| VIIa-a-46 | 2-(Allyloxy)-4-methylphenyl |
| VIIa-a-47 | 2-(Allyloxy)-5,6-dichlorphenyl |
| VIIa-a-48 | 2-(Allyloxy)-5,6-difluorphenyl |
| VIIa-a-49 | 2-(Allyloxy)-5-chlorphenyl |
| VIIa-a-50 | 2-(Allyloxy)-5-fluorphenyl |
| VIIa-a-51 | 2-(Allyloxy)-5-methylphenyl |
| VIIa-a-52 | 2-(Allyloxy)-6-chlorphenyl |
| VIIa-a-53 | 2-(Allyloxy)-6-fluorphenyl |
| VIIa-a-54 | 2-(Allyloxy)-6-methylphenyl |
| VIIa-a-55 | 2-(Allyloxy)phenyl |
| VIIa-a-56 | 2-(Cyanomethoxy)-3,4-dichlorphenyl |
| VIIa-a-57 | 2-(Cyanomethoxy)-3,4-difluorphenyl |
| VIIa-a-58 | 2-(Cyanomethoxy)-3,5-dichlorphenyl |
| VIIa-a-59 | 2-(Cyanomethoxy)-3,5-difluorphenyl |
| VIIa-a-60 | 2-(Cyanomethoxy)-3,6-dichlorphenyl |
| VIIa-a-61 | 2-(Cyanomethoxy)-3,6-difluorphenyl |
| VIIa-a-62 | 2-(Cyanomethoxy)-3-chlorphenyl |
| VIIa-a-63 | 2-(Cyanomethoxy)-3-fluorphenyl |
| VIIa-a-64 | 2-(Cyanomethoxy)-3-methylphenyl |
| VIIa-a-65 | 2-(Cyanomethoxy)-4,5-dichlorphenyl |
| VIIa-a-66 | 2-(Cyanomethoxy)-4,5-difluorphenyl |
| VIIa-a-67 | 2-(Cyanomethoxy)-4,6-dichlorphenyl |
| VIIa-a-68 | 2-(Cyanomethoxy)-4,6-difluorphenyl |
| VIIa-a-69 | 2-(Cyanomethoxy)-4-chlorphenyl |
| VIIa-a-70 | 2-(Cyanomethoxy)-4-fluorphenyl |
| VIIa-a-71 | 2-(Cyanomethoxy)-4-methylphenyl |
| VIIa-a-72 | 2-(Cyanomethoxy)-5,6-dichlorphenyl |
| VIIa-a-73 | 2-(Cyanomethoxy)-5,6-difluorphenyl |
| VIIa-a-74 | 2-(Cyanomethoxy)-5-chlorphenyl |
| VIIa-a-75 | 2-(Cyanomethoxy)-5-fluorphenyl |
| VIIa-a-76 | 2-(Cyanomethoxy)-5-methylphenyl |
| VIIa-a-77 | 2-(Cyanomethoxy)-6-chlorphenyl |
| VIIa-a-78 | 2-(Cyanomethoxy)-6-fluorphenyl |
| VIIa-a-79 | 2-(Cyanomethoxy)-6-methylphenyl |
| VIIa-a-80 | 2-(Cyanomethoxy)phenyl |
| VIIa-a-81 | 2-(Prop-2-in-1-yloxy)phenyl |
| VIIa-a-82 | 2-(Prop-2-in-1-yloxy)-4-(trifluormethyl)phenyl |
| VIIa-a-83 | 2-Chlor-3-(prop-2-in-1-yloxy)phenyl |
| VIIa-a-84 | 2-Chlor-3-[(methylsulfonyl)oxy]phenyl |
| VIIa-a-85 | 2-Chlor-3-formylphenyl |
| VIIa-a-86 | 2-Chlor-4-(prop-2-in-1-yloxy)phenyl |
| VIIa-a-87 | 2-Chlor-4-[(methylsulfonyl)oxy]phenyl |
| VIIa-a-88 | 2-Chlor-4-formylphenyl |
| VIIa-a-89 | 2-Fluor-3-(prop-2-in-1-yloxy)phenyl |
| VIIa-a-90 | 2-Fluor-3-[(methylsulfonyl)oxy]phenyl |
| VIIa-a-91 | 2-Fluor-3-formylphenyl |
| VIIa-a-92 | 2-Fluor-4-(prop-2-in-1-yloxy)phenyl |
| VIIa-a-93 | 2-Fluor-4-[(methylsulfonyl)oxy]phenyl |
| VIIa-a-94 | 2-Fluor-4-formylphenyl |
| VIIa-a-95 | 2-Formyl-3-methylphenyl |
| VIIa-a-96 | 2-Formyl-4-methylphenyl |
| VIIa-a-97 | 2-Formyl-5-methylphenyl |
| VIIa-a-98 | 2-Formyl-6-methylphenyl |
| VIIa-a-99 | 2-Formylphenyl |
| VIIa-a-100 | 2-Methyl-3-(prop-2-in-1-yloxy)phenyl |
| VIIa-a-101 | 2-Methyl-3-[(methylsulfonyl)oxy]phenyl |
| VIIa-a-102 | 2-Methyl-4-(prop-2-in-1-yloxy)phenyl |
| VIIa-a-103 | 2-Methyl-4-[(methylsulfonyl)oxy]phenyl |
| VIIa-a-104 | 2-[(Hydroxyimino)methyl]-3,4-difluorphenyl |
| VIIa-a-105 | 2-[(Hydroxyimino)methyl]-3,5-difluorphenyl |
| VIIa-a-106 | 2-[(Hydroxyimino)methyl]-3,6-difluorphenyl |
| VIIa-a-107 | 2-[(Hydroxyimino)methyl]-3-chlorphenyl |
| VIIa-a-08 | 2-[(Hydroxyimino)methyl]-3-fluorphenyl |
| VIIa-a-109 | 2-[(Hydroxyimino)methyl]-3-methylphenyl |
| VIIa-a-110 | 2-[(Hydroxyimino)methyl]-4,5-difluorphenyl |
| VIIa-a-111 | 2-[(Hydroxyimino)methyl]-4,6-difluorphenyl |
| VIIa-a-112 | 2-[(Hydroxyimino)methyl]-4-chlorphenyl |
| VIIa-a-113 | 2-[(Hydroxyimino)methyl]-4-fluorphenyl |
| VIIa-a-114 | 2-[(Hydroxyimino)methyl]-4-methylphenyl |
| VIIa-a-115 | 2-[(Hydroxyimino)methyl]-5,6-difluorphenyl |
| VIIa-a-116 | 2-[(Hydroxyimino)methyl]-5-chlorphenyl |
| VIIa-a-117 | 2-[(Hydroxyimino)methyl]-5-fluorphenyl |
| VIIa-a-118 | 2-[(Hydroxyimino)methyl]-5-methylphenyl |
| VIIa-a-119 | 2-[(Hydroxyimino)methyl]-6-chlorphenyl |
| VIIa-a-120 | 2-[(Hydroxyimino)methyl]-6-fluorphenyl |
| VIIa-a-121 | 2-[(Hydroxyimino)methyl]-6-methylphenyl |
| VIIa-a-122 | 2-[(Hydroxyimino)methyl]phenyl |
| VIIa-a-123 | 2-[(Methoxyimino)methyl]-3,4-difluorphenyl |
| VIIa-a-124 | 2-[(Methoxyimino)methyl]-3,5-difluorphenyl |
| VIIa-a-125 | 2-[(Methoxyimino)methyl]-3,6-difluorphenyl |
| VIIa-a-126 | 2-[(Methoxyimino)methyl]-3-chlorphenyl |
| VIIa-a-127 | 2-[(Methoxyimino)methyl]-3-fluorphenyl |
| VIIa-a-128 | 2-[(Methoxyimino)methyl]-3-methylphenyl |
| VIIa-a-129 | 2-[(Methoxyimino)methyl]-4,5-difluorphenyl |
| VIIa-a-130 | 2-[(Methoxyimino)methyl]-4,6-difluorphenyl |
| VIIa-a-131 | 2-[(Methoxyimino)methyl]-4-chlorphenyl |
| VIIa-a-132 | 2-[(Methoxyimino)methyl]-4-fluorphenyl |
| VIIa-a-133 | 2-[(Methoxyimino)methyl]-4-methylphenyl |
| VIIa-a-134 | 2-[(Methoxyimino)methyl]-5,6-difluorphenyl |
| VIIa-a-135 | 2-[(Methoxyimino)methyl]-5-chlorphenyl |
| VIIa-a-136 | 2-[(Methoxyimino)methyl]-5-fluorphenyl |
| VIIa-a-137 | 2-[(Methoxyimino)methyl]-5-methylphenyl |
| VIIa-a-138 | 2-[(Methoxyimino)methyl]-6-chlorphenyl |
| VIIa-a-139 | 2-[(Methoxyimino)methyl]-6-fluorphenyl |
| VIIa-a-140 | 2-[(Methoxyimino)methyl]-6-methylphenyl |
| VIIa-a-141 | 2-[(Methoxyimino)methyl]phenyl |
| VIIa-a-142 | 2-[(Methylsulfonyl)oxy]phenyl |
| VIIa-a-143 | 2-[(Methylsulfonyl)oxy]-4-(trifluormethyl)phenyl |
| VIIa-a-144 | 3,4-Dichlor-2-(prop-2-in-1-yloxy)phenyl |
| VIIa-a-145 | 3,4-Dichlor-2-[(methylsulfonyl)oxy]phenyl |
| VIIa-a-146 | 3,4-Difluor-2-(prop-2-in-1-yloxy)phenyl |
| VIIa-a-147 | 3,4-Difluor-2-[(methylsulfonyl)oxy]phenyl |
| VIIa-a-148 | 3,4-Difluor-2-formylphenyl |
| VIIa-a-149 | 3,5-Dichlor-2-(prop-2-in-1-yloxy)phenyl |
| VIIa-a-150 | 3,5-Dichlor-2-[(methylsulfonyl)oxy]phenyl |
| VIIa-a-151 | 3,5-Dichlor-4-(prop-2-in-1-yloxy)phenyl |
| VIIa-a-152 | 3,5-Dichlor-4-[(methylsulfonyl)oxy]phenyl |
| VIIa-a-153 | 3,5-Difluor-2-(prop-2-in-1-yloxy)phenyl |
| VIIa-a-154 | 3,5-Difluor-2-[(methylsulfonyl)oxy]phenyl |
| VIIa-a-155 | 3,5-Difluor-2-formylphenyl |
| VIIa-a-156 | 3,5-Difluor-4-(prop-2-in-1-yloxy)phenyl |
| VIIa-a-157 | 3,5-Difluor-4-[(methylsulfonyl)oxy]phenyl |
| VIIa-a-158 | 3,5-Difluor-4-formylphenyl |
| VIIa-a-159 | 3,6-Dichlor-2-(prop-2-in-1-yloxy)phenyl |
| VIIa-a-160 | 3,6-Dichlor-2-[(methylsulfonyl)oxy]phenyl |
| VIIa-a-161 | 3,6-Dichlor-4-(prop-2-in-1-yloxy)phenyl |
| VIIa-a-162 | 3,6-Dichlor-4-[(methylsulfonyl)oxy]phenyl |
| VIIa-a-163 | 3,6-Difluor-2-(prop-2-in-1-yloxy)phenyl |
| VIIa-a-164 | 3,6-Difluor-2-[(methylsulfonyl)oxy]phenyl |
| VIIa-a-165 | 3,6-Difluor-2-formylphenyl |
| VIIa-a-166 | 3,6-Difluor-4-(prop-2-in-1-yloxy)phenyl |
| VIIa-a-167 | 3,6-Difluor-4-[(methylsulfonyl)oxy]phenyl |
| VIIa-a-168 | 3,6-Difluor-4-formylphenyl |
| VIIa-a-169 | 3-(Allyloxy)-2,4-dichlorphenyl |
| VIIa-a-170 | 3-(Allyloxy)-2,4-difluorphenyl |
| VIIa-a-171 | 3-(Allyloxy)-2,5-dichlorphenyl |
| VIIa-a-172 | 3-(Allyloxy)-2,5-difluorphenyl |
| VIIa-a-173 | 3-(Allyloxy)-2,6-dichlorphenyl |
| VIIa-a-174 | 3-(Allyloxy)-2,6-difluorphenyl |
| VIIa-a-175 | 3-(Allyloxy)-2-chlorphenyl |
| VIIa-a-176 | 3-(Allyloxy)-2-fluorphenyl |
| VIIa-a-177 | 3-(Allyloxy)-2-methylphenyl |
| VIIa-a-178 | 3-(Allyloxy)-4,5-dichlorphenyl |
| VIIa-a-179 | 3-(Allyloxy)-4,5-difluorphenyl |
| VIIa-a-180 | 3-(Allyloxy)-4,6-dichlorphenyl |
| VIIa-a-181 | 3-(Allyloxy)-4,6-difluorphenyl |
| VIIa-a-182 | 3-(Allyloxy)-4-chlorphenyl |
| VIIa-a-183 | 3-(Allyloxy)-4-fluorphenyl |
| VIIa-a-184 | 3-(Allyloxy)-4-methylphenyl |
| VIIa-a-185 | 3-(Allyloxy)-5,6-dichlorphenyl |
| VIIa-a-186 | 3-(Allyloxy)-5,6-difluorphenyl |
| VIIa-a-187 | 3-(Allyloxy)-5-chlorphenyl |
| VIIa-a-188 | 3-(Allyloxy)-5-fluorphenyl |
| VIIa-a-189 | 3-(Allyloxy)-5-methylphenyl |
| VIIa-a-190 | 3-(Allyloxy)-6-chlorphenyl |
| VIIa-a-191 | 3-(Allyloxy)-6-fluorphenyl |
| VIIa-a-192 | 3-(Allyloxy)-6-methylphenyl |
| VIIa-a-193 | 3-(Allyloxy)phenyl |
| VIIa-a-194 | 3-(Cyanomethoxy)-2,4-dichlorphenyl |
| VIIa-a-195 | 3-(Cyanomethoxy)-2,4-difluorphenyl |
| VIIa-a-196 | 3-(Cyanomethoxy)-2,5-dichlorphenyl |
| VIIa-a-197 | 3-(Cyanomethoxy)-2,5-difluorphenyl |
| VIIa-a-198 | 3-(Cyanomethoxy)-2,6-dichlorphenyl |
| VIIa-a-199 | 3-(Cyanomethoxy)-2,6-difluorphenyl |
| VIIa-a-200 | 3-(Cyanomethoxy)-2-chlorphenyl |
| VIIa-a-201 | 3-(Cyanomethoxy)-2-fluorphenyl |
| VIIa-a-202 | 3-(Cyanomethoxy)-2-methylphenyl |
| VIIa-a-203 | 3-(Cyanomethoxy)-4,5-dichlorphenyl |
| VIIa-a-204 | 3-(Cyanomethoxy)-4,5-difluorphenyl |
| VIIa-a-205 | 3-(Cyanomethoxy)-4,6-dichlorphenyl |
| VIIa-a-206 | 3-(Cyanomethoxy)-4,6-difluorphenyl |
| VIIa-a-207 | 3-(Cyanomethoxy)-4-chlorphenyl |
| VIIa-a-208 | 3-(Cyanomethoxy)-4-fluorphenyl |
| VIIa-a-209 | 3-(Cyanomethoxy)-4-methylphenyl |
| VIIa-a-210 | 3-(Cyanomethoxy)-5,6-dichlorphenyl |
| VIIa-a-211 | 3-(Cyanomethoxy)-5,6-difluorphenyl |
| VIIa-a-212 | 3-(Cyanomethoxy)-5-chlorphenyl |
| VIIa-a-213 | 3-(Cyanomethoxy)-5-fluorphenyl |
| VIIa-a-214 | 3-(Cyanomethoxy)-5-methylphenyl |
| VIIa-a-215 | 3-(Cyanomethoxy)-6-chlorphenyl |
| VIIa-a-216 | 3-(Cyanomethoxy)-6-fluorphenyl |
| VIIa-a-217 | 3-(Cyanomethoxy)-6-methylphenyl |
| VIIa-a-218 | 3-(Cyanomethoxy)phenyl |
| VIIa-a-219 | 3-(Prop-2-in-1-yloxy)phenyl |
| VIIa-a-220 | 3-Chlor-2-(prop-2-in-1-yloxy)phenyl |
| VIIa-a-221 | 3-Chlor-2-[(methylsulfonyl)oxy]phenyl |
| VIIa-a-222 | 3-Chlor-2-formylphenyl |
| VIIa-a-223 | 3-Chlor-4-(prop-2-in-1-yloxy)phenyl |
| VIIa-a-224 | 3-Chlor-4-[(methylsulfonyl)oxy]phenyl |
| VIIa-a-225 | 3-Chlor-4-formylphenyl |
| VIIa-a-226 | 3-Fluor-2-(prop-2-in-1-yloxy)phenyl |
| VIIa-a-227 | 3-Fluor-2-[(methylsulfonyl)oxy]phenyl |
| VIIa-a-228 | 3-Fluor-2-formylphenyl |
| VIIa-a-229 | 3-Fluor-4-(prop-2-in-1-yloxy)phenyl |
| VIIa-a-230 | 3-Fluor-4-[(methylsulfonyl)oxy]phenyl |
| VIIa-a-231 | 3-Fluor-4-formylphenyl |
| VIIa-a-232 | 3-Formyl-2-methylphenyl |
| VIIa-a-233 | 3-Formyl-4-methylphenyl |
| VIIa-a-234 | 3-Formyl-5-methylphenyl |
| VIIa-a-235 | 3-Formyl-6-methylphenyl |
| VIIa-a-236 | 3-Formylphenyl |
| VIIa-a-237 | 3-Methyl-2-(prop-2-in-1-yloxy)phenyl |
| VIIa-a-238 | 3-Methyl-2-[(methylsulfonyl)oxy]phenyl |
| VIIa-a-239 | 3-Methyl-4-(prop-2-in-1-yloxy)phenyl |
| VIIa-a-240 | 3-Methyl-4-[(methylsulfonyl)oxy]phenyl |
| VIIa-a-241 | 3-[(Hydroxyimino)methyl]-2,4-difluorphenyl |
| VIIa-a-242 | 3-[(Hydroxyimino)methyl]-2,5-difluorphenyl |
| VIIa-a-243 | 3-[(Hydroxyimino)methyl]-2,6-difluorphenyl |
| VIIa-a-244 | 3-[(Hydroxyimino)methyl]-2-chlorphenyl |
| VIIa-a-245 | 3-[(Hydroxyimino)methyl]-2-fluorphenyl |
| VIIa-a-246 | 3-[(Hydroxyimino)methyl]-2-methylphenyl |
| VIIa-a-247 | 3-[(Hydroxyimino)methyl]-4,5-difluorphenyl |
| VIIa-a-248 | 3-[(Hydroxyimino)methyl]-4,6-difluorphenyl |
| VIIa-a-249 | 3-[(Hydroxyimino)methyl]-4-chlorphenyl |
| VIIa-a-250 | 3-[(Hydroxyimino)methyl]-4-fluorphenyl |
| VIIa-a-251 | 3-[(Hydroxyimino)methyl]-4-methylphenyl |
| VIIa-a-252 | 3-[(Hydroxyimino)methyl]-5,6-difluorphenyl |
| VIIa-a-253 | 3-[(Hydroxyimino)methyl]-5-chlorphenyl |
| VIIa-a-254 | 3-[(Hydroxyimino)methyl]-5-fluorphenyl |
| VIIa-a-255 | 3-[(Hydroxyimino)methyl]-5-methylphenyl |
| VIIa-a-256 | 3-[(Hydroxyimino)methyl]-6-chlorphenyl |
| VIIa-a-257 | 3-[(Hydroxyimino)methyl]-6-fluorphenyl |
| VIIa-a-258 | 3-[(Hydroxyimino)methyl]-6-methylphenyl |
| VIIa-a-259 | 3-[(Hydroxyimino)methyl]phenyl |
| VIIa-a-260 | 3-[(Methoxyimino)methyl]-2,4-difluorphenyl |
| VIIa-a-261 | 3-[(Methoxyimino)methyl]-2,5-difluorphenyl |
| VIIa-a-262 | 3-[(Methoxyimino)methyl]-2,6-difluorphenyl |
| VIIa-a-263 | 3-[(Methoxyimino)methyl]-2-chlorphenyl |
| VIIa-a-264 | 3-[(Methoxyimino)methyl]-2-fluorphenyl |
| VIIa-a-265 | 3-[(Methoxyimino)methyl]-2-methylphenyl |
| VIIa-a-266 | 3-[(Methoxyimino)methyl]-4,5-difluorphenyl |
| VIIa-a-267 | 3-[(Methoxyimino)methyl]-4,6-difluorphenyl |
| VIIa-a-268 | 3-[(Methoxyimino)methyl]-4-chlorphenyl |
| VIIa-a-269 | 3-[(Methoxyimino)methyl]-4-fluorphenyl |
| VIIa-a-270 | 3-[(Methoxyimino)methyl]-4-methylphenyl |
| VIIa-a-271 | 3-[(Methoxyimino)methyl]-5,6-difluorphenyl |
| VIIa-a-272 | 3-[(Methoxyimino)methyl]-5-chlorphenyl |
| VIIa-a-273 | 3-[(Methoxyimino)methyl]-5-fluorphenyl |
| VIIa-a-274 | 3-[(Methoxyimino)methyl]-5-methylphenyl |
| VIIa-a-275 | 3-[(Methoxyimino)methyl]-6-chlorphenyl |
| VIIa-a-276 | 3-[(Methoxyimino)methyl]-6-fluorphenyl |
| VIIa-a-277 | 3-[(Methoxyimino)methyl]-6-methylphenyl |
| VIIa-a-278 | 3-[(Methoxyimino)methyl]phenyl |
| VIIa-a-279 | 3-[(Methylsulfonyl)oxy]phenyl |
| VIIa-a-280 | 4,5-Dichlor-2-(prop-2-in-1-yloxy)phenyl |
| VIIa-a-281 | 4,5-Dichlor-2-[(methylsulfonyl)oxy]phenyl |
| VIIa-a-282 | 4,5-Dichlor-3-(prop-2-in-1-yloxy)phenyl |
| VIIa-a-283 | 4,5-Dichlor-3-[(methylsulfonyl)oxy]phenyl |
| VIIa-a-284 | 4,5-Difluor-2-(prop-2-in-1-yloxy)phenyl |
| VIIa-a-285 | 4,5-Difluor-2-[(methylsulfonyl)oxy]phenyl |
| VIIa-a-286 | 4,5-Difluor-2-formylphenyl |
| VIIa-a-287 | 4,5-Difluor-3-(prop-2-in-1-yloxy)phenyl |
| VIIa-a-288 | 4,5-Difluor-3-[(methylsulfonyl)oxy] phenyl |
| VIIa-a-289 | 4,5-Difluor-3-formylphenyl |
| VIIa-a-290 | 4,6-Dichlor-2-(prop-2-in-1-yloxy)phenyl |
| VIIa-a-291 | 4,6-Dichlor-2-[(methylsulfonyl)oxy]phenyl |
| VIIa-a-292 | 4,6-Dichlor-3-(prop-2-in-1-yloxy)phenyl |
| VIIa-a-293 | 4,6-Dichlor-3-[(methylsulfonyl)oxy]phenyl |
| VIIa-a-294 | 4,6-Difluor-2-(prop-2-in-1-yloxy)phenyl |
| VIIa-a-295 | 4,6-Difluor-2-[(methylsulfonyl)oxy]phenyl |
| VIIa-a-296 | 4,6-Difluor-2-formylphenyl |
| VIIa-a-297 | 4,6-Difluor-3-(prop-2-in-1-yloxy)phenyl |
| VIIa-a-298 | 4,6-Difluor-3-[(methylsulfonyl)oxy]phenyl |
| VIIa-a-299 | 4,6-Difluor-3-formylphenyl |
| VIIa-a-300 | 4-(Allyloxy)-2,3-dichlorphenyl |
| VIIa-a-301 | 4-(Allyloxy)-2,3-difluorphenyl |
| VIIa-a-302 | 4-(Allyloxy)-2,5-dichlorphenyl |
| VIIa-a-303 | 4-(Allyloxy)-2,5-difluorphenyl |
| VIIa-a-304 | 4-(Allyloxy)-2,6-dichlorphenyl |
| VIIa-a-305 | 4-(Allyloxy)-2,6-difluorphenyl |
| VIIa-a-306 | 4-(Allyloxy)-2-chlorphenyl |
| VIIa-a-307 | 4-(Allyloxy)-2-fluorphenyl |
| VIIa-a-308 | 4-(Allyloxy)-2-methylphenyl |
| VIIa-a-309 | 4-(Allyloxy)-3,5-dichlorphenyl |
| VIIa-a-310 | 4-(Allyloxy)-3,5-difluorphenyl |
| VIIa-a-311 | 4-(Allyloxy)-3,6-dichlorphenyl |
| VIIa-a-312 | 4-(Allyloxy)-3,6-difluorphenyl |
| VIIa-a-313 | 4-(Allyloxy)-3-chlorphenyl |
| VIIa-a-314 | 4-(Allyloxy)-3-fluorphenyl |
| VIIa-a-315 | 4-(Allyloxy)-3-methylphenyl |
| VIIa-a-316 | 4-(Allyloxy)phenyl |
| VIIa-a-317 | 4-(Cyanomethoxy)-2,3-dichlorphenyl |
| VIIa-a-318 | 4-(Cyanomethoxy)-2,3-difluorphenyl |
| VIIa-a-319 | 4-(Cyanomethoxy)-2,5-dichlorphenyl |
| VIIa-a-320 | 4-(Cyanomethoxy)-2,5-difluorphenyl |
| VIIa-a-321 | 4-(Cyanomethoxy)-2,6-dichlorphenyl |
| VIIa-a-322 | 4-(Cyanomethoxy)-2,6-difluorphenyl |
| VIIa-a-323 | 4-(Cyanomethoxy)-2-chlorphenyl |
| VIIa-a-324 | 4-(Cyanomethoxy)-2-fluorphenyl |
| VIIa-a-325 | 4-(Cyanomethoxy)-2-methylphenyl |
| VIIa-a-326 | 4-(Cyanomethoxy)-3,5-dichlorphenyl |
| VIIa-a-327 | 4-(Cyanomethoxy)-3,5-difluorphenyl |
| VIIa-a-328 | 4-(Cyanomethoxy)-3,6-dichlorphenyl |
| VIIa-a-329 | 4-(Cyanomethoxy)-3,6-difluorphenyl |
| VIIa-a-3 30 | 4-(Cyanomethoxy)-3-chlorphenyl |
| VIIa-a-3 31 | 4-(Cyanomethoxy)-3-fluorphenyl |
| VIIa-a-332 | 4-(Cyanomethoxy)-3-methylphenyl |
| VIIa-a-333 | 4-(Cyanomethoxy)phenyl |
| VIIa-a-334 | 4-(Prop-2-in-1-yloxy)phenyl |
| VIIa-a-335 | 4-Chlor-2-(prop-2-in-1-yloxy)phenyl |
| VIIa-a-3 36 | 4-Chlor-2-[(methylsulfonyl)oxy]phenyl |
| VIIa-a-337 | 4-Chlor-2-formylphenyl |
| VIIa-a-338 | 4-Chlor-3-(prop-2-in-1-yloxy)phenyl |
| VIIa-a-3 39 | 4-Chlor-3-[(methylsulfonyl)oxy]phenyl |
| VIIa-a-340 | 4-Chlor-3-formylphenyl |
| VIIa-a-341 | 4-Fluor-2-(prop-2-in-1-yloxy)phenyl |
| VIIa-a-342 | 4-Fluor-2-[(methylsulfonyl)oxy]phenyl |
| VIIa-a-343 | 4-Fluor-2-formylphenyl |
| VIIa-a-344 | 4-Fluor-3-(prop-2-in-1-yloxy)phenyl |
| VIIa-a-345 | 4-Fluor-3-[(methylsulfonyl)oxy]phenyl |
| VIIa-a-346 | 4-Fluor-3-formylphenyl |
| VIIa-a-347 | 4-Formyl-2-methylphenyl |
| VIIa-a-348 | 4-Formyl-3-methylphenyl |
| VIIa-a-349 | 4-Formylphenyl |
| VIIa-a-350 | 4-Methyl-2-(prop-2-in-1-yloxy)phenyl |
| VIIa-a-3 51 | 4-Methyl-2-[(methylsulfonyl)oxy]phenyl |
| VIIa-a-352 | 4-Methyl-3-(prop-2-in-1-yloxy)phenyl |
| VIIa-a-353 | 4-Methyl-3-[(methylsulfonyl)oxy]phenyl |
| VIIa-a-3 54 | 4-[(Hydroxyimino)methyl]-2,3-difluorphenyl |
| VIIa-a-355 | 4-[(Hydroxyimino)methyl]-2,5-difluorphenyl |
| VIIa-a-356 | 4-[(Hydroxyimino)methyl]-2,6-difluorphenyl |
| VIIa-a-357 | 4-[(Hydroxyimino)methyl]-2-chlorphenyl |
| VIIa-a-3 58 | 4-[(Hydroxyimino)methyl]-2-fluorphenyl |
| VIIa-a-359 | 4-[(Hydroxyimino)methyl]-2-methylphenyl |
| VIIa-a-360 | 4-[(Hydroxyimino)methyl]-3,5-difluorphenyl |
| VIIa-a-3 61 | 4-[(Hydroxyimino)methyl]-3,6-difluorphenyl |
| VIIa-a-362 | 4-[(Hydroxyimino)methyl]-3-chlorphenyl |
| VIIa-a-363 | 4-[(Hydroxyimino)methyl]-3-fluorphenyl |
| VIIa-a-364 | 4-[(Hydroxyimino)methyl]-3-methylphenyl |
| VIIa-a-365 | 4-[(Hydroxyimino)methyl]phenyl |
| VIIa-a-366 | 4-[(Methoxyimino)methyl]-2,3-difluorphenyl |
| VIIa-a-367 | 4-[(Methoxyimino)methyl]-2,5-difluorphenyl |
| VIIa-a-368 | 4-[(Methoxyimino)methyl]-2,6-difluorphenyl |
| VIIa-a-3 69 | 4-[(Methoxyimino)methyl]-2-chlorphenyl |
| VIIa-a-3 70 | 4-[(Methoxyimino)methyl]-2-fluorphenyl |
| VIIa-a-3 71 | 4-[(Methoxyimino)methyl]-2-methylphenyl |
| VIIa-a-372 | 4-[(Methoxyimino)methyl]-3,5-difluorphenyl |
| VIIa-a-373 | 4-[(Methoxyimino)methyl]-3,6-difluorphenyl |
| VIIa-a-3 74 | 4-[(Methoxyimino)methyl]-3-chlorphenyl |
| VIIa-a-375 | 4-[(Methoxyimino)methyl]-3-fluorphenyl |
| VIIa-a-376 | 4-[(Methoxyimino)methyl]-3-methylphenyl |
| VIIa-a-377 | 4-[(Methoxyimino)methyl]phenyl |
| VIIa-a-378 | 4-[(Methylsulfonyl)oxy]phenyl |
| VIIa-a-379 | 5,6-Dichlor-2-(prop-2-in-1-yloxy)phenyl |
| VIIa-a-380 | 5,6-Dichlor-2-[(methylsulfonyl)oxy]phenyl |
| VIIa-a-381 | 5,6-Dichlor-3-(prop-2-in-1-yloxy)phenyl |
| VIIa-a-382 | 5,6-Dichlor-3-[(methylsulfonyl)oxy]phenyl |
| VIIa-a-3 83 | 5,6-Difluor-2-(prop-2-in-1-yloxy)phenyl |
| VIIa-a-384 | 5,6-Difluor-2-[(methylsulfonyl)oxy]phenyl |
| VIIa-a-385 | 5,6-Difluor-2-formylphenyl |
| VIIa-a-386 | 5,6-Difluor-3-(prop-2-in-1-yloxy)phenyl |
| VIIa-a-387 | 5,6-Difluor-3-[(methylsulfonyl)oxy]phenyl |
| VIIa-a-388 | 5,6-Difluor-3-formylphenyl |
| VIIa-a-389 | 5-Chlor-2-(prop-2-in-1-yloxy)phenyl |
| VIIa-a-390 | 5-Chlor-2-[(methylsulfonyl)oxy]phenyl |
| VIIa-a-391 | 5-Chlor-2-formylphenyl |
| VIIa-a-392 | 5-Chlor-3-(prop-2-in-1-yloxy)phenyl |
| VIIa-a-393 | 5-Chlor-3-[(methylsulfonyl)oxy]phenyl |
| VIIa-a-394 | 5-Chlor-3-formylphenyl |
| VIIa-a-395 | 5-Fluor-2-(prop-2-in-1-yloxy)phenyl |
| VIIa-a-396 | 5-Fluor-2-[(methylsulfonyl)oxy]phenyl |
| VIIa-a-397 | 5-Fluor-2-formylphenyl |
| VIIa-a-398 | 5-Fluor-3-(prop-2-in-1-yloxy)phenyl |
| VIIa-a-399 | 5-Fluor-3-[(methylsulfonyl)oxy]phenyl |
| VIIa-a-400 | 5-Fluor-3-formylphenyl |
| VIIa-a-401 | 5-Methyl-2-(prop-2-in-1-yloxy)phenyl |
| VIIa-a-402 | 5-Methyl-2-[(methylsulfonyl)oxy]phenyl |
| VIIa-a-403 | 5-Methyl-3-(prop-2-in-1-yloxy)phenyl |
| VIIa-a-404 | 5-Methyl-3-[(methylsulfonyl)oxy]phenyl |
| VIIa-a-405 | 6-Chlor-2-(prop-2-in-1-yloxy)phenyl |
| VIIa-a-406 | 6-Chlor-2-[(methylsulfonyl)oxy]phenyl |
| VIIa-a-407 | 6-Chlor-2-formylphenyl |
| VIIa-a-408 | 6-Chlor-3 -(prop-2-in-1-yloxy)phenyl |
| VIIa-a-409 | 6-Chlor-3-[(methylsulfonyl)oxy]phenyl |
| VIIa-a-410 | 6-Chlor-3-formylphenyl |
| VIIa-a-411 | 6-Fluor-2-(prop-2-in-1-yloxy)phenyl |
| VIIa-a-412 | 6-Fluor-2-[(methylsulfonyl)oxy]phenyl |
| VIIa-a-413 | 6-Fluor-2-formylphenyl |
| VIIa-a-414 | 6-Fluor-3-(prop-2-in-1-yloxy)phenyl |
| VIIa-a-415 | 6-Fluor-3-[(methylsulfonyl)oxy]phenyl |
| VIIa-a-416 | 6-Fluor-3-formylphenyl |
| VIIa-a-417 | 6-Methyl-2-(prop-2-in-1-yloxy)phenyl |
| VIIa-a-418 | 6-Methyl-2-[(methylsulfonyl)oxy]phenyl |
| VIIa-a-419 | 6-Methyl-3-(prop-2-in-1-yloxy)phenyl |
| VIIa-a-420 | 6-Methyl-3-[(methylsulfonyl)oxy]phenyl |

**Tabelle 6:**

| | |
|---|---|
| 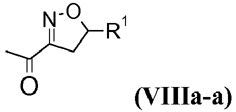 | |

| Bsp. | R¹ |
|---|---|
| VIIIa-a-1 | 2,3-Dichlor-4-(prop-2-in-1-yloxy)phenyl |
| VIIIa-a-2 | 2,3-Dichlor-4-[(methylsulfonyl)oxy]phenyl |
| VIIIa-a-3 | 2,3-Difluor-4-(prop-2-in-1-yloxy)phenyl |
| VIIIa-a-4 | 2,3-Difluor-4-[(methylsulfonyl)oxy]phenyl |
| VIIIa-a-5 | 2,3-Difluor-4-formylphenyl |
| VIIIa-a-6 | 2,4-Dichlor-3-(prop-2-in-1-yloxy)phenyl |
| VIIIa-a-7 | 2,4-Dichlor-3-[(methylsulfonyl)oxy]phenyl |
| VIIIa-a-8 | 2,4-Difluor-3-(prop-2-in-1-yloxy)phenyl |
| VIIIa-a-9 | 2,4-Difluor-3-[(methylsulfonyl)oxy]phenyl |
| VIIIa-a-10 | 2,4-Difluor-3-formylphenyl |
| VIIIa-a-11 | 2,5-Dichlor-3-(prop-2-in-1-yloxy)phenyl |
| VIIIa-a-12 | 2,5-Dichlor-3-[(methylsulfonyl)oxy]phenyl |
| VIIIa-a-13 | 2,5-Dichlor-4-(prop-2-in-1-yloxy)phenyl |
| VIIIa-a-14 | 2,5-Dichlor-4-[(methylsulfonyl)oxy]phenyl |
| VIIIa-a-15 | 2,5-Difluor-3-(prop-2-in-1-yloxy)phenyl |
| VIIIa-a-16 | 2,5-Difluor-3-[(methylsulfonyl)oxy]phenyl |
| VIIIa-a-17 | 2,5-Difluor-3-formylphenyl |
| VIIIa-a-18 | 2,5-Difluor-4-(prop-2-in-1-yloxy)phenyl |
| VIIIa-a-19 | 2,5-Difluor-4-[(methylsulfonyl)oxy]phenyl |
| VIIIa-a-20 | 2,5-Difluor-4-formylphenyl |
| VIIIa-a-21 | 2,6-Dichlor-3-(prop-2-in-1-yloxy)phenyl |
| VIIIa-a-22 | 2,6-Dichlor-3-[(methylsulfonyl)oxy]phenyl |
| VIIIa-a-23 | 2,6-Dichlor-4-(prop-2-in-1-yloxy)phenyl |
| VIIIa-a-24 | 2,6-Dichlor-4-[(methylsulfonyl)oxy]phenyl |
| VIIIa-a-25 | 2,6-Difluor-3-(prop-2-in-1-yloxy)phenyl |
| VIIIa-a-26 | 2,6-Difluor-3-[(methylsulfonyl)oxy]phenyl |
| VIIIa-a-27 | 2,6-Difluor-3-formylphenyl |
| VIIIa-a-28 | 2,6-Difluor-4-(prop-2-in-1-yloxy)phenyl |
| VIIIa-a-29 | 2,6-Difluor-4-[(methylsulfonyl)oxy]phenyl |
| VIIIa-a-30 | 2,6-Difluor-4-formylphenyl |
| VIIIa-a-31 | 2-(Allyloxy)-3,4-dichlorphenyl |
| VIIIa-a-32 | 2-(Allyloxy)-3,4-difluorphenyl |
| VIIIa-a-33 | 2-(Allyloxy)-3,5-dichlorphenyl |
| VIIIa-a-34 | 2-(Allyloxy)-3,5-difluorphenyl |
| VIIIa-a-35 | 2-(Allyloxy)-3,6-dichlorphenyl |
| VIIIa-a-36 | 2-(Allyloxy)-3,6-difluorphenyl |
| VIIIa-a-37 | 2-(Allyloxy)-3-chlorphenyl |
| VIIIa-a-38 | 2-(Allyloxy)-3-fluorphenyl |
| VIIIa-a-39 | 2-(Allyloxy)-3 -methylphenyl |
| VIIIa-a-40 | 2-(Allyloxy)-4,5-dichlorphenyl |
| VIIIa-a-41 | 2-(Allyloxy)-4,5-difluorphenyl |
| VIIIa-a-42 | 2-(Allyloxy)-4,6-dichlorphenyl |
| VIIIa-a-43 | 2-(Allyloxy)-4,6-difluorphenyl |
| VIIIa-a-44 | 2-(Allyloxy)-4-chlorphenyl |
| VIIIa-a-45 | 2-(Allyloxy)-4-fluorphenyl |
| VIIIa-a-46 | 2-(Allyloxy)-4-methylphenyl |
| VIIIa-a-47 | 2-(Allyloxy)-5,6-dichlorphenyl |
| VIIIa-a-48 | 2-(Allyloxy)-5,6-difluorphenyl |
| VIIIa-a-49 | 2-(Allyloxy)-5-chlorphenyl |
| VIIIa-a-50 | 2-(Allyloxy)-5-fluorphenyl |
| VIIIa-a-51 | 2-(Allyloxy)-5-methylphenyl |
| VIIIa-a-52 | 2-(Allyloxy)-6-chlorphenyl |
| VIIIa-a-53 | 2-(Allyloxy)-6-fluorphenyl |
| VIIIa-a-54 | 2-(Allyloxy)-6-methylphenyl |
| VIIIa-a-55 | 2-(Allyloxy)phenyl |
| VIIIa-a-56 | 2-(Cyanomethoxy)-3,4-dichlorphenyl |
| VIIIa-a-57 | 2-(Cyanomethoxy)-3,4-difluorphenyl |
| VIIIa-a-58 | 2-(Cyanomethoxy)-3,5-dichlorphenyl |
| VIIIa-a-59 | 2-(Cyanomethoxy)-3,5-difluorphenyl |
| VIIIa-a-60 | 2-(Cyanomethoxy)-3,6-dichlorphenyl |
| VIIIa-a-61 | 2-(Cyanomethoxy)-3,6-difluorphenyl |
| VIIIa-a-62 | 2-(Cyanomethoxy)-3-chlorphenyl |
| VIIIa-a-63 | 2-(Cyanomethoxy)-3-fluorphenyl |
| VIIIa-a-64 | 2-(Cyanomethoxy)-3 -methylphenyl |
| VIIIa-a-65 | 2-(Cyanomethoxy)-4,5-dichlorphenyl |
| VIIIa-a-66 | 2-(Cyanomethoxy)-4,5-difluorphenyl |
| VIIIa-a-67 | 2-(Cyanomethoxy)-4,6-dichlorphenyl |
| VIIIa-a-68 | 2-(Cyanomethoxy)-4,6-difluorphenyl |
| VIIIa-a-69 | 2-(Cyanomethoxy)-4-chlorphenyl |
| VIIIa-a-70 | 2-(Cyanomethoxy)-4-fluorphenyl |
| VIIIa-a-71 | 2-(Cyanomethoxy)-4-methylphenyl |
| VIIIa-a-72 | 2-(Cyanomethoxy)-5,6-dichlorphenyl |
| VIIIa-a-73 | 2-(Cyanomethoxy)-5,6-difluorphenyl |
| VIIIa-a-74 | 2-(Cyanomethoxy)-5-chlorphenyl |
| VIIIa-a-75 | 2-(Cyanomethoxy)-5-fluorphenyl |
| VIIIa-a-76 | 2-(Cyanomethoxy)-5-methylphenyl |
| VIIIa-a-77 | 2-(Cyanomethoxy)-6-chlorphenyl |
| VIIIa-a-78 | 2-(Cyanomethoxy)-6-fluorphenyl |
| VIIIa-a-79 | 2-(Cyanomethoxy)-6-methylphenyl |
| VIIIa-a-80 | 2-(Cyanomethoxy)phenyl |
| VIIIa-a-81 | 2-(Prop-2-in-1-yloxy)phenyl |
| VIIIa-a-82 | 2-(Prop-2-in-1-yloxy)-4-(trifluormethyl)phenyl |
| VIIIa-a-83 | 2-Chlor-3-(prop-2-in-1-yloxy)phenyl |
| VIIIa-a-84 | 2-Chlor-3-[(methylsulfonyl)oxy]phenyl |
| VIIIa-a-85 | 2-Chlor-3-formylphenyl |
| VIIIa-a-86 | 2-Chlor-4-(prop-2-in-1-yloxy)phenyl |
| VIIIa-a-87 | 2-Chlor-4-[(methylsulfonyl)oxy]phenyl |
| VIIIa-a-88 | 2-Chlor-4-formylphenyl |
| VIIIa-a-89 | 2-Fluor-3-(prop-2-in-1-yloxy)phenyl |
| VIIIa-a-90 | 2-Fluor-3-[(methylsulfonyl)oxy]phenyl |
| VIIIa-a-91 | 2-Fluor-3-formylphenyl |
| VIIIa-a-92 | 2-Fluor-4-(prop-2-in-1-yloxy)phenyl |
| VIIIa-a-93 | 2-Fluor-4-[(methylsulfonyl)oxy]phenyl |
| VIIIa-a-94 | 2-Fluor-4-formylphenyl |
| VIIIa-a-95 | 2-Formyl-3-methylphenyl |
| VIIIa-a-96 | 2-Formyl-4-methylphenyl |
| VIIIa-a-97 | 2-Formyl-5-methylphenyl |
| VIIIa-a-98 | 2-Formyl-6-methylphenyl |
| VIIIa-a-99 | 2-Formylphenyl |
| VIIIa-a-100 | 2-Methyl-3-(prop-2-in-1-yloxy)phenyl |
| VIIIa-a-101 | 2-Methyl-3-[(methylsulfonyl)oxy]phenyl |
| VIIIa-a-102 | 2-Methyl-4-(prop-2-in-1-yloxy)phenyl |
| VIIIa-a-103 | 2-Methyl-4-[(methylsulfonyl)oxy]phenyl |
| VIIIa-a-104 | 2-[(Hydroxyimino)methyl]-3,4-difluorphenyl |
| VIIIa-a-105 | 2-[(Hydroxyimino)methyl]-3,5-difluorphenyl |
| VIIIa-a-106 | 2-[(Hydroxyimino)methyl]-3,6-difluorphenyl |
| VIIIa-a-107 | 2-[(Hydroxyimino)methyl]-3-chlorphenyl |
| VIIIa-a-108 | 2-[(Hydroxyimino)methyl]-3-fluorphenyl |
| VIIIa-a-109 | 2-[(Hydroxyimino)methyl]-3-methylphenyl |
| VIIIa-a-110 | 2-[(Hydroxyimino)methyl]-4,5-difluorphenyl |
| VIIIa-a-111 | 2-[(Hydroxyimino)methyl]-4,6-difluorphenyl |
| VIIIa-a-112 | 2-[(Hydroxyimino)methyl]-4-chlorphenyl |
| VIIIa-a-113 | 2-[(Hydroxyimino)methyl]-4-fluorphenyl |
| VIIIa-a-114 | 2-[(Hydroxyimino)methyl]-4-methylphenyl |
| VIIIa-a-115 | 2-[(Hydroxyimino)methyl]-5,6-difluorphenyl |
| VIIIa-a-116 | 2-[(Hydroxyimino)methyl]-5-chlorphenyl |
| VIIIa-a-117 | 2-[(Hydroxyimino)methyl]-5-fluorphenyl |
| VIIIa-a-118 | 2-[(Hydroxyimino)methyl]-5-methylphenyl |
| VIIIa-a-119 | 2-[(Hydroxyimino)methyl]-6-chlorphenyl |
| VIIIa-a-120 | 2-[(Hydroxyimino)methyl]-6-fluorphenyl |
| VIIIa-a-121 | 2-[(Hydroxyimino)methyl]-6-methylphenyl |
| VIIIa-a-122 | 2-[(Hydroxyimino)methyl]phenyl |
| VIIIa-a-123 | 2-[(Methoxyimino)methyl]-3,4-difluorphenyl |
| VIIIa-a-124 | 2-[(Methoxyimino)methyl]-3,5-difluorphenyl |
| VIIIa-a-125 | 2-[(Methoxyimino)methyl]-3,6-difluorphenyl |
| VIIIa-a-126 | 2-[(Methoxyimino)methyl]-3-chlorphenyl |
| VIIIa-a-127 | 2-[(Methoxyimino)methyl]-3-fluorphenyl |
| VIIIa-a-128 | 2-[(Methoxyimino)methyl]-3-methylphenyl |
| VIIIa-a-129 | 2-[(Methoxyimino)methyl]-4,5-difluorphenyl |
| VIIIa-a-130 | 2-[(Methoxyimino)methyl]-4,6-difluorphenyl |
| VIIIa-a-131 | 2-[(Methoxyimino)methyl]-4-chlorphenyl |
| VIIIa-a-132 | 2-[(Methoxyimino)methyl]-4-fluorphenyl |
| VIIIa-a-133 | 2-[(Methoxyimino)methyl]-4-methylphenyl |
| VIIIa-a-134 | 2-[(Methoxyimino)methyl]-5,6-difluorphenyl |
| VIIIa-a-135 | 2-[(Methoxyimino)methyl]-5-chlorphenyl |
| VIIIa-a-136 | 2-[(Methoxyimino)methyl]-5-fluorphenyl |
| VIIIa-a-137 | 2-[(Methoxyimino)methyl]-5-methylphenyl |
| VIIIa-a-138 | 2-[(Methoxyimino)methyl]-6-chlorphenyl |
| VIIIa-a-139 | 2-[(Methoxyimino)methyl]-6-fluorphenyl |
| VIIIa-a-140 | 2-[(Methoxyimino)methyl]-6-methylphenyl |
| VIIIa-a-141 | 2-[(Methoxyimino)methyl]phenyl |
| VIIIa-a-142 | 2-[(Methylsulfonyl)oxy]phenyl |
| VIIIa-a-143 | 2-[(Methylsulfonyl)oxy]-4-(trifluormethyl)phenyl |
| VIIIa-a-144 | 3,4-Dichlor-2-(prop-2-in-1-yloxy)phenyl |
| VIIIa-a-145 | 3,4-Dichlor-2-[(methylsulfonyl)oxy]phenyl |
| VIIIa-a-146 | 3,4-Difluor-2-(prop-2-in-1-yloxy)phenyl |
| VIIIa-a-147 | 3,4-Difluor-2-[(methylsulfonyl)oxy]phenyl |
| VIIIa-a-148 | 3,4-Difluor-2-formylphenyl |
| VIIIa-a-149 | 3,5-Dichlor-2-(prop-2-in-1-yloxy)phenyl |
| VIIIa-a-150 | 3,5-Dichlor-2-[(methylsulfonyl)oxy]phenyl |
| VIIIa-a-151 | 3,5-Dichlor-4-(prop-2-in-1-yloxy)phenyl |
| VIIIa-a-152 | 3,5-Dichlor-4-[(methylsulfonyl)oxy]phenyl |
| VIIIa-a-153 | 3,5-Difluor-2-(prop-2-in-1-yloxy)phenyl |
| VIIIa-a-154 | 3,5-Difluor-2-[(methylsulfonyl)oxy]phenyl |
| VIIIa-a-155 | 3,5-Difluor-2-formylphenyl |
| VIIIa-a-156 | 3,5-Difluor-4-(prop-2-in-1-yloxy)phenyl |
| VIIIa-a-157 | 3,5-Difluor-4-[(methylsulfonyl)oxy]phenyl |
| VIIIa-a-158 | 3,5-Difluor-4-formylphenyl |
| VIIIa-a-159 | 3,6-Dichlor-2-(prop-2-in-1-yloxy)phenyl |
| VIIIa-a-160 | 3,6-Dichlor-2-[(methylsulfonyl)oxy]phenyl |
| VIIIa-a-161 | 3,6-Dichlor-4-(prop-2-in-1-yloxy)phenyl |
| VIIIa-a-162 | 3,6-Dichlor-4-[(methylsulfonyl)oxy]phenyl |
| VIIIa-a-163 | 3,6-Difluor-2-(prop-2-in-1-yloxy)phenyl |
| VIIIa-a-164 | 3,6-Difluor-2-[(methylsulfonyl)oxy]phenyl |
| VIIIa-a-165 | 3,6-Difluor-2-formylphenyl |
| VIIIa-a-166 | 3,6-Difluor-4-(prop-2-in-1-yloxy)phenyl |
| VIIIa-a-167 | 3,6-Difluor-4-[(methylsulfonyl)oxy]phenyl |
| VIIIa-a-168 | 3,6-Difluor-4-formylphenyl |
| VIIIa-a-169 | 3-(Allyloxy)-2,4-dichlorphenyl |
| VIIIa-a-170 | 3-(Allyloxy)-2,4-difluorphenyl |
| VIIIa-a-171 | 3-(Allyloxy)-2,5-dichlorphenyl |
| VIIIa-a-172 | 3-(Allyloxy)-2,5-difluorphenyl |
| VIIIa-a-173 | 3-(Allyloxy)-2,6-dichlorphenyl |
| VIIIa-a-174 | 3-(Allyloxy)-2,6-difluorphenyl |
| VIIIa-a-175 | 3-(Allyloxy)-2-chlorphenyl |
| VIIIa-a-176 | 3-(Allyloxy)-2-fluorphenyl |
| VIIIa-a-177 | 3-(Allyloxy)-2-methylphenyl |
| VIIIa-a-178 | 3-(Allyloxy)-4,5-dichlorphenyl |
| VIIIa-a-179 | 3-(Allyloxy)-4,5-difluorphenyl |
| VIIIa-a-180 | 3-(Allyloxy)-4,6-dichlorphenyl |
| VIIIa-a-181 | 3-(Allyloxy)-4,6-difluorphenyl |
| VIIIa-a-182 | 3-(Allyloxy)-4-chlorphenyl |
| VIIIa-a-183 | 3-(Allyloxy)-4-fluorphenyl |
| VIIIa-a-184 | 3-(Allyloxy)-4-methylphenyl |
| VIIIa-a-185 | 3-(Allyloxy)-5,6-dichlorphenyl |
| VIIIa-a-186 | 3-(Allyloxy)-5,6-difluorphenyl |
| VIIIa-a-187 | 3-(Allyloxy)-5-chlorphenyl |
| VIIIa-a-188 | 3-(Allyloxy)-5-fluorphenyl |
| VIIIa-a-189 | 3 -(Allyloxy)- 5- methylphenyl |
| VIIIa-a-190 | 3-(Allyloxy)-6-chlorphenyl |
| VIIIa-a-191 | 3-(Allyloxy)-6-fluorphenyl |
| VIIIa-a-192 | 3-(Allyloxy)-6-methylphenyl |
| VIIIa-a-193 | 3-(Allyloxy)phenyl |
| VIIIa-a-194 | 3-(Cyanomethoxy)-2,4-dichlorphenyl |
| VIIIa-a-195 | 3-(Cyanomethoxy)-2,4-difluorphenyl |
| VIIIa-a-196 | 3-(Cyanomethoxy)-2,5-dichlorphenyl |
| VIIIa-a-197 | 3-(Cyanomethoxy)-2,5-difluorphenyl |
| VIIIa-a-198 | 3-(Cyanomethoxy)-2,6-dichlorphenyl |
| VIIIa-a-199 | 3-(Cyanomethoxy)-2,6-difluorphenyl |
| VIIIa-a-200 | 3-(Cyanomethoxy)-2-chlorphenyl |
| VIIIa-a-201 | 3-(Cyanomethoxy)-2-fluorphenyl |
| VIIIa-a-202 | 3-(Cyanomethoxy)-2-methylphenyl |
| VIIIa-a-203 | 3-(Cyanomethoxy)-4,5-dichlorphenyl |
| VIIIa-a-204 | 3-(Cyanomethoxy)-4,5-difluorphenyl |
| VIIIa-a-205 | 3-(Cyanomethoxy)-4,6-dichlorphenyl |
| VIIIa-a-206 | 3-(Cyanomethoxy)-4,6-difluorphenyl |
| VIIIa-a-207 | 3-(Cyanomethoxy)-4-chlorphenyl |
| VIIIa-a-208 | 3-(Cyanomethoxy)-4-fluorphenyl |
| VIIIa-a-209 | 3-(Cyanomethoxy)-4-methylphenyl |
| VIIIa-a-210 | 3-(Cyanomethoxy)-5,6-dichlorphenyl |
| VIIIa-a-211 | 3-(Cyanomethoxy)-5,6-difluorphenyl |
| VIIIa-a-212 | 3-(Cyanomethoxy)-5-chlorphenyl |
| VIIIa-a-213 | 3-(Cyanomethoxy)-5-fluorphenyl |
| VIIIa-a-214 | 3-(Cyanomethoxy)-5-methylphenyl |
| VIIIa-a-215 | 3-(Cyanomethoxy)-6-chlorphenyl |
| VIIIa-a-216 | 3-(Cyanomethoxy)-6-fluorphenyl |
| VIIIa-a-217 | 3-(Cyanomethoxy)-6-methylphenyl |
| VIIIa-a-218 | 3-(Cyanomethoxy)phenyl |
| VIIIa-a-219 | 3-(Prop-2-in-1-yloxy)phenyl |
| VIIIa-a-220 | 3-Chlor-2-(prop-2-in-1-yloxy)phenyl |
| VIIIa-a-221 | 3-Chlor-2-[(methylsulfonyl)oxy]phenyl |
| VIIIa-a-222 | 3-Chlor-2-formylphenyl |
| VIIIa-a-223 | 3-Chlor-4-(prop-2-in-1-yloxy)phenyl |
| VIIIa-a-224 | 3-Chlor-4-[(methylsulfonyl)oxy]phenyl |
| VIIIa-a-225 | 3-Chlor-4-formylphenyl |
| VIIIa-a-226 | 3-Fluor-2-(prop-2-in-1-yloxy)phenyl |
| VIIIa-a-227 | 3-Fluor-2-[(methylsulfonyl)oxy]phenyl |
| VIIIa-a-228 | 3-Fluor-2-formylphenyl |
| VIIIa-a-229 | 3-Fluor-4-(prop-2-in-1-yloxy)phenyl |
| VIIIa-a-230 | 3-Fluor-4-[(methylsulfonyl)oxy]phenyl |
| VIIIa-a-231 | 3-Fluor-4-formylphenyl |
| VIIIa-a-232 | 3-Formyl-2-methylphenyl |
| VIIIa-a-233 | 3-Formyl-4-methylphenyl |
| VIIIa-a-234 | 3-Formyl-5-methylphenyl |
| VIIIa-a-235 | 3-Formyl-6-methylphenyl |
| VIIIa-a-236 | 3-Formylphenyl |
| VIIIa-a-237 | 3-Methyl-2-(prop-2-in-1-yloxy)phenyl |
| VIIIa-a-238 | 3-Methyl-2-[(methylsulfonyl)oxy]phenyl |
| VIIIa-a-239 | 3-Methyl-4-(prop-2-in-1-yloxy)phenyl |
| VIIIa-a-240 | 3-Methyl-4-[(methylsulfonyl)oxy]phenyl |
| VIIIa-a-241 | 3-[(Hydroxyimino)methyl]-2,4-difluorphenyl |
| VIIIa-a-242 | 3-[(Hydroxyimino)methyl]-2,5-difluorphenyl |
| VIIIa-a-243 | 3-[(Hydroxyimino)methyl]-2,6-difluorphenyl |
| VIIIa-a-244 | 3-[(Hydroxyimino)methyl]-2-chlorphenyl |
| VIIIa-a-245 | 3-[(Hydroxyimino)methyl]-2-fluorphenyl |
| VIIIa-a-246 | 3-[(Hydroxyimino)methyl]-2-methylphenyl |
| VIIIa-a-247 | 3-[(Hydroxyimino)methyl]-4,5-difluorphenyl |
| VIIIa-a-248 | 3-[(Hydroxyimino)methyl]-4,6-difluorphenyl |
| VIIIa-a-249 | 3-[(Hydroxyimino)methyl]-4-chlorphenyl |
| VIIIa-a-250 | 3-[(Hydroxyimino)methyl]-4-fluorphenyl |
| VIIIa-a-251 | 3-[(Hydroxyimino)methyl]-4-methylphenyl |
| VIIIa-a-252 | 3-[(Hydroxyimino)methyl]-5,6-difluorphenyl |
| VIIIa-a-253 | 3-[(Hydroxyimino)methyl]-5-chlorphenyl |
| VIIIa-a-254 | 3-[(Hydroxyimino)methyl]-5-fluorphenyl |
| VIIIa-a-255 | 3-[(Hydroxyimino)methyl]-5-methylphenyl |
| VIIIa-a-256 | 3-[(Hydroxyimino)methyl]-6-chlorphenyl |
| VIIIa-a-257 | 3-[(Hydroxyimino)methyl]-6-fluorphenyl |
| VIIIa-a-258 | 3-[(Hydroxyimino)methyl]-6-methylphenyl |
| VIIIa-a-259 | 3-[(Hydroxyimino)methyl]phenyl |
| VIIIa-a-260 | 3-[(Methoxyimino)methyl]-2,4-difluorphenyl |
| VIIIa-a-261 | 3-[(Methoxyimino)methyl]-2,5-difluorphenyl |
| VIIIa-a-262 | 3-[(Methoxyimino)methyl]-2,6-difluorphenyl |
| VIIIa-a-263 | 3-[(Methoxyimino)methyl]-2-chlorphenyl |
| VIIIa-a-264 | 3-[(Methoxyimino)methyl]-2-fluorphenyl |
| VIIIa-a-265 | 3-[(Methoxyimino)methyl]-2-methylphenyl |
| VIIIa-a-266 | 3-[(Methoxyimino)methyl]-4,5-difluorphenyl |
| VIIIa-a-267 | 3-[(Methoxyimino)methyl]-4,6-difluorphenyl |
| VIIIa-a-268 | 3-[(Methoxyimino)methyl]-4-chlorphenyl |
| VIIIa-a-269 | 3-[(Methoxyimino)methyl]-4-fluorphenyl |
| VIIIa-a-270 | 3-[(Methoxyimino)methyl]-4-methylphenyl |
| VIIIa-a-271 | 3-[(Methoxyimino)methyl]-5,6-difluorphenyl |
| VIIIa-a-272 | 3-[(Methoxyimino)methyl]-5-chlorphenyl |
| VIIIa-a-273 | 3-[(Methoxyimino)methyl]-5-fluorphenyl |
| VIIIa-a-274 | 3-[(Methoxyimino)methyl]-5-methylphenyl |
| VIIIa-a-275 | 3-[(Methoxyimino)methyl]-6-chlorphenyl |
| VIIIa-a-276 | 3-[(Methoxyimino)methyl]-6-fluorphenyl |
| VIIIa-a-277 | 3-[(Methoxyimino)methyl]-6-methylphenyl |
| VIIIa-a-278 | 3-[(Methoxyimino)methyl]phenyl |
| VIIIa-a-279 | 3-[(Methylsulfonyl)oxy]phenyl |
| VIIIa-a-280 | 4,5-Dichlor-2-(prop-2-in-1-yloxy)phenyl |
| VIIIa-a-281 | 4,5-Dichlor-2-[(methylsulfonyl)oxy]phenyl |
| VIIIa-a-282 | 4,5-Dichlor-3-(prop-2-in-1-yloxy)phenyl |
| VIIIa-a-283 | 4,5-Dichlor-3-[(methylsulfonyl)oxy]phenyl |
| VIIIa-a-284 | 4,5-Difluor-2-(prop-2-in-1-yloxy)phenyl |
| VIIIa-a-285 | 4,5-Difluor-2-[(methylsulfonyl)oxy]phenyl |
| VIIIa-a-286 | 4,5-Difluor-2-formylphenyl |
| VIIIa-a-287 | 4,5-Difluor-3-(prop-2-in-1-yloxy)phenyl |
| VIIIa-a-288 | 4,5-Difluor-3-[(methylsulfonyl)oxy]phenyl |
| VIIIa-a-289 | 4,5-Difluor-3-formylphenyl |
| VIIIa-a-290 | 4,6-Dichlor-2-(prop-2-in-1-yloxy)phenyl |
| VIIIa-a-291 | 4,6-Dichlor-2-[(methylsulfonyl)oxy]phenyl |
| VIIIa-a-292 | 4,6-Dichlor-3-(prop-2-in-1-yloxy)phenyl |
| VIIIa-a-293 | 4,6-Dichlor-3-[(methylsulfonyl)oxy]phenyl |
| VIIIa-a-294 | 4,6-Difluor-2-(prop-2-in-1-yloxy)phenyl |
| VIIIa-a-295 | 4,6-Difluor-2-[(methylsulfonyl)oxy]phenyl |
| VIIIa-a-296 | 4,6-Difluor-2-formylphenyl |
| VIIIa-a-297 | 4,6-Difluor-3-(prop-2-in-1-yloxy)phenyl |
| VIIIa-a-298 | 4,6-Difluor-3-[(methylsulfonyl)oxy]phenyl |
| VIIIa-a-299 | 4,6-Difluor-3-formylphenyl |
| VIIIa-a-300 | 4-(Allyloxy)-2,3-dichlorphenyl |
| Vina-a-301 | 4-(Allyloxy)-2,3-difluorphenyl |
| VIIIa-a-302 | 4-(Allyloxy)-2,5-dichlorphenyl |
| VIIIa-a-303 | 4-(Allyloxy)-2,5-difluorphenyl |
| VIIIa-a-304 | 4-(Allyloxy)-2,6-dichlorphenyl |
| VIIIa-a-305 | 4-(Allyloxy)-2,6-difluorphenyl |
| VIIIa-a-306 | 4-(Allyloxy)-2-chlorphenyl |
| VIIIa-a-307 | 4-(Allyloxy)-2-fluorphenyl |
| VIIIa-a-308 | 4-(Allyloxy)-2-methylphenyl |
| VIIIa-a-309 | 4-(Allyloxy)-3,5-dichlorphenyl |
| VIIIa-a-310 | 4-(Allyloxy)-3,5-difluorphenyl |
| VIIIa-a-311 | 4-(Allyloxy)-3,6-dichlorphenyl |
| VIIIa-a-312 | 4-(Allyloxy)-3,6-difluorphenyl |
| VIIIa-a-313 | 4-(Allyloxy)-3-chlorphenyl |
| VIIIa-a-314 | 4-(Allyloxy)-3-fluorphenyl |
| VIIIa-a-315 | 4-(Allyloxy)-3-methylphenyl |
| VIIIa-a-316 | 4-(Allyloxy)phenyl |
| VIIIa-a-317 | 4-(Cyanomethoxy)-2,3-dichlorphenyl |
| VIIIa-a-318 | 4-(Cyanomethoxy)-2,3-difluorphenyl |
| VIIIa-a-319 | 4-(Cyanomethoxy)-2,5-dichlorphenyl |
| VIIIa-a-320 | 4-(Cyanomethoxy)-2,5-difluorphenyl |
| VIIIa-a-321 | 4-(Cyanomethoxy)-2,6-dichlorphenyl |
| VIIIa-a-322 | 4-(Cyanomethoxy)-2,6-difluorphenyl |
| VIIIa-a-323 | 4-(Cyanomethoxy)-2-chlorphenyl |
| VIIIa-a-324 | 4-(Cyanomethoxy)-2-fluorphenyl |
| VIIIa-a-325 | 4-(Cyanomethoxy)-2-methylphenyl |
| VIIIa-a-326 | 4-(Cyanomethoxy)-3,5-dichlorphenyl |
| VIIIa-a-327 | 4-(Cyanomethoxy)-3,5-difluorphenyl |
| VIIIa-a-328 | 4-(Cyanomethoxy)-3,6-dichlorphenyl |
| VIIIa-a-329 | 4-(Cyanomethoxy)-3,6-difluorphenyl |
| VIIIa-a-330 | 4-(Cyanomethoxy)-3-chlorphenyl |
| VIIIa-a-331 | 4-(Cyanomethoxy)-3-fluorphenyl |
| VIIIa-a-332 | 4-(Cyanomethoxy)-3-methylphenyl |
| VIIIa-a-333 | 4-(Cyanomethoxy)phenyl |
| VIIIa-a-334 | 4-(Prop-2-in-1-yloxy)phenyl |
| VIIIa-a-335 | 4-Chlor-2-(prop-2-in-1-yloxy)phenyl |
| VIIIa-a-336 | 4-Chlor-2-[(methylsulfonyl)oxy]phenyl |
| VIIIa-a-337 | 4-Chlor-2-formylphenyl |
| VIIIa-a-338 | 4-Chlor-3-(prop-2-in-1-yloxy)phenyl |
| VIIIa-a-339 | 4-Chlor-3-[(methylsulfonyl)oxy]phenyl |
| VIIIa-a-340 | 4-Chlor-3-formylphenyl |
| VIIIa-a-341 | 4-Fluor-2-(prop-2-in-1-yloxy)phenyl |
| VIIIa-a-342 | 4-Fluor-2-[(methylsulfonyl)oxy]phenyl |
| VIIIa-a-343 | 4-Fluor-2-formylphenyl |
| VIIIa-a-344 | 4-Fluor-3-(prop-2-in-1-yloxy)phenyl |
| VIIIa-a-345 | 4-Fluor-3-[(methylsulfonyl)oxy]phenyl |
| VIIIa-a-346 | 4-Fluor-3-formylphenyl |
| VIIIa-a-347 | 4-Formyl-2-methylphenyl |
| VIIIa-a-348 | 4-Formyl-3-methylphenyl |
| VIIIa-a-349 | 4-Formylphenyl |
| VIIIa-a-350 | 4-Methyl-2-(prop-2-in-1-yloxy)phenyl |
| VIIIa-a-351 | 4-Methyl-2-[(methylsulfonyl)oxy] phenyl |
| VIIIa-a-352 | 4-Methyl-3-(prop-2-in-1-yloxy)phenyl |
| VIIIa-a-353 | 4-Methyl-3-[(methylsulfonyl)oxy]phenyl |
| VIIIa-a-354 | 4-[(Hydroxyimino)methyl]-2,3-difluorphenyl |
| VIIIa-a-355 | 4-[(Hydroxyimino)methyl]-2,5-difluorphenyl |
| VIIIa-a-356 | 4-[(Hydroxyimino)methyl]-2,6-difluorphenyl |
| VIIIa-a-357 | 4-[(Hydroxyimino)methyl]-2-chlorphenyl |
| VIIIa-a-358 | 4-[(Hydroxyimino)methyl]-2-fluorphenyl |
| VIIIa-a-359 | 4-[(Hydroxyimino)methyl]-2-methylphenyl |
| VIIIa-a-360 | 4-[(Hydroxyimino)methyl]-3,5-difluorphenyl |
| VIIIa-a-361 | 4-[(Hydroxyimino)methyl]-3,6-difluorphenyl |
| VIIIa-a-362 | 4-[(Hydroxyimino)methyl]-3-chlorphenyl |
| VIIIa-a-363 | 4-[(Hydroxyimino)methyl]-3-fluorphenyl |
| VIIIa-a-364 | 4-[(Hydroxyimino)methyl]-3-methylphenyl |
| VIIIa-a-365 | 4-[(Hydroxyimino)methyl]phenyl |
| VIIIa-a-366 | 4-[(Methoxyimino)methyl]-2,3-difluorphenyl |
| VIIIa-a-367 | 4-[(Methoxyimino)methyl]-2,5-difluorphenyl |
| VIIIa-a-368 | 4-[(Methoxyimino)methyl]-2,6-difluorphenyl |
| VIIIa-a-369 | 4-[(Methoxyimino)methyl]-2-chlorphenyl |
| VIIIa-a-370 | 4-[(Methoxyimino)methyl]-2-fluorphenyl |
| VIIIa-a-371 | 4-[(Methoxyimino)methyl]-2-methylphenyl |
| VIIIa-a-372 | 4-[(Methoxyimino)methyl]-3,5-difluorphenyl |
| VIIIa-a-373 | 4-[(Methoxyimino)methyl]-3,6-difluorphenyl |
| VIIIa-a-374 | 4-[(Methoxyimino)methyl]-3-chlorphenyl |
| VIIIa-a-375 | 4-[(Methoxyimino)methyl]-3-fluorphenyl |
| VIIIa-a-376 | 4-[(Methoxyimino)methyl]-3-methylphenyl |
| VIIIa-a-377 | 4-[(Methoxyimino)methyl]phenyl |
| VIIIa-a-378 | 4-[(Methylsulfonyl)oxy]phenyl |
| VIIIa-a-379 | 5,6-Dichlor-2-(prop-2-in-1-yloxy)phenyl |
| VIIIa-a-380 | 5,6-Dichlor-2-[(methylsulfonyl)oxy]phenyl |
| VIIIa-a-381 | 5,6-Dichlor-3-(prop-2-in-1-yloxy)phenyl |
| VIIIa-a-382 | 5,6-Dichlor-3-[(methylsulfonyl)oxy]phenyl |
| VIIIa-a-383 | 5,6-Difluor-2-(prop-2-in-1-yloxy)phenyl |
| VIIIa-a-384 | 5,6-Difluor-2-[(methylsulfonyl)oxy]phenyl |
| VIIIa-a-385 | 5,6-Difluor-2-formylphenyl |
| VIIIa-a-386 | 5,6-Difluor-3-(prop-2-in-1-yloxy)phenyl |
| VIIIa-a-387 | 5,6-Difluor-3-[(methylsulfonyl)oxy]phenyl |
| VIIIa-a-388 | 5,6-Difluor-3-formylphenyl |
| VIIIa-a-389 | 5-Chlor-2-(prop-2-in-1-yloxy)phenyl |
| VIIIa-a-390 | 5-Chlor-2-[(methylsulfonyl)oxy]phenyl |
| VIIIa-a-391 | 5-Chlor-2-formylphenyl |
| VIIIa-a-392 | 5-Chlor-3-(prop-2-in-1-yloxy)phenyl |
| VIIIa-a-393 | 5-Chlor-3-[(methylsulfonyl)oxy]phenyl |
| VIIIa-a-394 | 5-Chlor-3-formylphenyl |
| VIIIa-a-395 | 5-Fluor-2-(prop-2-in-1-yloxy)phenyl |
| VIIIa-a-396 | 5-Fluor-2-[(methylsulfonyl)oxy]phenyl |
| VIIIa-a-397 | 5-Fluor-2-formylphenyl |
| VIIIa-a-398 | 5-Fluor-3-(prop-2-in-1-yloxy)phenyl |
| VIIIa-a-399 | 5-Fluor-3-[(methylsulfonyl)oxy]phenyl |
| VIIIa-a-400 | 5-Fluor-3-formylphenyl |
| VIIIa-a-401 | 5-Methyl-2-(prop-2-in-1-yloxy)phenyl |
| VIIIa-a-402 | 5-Methyl-2-[(methylsulfonyl)oxy]phenyl |
| VIIIa-a-403 | 5-Methyl-3-(prop-2-in-1-yloxy)phenyl |
| VIIIa-a-404 | 5-Methyl-3-[(methylsulfonyl)oxy]phenyl |
| VIIIa-a-405 | 6-Chlor-2-(prop-2-in-1-yloxy)phenyl |
| VIIIa-a-406 | 6-Chlor-2-[(methylsulfonyl)oxy]phenyl |
| VIIIa-a-407 | 6-Chlor-2-formylphenyl |
| VIIIa-a-408 | 6-Chlor-3-(prop-2-in-1-yloxy)phenyl |
| VIIIa-a-409 | 6-Chlor-3-[(methylsulfonyl)oxy]phenyl |
| VIIIa-a-410 | 6-Chlor-3-formylphenyl |
| VIIIa-a-411 | 6-Fluor-2-(prop-2-in-1-yloxy)phenyl |
| VIIIa-a-412 | 6-Fluor-2-[(methylsulfonyl)oxy]phenyl |
| VIIIa-a-413 | 6-Fluor-2-formylphenyl |
| VIIIa-a-414 | 6-Fluor-3-(prop-2-in-1-yloxy)phenyl |
| VIIIa-a-415 | 6-Fluor-3-[(methylsulfonyl)oxy]phenyl |
| VIIIa-a-416 | 6-Fluor-3-formylphenyl |
| VIIIa-a-417 | 6-Methyl-2-(prop-2-in-1-yloxy)phenyl |
| VIIIa-a-418 | 6-Methyl-2-[(methylsulfonyl)oxy]phenyl |
| VIIIa-a-419 | 6-Methyl-3-(prop-2-in-1-yloxy)phenyl |
| VIIIa-a-420 | 6-Methyl-3-[(methylsulfonyl)oxy]phenyl |

**Die Messung der logP Werte** erfolgte gemäß EEC Directive 79/831 Annex V.A8 durch HPLC (High Performance Liquid Chromatography) an reversed-phase Säulen (C 18), mit nachfolgenden Methoden:
^{[a]} Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril
^{[b]} Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die lambda-maX Werte wurden an Hand der UV-Spektren von 200 nm bis 400 nm in den Maxima der chromatographischen Signale ermittelt.

### NMR-Daten ausgewählter Beispiele

### NMR-Peak-Listenverfahren

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁); δ₂ (Intensität₂);........; δᵢ (Intensitätᵢ);......; δₙ (Intensitätₙ)

### Bsp. I-1, Lösungsmittel: DMSO-d6, Spektrometer: 399.95 MHz

7.9545 (3.60); 7.4134 (0.43); 7.3965 (0.44); 7.1822 (0.83); 7.1782 (1.01); 7.1516 (0.33); 7.1314 (0.39); 7.0088 (0.67); 6.9876 (0.60); 6.9104 (0.58); 6.8890 (0.39); 6.8848 (0.41); 6.0411 (1.73); 6.0305 (0.76); 4.8750 (0.67); 4.8476 (0.96); 4.8430 (1.60); 4.8377 (1.51); 3.5424 (0.37); 3.5368 (0.73); 3.5312 (0.35); 3.3287 (11.63); 2.8904 (16.00); 2.7310 (13.61); 2.6890 (0.37); 2.5110 (4.13); 2.5066 (8.51); 2.5021 (11.46); 2.4976 (8.43); 2.4933 (4.16); 2.1951 (4.61); 2.0941 (0.33); 2.0668 (0.35); -0.0002 (1.94)

### Bsp. I-2, Lösungsmittel: DMSO-d6, Spektrometer: 399.95 MHz

8.7704 (0.40); 7.9674 (7.50); 7.9568 (14.86); 7.4320 (2.09); 7.4148 (2.86); 7.4110 (4.55); 7.3941 (4.71); 7.3901 (3.04); 7.3731 (2.52); 7.3581 (4.38); 7.3361 (5.35); 7.3250 (10.03); 7.3031 (10.99); 7.0071 (6.19); 6.9860 (5.62); 6.9292 (5.16); 6.9241 (2.51); 6.9113 (10.58); 6.9073 (11.20); 6.8896 (8.88); 6.8594 (2.99); 6.0694 (0.97); 6.0558 (1.97); 6.0472 (1.41); 6.0337 (2.54); 6.0256 (2.36); 6.0168 (1.28); 6.0031 (1.97); 4.8896 (0.32); 4.8834 (0.74); 4.8772 (0.76); 4.8656 (1.59); 4.8492 (4.59); 4.8436 (12.06); 4.8381 (14.93); 4.8323 (10.43); 4.8048 (0.62); 4.7986 (0.99); 4.7923 (0.83); 4.7709 (3.30); 4.7542 (0.83); 4.7321 (1.79); 4.6953 (1.81); 4.6238 (1.78); 4.5884 (0.76); 4.4207 (1.52); 4.4000 (1.80); 4.3934 (1.73); 4.0553 (0.87); 4.0376 (3.47); 4.0330 (2.34); 4.0245 (7.36); 4.0200 (5.20); 4.0159 (5.42); 4.0073 (7.33); 3.9985 (5.23); 3.9898 (3.31); 3.9811 (2.74); 3.9477 (1.45); 3.8563 (0.63); 3.8236 (0.70); 3.8067 (0.86); 3.7756 (1.65); 3.7647 (1.16); 3.7337 (2.17); 3.7023 (1.19); 3.5447 (2.02); 3.5394 (5.22); 3.5351 (8.28); 3.5292 (3.52); 3.5185 (1.34); 3.4974 (2.51); 3.4824 (3.33); 3.4765 (2.53); 3.4648 (4.49); 3.4535 (2.24); 3.4470 (3.58); 3.4292 (1.81); 3.4119 (0.33); 3.4007 (0.64); 3.3917 (1.11); 3.3816 (0.83); 3.3719 (1.35); 3.3629 (2.44); 3.3537 (1.84); 3.3270 (73.42); 3.3064 (0.72); 3.2965 (0.67); 3.2873 (0.41); 3.2150 (0.97); 3.1832 (1.71); 3.1539 (1.00); 3.1259 (0.53); 3.0951 (0.78); 3.0660 (0.44); 2.9458 (0.50); 2.8589 (0.43); 2.8419 (1.16); 2.8248 (1.83); 2.8184 (1.23); 2.8078 (2.05); 2.7794 (2.09); 2.7501 (1.64); 2.7210 (0.51); 2.6754 (0.60); 2.6709 (0.81); 2.6663 (0.59); 2.5411 (0.32); 2.5241 (2.86); 2.5107 (45.32); 2.5063 (87.61); 2.5018 (114.13); 2.4973 (83.01); 2.4929 (40.26); 2.3373 (0.35); 2.3330 (0.64); 2.3286 (0.84); 2.3241 (0.63); 2.0941 (2.96); 2.0679 (3.61); 1.9892 (9.12); 1.9618 (0.66); 1.7451 (0.50); 1.7164 (1.15); 1.6926 (1.08); 1.6868 (1.06); 1.6630 (0.48); 1.5840 (0.92); 1.5537 (1.64); 1.5224 (1.55); 1.4918 (0.83); 1.3970 (7.54); 1.3356 (7.58); 1.3299 (6.61); 1.3183 (14.57); 1.3126 (11.93); 1.3009 (7.37); 1.2952 (5.52); 1.2490 (0.42); 1.1921 (2.98); 1.1745 (12.90); 1.1567 (16.00); 1.1374 (7.43); 1.0126 (11.42); 0.9956 (11.25); 0.0079 (1.26); -0.0002 (27.88); -0.0085 (1.05)

### Bsp. I-3, Lösungsmittel: DMSO-d6, Spektrometer: 399.95 MHz

7.9542 (6.35); 7.5019 (0.97); 7.4823 (2.04); 7.4638 (0.97); 7.4485 (2.11); 7.4441 (2.24); 7.4361 (1.00); 7.4282 (2.01); 7.4200 (1.99); 7.4158 (2.15); 7.4097 (0.92); 7.3982 (1.43); 7.3944 (0.97); 7.3773 (0.73); 7.2380 (0.71); 7.2200 (0.68); 7.0109 (2.14); 6.9897 (1.93); 6.9118 (1.09); 6.8863 (1.32); 6.8649 (1.02); 6.0571 (0.93); 6.0347 (1.14); 6.0267 (1.10); 6.0041 (0.97); 4.9751 (0.54); 4.9393 (2.21); 4.8987 (2.15); 4.8847 (0.37); 4.8619 (0.69); 4.8500 (3.18); 4.8452 (4.90); 4.8404 (3.23); 4.4223 (0.59); 4.3898 (0.64); 4.0557 (1.11); 4.0379 (3.38); 4.0201 (3.44); 4.0023 (1.19); 3.9716 (0.57); 3.9378 (0.63); 3.7681 (0.61); 3.7375 (0.70); 3.7262 (0.86); 3.6955 (0.76); 3.5451 (1.35); 3.5393 (2.76); 3.5337 (1.30); 3.4808 (0.60); 3.4595 (0.60); 3.4396 (0.46); 3.4174 (0.45); 3.3913 (0.47); 3.3817 (0.33); 3.3721 (0.55); 3.3626 (0.96); 3.3535 (0.61); 3.3283 (18.68); 3.2282 (0.43); 3.1978 (0.77); 3.1681 (0.44); 2.8255 (0.39); 2.8204 (0.41); 2.7906 (0.75); 2.7627 (0.42); 2.5111 (12.72); 2.5069 (24.81); 2.5024 (32.41); 2.4980 (24.08); 2.2501 (16.00); 2.0863 (1.06); 2.0698 (1.09); 1.9895 (14.31); 1.7192 (0.49); 1.6894 (0.48); 1.5510 (0.51); 1.5202 (0.48); 1.1924 (3.91); 1.1745 (7.74); 1.1568 (3.84); -0.0002 (5.50)

### Bsp. I-4, Lösungsmittel: DMSO-d6, Spektrometer: 399.95 MHz

7.9541 (4.56); 7.4349 (0.46); 7.4130 (2.11); 7.3972 (1.13); 7.3933 (0.72); 7.3762 (0.52); 7.3518 (0.76); 7.3325 (0.92); 7.1471 (0.94); 7.1284 (0.78); 7.0095 (1.47); 6.9883 (1.33); 6.9105 (0.74); 6.8852 (0.91); 6.8637 (0.69); 6.0562 (0.54); 6.0339 (0.68); 6.0262 (0.65); 6.0034 (0.56); 5.7593 (2.88); 4.9251 (0.42); 4.8892 (1.54); 4.8423 (4.60); 4.8048 (0.46); 4.4214 (0.40); 4.3913 (0.45); 4.0553 (0.49); 4.0375 (1.49); 4.0197 (1.55); 4.0019 (0.81); 3.9619 (0.44); 3.7305 (0.47); 3.5436 (0.89); 3.5378 (1.80); 3.5321 (0.84); 3.4860 (0.65); 3.4635 (0.65); 3.4431 (0.50); 3.4211 (0.49); 3.3685 (0.39); 3.3592 (0.66); 3.3498 (0.42); 3.3270 (13.45); 3.1941 (0.53); 2.8901 (1.43); 2.7854 (0.52); 2.7310 (1.19); 2.5105 (11.52); 2.5063 (22.30); 2.5018 (29.06); 2.4973 (21.43); 2.4931 (10.72); 2.2184 (10.46); 2.2101 (16.00); 2.0946 (0.80); 2.0695 (0.81); 1.9892 (6.42); 1.7172 (0.34); 1.6895 (0.33); 1.5506 (0.35); 1.5203 (0.34); 1.1921 (1.74); 1.1743 (3.47); 1.1565 (1.71); -0.0002 (6.69)

### Bsp. I-5, Lösungsmittel: DMSO-d6, Spektrometer: 399.95 MHz

7.9523 (6.32); 7.6893 (1.32); 7.6698 (1.71); 7.5807 (2.39); 7.5613 (1.28); 7.5412 (2.22); 7.5213 (1.15); 7.4361 (0.74); 7.4188 (1.57); 7.4151 (2.00); 7.3983 (2.20); 7.3774 (1.00); 7.0114 (2.15); 6.9902 (1.94); 6.9109 (1.08); 6.8854 (1.35); 6.8640 (1.02); 6.0567 (0.92); 6.0343 (1.13); 6.0264 (1.10); 6.0037 (0.96); 5.7598 (1.88); 4.9935 (0.58); 4.9577 (2.20); 4.9145 (2.12); 4.8908 (0.35); 4.8797 (0.65); 4.8505 (3.16); 4.8457 (4.91); 4.8408 (3.18); 4.4224 (0.61); 4.3902 (0.67); 4.0560 (0.79); 4.0382 (2.41); 4.0204 (2.45); 4.0026 (0.85); 3.9687 (0.59); 3.9353 (0.66); 3.7654 (0.64); 3.7334 (0.72); 3.7233 (0.90); 3.6918 (0.79); 3.5457 (1.30); 3.5399 (2.71); 3.5342 (1.28); 3.4790 (0.55); 3.4564 (0.55); 3.4410 (0.42); 3.4139 (0.41); 3.3912 (0.48); 3.3814 (0.36); 3.3717 (0.57); 3.3623 (0.97); 3.3532 (0.61); 3.3429 (0.45); 3.3286 (11.09); 3.2288 (0.44); 3.1983 (0.80); 3.1689 (0.44); 2.8263 (0.47); 2.8214 (0.43); 2.7907 (0.79); 2.7622 (0.44); 2.5251 (0.45); 2.5073 (19.73); 2.5028 (25.92); 2.4984 (19.39); 2.2689 (16.00); 2.1042 (1.05); 2.0709 (1.17); 1.9897 (10.36); 1.7171 (0.52); 1.6895 (0.50); 1.5492 (0.53); 1.5205 (0.50); 1.3969 (2.38); 1.1926 (2.89); 1.1748 (5.76); 1.1570 (2.84); -0.0002 (6.56); -0.0084 (0.35)

### Bsp. I-6, Lösungsmittel: DMSO-d6, Spektrometer: 399.95 MHz

8.7705 (0.42); 8.0099 (3.69); 7.4036 (1.92); 7.3536 (0.94); 7.3499 (0.82); 7.3340 (1.13); 7.3301 (1.00); 7.1772 (2.14); 7.1651 (2.47); 7.1611 (2.22); 7.1538 (1.50); 7.1340 (1.17); 7.0888 (0.77); 7.0667 (0.89); 5.8531 (0.60); 5.8355 (0.71); 5.8255 (0.74); 5.8078 (0.62); 4.9178 (0.40); 4.8774 (4.12); 4.8717 (4.06); 4.8408 (1.59); 4.8165 (0.33); 4.8050 (0.44); 4.4103 (0.40); 4.3795 (0.46); 4.0558 (0.43); 4.0380 (1.31); 4.0202 (1.34); 4.0023 (0.51); 3.9802 (0.41); 3.9468 (0.46); 3.9011 (0.54); 3.8735 (0.56); 3.8579 (0.65); 3.8304 (0.54); 3.6078 (0.75); 3.6020 (1.51); 3.5968 (0.75); 3.3502 (0.40); 3.3408 (0.75); 3.3282 (13.10); 3.3124 (0.41); 3.2678 (0.69); 3.2503 (0.67); 3.2245 (0.70); 3.2073 (0.72); 3.1835 (0.56); 3.1533 (0.35); 2.7724 (0.55); 2.7438 (0.35); 2.5246 (0.64); 2.5112 (11.67); 2.5070 (22.74); 2.5025 (29.67); 2.4982 (21.87); 2.2281 (3.23); 2.2182 (16.00); 2.2074 (13.09); 2.1996 (2.30); 2.1902 (0.67); 2.0916 (0.70); 2.0820 (0.72); 2.0499 (0.83); 1.9897 (5.58); 1.9092 (1.42); 1.7042 (0.33); 1.6968 (0.34); 1.6732 (0.34); 1.5307 (0.33); 1.5202 (0.35); 1.5001 (0.34); 1.4907 (0.34); 1.3969 (4.19); 1.1925 (1.54); 1.1747 (3.02); 1.1569 (1.51); -0.0002 (6.53)

### Bsp. I-7, Lösungsmittel: DMSO-d6, Spektrometer: 399.95 MHz

12.9922 (0.38); 8.7715 (0.37); 8.6617 (1.17); 8.0222 (3.57); 8.0128 (9.56); 7.9937 (0.35); 7.3508 (1.87); 7.3231 (7.08); 7.3012 (7.21); 7.1746 (6.71); 7.1621 (7.00); 7.1585 (6.15); 7.0890 (2.56); 7.0861 (2.56); 7.0636 (2.71); 6.9204 (3.70); 6.9137 (6.26); 6.8981 (4.17); 6.8921 (5.48); 5.8648 (0.63); 5.8525 (1.65); 5.8357 (2.26); 5.8251 (1.92); 5.8076 (1.59); 4.8718 (11.07); 4.8662 (10.08); 4.8259 (3.15); 4.8014 (0.46); 4.7948 (0.36); 4.7674 (3.06); 4.7309 (1.39); 4.6919 (1.12); 4.6139 (1.01); 4.5791 (0.47); 4.4126 (1.20); 4.4035 (1.23); 4.3825 (1.37); 4.0563 (0.82); 4.0470 (1.50); 4.0386 (2.60); 4.0305 (4.26); 4.0211 (3.54); 4.0130 (4.45); 4.0056 (3.60); 3.9955 (1.88); 3.9881 (2.69); 3.9706 (1.68); 3.9259 (1.56); 3.9044 (1.84); 3.8977 (1.24); 3.8820 (1.30); 3.8765 (1.96); 3.8611 (2.11); 3.8552 (1.16); 3.8334 (1.85); 3.8136 (0.48); 3.6065 (2.67); 3.6008 (5.18); 3.5953 (2.56); 3.5802 (0.39); 3.4978 (0.71); 3.4801 (1.85); 3.4623 (2.60); 3.4445 (2.00); 3.4340 (0.43); 3.4268 (0.82); 3.3819 (0.51); 3.3731 (0.82); 3.3635 (0.69); 3.3535 (1.10); 3.3442 (2.07); 3.3304 (20.08); 3.3156 (1.38); 3.3057 (1.23); 3.2835 (1.13); 3.2674 (2.56); 3.2501 (1.95); 3.2403 (0.91); 3.2242 (2.21); 3.2069 (2.25); 3.1709 (1.36); 3.1419 (0.82); 3.1111 (0.45); 3.0754 (0.71); 3.0483 (0.44); 3.0138 (0.53); 2.9472 (0.39); 2.8372 (0.51); 2.8205 (0.92); 2.8016 (1.01); 2.7648 (1.65); 2.7342 (1.22); 2.7036 (0.40); 2.6771 (0.33); 2.6724 (0.45); 2.5080 (36.63); 2.5036 (46.90); 2.4992 (34.39); 2.3305 (0.33); 2.0721 (2.16); 2.0460 (2.77); 1.9904 (5.45); 1.9584 (0.71); 1.9316 (0.42); 1.7294 (0.39); 1.7003 (0.88); 1.6773 (0.85); 1.6485 (0.39); 1.5890 (0.35); 1.5595 (0.97); 1.5289 (1.45); 1.4981 (1.32); 1.4631 (0.69); 1.4041 (11.07); 1.3961 (3.74); 1.3397 (7.11); 1.3224 (16.00); 1.3052 (11.20); 1.2880 (2.43); 1.2491 (0.35); 1.2342 (0.35); 1.1932 (1.83); 1.1753 (10.41); 1.1572 (12.78); 1.1348 (7.30); 1.0097 (5.45); 0.9929 (5.28); 0.0079 (0.57); -0.0002 (12.39); -0.0085 (0.54)

### Bsp. I-8, Lösungsmittel: CD3CN, Spektrometer: 399.95 MHz

10.1384 (4.87); 7.9635 (1.51); 7.9450 (1.44); 7.6833 (2.36); 7.6811 (2.86); 7.6714 (3.22); 7.6684 (1.83); 7.6487 (5.99); 7.5976 (0.76); 7.5861 (1.11); 7.5783 (0.82); 7.5664 (0.76); 7.5568 (0.48); 6.4608 (1.02); 6.4443 (1.03); 6.4329 (1.06); 6.4162 (1.04); 4.6285 (2.33); 4.6116 (2.40); 4.4785 (0.38); 4.4512 (0.39); 4.3267 (0.42); 4.0990 (1.23); 4.0864 (0.94); 4.0686 (2.80); 4.0554 (1.49); 4.0507 (2.75); 4.0329 (0.94); 4.0275 (1.36); 3.9830 (0.37); 3.9498 (0.40); 3.3104 (0.47); 3.2908 (0.48); 3.2811 (0.95); 3.2712 (0.51); 3.2524 (0.50); 3.1672 (1.48); 3.1505 (1.74); 3.1236 (1.53); 3.1069 (1.36); 2.7614 (0.48); 2.1913 (0.46); 2.1898 (0.51); 2.1692 (146.43); 2.1651 (275.77); 2.1639 (218.61); 2.1469 (1.17); 2.1323 (0.61); 2.1199 (0.97); 2.1139 (1.36); 2.1076 (1.38); 2.1015 (1.04); 2.0954 (0.76); 2.0812 (0.94); 1.9721 (12.09); 1.9645 (14.96); 1.9584 (2.42); 1.9526 (40.42); 1.9464 (79.16); 1.9402 (115.91); 1.9340 (78.66); 1.9279 (39.79); 1.9150 (0.58); 1.8888 (0.58); 1.8536 (15.08); 1.8373 (0.55); 1.8175 (0.82); 1.8083 (16.00); 1.7748 (0.55); 1.7686 (0.77); 1.7623 (0.56); 1.7565 (0.44); 1.7305 (0.37); 1.6986 (0.38); 1.6154 (0.35); 1.5913 (0.33); 1.5812 (0.32); 1.3349 (0.33); 1.2218 (3.22); 1.2040 (6.45); 1.1861 (3.15); -0.0002 (1.13)

### Bsp. I-9, Lösungsmittel: DMSO-d6, Spektrometer: 399.95 MHz

8.0327 (6.22); 7.5906 (0.54); 7.5747 (0.64); 7.5696 (1.15); 7.5538 (1.19); 7.5487 (0.76); 7.5329 (0.70); 7.3481 (0.81); 7.3300 (2.48); 7.3238 (1.11); 7.3092 (1.66); 7.3025 (0.75); 6.0465 (0.62); 6.0230 (0.78); 6.0166 (0.74); 5.9930 (0.65); 5.0844 (0.38); 5.0466 (1.73); 5.0071 (1.67); 4.9696 (0.39); 4.3987 (0.40); 4.3659 (0.43); 4.0380 (0.51); 4.0202 (0.52); 3.8983 (0.43); 3.8942 (0.40); 3.8679 (0.49); 3.8636 (0.49); 3.8550 (0.59); 3.8506 (0.60); 3.8242 (0.85); 3.8205 (0.87); 3.7880 (0.43); 3.5470 (16.00); 3.5275 (0.76); 3.5040 (0.72); 3.4826 (0.57); 3.4598 (0.56); 3.3900 (0.34); 3.3702 (0.45); 3.3613 (0.73); 3.3519 (0.42); 3.3245 (11.87); 3.1663 (0.53); 2.7902 (0.52); 2.5245 (0.41); 2.5197 (0.70); 2.5112 (8.64); 2.5067 (17.31); 2.5021 (22.66); 2.4974 (16.20); 2.4929 (7.58); 2.0999 (0.80); 2.0945 (0.84); 2.0629 (15.57); 1.9891 (2.31); 1.7285 (0.35); 1.6980 (0.32); 1.5563 (0.33); 1.5271 (0.32); 1.2496 (0.35); 1.1924 (0.64); 1.1746 (1.29); 1.1568 (0.62); 0.0080 (0.52); -0.0002 (14.82); -0.0086 (0.44)

### Bsp. I-10, Lösungsmittel: DMSO-d6, Spektrometer: 399.95 MHz

8.0469 (5.74); 7.5142 (0.80); 7.5100 (0.92); 7.4931 (1.33); 7.4682 (0.81); 7.4637 (0.99); 7.4529 (2.98); 7.4486 (3.63); 7.4345 (0.58); 7.4232 (1.09); 7.4170 (0.76); 7.4070 (0.65); 7.4046 (0.79); 7.4015 (0.59); 7.3979 (0.57); 7.3893 (0.39); 7.3827 (0.36); 6.0059 (0.79); 5.9865 (0.93); 5.9780 (0.90); 5.9586 (0.81); 5.0803 (0.36); 5.0426 (1.65); 5.0030 (1.60); 4.9651 (0.36); 4.3930 (0.37); 4.3602 (0.40); 4.0381 (0.64); 4.0203 (0.65); 3.9914 (0.81); 3.9634 (0.95); 3.9479 (1.06); 3.9200 (0.87); 3.8135 (0.36); 3.7798 (0.40); 3.5526 (16.00); 3.3767 (0.36); 3.3561 (1.28); 3.3483 (0.78); 3.3367 (1.27); 3.3240 (8.73); 3.3126 (1.08); 3.2932 (0.91); 3.1569 (0.50); 2.7799 (0.48); 2.5247 (0.40); 2.5199 (0.61); 2.5113 (8.66); 2.5067 (17.70); 2.5021 (23.44); 2.4975 (16.86); 2.4930 (7.97); 2.0767 (0.90); 2.0611 (14.17); 2.0522 (1.34); 1.9892 (2.84); 1.2495 (0.33); 1.1927 (0.79); 1.1749 (1.58); 1.1571 (0.78); 0.0080 (0.43); -0.0002 (14.15); -0.0085 (0.45)

### Bsp. I-11, Lösungsmittel: DMSO-d6, Spektrometer: 399.95 MHz

7.9982 (5.94); 7.3449 (0.52); 7.3407 (0.70); 7.3199 (1.87); 7.3060 (0.69); 7.3018 (1.61); 7.2985 (1.84); 7.1486 (1.75); 7.1285 (1.48); 7.0156 (0.96); 6.9981 (1.67); 6.9795 (0.77); 5.8806 (0.85); 5.8628 (0.97); 5.8529 (0.94); 5.8350 (0.86); 5.7577 (1.13); 4.8781 (4.66); 4.8722 (4.67); 4.6666 (2.44); 4.6483 (2.43); 4.5951 (0.67); 4.3878 (0.43); 4.3549 (0.47); 4.0557 (0.38); 4.0379 (1.14); 4.0201 (1.16); 4.0023 (0.39); 3.9284 (0.41); 3.8900 (1.26); 3.8621 (1.05); 3.8470 (1.15); 3.8192 (0.96); 3.5879 (1.22); 3.5821 (2.71); 3.5762 (1.21); 3.3571 (0.39); 3.3375 (0.56); 3.3240 (19.15); 3.3094 (0.40); 3.2993 (0.51); 3.2588 (1.16); 3.2409 (1.12); 3.2158 (1.04); 3.1981 (1.00); 3.1408 (0.57); 2.7458 (0.55); 2.5243 (0.43); 2.5194 (0.75); 2.5108 (10.95); 2.5064 (22.13); 2.5018 (29.24); 2.4973 (21.30); 2.4928 (10.34); 2.0607 (0.89); 2.0308 (1.05); 1.9891 (5.09); 1.8288 (15.58); 1.8235 (1.85); 1.8196 (1.01); 1.7932 (16.00); 1.7823 (0.95); 1.6664 (0.46); 1.6433 (0.37); 1.6320 (0.39); 1.5163 (0.36); 1.5084 (0.38); 1.4872 (0.37); 1.4792 (0.35); 1.1924 (1.33); 1.1746 (2.66); 1.1568 (1.31); -0.0002 (2.35)

### Bsp. I-12, Lösungsmittel: DMSO-d6, Spektrometer: 399.95 MHz

8.0416 (5.69); 7.5142 (0.85); 7.5102 (0.98); 7.4930 (1.44); 7.4727 (0.34); 7.4683 (0.84); 7.4638 (1.03); 7.4530 (3.23); 7.4486 (3.86); 7.4344 (0.68); 7.4234 (1.18); 7.4173 (0.86); 7.4048 (0.89); 7.4018 (0.70); 7.3983 (0.66); 7.3896 (0.45); 7.3830 (0.41); 6.0049 (0.84); 5.9855 (1.01); 5.9772 (0.97); 5.9577 (0.88); 5.7581 (0.70); 4.6691 (2.29); 4.6515 (2.34); 4.3911 (0.40); 4.3593 (0.44); 4.0558 (0.32); 4.0380 (0.93); 4.0202 (0.95); 4.0024 (0.35); 3.9909 (0.84); 3.9629 (1.00); 3.9474 (1.24); 3.9323 (0.43); 3.9196 (1.13); 3.8993 (0.45); 3.5533 (16.00); 3.3691 (0.43); 3.3555 (1.19); 3.3501 (0.58); 3.3360 (1.53); 3.3254 (14.63); 3.3120 (1.37); 3.2927 (0.98); 3.1461 (0.53); 2.7512 (0.53); 2.5247 (0.41); 2.5198 (0.67); 2.5113 (7.79); 2.5068 (15.48); 2.5023 (20.20); 2.4977 (14.58); 2.4932 (6.96); 2.0677 (0.85); 2.0372 (0.99); 1.9893 (4.12); 1.8296 (14.61); 1.8187 (0.70); 1.7936 (15.22); 1.7815 (0.68); 1.6721 (0.35); 1.6676 (0.35); 1.6494 (0.33); 1.6417 (0.33); 1.5159 (0.35); 1.4932 (0.33); 1.1925 (1.10); 1.1747 (2.18); 1.1569 (1.07); -0.0002 (1.61)

### Bsp. I-13, Lösungsmittel: DMSO-d6, Spektrometer: 399.95 MHz

8.0271 (6.25); 7.5903 (0.53); 7.5745 (0.62); 7.5694 (1.14); 7.5535 (1.18); 7.5484 (0.76); 7.5326 (0.69); 7.3484 (0.80); 7.3297 (2.54); 7.3243 (1.19); 7.3088 (1.66); 7.3029 (0.80); 6.0454 (0.63); 6.0220 (0.78); 6.0155 (0.75); 5.9920 (0.66); 4.6725 (2.29); 4.6557 (2.27); 4.3964 (0.39); 4.3649 (0.42); 4.0379 (0.66); 4.0201 (0.66); 3.9404 (0.38); 3.8978 (0.69); 3.8939 (0.59); 3.8672 (0.49); 3.8630 (0.49); 3.8543 (0.58); 3.8499 (0.59); 3.8238 (0.49); 3.8197 (0.51); 3.5471 (16.00); 3.5264 (0.76); 3.5028 (0.73); 3.4818 (0.56); 3.4586 (0.56); 3.3817 (0.36); 3.3623 (0.42); 3.3528 (0.77); 3.3433 (0.46); 3.3250 (15.50); 3.1551 (0.51); 2.7608 (0.50); 2.5245 (0.34); 2.5197 (0.56); 2.5111 (7.41); 2.5066 (14.97); 2.5020 (19.76); 2.4974 (14.25); 2.4929 (6.74); 2.0859 (0.82); 2.0555 (0.94); 1.9891 (2.95); 1.8325 (15.49); 1.7955 (15.54); 1.6908 (0.33); 1.6698 (0.34); 1.6620 (0.32); 1.5326 (0.33); 1.1924 (0.80); 1.1745 (1.59); 1.1568 (0.78); -0.0002 (1.73)

### Bsp. I-14, Lösungsmittel: CDCl₃, Spektrometer: 250.13 MHz

7.6093 (1.70); 7.5932 (0.43); 7.5684 (0.47); 7.5599 (0.47); 7.4024 (0.76); 7.3874 (1.25); 7.3561 (0.38); 7.3447 (0.46); 7.2658 (5.88); 7.2635 (5.81); 6.0595 (0.34); 6.0271 (0.40); 6.0145 (0.39); 5.9817 (0.38); 5.3038 (3.98); 5.3015 (3.99); 4.7093 (1.76); 4.7048 (1.79); 3.9822 (0.33); 3.9378 (0.34); 3.9131 (0.46); 3.8680 (0.42); 3.4797 (0.45); 3.4464 (0.43); 3.4092 (0.35); 3.3769 (0.35); 3.2957 (0.33); 3.2640 (5.71); 3.2621 (5.51); 2.8063 (0.39); 2.1921 (0.35); 2.1471 (0.46); 1.8761 (0.37); 1.8514 (5.47); 1.8494 (5.40); 1.7818 (0.42); 1.7665 (0.44); 1.7310 (0.37); 1.7150 (0.36); 1.6003 (5.45); 1.2560 (0.32); 1.0915 (16.00); 1.0898 (15.63); 0.0020 (3.70); -0.0002 (3.83)

### Bsp. XVI-81, Lösungsmittel: DMSO-d6, Spektrometer: 399.95 MHz

8.0023 (2.21); 7.3274 (0.63); 7.3094 (0.52); 7.3064 (0.61); 7.1559 (0.55); 7.1361 (0.48); 7.0063 (0.54); 4.8848 (1.59); 4.8789 (1.58); 4.0457 (0.33); 4.0280 (0.46); 3.8681 (0.37); 3.8529 (0.41); 3.8252 (0.34); 3.5917 (0.42); 3.5858 (0.91); 3.5799 (0.39); 3.3325 (21.59); 3.2674 (0.44); 3.2496 (0.49); 3.2243 (0.41); 3.2066 (0.40); 2.5323 (0.43); 2.5275 (0.67); 2.5189 (7.55); 2.5144 (15.19); 2.5098 (20.02); 2.5052 (14.38); 2.5006 (6.78); 2.0156 (0.36); 1.9968 (1.09); 1.5688 (0.37); 1.5590 (0.33); 1.5375 (0.32); 1.4130 (16.00); 1.4055 (4.84); 1.3983 (0.54); 1.1828 (0.57)

### Bsp. XVI-96, Lösungsmittel: DMSO-d6, Spektrometer: 399.95 MHz

10.1331 (1.80); 7.9900 (2.32); 7.8225 (0.80); 7.5122 (0.57); 7.5092 (0.58); 7.4848 (0.99); 7.4649 (0.42); 6.3801 (0.33); 6.3686 (0.33); 4.0440 (0.39); 4.0162 (0.67); 4.0004 (0.66); 3.9728 (0.57); 3.3355 (5.02); 3.2206 (0.32); 3.1791 (0.44); 3.1628 (0.41); 3.1354 (0.38); 3.1192 (0.38); 2.5121 (5.31); 2.5077 (10.74); 2.5032 (14.32); 2.4987 (10.76); 2.4943 (5.47); 2.3956 (3.63); 2.0202 (0.40); 1.9900 (0.60); 1.5485 (0.36); 1.5391 (0.41); 1.5179 (0.35); 1.5082 (0.34); 1.4022 (16.00); 1.3969 (13.31); 1.3823 (0.80); -0.0002 (6.28)

### Bsp. XVI-99, Lösungsmittel: DMSO-d6, Spektrometer: 399.95 MHz

10.1791 (1.59); 8.0298 (0.44); 8.0257 (0.50); 8.0094 (0.57); 8.0067 (0.52); 7.9879 (2.03); 7.7141 (0.51); 7.6953 (0.37); 7.6920 (0.34); 7.6213 (0.41); 7.6054 (0.91); 7.5869 (0.61); 4.0770 (0.34); 4.0490 (0.43); 4.0400 (0.56); 4.0332 (0.54); 4.0222 (0.72); 4.0050 (0.74); 3.9820 (0.36); 3.3146 (76.20); 3.2228 (0.38); 3.2155 (0.57); 3.1993 (0.48); 3.1719 (0.40); 3.1557 (0.39); 2.5109 (4.05); 2.5066 (7.25); 2.5021 (9.26); 2.4977 (6.41); 2.4933 (3.09); 2.0288 (0.39); 1.9877 (2.29); 1.5561 (0.38); 1.5457 (0.37); 1.5242 (0.34); 1.4203 (0.57); 1.4095 (3.12); 1.4028 (16.00); 1.3837 (0.49); 1.1936 (0.57); 1.1758 (1.11); 1.1580 (0.55); -0.0002 (0.49)

### Bsp. XVI-142, Lösungsmittel: DMSO-d6, Spektrometer: 399.95 MHz

8.0379 (2.21); 7.5135 (0.38); 7.5098 (0.43); 7.4923 (0.63); 7.4673 (0.37); 7.4629 (0.44); 7.4520 (1.34); 7.4477 (1.52); 7.4226 (0.47); 7.4165 (0.33); 7.4040 (0.37); 6.0031 (0.35); 5.9837 (0.42); 5.9753 (0.40); 5.9558 (0.36); 4.0252 (0.33); 3.9887 (0.66); 3.9606 (0.44); 3.9452 (0.47); 3.9173 (0.39); 3.5512 (6.33); 3.3554 (0.46); 3.3357 (0.63); 3.3237 (9.19); 3.3121 (0.51); 3.2926 (0.41); 3.2460 (0.35); 2.5108 (5.80); 2.5066 (10.93); 2.5021 (14.10); 2.4976 (10.36); 2.4933 (5.17); 2.0453 (0.42); 2.0403 (0.42); 2.0134 (0.49); 1.5667 (0.40); 1.5584 (0.44); 1.5366 (0.40); 1.5274 (0.37); 1.4061 (16.00); 1.0435 (0.44); 1.0283 (0.43)

### Bsp. XVI-405, Lösungsmittel: DMSO-d6, Spektrometer: 399.95 MHz

7.9403 (2.52); 7.4067 (0.50); 7.3861 (0.88); 7.3654 (0.70); 7.1577 (0.74); 7.1497 (0.90); 7.1364 (0.57); 7.1297 (0.75); 7.1275 (0.53); 6.2254 (0.35); 6.2008 (0.46); 6.1946 (0.42); 6.1699 (0.36); 4.7922 (0.79); 4.7859 (1.02); 4.7840 (0.98); 4.7776 (0.78); 4.0380 (1.03); 4.0202 (0.99); 4.0024 (0.55); 3.7230 (0.34); 3.7118 (0.48); 3.6810 (0.39); 3.5723 (0.44); 3.5476 (0.44); 3.4430 (0.48); 3.4371 (1.11); 3.4312 (0.46); 3.3224 (6.80); 2.5112 (3.77); 2.5066 (7.61); 2.5020 (10.08); 2.4974 (7.28); 2.4929 (3.45); 2.0660 (0.34); 2.0609 (0.36); 2.0341 (0.41); 2.0288 (0.39); 1.9887 (3.52); 1.5770 (0.32); 1.4097 (16.00); 1.1930 (0.97); 1.1752 (1.92); 1.1573 (0.95); -0.0002 (0.69)

### Bsp. XVI-411, Lösungsmittel: DMSO-d6, Spektrometer: 399.95 MHz

7.9551 (2.65); 7.4104 (0.46); 7.3936 (0.47); 7.0086 (0.64); 6.9874 (0.58); 6.9077 (0.32); 6.8862 (0.35); 6.8829 (0.37); 6.0427 (0.34); 6.0346 (0.32); 4.8486 (0.94); 4.8431 (1.62); 4.8375 (0.91); 3.5414 (0.53); 3.5355 (1.20); 3.5296 (0.51); 3.5158 (0.33); 3.4932 (0.33); 3.3217 (3.50); 3.2549 (0.33); 2.8906 (0.76); 2.7311 (0.48); 2.5109 (3.82); 2.5064 (7.66); 2.5018 (10.06); 2.4972 (7.17); 2.4926 (3.38); 2.0629 (0.34); 2.0576 (0.35); 2.0311 (0.41); 2.0258 (0.40); 1.5840 (0.32); 1.5741 (0.35); 1.5532 (0.33); 1.4094 (16.00); 1.3974 (4.87); -0.0002 (7.08)

### Bsp. XVI-412, Lösungsmittel: DMSO-d6, Spektrometer: 399.95 MHz

8.0235 (2.59); 7.5685 (0.48); 7.5526 (0.49); 7.3468 (0.35); 7.3290 (0.99); 7.3225 (0.47); 7.3083 (0.67); 6.0203 (0.33); 4.0380 (0.97); 4.0202 (0.94); 4.0024 (0.54); 3.5455 (6.73); 3.5261 (0.33); 3.3235 (9.89); 3.2588 (0.34); 2.5195 (0.38); 2.5111 (4.57); 2.5066 (9.08); 2.5020 (11.93); 2.4975 (8.59); 2.4930 (4.09); 2.0632 (0.36); 2.0576 (0.37); 2.0310 (0.42); 1.9889 (3.21); 1.5730 (0.32); 1.4085 (16.00); 1.1928 (0.84); 1.1750 (1.67); 1.1572 (0.82); -0.0002 (1.02)

### Bsp. XIIIa-81, Lösungsmittel: DMSO-d6, Spektrometer: 250.13 MHz

8.0476 (3.47); 7.3672 (0.36); 7.3353 (1.41); 7.3054 (1.33); 7.1686 (0.81); 7.1370 (0.63); 7.0414 (0.47); 7.0114 (0.78); 6.9820 (0.33); 5.9127 (0.37); 5.8843 (0.48); 5.8686 (0.45); 5.8401 (0.38); 4.8933 (2.26); 4.8838 (2.22); 3.9297 (0.40); 3.8852 (0.47); 3.8609 (0.55); 3.8164 (0.46); 3.6534 (0.50); 3.6266 (0.34); 3.6146 (0.78); 3.6053 (1.72); 3.5958 (0.80); 3.5776 (16.00); 3.4196 (0.66); 3.4064 (0.47); 3.3943 (0.67); 3.3770 (0.53); 3.3618 (0.48); 3.3296 (0.55); 3.2946 (0.60); 3.2661 (0.51); 3.2261 (0.44); 3.1973 (0.43); 3.0480 (0.41); 3.0062 (0.39); 2.5269 (2.31); 2.5197 (4.97); 2.5123 (6.87); 2.5050 (4.89); 2.4977 (2.22); 2.2226 (0.38); 2.1762 (0.54); 1.9494 (0.44); 1.9198 (0.45); 1.9077 (0.37)

### Bsp. XIIIa-99, Lösungsmittel: DMSO-d6, Spektrometer: 399.95 MHz

10.1852 (0.84); 8.0314 (1.21); 7.6096 (0.56); 7.5908 (0.35); 4.9198 (0.83); 3.5685 (16.00); 2.5153 (1.65); 2.5110 (3.07); 2.5066 (4.02); 2.5022 (2.88); 2.4979 (1.45)

### Bsp. XIIIa-142, Lösungsmittel: DMSO-d6, Spektrometer: 399.95 MHz

8.0794 (1.10); 7.4568 (0.52); 7.4530 (0.70); 3.5680 (16.00); 3.5561 (3.16); 3.3620 (0.33); 3.3187 (0.33); 2.5122 (3.27); 2.5077 (6.64); 2.5031 (8.90); 2.4985 (6.58); 2.4940 (3.22); 1.5955 (0.36)

### Bsp. XIIIa-236, Lösungsmittel: DMSO-d6, Spektrometer: 399.95 MHz

10.0393 (1.14); 10.0353 (1.07); 8.0737 (1.13); 8.0695 (1.10); 7.9323 (0.83); 7.9014 (0.45); 7.8983 (0.42); 7.8826 (0.51); 7.7509 (0.36); 7.7316 (0.53); 7.6643 (0.37); 7.6455 (0.55); 6.1538 (0.93); 5.8819 (0.37); 3.5694 (16.00); 3.5671 (12.49); 3.5649 (15.32); 3.4501 (0.42); 3.4466 (0.44); 3.4306 (0.45); 3.4269 (0.47); 3.4123 (0.43); 3.4073 (0.49); 3.3878 (0.48); 3.3347 (0.59); 3.3038 (0.70); 3.0320 (0.55); 3.0039 (0.53); 2.5163 (4.08); 2.5125 (5.39); 2.5084 (4.95); 2.2135 (0.49); 2.1809 (0.67); 2.0048 (0.54); 1.9753 (0.48); -0.0002 (0.36); -0.0047 (0.34)

### Bsp. XIIIa-349, Lösungsmittel: DMSO-d6, Spektrometer: 399.95 MHz

10.0194 (2.71); 8.0738 (3.28); 7.9545 (1.88); 7.9378 (0.78); 7.9339 (2.11); 7.6260 (1.88); 7.6055 (1.65); 5.8958 (0.45); 5.8766 (0.52); 5.8683 (0.50); 5.8490 (0.45); 3.9953 (0.51); 3.9675 (0.57); 3.9521 (0.66); 3.9244 (0.56); 3.5689 (16.00); 3.4221 (0.79); 3.4095 (0.55); 3.4028 (0.83); 3.3915 (0.34); 3.3792 (0.76); 3.3597 (0.82); 3.3475 (0.61); 3.3157 (0.71); 3.0334 (0.58); 3.0066 (0.56); 2.5679 (0.44); 2.5157 (4.27); 2.5113 (8.22); 2.5068 (10.52); 2.5022 (7.51); 2.4978 (3.56); 2.2079 (0.52); 2.1829 (0.68); 2.1792 (0.69); 1.9731 (0.55); 1.9474 (0.49); 1.3565 (0.95); 0.0079 (0.43); -0.0002 (10.08); -0.0085 (0.34)

### Bsp. XIIIa-405, Lösungsmittel: DMSO-d6, Spektrometer: 399.95 MHz

7.9866 (2.24); 7.4120 (0.45); 7.3913 (0.91); 7.3707 (0.66); 7.1635 (0.77); 7.1531 (0.89); 7.1425 (0.65); 7.1331 (0.74); 6.2352 (0.36); 6.2106 (0.47); 6.2044 (0.43); 6.1797 (0.37); 4.7980 (0.90); 4.7920 (1.02); 4.7883 (1.01); 4.7821 (0.87); 3.7339 (0.37); 3.7229 (0.50); 3.6921 (0.42); 3.5774 (0.70); 3.5680 (16.00); 3.5532 (0.52); 3.5356 (0.34); 3.5111 (0.34); 3.4624 (0.59); 3.4567 (1.14); 3.4508 (0.58); 3.4175 (0.36); 3.3598 (0.44); 3.3279 (0.54); 3.0421 (0.44); 3.0155 (0.43); 2.5130 (3.61); 2.5087 (7.01); 2.5042 (9.07); 2.4996 (6.63); 2.4953 (3.26); 2.2274 (0.40); 2.1984 (0.54); 1.9769 (0.40); 1.9498 (0.38); -0.0002 (5.24)

### Bsp. XIIIa-411, Lösungsmittel: DMSO-d6, Spektrometer: 399.95 MHz

8.0015 (2.35); 7.4157 (0.58); 7.3985 (0.55); 7.0128 (0.89); 6.9917 (0.82); 6.9107 (0.47); 6.8864 (0.61); 6.8637 (0.43); 6.0736 (0.39); 6.0514 (0.48); 6.0436 (0.47); 6.0210 (0.41); 5.7614 (0.38); 4.8476 (2.03); 3.7794 (0.35); 3.7680 (0.43); 3.7375 (0.39); 3.5680 (16.00); 3.5655 (11.38); 3.5537 (1.28); 3.5212 (0.47); 3.4989 (0.47); 3.4787 (0.37); 3.4564 (0.44); 3.4173 (0.45); 3.3537 (0.58); 3.3226 (0.71); 3.0381 (0.58); 3.0107 (0.56); 2.8913 (0.38); 2.7306 (0.35); 2.5089 (10.65); 2.5049 (14.30); 2.2234 (0.55); 2.1921 (0.72); 1.9766 (0.58); 1.9481 (0.51); -0.0002 (3.54); -0.0027 (2.58)

### Bsp. XIIIa-412, Lösungsmittel: DMSO-d6, Spektrometer: 399.95 MHz

8.0656 (0.97); 7.3355 (0.35); 3.5681 (16.00); 3.5526 (2.63); 2.5128 (2.19); 2.5085 (4.36); 2.5039 (5.79); 2.4994 (4.34); 2.4951 (2.19)

### Bsp. VIIa-a-143, Lösungsmittel: DMSO-d6, Spektrometer: 399.95 MHz

13.7691 (1.28); 8.0269 (0.33); 7.8403 (1.07); 7.8195 (1.73); 7.8042 (2.66); 7.7584 (2.21); 7.7381 (1.42); 6.1631 (0.88); 6.1492 (0.57); 6.1423 (1.04); 6.1335 (0.98); 6.1126 (0.91); 6.1064 (0.46); 5.6296 (0.44); 5.6015 (0.41); 5.0141 (1.08); 4.9727 (4.57); 4.9653 (2.84); 4.9504 (4.46); 4.9090 (1.05); 4.2764 (1.13); 3.8091 (1.02); 3.7793 (1.13); 3.7648 (1.26); 3.7351 (1.12); 3.6493 (16.00); 3.5693 (3.62); 3.3743 (5.39); 3.1903 (1.20); 3.1695 (1.19); 3.1460 (1.06); 3.1253 (1.05); 2.5114 (7.75); 2.5070 (10.22); 2.5027 (7.52)

### Bsp. VIIIa-a-81, Lösungsmittel: DMSO-d6, Spektrometer: 399.95 MHz

7.3037 (0.63); 7.2995 (0.72); 7.2809 (1.24); 7.2646 (0.85); 7.2604 (1.00); 7.2462 (1.33); 7.2423 (1.23); 7.2273 (1.52); 7.2234 (1.28); 7.0579 (1.80); 7.0379 (1.52); 6.9489 (0.97); 6.9473 (0.98); 6.9303 (1.69); 6.9287 (1.66); 6.9116 (0.81); 6.9100 (0.78); 5.8459 (0.94); 5.8251 (1.09); 5.8160 (1.08); 5.7952 (0.98); 4.7535 (4.77); 4.7476 (4.83); 3.5303 (1.35); 3.5244 (2.79); 3.5186 (1.36); 3.4687 (1.12); 3.4387 (1.19); 3.4251 (1.39); 3.3952 (1.25); 3.2600 (4.66); 2.9524 (1.32); 2.9315 (1.30); 2.9089 (1.14); 2.8880 (1.13); 2.4336 (5.79); 2.4292 (7.64); 2.4248 (5.66); 2.3714 (16.00)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

### Verwendungsbeispiele

### Phytophthora-Test (Tomate) / protektiv

| | | |
|---|---|---|
| Lösungsmittel: | 49 | Gewichtsteile N, N - Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Tomatenpflanzen mit der Wirkstoffzubereitung in der angegebenen Aufwandmenge besprüht. 1 Tag nach der Behandlung werden die Pflanzen mit einer Sporensuspension von ***Phytophthora infestans*** inokuliert und stehen dann 24h bei 100% rel. Feuchte und 22°C. Anschließend werden die Pflanzen in einer Klimazelle bei ca. 96% relativer Luftfeuchtigkeit und einer Temperatur von ca. 20°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die nachfolgenden erfindungsgemäßen Verbindungen bei einer Konzentration an Wirkstoff von 100ppm einen Wirkungsgrad von 70% oder mehr.

| **Bsp.** | **Eff.%** |
|---|---|
| I-1 | 94 |
| I-2 | 89 |
| I-3 | 83 |
| I-4 | 78 |
| I-8 | 89 |

## Patentansprüche

1. Verbindungen der Formel **(VIIIa)** und **(VIIIb),** 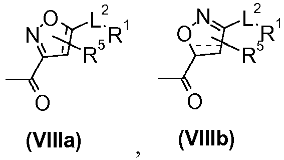 wobei
R⁵ steht für Wasserstoff,
L² steht für eine direkte Bindung,
R¹ steht für Phenyl, das 1, 2 oder 3 Substituenten enthält, wobei die Substituenten unabhängig voneinander mindestens einmal aus Z⁴ und gegebenenfalls aus folgender Liste ausgewählt sind:
Fluor, Chlor, Brom, Iod, Cyano, Nitro, Hydroxy, Amino, Methyl, Ethyl, *n*-Propyl, 1-Methylethyl, *n*-Butyl, 1,1-Dimethylethyl, 1,2-Dimethylethyl, Ethenyl, Ethinyl, Trifluormethyl, Difluormethyl, Trichlormethyl, Dichlormethyl, Cyclopropyl, Methoxy, Ethoxy, *n*-Propoxy, 1-Methylethoxy, 1,1-Dimethylethoxy, Methylcarbonyl, Ethylcarbonyl, Methoxycarbonyl, Ethoxycarbonyl, *n*-Propoxycarbonyl, 1-Methylethoxycarbonyl, 1,1-Dimethylethoxycarbonyl, 1-Methylcarbonyloxy, Methylthio, Ethylthio, Methylsulfonyl oder -L³R³,
L³ steht für eine direkte Bindung,
R³ steht für Wasserstoff, C₁-C₆-Alkyl, C₁-C₆-Halogenalkyl, C₃-C₈-Cycloalkyl, C₁-C₄-Alkylcarbonyl, C₁-C₄-Halogenalkylcarbonyl, C₁-C₄-Alkoxycarbonyl oder C₁-C₄-Halogen-alkoxycarbonyl,
Z³ steht für einen Phenylrest, der bis zu zwei Substituenten enthalten kann, wobei die Substituenten unabhängig voneinander ausgewählt sind aus folgender Liste:
Chlor, Brom, Iod, Fluor, Cyano, Nitro, Hydroxy, Amino, -SH, Methyl, Ethyl, *n*-Propyl, 1-Methylethyl, 1,1-Dimethylethyl, Ethenyl, Propen-2-yl, Ethinyl, Propin-2-yl, Trifluormethyl, Difluormethyl, Methoxymethyl, Methylcarbonyl, Ethylcarbonyl, Trifluormethylcarbonyl, Methoxycarbonyl, Ethoxycarbonyl, *n*-Propoxycarbonyl, 1-Methylethoxycarbonyl, 1,1-Dimethylethoxycarbonyl, Methoxy, Ethoxy, *n*-Propoxy, 1-Methylethoxy, 1,1-Dimethylethoxy, Trifluormethoxy, Ethenyloxy, 2-Propenyloxy, Ethinyloxy, 2-Propinyloxy, Methylthio, Ethylthio, Trifluormethylthio, Methylsulfonyl, Ethylsulfonyl, Propylthionyl, 1-Methylethylthio, Trifluormethylsulfonyl, Methylamino, Ethylamino, *n*-Propylamino, 1-Methylethylamino, 1,1-Dimethylethylamino oder Dimethylamino, oder
Z³ steht für Naphthalenyl,
R¹³ und R¹⁴ stehen gleich oder verschieden unabhängig voneinander für Wasserstoff, Methyl, Ethyl, *n*-Propyl, 1-Methylethyl, *n*-Butyl oder 1,1-Dimethylethyl,
Z⁴ steht für -Formyl, Methoxymethoxy, 2-Methoxyethoxy, Allyloxy, 2-Fluorprop-2-en-1-yloxy, 2-Chlorprop-2-en-1-yloxy, 3-Chlorprop-2-en-1-yloxy, 2-Bromprop-2-en-1-yloxy, 2-Methylprop-2-en-1-yloxy, 3,3-Dichlorprop-2-en-1-yloxy, 3,3-Dichlor-2-fluorprop-2-en-1-yloxy, But-2-en-1-yloxy, But-3-en-2-yloxy, But-3-en-1-yloxy, 3-Chlorbut-2-en-1-yloxy 3-Methylbut-2-en-1-yloxy, 4,4,4-Trifluorbut-2-en-1-yloxy, Prop-2-in-1-yloxy, 3-Chlorprop-2-in-1-yloxy, 3-Bromprop-2-in-1-yloxy, But-2-in-1-yloxy, Pent-2-in-1-yloxy, 2-Fluor-2-methylpropanoyloxy, 3,3,3-Trifluorpropanoyloxy, Cyclopropylcarbonyloxy, Cyclohexylcarbonyloxy, (1-Chlorcyclopropyl)carbonyloxy, But-2-enoyloxy, Acryloyloxy, Cyanomethoxy, Methylsulfonyloxy, Ethylsulfonyloxy, Trifluormethylsulfonyloxy, Cyclopropylsulfonyloxy, 2-Methoxyethoxymethyl, Allyloxymethyl, Prop-2-in-1-yloxymethyl, Methylsulfonylmethyl, Methylcarbonylaminomethyl, Methylsulfonylaminomethyl, -C(=NOR⁷)R⁸, Dimethylaminosulfonyl, Ethylaminosulfonyl, Trimethylsilylethinyl, Diethylaminosulfonyl, Methylaminosulfonyl, Trimethylsilyloxy, Trimethylsilylprop-2-in-1-yloxy, Trifluormethylamino, Dimethylaminocarbonylamino, -C(=O)OH, -NHC(=O)H, -C(=O)NH₂, -C(=S)NR¹³R¹⁴ 1,1-Dimethylethylcarbonylamino, Chlormethylcarbonylamino, Trifluormethylcarbonylamino, 1,1-Dimethylethoxycarbonylamino, Ethylcarbonylamino, 1-Methylethoxycarbonylamino, Trifluormethylcarbonylamino, Methylcarbonylamino, Methoxycarbonylamino, Ethoxycarbonylamino, *iso*-Propoxycarbonylamino, 1-Methylethylcarbonylamino, Methylsulfonylamino oder Phenylsulfonylamino, 3-Bromprop-2-en-1-yloxy, oder -L⁴Z³,
L⁴ steht für -C(=O)O- oder -OCH₂C≡C-,
R⁷ steht für Wasserstoff, Methyl, Ethyl, *n*-Propyl, 1-Methylethyl, *n*-Butyl, 1,1-Dimethylethyl oder 2-Methylpropyl,
R⁸ steht für Wasserstoff, Methyl, Ethyl, *n*-Propyl, 1-Methylethyl, *n*-Butyl, 1,1-Dimethylethyl oder 2-Methylpropyl,
sowie Salze davon.

2. Verbindung nach Anspruch 1, wobei R¹ für 6-Chlor-2-[(methylsulfonyl)oxy]phenyl steht.

## Claims

1. Compounds of the formula **(VIIIa)** and **(VIIIb),** 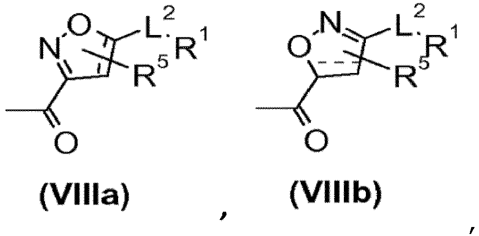 wherein
R⁵ represents hydrogen,
L² represents a direct bond,
R¹ represents phenyl which contains 1, 2 or 3 substituents, where the substituents independently of one another are selected at least once from Z⁴ and optionally from the list below:
fluorine, chlorine, bromine, iodine, cyano, nitro, hydroxy, amino, methyl, ethyl, n-propyl, 1-methylethyl, n-butyl, 1,1-dimethylethyl, 1,2-dimethylethyl, ethenyl, ethynyl, trifluoromethyl, difluoromethyl, trichloromethyl, dichloromethyl, cyclopropyl, methoxy, ethoxy, n-propoxy, 1-methylethoxy, 1,1-dimethylethoxy, methylcarbonyl, ethylcarbonyl, methoxycarbonyl, ethoxycarbonyl, n-propoxycarbonyl, 1-methylethoxycarbonyl, 1,1-dimethylethoxycarbonyl, 1-methylcarbonyloxy, methylthio, ethylthio, methylsulfonyl or -L³R³,
L³ represents a direct bond,
R³ represents hydrogen, C₁-C₆-alkyl, C₁-C₆-haloalkyl, C₃-C₈-cycloalkyl, C₁-C₄-alkylcarbonyl, C₁-C₄-haloalkylcarbonyl, C₁-C₄-alkoxycarbonyl or C₁-C₄-haloalkoxycarbonyl,
Z³ represents a phenyl radical which may contain up to two substituents, where the substituents independently of one another are selected from the list below:
chlorine, bromine, iodine, fluorine, cyano, nitro, hydroxy, amino, -SH, methyl, ethyl, n-propyl, 1-methylethyl, 1,1-dimethylethyl, ethenyl, propen-2-yl, ethynyl, propyn-2-yl, trifluoromethyl, difluoromethyl, methoxymethyl, methylcarbonyl, ethylcarbonyl, trifluoromethyl-carbonyl, methoxycarbonyl, ethoxycarbonyl, n-propoxycarbonyl, 1-methylethoxycarbonyl, 1,1-dimethylethoxycarbonyl, methoxy, ethoxy, n-propoxy, 1-methylethoxy, 1,1-dimethylethoxy, trifluoromethoxy, ethenyloxy, 2-propenyloxy, ethynyloxy, 2-propynyloxy, methylthio, ethylthio, trifluoromethylthio, methylsulfonyl, ethylsulfonyl, propylthionyl, 1-methylethylthio, trifluoromethylsulfonyl, methylamino, ethylamino, n-propylamino, 1-methylethylamino, 1,1-dimethylethylamino or dimethylamino or
Z³ represents naphthalenyl,
R¹³ and R¹⁴ are identical or different and independently of one another represent hydrogen, methyl, ethyl, n-propyl, 1-methylethyl, n-butyl or 1,1-dimethylethyl,
Z⁴ represents -formyl, methoxymethoxy, 2-methoxy-ethoxy, allyloxy, 2-fluoroprop-2-en-1-yloxy, 2-chloroprop-2-en-1-yloxy, 3-chloroprop-2-en-1-yloxy, 2-bromoprop-2-en-1-yloxy, 2-methylprop-2-en-1-yloxy, 3,3-dichloroprop-2-en-1-yloxy, 3,3-dichloro-2-fluoroprop-2-en-1-yloxy, but-2-en-1-yloxy, but-3-en-2-yloxy, but-3-en-1-yloxy, 3-chlorobut-2-en-1-yloxy, 3-methylbut-2-en-1-yloxy, 4,4,4-trifluorobut-2-en-1-yloxy, prop-2-yn-1-yloxy, 3-chloroprop-2-yn-1-yloxy, 3-bromoprop-2-yn-1-yloxy, but-2-yn-1-yloxy, pent-2-yn-1-yloxy, 2-fluoro-2-methyl-propanoyloxy, 3,3,3-trifluoropropanoyloxy, cyclopropylcarbonyloxy, cyclohexylcarbonyloxy, (1-chlorocyclopropyl)carbonyloxy, but-2-enoyloxy, acryloyloxy, cyanomethoxy, methylsulfonyloxy, ethylsulfonyloxy, trifluoromethylsulfonyloxy, cyclopropylsulfonyloxy, 2-methoxyethoxymethyl, allyloxymethyl, prop-2-yn-1-yloxymethyl, methylsulfonylmethyl, methylcarbonylaminomethyl, methylsulfonylaminomethyl, -C(=NOR⁷)R⁸, dimethylaminosulfonyl, ethylaminosulfonyl, trimethylsilylethynyl, diethylamino-sulfonyl, methylaminosulfonyl, trimethylsilyloxy, trimethylsilylprop-2-yn-1-yloxy, trifluoromethylamino, dimethylaminocarbonylamino, -C(=O)OH, -NHC(=O)H, -C(=O)NH₂, -C(=S)NR¹³R¹⁴, 1,1-dimethylethylcarbonylamino, chloromethylcarbonylamino, trifluoromethylcarbonylamino, 1,1-dimethylethoxycarbonylamino, ethylcarbonylamino, 1-methylethoxycarbonylamino, trifluoromethylcarbonylamino, methylcarbonylamino, methoxycarbonylamino, ethoxycarbonylamino, isopropoxycarbonylamino, 1-methylethylcarbonylamino, methylsulfonylamino or phenylsulfonylamino, 3-bromoprop-2-en-1-yloxy or -L⁴Z³,
L⁴ represents -C(=O)O- or -OCH₂C≡C-,
R⁷ represents hydrogen, methyl, ethyl, n-propyl, 1-methylethyl, n-butyl, 1,1-dimethylethyl or 2-methylpropyl,
R⁸ represents hydrogen, methyl, ethyl, n-propyl, 1-methylethyl, n-butyl, 1,1-dimethylethyl or 2-methylpropyl,
and salts thereof.

2. Compound according to Claim 1, where R¹ represents 6-chloro-2-[(methylsulfonyl)oxy]phenyl.

## Revendications

1. Composés de formule (VIIIa) et (VIIIb) 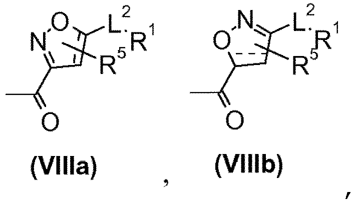 dans lesquelles
R⁵ représente hydrogène,
L² représente une liaison directe,
R¹ représente phényle, qui contient 1, 2 ou 3 substituants, les substituants étant choisis indépendamment les uns des autres au moins une fois parmi Z⁴ et éventuellement dans la liste suivante : fluor, chlore, brome, iode, cyano, nitro, hydroxy, amino, méthyle, éthyle, n-propyle, 1-méthyléthyle, n-butyle, 1,1-diméthyléthyle, 1,2-diméthyléthyle, éthényle, éthynyle, trifluorométhyle, difluorométhyle, trichlorométhyle, dichlorométhyle, cyclopropyle, méthoxy, éthoxy, n-propoxy, 1-méthyléthoxy, 1,1-diméthyléthoxy, méthylcarbonyle, éthylcarbonyle, méthoxycarbonyle, éthoxycarbonyle, n-propoxycarbonyle, 1-méthyléthoxycarbonyle, 1,1-diméthyléthoxycarbonyle, 1-méthylcarbonyloxy, méthylthio, éthylthio, méthylsulfonyle ou -L³R³,
L³ représente une liaison directe,
R³ représente hydrogène, alkyle en C₁-C₆, halogénoalkyle en C₁-C₆, cycloalkyle en C₃-C₈, alkylcarbonyle en C₁-C₄, halogénoalkylcarbonyle en C₁-C₄, alcoxycarbonyle en C₁-C₄ ou halogénoalcoxycarbonyle en C₁-C₄,
Z³ représente un radical phényle qui peut contenir jusqu'à deux substituants, les substituants étant choisis indépendamment l'un de l'autre dans la liste suivante :
chlore, brome, iode, fluore, cyano, nitro, hydroxy, amino, -SH, méthyle, éthyle, n-propyle, 1-méthyléthyle, 1,1-diméthyléthyle, éthényle, propén-2-yle, éthynyle, propyn-2-yle, trifluorométhyle, difluorométhyle, méthoxyméthyle, méthylcarbonyle, éthylcarbonyle, trifluorométhylcarbonyle, méthoxycarbonyle, éthoxycarbonyle, n-propoxycarbonyle, 1-méthyléthoxycarbonyle, 1,1-diméthyléthoxycarbonyle, méthoxy, éthoxy, n-propoxy, 1-méthyléthoxy, 1,1-diméthyléthoxy, trifluorométhoxy, éthényloxy, 2-propényloxy, éthynyloxy, 2-propynyloxy, méthylthio, éthylthio, trifluorométhylthio, méthylsulfonyle, éthylsulfonyle, propylthionyle, 1-méthyléthylthio, trifluorométhylsulfonyle, méthylamino, éthylamino, n-propylamino, 1-méthyléthylamino, 1,1-diméthyléthylamino ou diméthylamino, ou
Z³ représente naphtalényle,
R¹³ et R¹⁴, identiques ou différents, représentent, indépendamment les uns des autres, hydrogène, méthyle, éthyle, n-propyle, 1-méthyléthyle, n-butyle, ou 1,1-diméthyléthyle,
Z⁴ représente formyle, méthoxyméthoxy, 2-méthoxyéthoxy, allyloxy, 2-fluoroprop-2-én-1-yloxy, 2-chloroprop-2-én-1-yloxy, 3-chloroprop-2-én-1-yloxy, 2-bromoprop-2-én-1-yloxy, 2-méthylprop-2-én-1-yloxy, 3,3-dichloroprop-2-én-1-yloxy, 3,3-dichloro-2-fluoroprop-2-én-1-yloxy, but-2-én-1-yloxy, but-3-én-2-yloxy, but-3-én-1-yloxy, 3-chlorobut-2-én-1-yloxy, 3-méthylbut-2-én-1-yloxy, 4,4,4-trifluorobut-2-én-1-yloxy, prop-2-yn-1-yloxy, 3-chloroprop-2-yn-1-yloxy, 3-bromoprop-2-yn-1-yloxy, but-2-yn-1-yloxy, pent-2-yn-1-yloxy, 2-fluoro-2-méthylpropanoyloxy, 3,3,3-trifluoropropanoyloxy, cyclopropylcarbonyloxy, cyclohexylcarbonyloxy, (1-chlorocyclopropyl)carbonyloxy, but-2-énoyloxy, acryloyloxy, cyanométhoxy, méthylsulfonyloxy, éthylsulfonyloxy, trifluorométhylsulfonyloxy, cyclopropylsulfonyloxy, 2-méthoxyéthoxyméthyle, allyloxyméthyle, prop-2-yn-1-yloxyméthyle, méthylsulfonylméthyle, méthylcarbonylaminométhyle, méthylsulfonylaminométhyle, -C(=NOR⁷)R⁸, diméthylaminosulfonyle, éthylaminosulfonyle, triméthylsilyléthinyle, diéthylaminosulfonyle, méthylaminosulfonyle, triméthylsilyloxy, triméthylsilylprop-2-yn-1-yloxy, trifluorométhylamino, diméthylaminocarbonylamino, -C(=O)OH, -NHC(=O)H, - C(=O)NH₂, -C(=S)NR¹³R¹⁴, 1,1-diméthyléthylcarbonylamino, chlorométhylcarbonylamino, trifluorométhylcarbonylamino, 1,1-diméthyléthoxycarbonylamino, éthylcarbonylamino, 1-méthyléthoxycarbonylamino, trifluorométhylcarbonylamino, méthylcarbonylamino, méthoxycarbonylamino, éthoxycarbonylamino, *iso-*propoxycarbonylamino, 1-méthyléthylcarbonylamino, méthylsulfonylamino ou phénylsulfonylamino, 3-bromoprop-2-én-1-yloxy ou -L⁴Z³,
L⁴ représente -C(=O)O- ou -OCH₂C≡C-,
R⁷ représente hydrogène, méthyle, éthyle, n-propyle, 1-méthyléthyle, n-butyle, 1,1-diméthyléthyle ou 2-méthylpropyle,
R⁸ représente hydrogène, méthyle, éthyle, n-propyle, 1-méthyléthyle, n-butyle, 1,1-diméthyléthyle ou 2-méthylpropyle,
ainsi que leurs sels.

2. Composé selon la revendication 1, dans lequel R¹ représente 6-chloro-2-[(méthylsulfonyl)oxy]phényle.
